# EUROPEAN PATENT APPLICATION

(11) **EP 3 939 604 A2**
(43) Date of publication of application: **19.01.2022**
(21) Application number: 21171514.9
(22) Date of filing: 18.10.2017
(51) Int. Cl.: A61K 38/00, A61K 39/00, A61K 39/12, A61K 39/145, A61P 31/16

(54) **INFLUENZA HEMAGGLUTININ PROTEIN VACCINES**

(30) Priority: 21.10.2016 US 201662411240 P; 26.04.2017 US 201762490086 P
(62) Divisional of application: 17862887.1
(71) Applicant: Merck Sharp & Dohme Corp., Rahway, New Jersey 07065-0907 (US)
(72) Inventor: Flynn, Jessica Anne, West Point, 19486 (US); Zhang, Lan, West Point, 19486 (US); Babaoglu, Kerim, West Point, 19486 (US); Fridman, Arthur, Rahway, 07065-0907 (US); Nickle, David, Seattle, 98117 (US)
(74) Representative: Böhles, Elena

(57) **Abstract**

The disclosure relates to influenza virus hemagglutinin protein and DNA vaccines, as well as methods of using the vaccines and compositions comprising the vaccines.

SEQUENCE LISTING

<110>  Flynn, Jessica Anne
       Babaoglu, Kerim
       Fridman, Arthur
       Nickle, David
       Zhang, Lan

<120>  INFLUENZA HEMAGGLUTININ PROTEIN VACCINES

<130>  24378

<140>  62/490,086
<141>  2017-04-26

<150>  62/411,240
<151>  2016-10-21

<160>  88

<170>  PatentIn version 3.5

<210>  1
<211>  191
<212>  PRT
<213>  Artificial Sequence

<220>
<223>  BHA10-2: HA10

<400>  1

Met Glu Thr Pro Ala Gln Leu Leu Phe Leu Leu Leu Leu Trp Leu Pro
1               5                   10                  15

Asp Thr Thr Gly His Val Val Lys Thr Ala Thr Gln Gly Glu Val Asn
            20                  25                  30

Val Thr Gly Val Ile Pro Leu Thr Thr Thr Pro Thr Gly Ser Ala Asn
        35                  40                  45

Lys Ser Lys Pro Tyr Tyr Thr Gly Glu His Ala Lys Ala Thr Gly Asn
    50                  55                  60

Cys Pro Ile Trp Val Lys Thr Pro Leu Lys Leu Ala Asn Gly Thr Lys
65                  70                  75                  80

Tyr Gly Ser Ala Gly Ser Ala Thr Gln Glu Ala Ile Asn Lys Ile Thr
                85                  90                  95

Lys Asn Leu Asn Ser Leu Ser Glu Leu Glu Val Lys Asn Leu Gln Arg
            100                 105                 110

Leu Ser Gly Ala Ser Asp Glu Thr His Asn Glu Ile Leu Glu Leu Asp
        115                 120                 125

Glu Lys Val Asp Asp Leu Arg Ala Asp Thr Ile Ser Ser Gln Ile Glu
    130                 135                 140

Leu Ala Val Leu Leu Ser Asn Glu Gly Ile Ile Asn Ser Glu Asp Glu
145                 150                 155                 160

Gly Thr Gly Gly Gly Tyr Ile Pro Glu Ala Pro Arg Asp Gly Gln Ala
                165                 170                 175

Tyr Val Arg Lys Asp Gly Glu Trp Val Leu Leu Ser Thr Phe Leu
            180                 185                 190

<210>  2
<211>  160
<212>  PRT
<213>  Artificial Sequence

<220>
<223>  BHA10-3:BHA10-2

<400>  2

Met Glu Thr Pro Ala Gln Leu Leu Phe Leu Leu Leu Leu Trp Leu Pro
1               5                   10                  15

Asp Thr Thr Gly His Val Val Lys Thr Ala Thr Gln Gly Glu Val Asn
            20                  25                  30

Val Thr Gly Val Ile Pro Leu Thr Thr Thr Pro Thr Gly Ser Ala Asn
        35                  40                  45

Lys Ser Lys Pro Tyr Tyr Thr Gly Glu His Ala Lys Ala Thr Gly Asn
    50                  55                  60

Cys Pro Ile Trp Val Lys Thr Pro Leu Lys Leu Ala Asn Gly Thr Lys
65                  70                  75                  80

Tyr Gly Ser Ala Gly Ser Ala Thr Gln Glu Ala Ile Asn Lys Ile Thr
                85                  90                  95

Lys Asn Leu Asn Ser Leu Ser Glu Leu Glu Val Lys Asn Leu Gln Arg
            100                 105                 110

Leu Ser Cys Ala Ser Asp Glu Thr His Asn Cys Ile Leu Glu Leu Asp
        115                 120                 125

Glu Lys Val Asp Asp Leu Arg Ala Asp Thr Ile Ser Ser Leu Ile Glu
    130                 135                 140

Leu Ala Val Leu Leu Ser Asn Glu Gly Ile Ile Asn Ser Glu Asp Glu
145                 150                 155                 160

<210>  3
<211>  260
<212>  PRT
<213>  Artificial Sequence

<220>
<223>  NIHGen6HASS-TM

<400>  3

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Gly Ser Gly Leu Arg Met Val Thr Gly Leu Arg Asn
    50                  55                  60

Ile Pro Gln Arg Glu Thr Arg Gly Leu Phe Gly Ala Ile Ala Gly Phe
65                  70                  75                  80

Ile Glu Gly Gly Trp Thr Gly Met Val Asp Gly Trp Tyr Gly Tyr His
                85                  90                  95

His Gln Asn Glu Gln Gly Ser Gly Tyr Ala Ala Asp Gln Lys Ser Thr
            100                 105                 110

Gln Asn Ala Ile Asn Gly Ile Thr Asn Met Val Asn Ser Val Ile Glu
        115                 120                 125

Lys Met Gly Ser Gly Gly Ser Gly Thr Asp Leu Ala Glu Leu Leu Val
    130                 135                 140

Leu Leu Leu Asn Glu Arg Thr Leu Asp Phe His Asp Ser Asn Val Lys
145                 150                 155                 160

Asn Leu Tyr Glu Lys Val Lys Ser Gln Leu Lys Asn Asn Ala Lys Glu
                165                 170                 175

Ile Gly Asn Gly Cys Phe Glu Phe Tyr His Lys Cys Asn Asn Glu Cys
            180                 185                 190

Met Glu Ser Val Lys Asn Gly Thr Tyr Asp Tyr Pro Lys Tyr Ser Glu

195                 200                 205

Glu Ser Lys Leu Asn Arg Glu Lys Ile Asp Gln Gly Thr Gly Gly Ile
    210                 215                 220

Leu Ala Ile Tyr Ser Thr Val Ala Ser Ser Leu Val Leu Leu Val Ser
225                 230                 235                 240

Leu Gly Ala Ile Ser Phe Trp Met Cys Ser Asn Gly Ser Leu Gln Cys
                245                 250                 255

Arg Ile Cys Ile
            260

<210>  4
<211>  266
<212>  PRT
<213>  Artificial Sequence

<220>
<223>  NIHGen6HASS-TM2

<400>  4

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Gly Ser Gly Leu Arg Met Val Thr Gly Leu Arg Asn
    50                  55                  60

Ile Pro Gln Arg Glu Thr Arg Gly Leu Phe Gly Ala Ile Ala Gly Phe
65                  70                  75                  80

Ile Glu Gly Gly Trp Thr Gly Met Val Asp Gly Trp Tyr Gly Tyr His
                85                  90                  95

His Gln Asn Glu Gln Gly Ser Gly Tyr Ala Ala Asp Gln Lys Ser Thr
            100                 105                 110

Gln Asn Ala Ile Asn Gly Ile Thr Asn Met Val Asn Ser Val Ile Glu
        115                 120                 125

Lys Met Gly Ser Gly Gly Ser Gly Thr Asp Leu Ala Glu Leu Leu Val
    130                 135                 140

Leu Leu Leu Asn Glu Arg Thr Leu Asp Phe His Asp Ser Asn Val Lys
145                 150                 155                 160

Asn Leu Tyr Glu Lys Val Lys Ser Gln Leu Lys Asn Asn Ala Lys Glu
                165                 170                 175

Ile Gly Asn Gly Cys Phe Glu Phe Tyr His Lys Cys Asn Asn Glu Cys
            180                 185                 190

Met Glu Ser Val Lys Asn Gly Thr Tyr Asp Tyr Pro Lys Tyr Ser Glu
        195                 200                 205

Glu Ser Lys Leu Asn Arg Glu Lys Ile Asp Gly Val Lys Leu Glu Ser
    210                 215                 220

Met Gly Val Tyr Gln Ile Leu Ala Ile Tyr Ser Thr Val Ala Ser Ser
225                 230                 235                 240

Leu Val Leu Leu Val Ser Leu Gly Ala Ile Ser Phe Trp Met Cys Ser
                245                 250                 255

Asn Gly Ser Leu Gln Cys Arg Ile Cys Ile
            260                 265

<210>  5
<211>  248
<212>  PRT
<213>  Artificial Sequence

<220>
<223>  NIHGen6HASS

<400>  5

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Gly Ser Gly Leu Arg Met Val Thr Gly Leu Arg Asn
    50                  55                  60

Ile Pro Gln Arg Glu Thr Arg Gly Leu Phe Gly Ala Ile Ala Gly Phe
65                  70                  75                  80

Ile Glu Gly Gly Trp Thr Gly Met Val Asp Gly Trp Tyr Gly Tyr His
                85                  90                  95

His Gln Asn Glu Gln Gly Ser Gly Tyr Ala Ala Asp Gln Lys Ser Thr
            100                 105                 110

Gln Asn Ala Ile Asn Gly Ile Thr Asn Met Val Asn Ser Val Ile Glu
        115                 120                 125

Lys Met Gly Ser Gly Gly Ser Gly Thr Asp Leu Ala Glu Leu Leu Val
    130                 135                 140

Leu Leu Leu Asn Glu Arg Thr Leu Asp Phe His Asp Ser Asn Val Lys
145                 150                 155                 160

Asn Leu Tyr Glu Lys Val Lys Ser Gln Leu Lys Asn Asn Ala Lys Glu
                165                 170                 175

Ile Gly Asn Gly Cys Phe Glu Phe Tyr His Lys Cys Asn Asn Glu Cys
            180                 185                 190

Met Glu Ser Val Lys Asn Gly Thr Tyr Asp Tyr Pro Lys Tyr Ser Glu
        195                 200                 205

Glu Ser Lys Leu Asn Arg Glu Lys Ile Asp Pro Gly Ser Gly Tyr Ile
    210                 215                 220

Pro Glu Ala Pro Arg Asp Gly Gln Ala Tyr Val Arg Lys Asp Gly Glu
225                 230                 235                 240

Trp Val Leu Leu Ser Thr Phe Leu
                245

<210>  6
<211>  566
<212>  PRT
<213>  Artificial Sequence

<220>
<223>  ConH1

<400>  6

Met Lys Ala Lys Leu Leu Val Leu Leu Cys Ala Phe Thr Ala Thr Asp
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Lys Leu Lys Gly Ile
    50                  55                  60

Ala Pro Leu Gln Leu Gly Lys Cys Asn Ile Ala Gly Trp Ile Leu Gly
65                  70                  75                  80

Asn Pro Glu Cys Glu Ser Leu Ile Ser Lys Arg Ser Trp Ser Tyr Ile
                85                  90                  95

Val Glu Thr Pro Asn Ser Glu Asn Gly Thr Cys Tyr Pro Gly Asp Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Asn
    130                 135                 140

Val Thr Lys Gly Val Thr Ala Ala Cys Ser His Ala Gly Lys Ser Ser
145                 150                 155                 160

Phe Tyr Arg Asn Leu Leu Trp Leu Thr Glu Lys Asn Gly Ser Tyr Pro
                165                 170                 175

Lys Leu Ser Lys Ser Tyr Val Asn Asn Lys Glu Lys Glu Val Leu Val
            180                 185                 190

Leu Trp Gly Val His His Pro Ser Asn Ile Thr Asp Gln Arg Thr Leu
        195                 200                 205

Tyr Gln Asn Glu Asn Ala Tyr Val Ser Val Val Ser Ser His Tyr Asn
    210                 215                 220

Arg Arg Phe Thr Pro Glu Ile Ala Lys Arg Pro Lys Val Arg Gly Gln
225                 230                 235                 240

Ala Gly Arg Ile Asn Tyr Tyr Trp Thr Leu Leu Glu Pro Gly Asp Thr
                245                 250                 255

Ile Ile Phe Glu Ala Asn Gly Asn Leu Ile Ala Pro Trp Tyr Ala Phe
            260                 265                 270

Ala Leu Ser Arg Gly Phe Gly Ser Gly Ile Ile Thr Ser Asn Ala Pro
        275                 280                 285

Met His Glu Cys Asp Thr Lys Cys Gln Thr Pro Gln Gly Ala Ile Asn
    290                 295                 300

Ser Ser Leu Pro Phe Gln Asn Val His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Ile Asp Gly Trp Tyr Gly Tyr
        355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Lys
                405                 410                 415

Leu Glu Lys Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Lys Asn Asn Ala Lys Glu Ile Gly Asn Gly
465                 470                 475                 480

Cys Phe Glu Phe Tyr His Lys Cys Asn Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Gly Val Lys Leu Glu Ser Met Gly Val Tyr
        515                 520                 525

Gln Ile Leu Ala Ile Tyr Ser Thr Val Ala Ser Ser Leu Val Leu Leu
    530                 535                 540

Val Ser Leu Gly Ala Ile Ser Phe Trp Met Cys Ser Asn Gly Ser Leu
545                 550                 555                 560

Gln Cys Arg Ile Cys Ile
                565

<210>  7
<211>  566
<212>  PRT
<213>  Artificial Sequence

<220>
<223>  ConH3

<400>  7

Met Lys Thr Ile Ile Ala Leu Ser Tyr Ile Phe Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Leu Val Lys Thr Ile Thr Asn Asp
        35                  40                  45

Gln Ile Glu Val Thr Asn Ala Thr Glu Leu Val Gln Ser Ser Ser Thr
    50                  55                  60

Gly Arg Ile Cys Asp Ser Pro His Arg Ile Leu Asp Gly Thr Asn Cys
65                  70                  75                  80

Thr Leu Ile Asp Ala Leu Leu Gly Asp Pro His Cys Asp Gly Phe Gln
                85                  90                  95

Asn Lys Glu Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Gly Phe Asn Trp Thr
    130                 135                 140

Gly Val Thr Gln Asn Gly Gly Ser Ser Ala Cys Lys Arg Gly Ser Asp
145                 150                 155                 160

Lys Ser Phe Phe Ser Arg Leu Asn Trp Leu His Lys Leu Lys Tyr Lys
                165                 170                 175

Tyr Pro Ala Leu Asn Val Thr Met Pro Asn Asn Asp Lys Phe Asp Lys
        180                 185                 190

Leu Tyr Ile Trp Gly Val His His Pro Ser Thr Asp Ser Asp Gln Thr
    195                 200                 205

Ser Leu Tyr Val Gln Ala Ser Gly Arg Val Thr Val Ser Thr Lys Arg
    210                 215                 220

Ser Gln Gln Thr Val Ile Pro Asn Ile Gly Ser Arg Pro Trp Val Arg
225                 230                 235                 240

Gly Leu Ser Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Thr Cys Asn Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Asn Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Thr Gly Gln Ala Ala Asp Leu Lys
    370                 375                 380

Ser Thr Gln Ala Ala Ile Asp Gln Ile Asn Gly Lys Leu Asn Arg Leu
385                 390                 395                 400

Ile Glu Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Arg Thr Arg Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Val Leu Leu Gly Phe Ile Met Trp Ala Cys Gln Lys Gly Asn Ile
545                 550                 555                 560

Arg Cys Asn Ile Cys Ile
                565

<210>  8
<211>  566
<212>  PRT
<213>  Artificial Sequence

<220>
<223>  MRK_pH1_Con

<400>  8

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Ser Ser Ser Asp Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Ala Tyr Val Phe Val Gly Thr Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Val Pro Ser Ile Gln Ser Arg Gly Leu Phe Gly Ala Ile Ala Gly
            340                 345                 350

Phe Ile Glu Gly Gly Trp Thr Gly Met Val Asp Gly Trp Tyr Gly Tyr
        355                 360                 365

His His Gln Asn Glu Gln Gly Ser Gly Tyr Ala Ala Asp Leu Lys Ser
    370                 375                 380

Thr Gln Asn Ala Ile Asp Lys Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Tyr His Asp Ser Asn Val Lys Asn Leu Tyr Glu
    450                 455                 460

Lys Val Arg Ser Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
            500                 505                 510

Asn Arg Glu Glu Ile Asp Gly Val Lys Leu Glu Ser Thr Arg Ile Tyr
        515                 520                 525

Gln Ile Leu Ala Ile Tyr Ser Thr Val Ala Ser Ser Leu Val Leu Val
    530                 535                 540

Val Ser Leu Gly Ala Ile Ser Phe Trp Met Cys Ser Asn Gly Ser Leu
545                 550                 555                 560

Gln Cys Arg Ile Cys Ile

                565

<210>  9
<211>  529
<212>  PRT
<213>  Artificial Sequence

<220>
<223>  MRK_pH1_Con

<400>  9

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Ser Ser Ser Asp Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Ala Tyr Val Phe Val Gly Thr Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Val Pro Ser Ile Gln Ser Arg Gly Leu Phe Gly Ala Ile Ala Gly
            340                 345                 350

Phe Ile Glu Gly Gly Trp Thr Gly Met Val Asp Gly Trp Tyr Gly Tyr
        355                 360                 365

His His Gln Asn Glu Gln Gly Ser Gly Tyr Ala Ala Asp Leu Lys Ser
    370                 375                 380

Thr Gln Asn Ala Ile Asp Lys Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Tyr His Asp Ser Asn Val Lys Asn Leu Tyr Glu

450                 455                 460

Lys Val Arg Ser Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
            500                 505                 510

Asn Arg Glu Glu Ile Asp Gly Val Lys Leu Glu Ser Thr Arg Ile Tyr
        515                 520                 525

Gln

<210>  10
<211>  565
<212>  PRT
<213>  Artificial Sequence

<220>
<223>  MRK_sH1_Con

<400>  10

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Lys Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Arg Ala Leu Tyr
        195                 200                 205

His Thr Glu Asn Ala Tyr Val Ser Val Val Ser Ser His Tyr Ser Arg
    210                 215                 220

Arg Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala
            260                 265                 270

Leu Ser Arg Gly Phe Gly Ser Gly Ile Ile Thr Ser Asn Ala Pro Met
        275                 280                 285

Asp Glu Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Val Asp Gly Trp Tyr Gly Tyr His
        355                 360                 365

His Gln Asn Glu Gln Gly Ser Gly Tyr Ala Ala Asp Gln Lys Ser Thr

370                 375                 380

Gln Asn Ala Ile Asn Gly Ile Thr Asn Lys Val Asn Ser Val Ile Glu
385                 390                 395                 400

Lys Met Asn Thr Gln Phe Thr Ala Val Gly Lys Glu Phe Asn Lys Leu
                405                 410                 415

Glu Arg Arg Met Glu Asn Leu Asn Lys Lys Val Asp Asp Gly Phe Leu
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asn Asp Glu Cys Met Glu Ser Val Lys
                485                 490                 495

Asn Gly Thr Tyr Asp Tyr Pro Lys Tyr Ser Glu Glu Ser Lys Leu Asn
            500                 505                 510

Arg Glu Lys Ile Asp Gly Val Lys Leu Glu Ser Met Gly Val Tyr Gln
        515                 520                 525

Ile Leu Ala Ile Tyr Ser Thr Val Ala Ser Ser Leu Val Leu Leu Val
    530                 535                 540

Ser Leu Gly Ala Ile Ser Phe Trp Met Cys Ser Asn Gly Ser Leu Gln
545                 550                 555                 560

Cys Arg Ile Cys Ile
                565

<210>  11
<211>  528
<212>  PRT
<213>  Artificial Sequence

<220>
<223>  MRK_sH1_Con

<400>  11

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Lys Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Arg Ala Leu Tyr
        195                 200                 205

His Thr Glu Asn Ala Tyr Val Ser Val Val Ser Ser His Tyr Ser Arg
    210                 215                 220

Arg Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala

260                 265                 270

Leu Ser Arg Gly Phe Gly Ser Gly Ile Ile Thr Ser Asn Ala Pro Met
        275                 280                 285

Asp Glu Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Val Asp Gly Trp Tyr Gly Tyr His
        355                 360                 365

His Gln Asn Glu Gln Gly Ser Gly Tyr Ala Ala Asp Gln Lys Ser Thr
    370                 375                 380

Gln Asn Ala Ile Asn Gly Ile Thr Asn Lys Val Asn Ser Val Ile Glu
385                 390                 395                 400

Lys Met Asn Thr Gln Phe Thr Ala Val Gly Lys Glu Phe Asn Lys Leu
                405                 410                 415

Glu Arg Arg Met Glu Asn Leu Asn Lys Lys Val Asp Asp Gly Phe Leu
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asn Asp Glu Cys Met Glu Ser Val Lys
                485                 490                 495

Asn Gly Thr Tyr Asp Tyr Pro Lys Tyr Ser Glu Glu Ser Lys Leu Asn
            500                 505                 510

Arg Glu Lys Ile Asp Gly Val Lys Leu Glu Ser Met Gly Val Tyr Gln
        515                 520                 525

<210>  12
<211>  566
<212>  PRT
<213>  Artificial Sequence

<220>
<223>  Cobra_P1

<400>  12

Met Lys Ala Arg Leu Leu Val Leu Leu Cys Ala Leu Ala Ala Thr Asp
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Lys Leu Lys Gly Ile
    50                  55                  60

Ala Pro Leu Gln Leu Gly Lys Cys Asn Ile Ala Gly Trp Leu Leu Gly
65                  70                  75                  80

Asn Pro Glu Cys Glu Ser Leu Leu Ser Ala Arg Ser Trp Ser Tyr Ile
                85                  90                  95

Val Glu Thr Pro Asn Ser Glu Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Asn
    130                 135                 140

Thr Thr Lys Gly Val Thr Ala Ala Cys Ser His Ala Gly Lys Ser Ser
145                 150                 155                 160

Phe Tyr Arg Asn Leu Leu Trp Leu Thr Lys Lys Gly Gly Ser Tyr Pro
                165                 170                 175

Lys Leu Ser Lys Ser Tyr Val Asn Asn Lys Gly Lys Glu Val Leu Val
            180                 185                 190

Leu Trp Gly Val His His Pro Ser Thr Ser Thr Asp Gln Gln Ser Leu

195                 200                 205

Tyr Gln Asn Glu Asn Ala Tyr Val Ser Val Val Ser Ser Asn Tyr Asn
    210                 215                 220

Arg Arg Phe Thr Pro Glu Ile Ala Glu Arg Pro Lys Val Arg Gly Gln
225                 230                 235                 240

Ala Gly Arg Met Asn Tyr Tyr Trp Thr Leu Leu Glu Pro Gly Asp Thr
                245                 250                 255

Ile Ile Phe Glu Ala Thr Gly Asn Leu Ile Ala Pro Trp Tyr Ala Phe
            260                 265                 270

Ala Leu Ser Arg Gly Ser Gly Ser Gly Ile Ile Thr Ser Asn Ala Ser
        275                 280                 285

Met His Glu Cys Asn Thr Lys Cys Gln Thr Pro Gln Gly Ala Ile Asn
    290                 295                 300

Ser Ser Leu Pro Phe Gln Asn Ile His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Ile Asp Gly Trp Tyr Gly Tyr
        355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Asn
                405                 410                 415

Leu Glu Lys Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Arg Asn Asn Ala Lys Glu Ile Gly Asn Gly
465                 470                 475                 480

Cys Phe Glu Phe Tyr His Lys Cys Asp Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Gly Val Lys Leu Glu Ser Met Gly Val Tyr
        515                 520                 525

Gln Ile Leu Ala Ile Tyr Ser Thr Val Ala Ser Ser Leu Val Leu Leu
    530                 535                 540

Val Ser Leu Gly Ala Ile Ser Phe Trp Met Cys Ser Asn Gly Ser Leu
545                 550                 555                 560

Gln Cys Arg Ile Cys Ile
                565

<210>  13
<211>  566
<212>  PRT
<213>  Artificial Sequence

<220>
<223>  Cobra_X3

<400>  13

Met Glu Ala Arg Leu Leu Val Leu Leu Cys Ala Phe Ala Ala Thr Asn
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Arg Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Ser Leu Phe Ser Lys Glu Ser Trp Ser Tyr Ile

                85                  90                  95

Ala Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Lys Gly Val Thr Ala Ser Cys Ser His Asn Gly Lys Ser Ser
145                 150                 155                 160

Phe Tyr Arg Asn Leu Leu Trp Leu Thr Glu Lys Asn Gly Leu Tyr Pro
                165                 170                 175

Asn Leu Ser Lys Ser Tyr Val Asn Asn Lys Glu Lys Glu Val Leu Val
            180                 185                 190

Leu Trp Gly Val His His Pro Ser Asn Ile Gly Asp Gln Arg Ala Ile
        195                 200                 205

Tyr His Thr Glu Asn Ala Tyr Val Ser Val Val Ser Ser His Tyr Ser
    210                 215                 220

Arg Arg Phe Thr Pro Glu Ile Ala Lys Arg Pro Lys Val Arg Asp Gln
225                 230                 235                 240

Glu Gly Arg Ile Asn Tyr Tyr Trp Thr Leu Leu Glu Pro Gly Asp Thr
                245                 250                 255

Ile Ile Phe Glu Ala Asn Gly Asn Leu Ile Ala Pro Trp Tyr Ala Phe
            260                 265                 270

Ala Leu Ser Arg Gly Phe Gly Ser Gly Ile Ile Thr Ser Asn Ala Ser
        275                 280                 285

Met Asp Glu Cys Asp Ala Lys Cys Gln Thr Pro Gln Gly Ala Ile Asn
    290                 295                 300

Ser Ser Leu Pro Phe Gln Asn Val His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Ile Asp Gly Trp Tyr Gly Tyr
        355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Lys
                405                 410                 415

Leu Glu Arg Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Lys Asn Asn Ala Lys Glu Ile Gly Asn Gly
465                 470                 475                 480

Cys Phe Glu Phe Tyr His Lys Cys Asn Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Gly Val Lys Leu Glu Ser Met Gly Val Tyr
        515                 520                 525

Gln Ile Leu Ala Ile Tyr Ser Thr Val Ala Ser Ser Leu Val Leu Leu
    530                 535                 540

Val Ser Leu Gly Ala Ile Ser Phe Trp Met Cys Ser Asn Gly Ser Leu
545                 550                 555                 560

Gln Cys Arg Ile Cys Ile
                565

<210>  14
<211>  684
<212>  PRT

<213>  Artificial Sequence

<220>
<223>  ConH1_ferritin

<400>  14

Met Lys Ala Lys Leu Leu Val Leu Leu Cys Ala Phe Thr Ala Thr Asp
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Lys Leu Lys Gly Ile
    50                  55                  60

Ala Pro Leu Gln Leu Gly Lys Cys Asn Ile Ala Gly Trp Ile Leu Gly
65                  70                  75                  80

Asn Pro Glu Cys Glu Ser Leu Ile Ser Lys Arg Ser Trp Ser Tyr Ile
                85                  90                  95

Val Glu Thr Pro Asn Ser Glu Asn Gly Thr Cys Tyr Pro Gly Asp Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Asn
    130                 135                 140

Val Thr Lys Gly Val Thr Ala Ala Cys Ser His Ala Gly Lys Ser Ser
145                 150                 155                 160

Phe Tyr Arg Asn Leu Leu Trp Leu Thr Glu Lys Asn Gly Ser Tyr Pro
                165                 170                 175

Lys Leu Ser Lys Ser Tyr Val Asn Asn Lys Glu Lys Glu Val Leu Val
            180                 185                 190

Leu Trp Gly Val His His Pro Ser Asn Ile Thr Asp Gln Arg Thr Leu
        195                 200                 205

Tyr Gln Asn Glu Asn Ala Tyr Val Ser Val Val Ser Ser His Tyr Asn
    210                 215                 220

Arg Arg Phe Thr Pro Glu Ile Ala Lys Arg Pro Lys Val Arg Gly Gln
225                 230                 235                 240

Ala Gly Arg Ile Asn Tyr Tyr Trp Thr Leu Leu Glu Pro Gly Asp Thr
                245                 250                 255

Ile Ile Phe Glu Ala Asn Gly Asn Leu Ile Ala Pro Trp Tyr Ala Phe
            260                 265                 270

Ala Leu Ser Arg Gly Phe Gly Ser Gly Ile Ile Thr Ser Asn Ala Pro
        275                 280                 285

Met His Glu Cys Asp Thr Lys Cys Gln Thr Pro Gln Gly Ala Ile Asn
    290                 295                 300

Ser Ser Leu Pro Phe Gln Asn Val His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Ile Asp Gly Trp Tyr Gly Tyr
        355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Lys
                405                 410                 415

Leu Glu Lys Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Lys Asn Asn Ala Lys Glu Ile Gly Asn Gly
465                 470                 475                 480

Cys Phe Glu Phe Tyr His Lys Cys Asn Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Ser Gly Gly Asp Ile Ile Lys Leu Leu Asn
        515                 520                 525

Glu Gln Val Asn Lys Glu Met Gln Ser Ser Asn Leu Tyr Met Ser Met
    530                 535                 540

Ser Ser Trp Cys Tyr Thr His Ser Leu Asp Gly Ala Gly Leu Phe Leu
545                 550                 555                 560

Phe Asp His Ala Ala Glu Glu Tyr Glu His Ala Lys Lys Leu Ile Ile
                565                 570                 575

Phe Leu Asn Glu Asn Asn Val Pro Val Gln Leu Thr Ser Ile Ser Ala
            580                 585                 590

Pro Glu His Lys Phe Glu Gly Leu Thr Gln Ile Phe Gln Lys Ala Tyr
        595                 600                 605

Glu His Glu Gln His Ile Ser Glu Ser Ile Asn Asn Ile Val Asp His
    610                 615                 620

Ala Ile Lys Ser Lys Asp His Ala Thr Phe Asn Phe Leu Gln Trp Tyr
625                 630                 635                 640

Val Ala Glu Gln His Glu Glu Glu Val Leu Phe Lys Asp Ile Leu Asp
                645                 650                 655

Lys Ile Glu Leu Ile Gly Asn Glu Asn His Gly Leu Tyr Leu Ala Asp
            660                 665                 670

Gln Tyr Val Lys Gly Ile Ala Lys Ser Arg Lys Ser
        675                 680

<210>  15
<211>  685
<212>  PRT
<213>  Artificial Sequence

<220>
<223>  ConH3_ferritin

<400>  15

Met Lys Thr Ile Ile Ala Leu Ser Tyr Ile Phe Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Leu Val Lys Thr Ile Thr Asn Asp
        35                  40                  45

Gln Ile Glu Val Thr Asn Ala Thr Glu Leu Val Gln Ser Ser Ser Thr
    50                  55                  60

Gly Arg Ile Cys Asp Ser Pro His Arg Ile Leu Asp Gly Thr Asn Cys
65                  70                  75                  80

Thr Leu Ile Asp Ala Leu Leu Gly Asp Pro His Cys Asp Gly Phe Gln
                85                  90                  95

Asn Lys Glu Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Gly Phe Asn Trp Thr
    130                 135                 140

Gly Val Thr Gln Asn Gly Gly Ser Ser Ala Cys Lys Arg Gly Ser Asp
145                 150                 155                 160

Lys Ser Phe Phe Ser Arg Leu Asn Trp Leu His Lys Leu Lys Tyr Lys
                165                 170                 175

Tyr Pro Ala Leu Asn Val Thr Met Pro Asn Asn Asp Lys Phe Asp Lys
            180                 185                 190

Leu Tyr Ile Trp Gly Val His His Pro Ser Thr Asp Ser Asp Gln Thr
        195                 200                 205

Ser Leu Tyr Val Gln Ala Ser Gly Arg Val Thr Val Ser Thr Lys Arg
    210                 215                 220

Ser Gln Gln Thr Val Ile Pro Asn Ile Gly Ser Arg Pro Trp Val Arg
225                 230                 235                 240

Gly Leu Ser Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Thr Cys Asn Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Asn Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Thr Gly Gln Ala Ala Asp Leu Lys
    370                 375                 380

Ser Thr Gln Ala Ala Ile Asp Gln Ile Asn Gly Lys Leu Asn Arg Leu
385                 390                 395                 400

Ile Glu Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Arg Thr Arg Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Ser Gly Gly Asp Ile Ile Lys Leu Leu
        515                 520                 525

Asn Glu Gln Val Asn Lys Glu Met Gln Ser Ser Asn Leu Tyr Met Ser
    530                 535                 540

Met Ser Ser Trp Cys Tyr Thr His Ser Leu Asp Gly Ala Gly Leu Phe
545                 550                 555                 560

Leu Phe Asp His Ala Ala Glu Glu Tyr Glu His Ala Lys Lys Leu Ile
                565                 570                 575

Ile Phe Leu Asn Glu Asn Asn Val Pro Val Gln Leu Thr Ser Ile Ser
            580                 585                 590

Ala Pro Glu His Lys Phe Glu Gly Leu Thr Gln Ile Phe Gln Lys Ala
        595                 600                 605

Tyr Glu His Glu Gln His Ile Ser Glu Ser Ile Asn Asn Ile Val Asp
    610                 615                 620

His Ala Ile Lys Ser Lys Asp His Ala Thr Phe Asn Phe Leu Gln Trp
625                 630                 635                 640

Tyr Val Ala Glu Gln His Glu Glu Glu Val Leu Phe Lys Asp Ile Leu
                645                 650                 655

Asp Lys Ile Glu Leu Ile Gly Asn Glu Asn His Gly Leu Tyr Leu Ala
            660                 665                 670

Asp Gln Tyr Val Lys Gly Ile Ala Lys Ser Arg Lys Ser
        675                 680                 685

<210>  16
<211>  684
<212>  PRT
<213>  Artificial Sequence

<220>
<223>  Merck_pH1_Con_ferritin

<400>  16

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Ser Ser Ser Asp Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Ala Tyr Val Phe Val Gly Thr Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Val Pro Ser Ile Gln Ser Arg Gly Leu Phe Gly Ala Ile Ala Gly
            340                 345                 350

Phe Ile Glu Gly Gly Trp Thr Gly Met Val Asp Gly Trp Tyr Gly Tyr
        355                 360                 365

His His Gln Asn Glu Gln Gly Ser Gly Tyr Ala Ala Asp Leu Lys Ser
    370                 375                 380

Thr Gln Asn Ala Ile Asp Lys Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Tyr His Asp Ser Asn Val Lys Asn Leu Tyr Glu
    450                 455                 460

Lys Val Arg Ser Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
            500                 505                 510

Asn Arg Glu Glu Ile Asp Ser Gly Gly Asp Ile Ile Lys Leu Leu Asn
        515                 520                 525

Glu Gln Val Asn Lys Glu Met Gln Ser Ser Asn Leu Tyr Met Ser Met

530                 535                 540

Ser Ser Trp Cys Tyr Thr His Ser Leu Asp Gly Ala Gly Leu Phe Leu
545                 550                 555                 560

Phe Asp His Ala Ala Glu Glu Tyr Glu His Ala Lys Lys Leu Ile Ile
                565                 570                 575

Phe Leu Asn Glu Asn Asn Val Pro Val Gln Leu Thr Ser Ile Ser Ala
            580                 585                 590

Pro Glu His Lys Phe Glu Gly Leu Thr Gln Ile Phe Gln Lys Ala Tyr
        595                 600                 605

Glu His Glu Gln His Ile Ser Glu Ser Ile Asn Asn Ile Val Asp His
    610                 615                 620

Ala Ile Lys Ser Lys Asp His Ala Thr Phe Asn Phe Leu Gln Trp Tyr
625                 630                 635                 640

Val Ala Glu Gln His Glu Glu Glu Val Leu Phe Lys Asp Ile Leu Asp
                645                 650                 655

Lys Ile Glu Leu Ile Gly Asn Glu Asn His Gly Leu Tyr Leu Ala Asp
            660                 665                 670

Gln Tyr Val Lys Gly Ile Ala Lys Ser Arg Lys Ser
        675                 680

<210>  17
<211>  683
<212>  PRT
<213>  Artificial Sequence

<220>
<223>  Merck_sH1_Con_ferritin

<400>  17

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Lys Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Arg Ala Leu Tyr
        195                 200                 205

His Thr Glu Asn Ala Tyr Val Ser Val Val Ser Ser His Tyr Ser Arg
    210                 215                 220

Arg Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala
            260                 265                 270

Leu Ser Arg Gly Phe Gly Ser Gly Ile Ile Thr Ser Asn Ala Pro Met
        275                 280                 285

Asp Glu Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro

305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Val Asp Gly Trp Tyr Gly Tyr His
        355                 360                 365

His Gln Asn Glu Gln Gly Ser Gly Tyr Ala Ala Asp Gln Lys Ser Thr
    370                 375                 380

Gln Asn Ala Ile Asn Gly Ile Thr Asn Lys Val Asn Ser Val Ile Glu
385                 390                 395                 400

Lys Met Asn Thr Gln Phe Thr Ala Val Gly Lys Glu Phe Asn Lys Leu
                405                 410                 415

Glu Arg Arg Met Glu Asn Leu Asn Lys Lys Val Asp Asp Gly Phe Leu
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asn Asp Glu Cys Met Glu Ser Val Lys
                485                 490                 495

Asn Gly Thr Tyr Asp Tyr Pro Lys Tyr Ser Glu Glu Ser Lys Leu Asn
            500                 505                 510

Arg Glu Lys Ile Asp Ser Gly Gly Asp Ile Ile Lys Leu Leu Asn Glu
        515                 520                 525

Gln Val Asn Lys Glu Met Gln Ser Ser Asn Leu Tyr Met Ser Met Ser
    530                 535                 540

Ser Trp Cys Tyr Thr His Ser Leu Asp Gly Ala Gly Leu Phe Leu Phe
545                 550                 555                 560

Asp His Ala Ala Glu Glu Tyr Glu His Ala Lys Lys Leu Ile Ile Phe
                565                 570                 575

Leu Asn Glu Asn Asn Val Pro Val Gln Leu Thr Ser Ile Ser Ala Pro
            580                 585                 590

Glu His Lys Phe Glu Gly Leu Thr Gln Ile Phe Gln Lys Ala Tyr Glu
        595                 600                 605

His Glu Gln His Ile Ser Glu Ser Ile Asn Asn Ile Val Asp His Ala
    610                 615                 620

Ile Lys Ser Lys Asp His Ala Thr Phe Asn Phe Leu Gln Trp Tyr Val
625                 630                 635                 640

Ala Glu Gln His Glu Glu Glu Val Leu Phe Lys Asp Ile Leu Asp Lys
                645                 650                 655

Ile Glu Leu Ile Gly Asn Glu Asn His Gly Leu Tyr Leu Ala Asp Gln
            660                 665                 670

Tyr Val Lys Gly Ile Ala Lys Ser Arg Lys Ser
        675                 680

<210>  18
<211>  684
<212>  PRT
<213>  Artificial Sequence

<220>
<223>  Cobra_P1_ferritin

<400>  18

Met Lys Ala Arg Leu Leu Val Leu Leu Cys Ala Leu Ala Ala Thr Asp
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Lys Leu Lys Gly Ile
    50                  55                  60

Ala Pro Leu Gln Leu Gly Lys Cys Asn Ile Ala Gly Trp Leu Leu Gly
65                  70                  75                  80

Asn Pro Glu Cys Glu Ser Leu Leu Ser Ala Arg Ser Trp Ser Tyr Ile

                85                  90                  95

Val Glu Thr Pro Asn Ser Glu Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Asn
    130                 135                 140

Thr Thr Lys Gly Val Thr Ala Ala Cys Ser His Ala Gly Lys Ser Ser
145                 150                 155                 160

Phe Tyr Arg Asn Leu Leu Trp Leu Thr Lys Lys Gly Gly Ser Tyr Pro
                165                 170                 175

Lys Leu Ser Lys Ser Tyr Val Asn Asn Lys Gly Lys Glu Val Leu Val
            180                 185                 190

Leu Trp Gly Val His His Pro Ser Thr Ser Thr Asp Gln Gln Ser Leu
        195                 200                 205

Tyr Gln Asn Glu Asn Ala Tyr Val Ser Val Val Ser Ser Asn Tyr Asn
    210                 215                 220

Arg Arg Phe Thr Pro Glu Ile Ala Glu Arg Pro Lys Val Arg Gly Gln
225                 230                 235                 240

Ala Gly Arg Met Asn Tyr Tyr Trp Thr Leu Leu Glu Pro Gly Asp Thr
                245                 250                 255

Ile Ile Phe Glu Ala Thr Gly Asn Leu Ile Ala Pro Trp Tyr Ala Phe
            260                 265                 270

Ala Leu Ser Arg Gly Ser Gly Ser Gly Ile Ile Thr Ser Asn Ala Ser
        275                 280                 285

Met His Glu Cys Asn Thr Lys Cys Gln Thr Pro Gln Gly Ala Ile Asn
    290                 295                 300

Ser Ser Leu Pro Phe Gln Asn Ile His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
        340                 345                 350

Phe Ile Glu Gly Gly Trp Thr Gly Met Ile Asp Gly Trp Tyr Gly Tyr
    355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Asn
                405                 410                 415

Leu Glu Lys Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Arg Asn Asn Ala Lys Glu Ile Gly Asn Gly
465                 470                 475                 480

Cys Phe Glu Phe Tyr His Lys Cys Asp Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Ser Gly Gly Asp Ile Ile Lys Leu Leu Asn
        515                 520                 525

Glu Gln Val Asn Lys Glu Met Gln Ser Ser Asn Leu Tyr Met Ser Met
    530                 535                 540

Ser Ser Trp Cys Tyr Thr His Ser Leu Asp Gly Ala Gly Leu Phe Leu
545                 550                 555                 560

Phe Asp His Ala Ala Glu Glu Tyr Glu His Ala Lys Lys Leu Ile Ile
                565                 570                 575

Phe Leu Asn Glu Asn Asn Val Pro Val Gln Leu Thr Ser Ile Ser Ala
            580                 585                 590

Pro Glu His Lys Phe Glu Gly Leu Thr Gln Ile Phe Gln Lys Ala Tyr
        595                 600                 605

Glu His Glu Gln His Ile Ser Glu Ser Ile Asn Asn Ile Val Asp His
    610                 615                 620

Ala Ile Lys Ser Lys Asp His Ala Thr Phe Asn Phe Leu Gln Trp Tyr
625                 630                 635                 640

Val Ala Glu Gln His Glu Glu Glu Val Leu Phe Lys Asp Ile Leu Asp
                645                 650                 655

Lys Ile Glu Leu Ile Gly Asn Glu Asn His Gly Leu Tyr Leu Ala Asp
            660                 665                 670

Gln Tyr Val Lys Gly Ile Ala Lys Ser Arg Lys Ser
        675                 680

<210> 19
<211> 684
<212> PRT
<213> Artificial Sequence

<220>
<223> Cobra_X3_ferritin

<400> 19

Met Glu Ala Arg Leu Leu Val Leu Leu Cys Ala Phe Ala Ala Thr Asn
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Arg Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Ser Leu Phe Ser Lys Glu Ser Trp Ser Tyr Ile
                85                  90                  95

Ala Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Lys Gly Val Thr Ala Ser Cys Ser His Asn Gly Lys Ser Ser
145                 150                 155                 160

Phe Tyr Arg Asn Leu Leu Trp Leu Thr Glu Lys Asn Gly Leu Tyr Pro
                165                 170                 175

Asn Leu Ser Lys Ser Tyr Val Asn Asn Lys Glu Lys Glu Val Leu Val
            180                 185                 190

Leu Trp Gly Val His His Pro Ser Asn Ile Gly Asp Gln Arg Ala Ile
        195                 200                 205

Tyr His Thr Glu Asn Ala Tyr Val Ser Val Val Ser Ser His Tyr Ser
    210                 215                 220

Arg Arg Phe Thr Pro Glu Ile Ala Lys Arg Pro Lys Val Arg Asp Gln
225                 230                 235                 240

Glu Gly Arg Ile Asn Tyr Tyr Trp Thr Leu Leu Glu Pro Gly Asp Thr
                245                 250                 255

Ile Ile Phe Glu Ala Asn Gly Asn Leu Ile Ala Pro Trp Tyr Ala Phe
            260                 265                 270

Ala Leu Ser Arg Gly Phe Gly Ser Gly Ile Ile Thr Ser Asn Ala Ser
        275                 280                 285

Met Asp Glu Cys Asp Ala Lys Cys Gln Thr Pro Gln Gly Ala Ile Asn
    290                 295                 300

Ser Ser Leu Pro Phe Gln Asn Val His Pro Val Thr Ile Gly Glu Cys
305                 310                 315                 320

Pro Lys Tyr Val Arg Ser Thr Lys Leu Arg Met Val Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Ile Asp Gly Trp Tyr Gly Tyr
        355                 360                 365

His His Gln Asn Glu Gln Gly Ser Gly Tyr Ala Ala Asp Gln Lys Ser
    370                 375                 380

Thr Gln Asn Ala Ile Asn Gly Ile Thr Asn Lys Val Asn Ser Val Ile
385                 390                 395                 400

Glu Lys Met Asn Thr Gln Phe Thr Ala Val Gly Lys Glu Phe Asn Lys
                405                 410                 415

Leu Glu Arg Arg Met Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Phe His Asp Ser Asn Val Lys Asn Leu Tyr Glu
    450                 455                 460

Lys Val Lys Ser Gln Leu Lys Asn Asn Ala Lys Glu Ile Gly Asn Gly
465                 470                 475                 480

Cys Phe Glu Phe Tyr His Lys Cys Asn Asn Glu Cys Met Glu Ser Val
                485                 490                 495

Lys Asn Gly Thr Tyr Asp Tyr Pro Lys Tyr Ser Glu Glu Ser Lys Leu
            500                 505                 510

Asn Arg Glu Lys Ile Asp Ser Gly Gly Asp Ile Ile Lys Leu Leu Asn
        515                 520                 525

Glu Gln Val Asn Lys Glu Met Gln Ser Ser Asn Leu Tyr Met Ser Met
    530                 535                 540

Ser Ser Trp Cys Tyr Thr His Ser Leu Asp Gly Ala Gly Leu Phe Leu
545                 550                 555                 560

Phe Asp His Ala Ala Glu Glu Tyr Glu His Ala Lys Lys Leu Ile Ile
                565                 570                 575

Phe Leu Asn Glu Asn Asn Val Pro Val Gln Leu Thr Ser Ile Ser Ala
            580                 585                 590

Pro Glu His Lys Phe Glu Gly Leu Thr Gln Ile Phe Gln Lys Ala Tyr
        595                 600                 605

Glu His Glu Gln His Ile Ser Glu Ser Ile Asn Asn Ile Val Asp His
    610                 615                 620

Ala Ile Lys Ser Lys Asp His Ala Thr Phe Asn Phe Leu Gln Trp Tyr
625                 630                 635                 640

Val Ala Glu Gln His Glu Glu Glu Val Leu Phe Lys Asp Ile Leu Asp
                645                 650                 655

Lys Ile Glu Leu Ile Gly Asn Glu Asn His Gly Leu Tyr Leu Ala Asp
            660                 665                 670

Gln Tyr Val Lys Gly Ile Ala Lys Ser Arg Lys Ser
        675                 680

<210>  20
<211>  498
<212>  PRT
<213>  Artificial Sequence

<220>
<223>  Wildtype Sequence of Nucleoprotein

<400>  20

Met Ala Ser Gln Gly Thr Lys Arg Ser Tyr Glu Gln Met Glu Thr Asp
1               5                   10                  15

Gly Glu Arg Gln Asn Ala Thr Glu Ile Arg Ala Ser Val Gly Lys Met
            20                  25                  30

Ile Asp Gly Ile Gly Arg Phe Tyr Ile Gln Met Cys Thr Glu Leu Lys
        35                  40                  45

Leu Ser Asp Tyr Glu Gly Arg Leu Ile Gln Asn Ser Leu Thr Ile Glu
    50                  55                  60

Arg Met Val Leu Ser Ala Phe Asp Glu Arg Arg Asn Arg Tyr Leu Glu
65                  70                  75                  80

Glu His Pro Ser Ala Gly Lys Asp Pro Lys Lys Thr Gly Gly Pro Ile
                85                  90                  95

Tyr Lys Arg Val Asp Gly Arg Trp Met Arg Glu Leu Val Leu Tyr Asp
            100                 105                 110

Lys Glu Glu Ile Arg Arg Ile Trp Arg Gln Ala Asn Asn Gly Asp Asp
        115                 120                 125

Ala Thr Ala Gly Leu Thr His Met Met Ile Trp His Ser Asn Leu Asn
    130                 135                 140

Asp Thr Thr Tyr Gln Arg Thr Arg Ala Leu Val Arg Thr Gly Met Asp
145                 150                 155                 160

Pro Arg Met Cys Ser Leu Met Gln Gly Ser Thr Leu Pro Arg Arg Ser
                165                 170                 175

Gly Ala Ala Gly Ala Ala Val Lys Gly Ile Gly Thr Met Val Met Glu
            180                 185                 190

Leu Ile Arg Met Ile Lys Arg Gly Ile Asn Asp Arg Asn Phe Trp Arg
        195                 200                 205

Gly Glu Asn Gly Arg Lys Thr Arg Ser Ala Tyr Glu Arg Met Cys Asn
    210                 215                 220

Ile Leu Lys Gly Lys Phe Gln Thr Ala Ala Gln Arg Ala Met Met Asp
225                 230                 235                 240

Gln Val Arg Glu Ser Arg Asn Pro Gly Asn Ala Glu Ile Glu Asp Leu
                245                 250                 255

Ile Phe Ser Ala Arg Ser Ala Leu Ile Leu Arg Gly Ser Val Ala His
            260                 265                 270

Lys Ser Cys Leu Pro Ala Cys Val Tyr Gly Pro Ala Val Ser Ser Gly
        275                 280                 285

Tyr Asn Phe Glu Lys Glu Gly Tyr Ser Leu Val Gly Ile Asp Pro Phe
    290                 295                 300

Lys Leu Leu Gln Asn Ser Gln Val Tyr Ser Leu Ile Arg Pro Asn Glu
305                 310                 315                 320

Asn Pro Ala His Lys Ser Gln Leu Val Trp Met Ala Cys His Ser Ala
                325                 330                 335

Ala Phe Glu Asp Leu Arg Leu Leu Ser Phe Ile Arg Gly Thr Lys Val
            340                 345                 350

Ser Pro Arg Gly Lys Leu Ser Thr Arg Gly Val Gln Ile Ala Ser Asn
        355                 360                 365

Glu Asn Met Asp Asn Met Glu Ser Ser Thr Leu Glu Leu Arg Ser Arg
    370                 375                 380

Tyr Trp Ala Ile Arg Thr Arg Ser Gly Gly Asn Thr Asn Gln Gln Arg
385                 390                 395                 400

Ala Ser Ala Gly Gln Ile Ser Val Gln Pro Thr Phe Ser Val Gln Arg
                405                 410                 415

Asn Leu Pro Phe Glu Lys Ser Thr Val Met Ala Ala Phe Thr Gly Asn
            420                 425                 430

Thr Glu Gly Arg Thr Ser Asp Met Arg Ala Glu Ile Ile Arg Met Met
        435                 440                 445

Glu Gly Ala Lys Pro Glu Glu Val Ser Phe Arg Gly Arg Gly Val Phe
    450                 455                 460

Glu Leu Ser Asp Glu Lys Ala Thr Asn Pro Ile Val Pro Ser Phe Asp
465                 470                 475                 480

Met Ser Asn Glu Gly Ser Tyr Phe Phe Gly Asp Asn Ala Glu Glu Tyr
                485                 490                 495

Asp Asn

<210>  21
<211>  573
<212>  DNA
<213>  Artificial Sequence

<220>
<223>  BHA10-2

<400>  21
atggagaccc ccgcccagct gctgttcctg ctgctgctgt ggctgcccga caccaccggc      60

cacgtggtga agaccgccac ccagggcgag gtgaacgtga ccggcgtgat ccccctgacc     120

accaccccca ccggcagcgc caacaagagc aagccctact acaccggcga gcacgccaag     180

gccaccggca actgccccat ctgggtgaag acccccctga agctggccaa cggcaccaag     240

tacggcagcg ccggcagcgc cacccaggag gccatcaaca agatcaccaa gaacctgaac     300

agcctgagcg agctggaggt gaagaacctg cagaggctga gcggcgccag cgacgagacc     360

cacaacgaga tcctggagct ggacgagaag gtggacgacc tgagggccga caccatcagc     420

agccagatcg agctggccgt gctgctgagc aacgagggca tcatcaacag cgaggacgag     480

ggcaccggcg gcggctacat ccccgaggcc cccagggacg gccaggccta cgtgaggaag     540

gacggcgagt gggtgctgct gagcaccttc ctg                                  573

<210>  22
<211>  480
<212>  DNA

<213>  Artificial Sequence

<220>
<223>  BHA10-3

<400>  22
atggagaccc ccgcccagct gctgttcctg ctgctgctgt ggctgcccga caccaccggc      60

cacgtggtga agaccgccac ccagggcgag gtgaacgtga ccggcgtgat ccccctgacc      120

accaccccca ccggcagcgc caacaagagc aagccctact acaccggcga gcacgccaag      180

gccaccggca actgccccat ctgggtgaag acccccctga agctggccaa cggcaccaag      240

tacggcagcg ccggcagcgc cacccaggag gccatcaaca agatcaccaa gaacctgaac      300

agcctgagcg agctggaggt gaagaacctg cagaggctga gctgcgccag cgacgagacc      360

cacaactgca tcctggagct ggacgagaag gtggacgacc tgagggccga caccatcagc      420

agcctgatcg agctggccgt gctgctgagc aacgagggca tcatcaacag cgaggacgag      480


<210>  23
<211>  780
<212>  DNA
<213>  Artificial Sequence

<220>
<223>  NIHGen6HASS-TM

<400>  23
atggagaccc ccgcccagct gctgttcctg ctgctgctgt ggctgcccga caccaccggc      60

gacaccatct gcatcggcta ccacgccaac aacagcaccg acaccgtgga caccgtgctg      120

gagaagaacg tgaccgtgac ccacagcgtg aacctgggca gcggcctgag gatggtgacc      180

ggcctgagga acatccccca gagggagacc aggggcctgt tcggcgccat cgccggcttc      240

atcgagggcg gctggaccgg catggtggac ggctggtacg gctaccacca ccagaacgag      300

cagggcagcg gctacgccgc cgaccagaag agcacccaga acgccatcaa cggcatcacc      360

aacatggtga acagcgtgat cgagaagatg ggcagcggcg gcagcggcac cgacctggcc      420

gagctgctgg tgctgctgct gaacgagagg accctggact tccacgacag caacgtgaag      480

aacctgtacg agaaggtgaa gagccagctg aagaacaacg ccaaggagat cggcaacggc      540

tgcttcgagt tctaccacaa gtgcaacaac gagtgcatgg agagcgtgaa gaacggcacc      600

tacgactacc ccaagtacag cgaggagagc aagctgaaca gggagaagat cgaccagggc      660

accggcggca tcctggccat ctacagcacc gtggccagca gcctggtgct gctggtgagc      720

ctgggcgcca tcagcttctg gatgtgcagc aacggcagcc tgcagtgcag aatctgcatc      780


<210>  24
<211>  798
<212>  DNA
<213>  Artificial Sequence

<220>
<223>  NIHGen6HASS-TM2

<400>  24
atggagaccc ccgcccagct gctgttcctg ctgctgctgt ggctgcccga caccaccggc      60

gacaccatct gcatcggcta ccacgccaac aacagcaccg acaccgtgga caccgtgctg     120

gagaagaacg tgaccgtgac ccacagcgtg aacctgggca gcggcctgag gatggtgacc     180

ggcctgagga acatccccca gagggagacc aggggcctgt tcggcgccat cgccggcttc     240

atcgagggcg gctggaccgg catggtggac ggctggtacg gctaccacca ccagaacgag     300

cagggcagcg gctacgccgc cgaccagaag agcacccaga acgccatcaa cggcatcacc     360

aacatggtga acagcgtgat cgagaagatg ggcagcggcg gcagcggcac cgacctggcc     420

gagctgctgg tgctgctgct gaacgagagg accctggact tccacgacag caacgtgaag     480

aacctgtacg agaaggtgaa gagccagctg aagaacaacg ccaaggagat cggcaacggc     540

tgcttcgagt tctaccacaa gtgcaacaac gagtgcatgg agagcgtgaa gaacggcacc     600

tacgactacc ccaagtacag cgaggagagc aagctgaaca gggagaagat cgacggagtg     660

aaattggaat caatgggggt ctatcagatc ctggccatct acagcaccgt ggccagcagc     720

ctggtgctgc tggtgagcct gggcgccatc agcttctgga tgtgcagcaa cggcagcctg     780

cagtgcagaa tctgcatc                                                   798

<210>  25
<211>  744
<212>  DNA
<213>  Artificial Sequence

<220>
<223>  NIHGen6HASS-foldon

<400>  25
atggagaccc ccgcccagct gctgttcctg ctgctgctgt ggctgcccga caccaccggc      60

gacaccatct gcatcggcta ccacgccaac aacagcaccg acaccgtgga caccgtgctg     120

gagaagaacg tgaccgtgac ccacagcgtg aacctgggca gcggcctgag gatggtgacc     180

ggcctgagga acatccccca gagggagacc aggggcctgt tcggcgccat cgccggcttc     240

atcgagggcg gctggaccgg catggtggac ggctggtacg gctaccacca ccagaacgag     300

cagggcagcg gctacgccgc cgaccagaag agcacccaga acgccatcaa cggcatcacc     360

aacatggtga acagcgtgat cgagaagatg ggcagcggcg gcagcggcac cgacctggcc     420

gagctgctgg tgctgctgct gaacgagagg accctggact tccacgacag caacgtgaag     480

aacctgtacg agaaggtgaa gagccagctg aagaacaacg ccaaggagat cggcaacggc     540

tgcttcgagt tctaccacaa gtgcaacaac gagtgcatgg agagcgtgaa gaacggcacc     600

tacgactacc ccaagtacag cgaggagagc aagctgaaca gggagaagat cgaccccggc 660

agcggctaca tccccgaggc ccccagggac ggccaggcct acgtgaggaa ggacggcgag 720

tgggtgctgc tgagcacctt cctg 744

<210> 26
<211> 1698
<212> DNA
<213> Artificial Sequence

<220>
<223> ConH1

<400> 26
atgaaggcca agctgctggt gctgctgtgc gccttcaccg ccaccgacgc cgacaccatc 60

tgcattggct accatgccaa caacagcacc gacaccgtgg acaccgtgct cgagaagaac 120

gtgaccgtga cccactccgt gaatttgttg gaggacagcc acaacggcaa gctgtgtaag 180

ctgaagggca tcgcccccct gcagctgggc aagtgcaaca tcgccggctg gatcttgggc 240

aatcccgagt gcgaaagcct gatctccaag agaagctgga gctacatcgt ggagactccc 300

aacagcgaaa acggcacctg ctaccccggc gacttcgctg actacgagga actgagagag 360

cagctgagca gcgtgagcag ctttgagaga ttcgagatct tccccaaaga gagcagctgg 420

cccaaccaca acgtaaccaa gggcgtgaca gccgcctgca gccacgccgg taagagcagc 480

ttctacagaa acctgctgtg gctgacagag aagaacggca gctaccccaa gctgagcaag 540

agctatgtga acaacaagga gaaggaggtg ctggtcctgt ggggcgtaca ccaccccagc 600

aacattaccg atcagagaac cctgtaccag aacgagaatg cctacgtgag cgtggtgagc 660

agccactaca acagaagatt cacccccgag attgccaaga gaccgaaagt gagaggccag 720

gccggaagaa tcaactacta ctggaccctg ctggagcccg gcgacaccat catcttcgag 780

gccaacggca acctgatcgc cccctggtat gccttcgccc tgagcagagg cttcggaagc 840

ggcatcatca catccaacgc ccccatgcat gaatgcgaca caaagtgtca gaccccccag 900

ggcgccatca acagcagcct gcccttccag aacgtgcacc ctgtgaccat cggcgagtgc 960

cccaagtacg tgagaagcac caagctgaga atggtgaccg gcctgagaaa tatccccagt 1020

atccagagca gaggcctgtt cggcgccatc gccggcttca tcgagggcgg ctggaccggc 1080

atgatcgacg gctggtacgg ctaccaccac cagaacgagc agggcagcgg ctacgccgcc 1140

gaccagaaga gcactcagaa cgccatcaac ggcatcacca acaaggtgaa cagcgtgatc 1200

gagaagatga acacacagtt caccgccgtg ggcaaagagt tcaataagct cgagaagaga 1260

atggaaaacc tgaacaagaa ggttgacgac ggtttcctgg atatctggac ctacaacgcc 1320

gagctgcttg tgctgctgga gaacgagcgt accctggact ttcacgactc gaacgtgaag 1380

aacctgtacg aaaaggtgaa gtcccagctg aagaacaacg ccaaagaaat tggcaacggc 1440

tgcttcgagt tctaccacaa gtgtaacaac gagtgcatgg agagcgtgaa gaacggcacc 1500

tacgactatc ccaagtacag cgaggagagc aagctaaaca gagagaagat tgacggcgtg 1560

aaactggagt caatgggcgt gtaccagatc ctggccatct acagcaccgt ggccagcagc 1620

ctcgtgctgc tggtgagcct gggcgccata agcttctgga tgtgtagcaa cggcagcctg 1680

cagtgcagaa tctgcatc 1698

<210> 27
<211> 1698
<212> DNA
<213> Artificial Sequence

<220>
<223> ConH3

<400> 27
atgaagacca tcatcgctct gagctacata ttctgcctgg tgttcgccca gaagctgccc 60

ggcaacgaca acagcaccgc caccctgtgc ctgggccatc acgcagtgcc aaacggcacc 120

ttagttaaga ccatcaccaa cgaccagatc gaggtgacca acgccaccga gctggtgcag 180

agcagtagca ccggcagaat ctgcgacagc ccccaccgga tccttgacgg cactaactgc 240

accctgatcg acgccctgct gggcgacccc cactgcgacg ggttccagaa caaggagtgg 300

gacctgttcg tggagagaag caaggcctac agcaactgct acccctacga tgtgcccgac 360

tacgccagcc tgagatctct tgtggctagc agcggcaccc tggagttcaa caatgagggc 420

ttcaattgga caggcgtgac ccagaacggc ggcagcagcg cctgcaagag aggcagcgac 480

aagagcttct tcagcagact gaactggctg cacaagctga agtacaagta tcccgccctg 540

aacgtgacca tgcccaataa cgacaagttc gataagctgt atatttgggg cgtgcaccac 600

cccagcaccg acagcgacca gacctccctg tacgtccagg cgagcggcag agtgaccgtg 660

agcaccaaac ggagccagca aaccgtgatc cccaacatcg gcagcagacc ttgggtcaga 720

ggactgagca gcagaatcag catctactgg accatcgtga agcctggcga catcttgctg 780

atcaatagca ccggcaacct gatcgccccc agaggctact tcaagatcag aagcggcaag 840

agctcaatca tgagaagcga cgcccccata ggcacctgca acagcgagtg catcaccccg 900

aacggcagca tccccaacga caagcccttc cagaacgtga acagaatcac atacggcgcc 960

tgccccaggt acgtaaagca aaacaccctg aagctggcca ccggcatgag aaacgtaccc 1020

gagaagcaga ccagaggcat cttcggcgcc atcgcgggct tcatcgagaa tggctgggaa 1080

ggcatggtgg acggctggta cggcttcaga catcagaaca gcgagggcac cggccaggcc 1140

gccgacctga aaagcaccca ggccgccatc gaccagatca acggcaagct gaacagactg 1200

atcgaaaaga ccaacgagaa gttccaccag atcgagaagg agttcagcga agtggaggga 1260

agaatacagg acctggagaa atacgtggag gacaccaaga tcgacctgtg gtcgtacaac 1320

gccgagctgc tggtggccct ggaaaaccag cacaccattg acctgaccga tagcgagatg 1380

aacaagctgt tcgagagaac cagaaaacag ctgagagaga acgccgagga catgggcaac 1440

ggctgcttta agatctacca caagtgcgac aacgcctgca tcggcagcat cagaaacggc 1500

acctacgacc acgacgtgta cagagacgag gccctgaaca acagattcca gatcaagggc 1560

gtggagctga agagcggcta caaggactgg atcctgtgga tcagcttcgc catcagctgt 1620

ttcctgcttt gcgtagtgct gctgggcttc atcatgtggg cctgccagaa gggcaatatc 1680

agatgcaaca tttgcatc 1698

<210> 28
<211> 1698
<212> DNA
<213> Artificial Sequence

<220>
<223> MRK_pH1_Con

<400> 28
atgaaggcca tcctggtggt gctgctgtac accttcgcca cggccaacgc tgacaccctg 60

tgcatcggat accacgcgaa caacagcacc gacaccgttg acaccgtgct ggagaagaac 120

gtgaccgtga cccacagcgt gaacctcctg gaagacaagc acaacggcaa gctgtgcaag 180

ctgagaggcg tggcccccct gcacctgggc aagtgcaaca tcgccggctg gatcttaggc 240

aaccccgagt gcgagagcct gagcaccgcc agcagctgga gctacattgt ggagaccagc 300

agcagcgaca acggcacctg ctaccccggc gacttcatcg actacgagga gctgagagag 360

cagctgagca gcgtgagcag cttcgagaga ttcgagatct tccccaagac ctcaagctgg 420

cccaaccacg acagcaataa gggcgtgact gccgcctgcc cccacgccgg cgccaagagc 480

ttctacaaga acctgatctg gctggtgaag aaaggcaata gctaccccaa gctgagcaag 540

tcctatatca acgacaaggg caaggaggtg ttggttctgt ggggcatcca tcaccccagc 600

accagtgctg atcagcaaag cctgtaccaa aacgccgatg cctacgtgtt cgtgggcacc 660

tcaagataca gcaagaagtt caagcccgag atcgccatca gacccaaggt gagagaccag 720

gagggtagaa tgaactacta ctggaccctc gtggagccgg gcgacaagat caccttcgag 780

gccaccggca acctggtggt gcccagatac gccttcgcca tggagagaaa tgccggcagc 840

gggatcatca tctcggacac ccccgtgcac gattgtaaca ccacctgcca gacccccaag 900

ggcgccatca acacctccct gcccttccag aacatccacc ccatcaccat cggcaagtgc 960

cccaagtacg tgaagagcac caagctgaga ctggcgaccg gactgagaaa cgtgcccagc 1020

atccagtcaa gaggcctgtt cggcgccatc gccggcttca tcgagggcgg ctggacaggc 1080

atggtggacg gctggtacgg ctaccaccac cagaacgagc agggcagcgg atacgccgcc 1140

gacctgaaga gcacacaaaa cgccatcgac aagatcacca acaaggtgaa cagcgtgatc 1200

gaaaagatga acacccagtt caccgccgtg ggcaaggagt tcaaccacct ggagaagaga 1260

atcgagaacc tgaacaagaa agtggacgac ggcttcctgg acatctggac ctacaacgcc 1320

gagctgctgg tcctgctgga gaacgagaga accctggact accatgacag caacgtgaag 1380

aacctgtacg agaaggtgag aagccagctg aagaacaacg ccaaggagat aggcaacggc 1440

tgcttcgagt tctaccacaa gtgcgacaac acctgcatgg agagcgtgaa gaacggcacc 1500

tacgactacc caaagtatag cgaggaggca aagctgaaca gagaggagat cgacggcgtg 1560

aagctggaga gcaccaggat ctatcaaatc ctggccatat acagcaccgt ggccagcagc 1620

ctggtgttag tggtgagcct gggcgccatc agcttctgga tgtgtagcaa cggcagcctg 1680

cagtgcagaa tctgcatc 1698

<210> 29
<211> 1587
<212> DNA
<213> Artificial Sequence

<220>
<223> MRK_pH1_Con

<400> 29
atgaaggcca tcctggtggt gctgctgtac accttcgcca cggccaacgc tgacaccctg 60

tgcatcggat accacgcgaa caacagcacc gacaccgttg acaccgtgct ggagaagaac 120

gtgaccgtga cccacagcgt gaacctcctg gaagacaagc acaacggcaa gctgtgcaag 180

ctgagaggcg tggcccccct gcacctgggc aagtgcaaca tcgccggctg gatcttaggc 240

aaccccgagt gcgagagcct gagcaccgcc agcagctgga gctacattgt ggagaccagc 300

agcagcgaca acggcacctg ctaccccggc gacttcatcg actacgagga gctgagagag 360

cagctgagca gcgtgagcag cttcgagaga ttcgagatct tccccaagac ctcaagctgg 420

cccaaccacg acagcaataa gggcgtgact gccgcctgcc cccacgccgg cgccaagagc 480

ttctacaaga acctgatctg gctggtgaag aaaggcaata gctaccccaa gctgagcaag 540

tcctatatca acgacaaggg caaggaggtg ttggttctgt ggggcatcca tcaccccagc 600

accagtgctg atcagcaaag cctgtaccaa aacgccgatg cctacgtgtt cgtgggcacc 660

tcaagataca gcaagaagtt caagcccgag atcgccatca gacccaaggt gagagaccag 720

gagggtagaa tgaactacta ctggaccctc gtggagccgg gcgacaagat caccttcgag 780

gccaccggca acctggtggt gcccagatac gccttcgcca tggagagaaa tgccggcagc 840

gggatcatca tctcggacac ccccgtgcac gattgtaaca ccacctgcca gacccccaag 900

ggcgccatca acacctccct gcccttccag aacatccacc ccatcaccat cggcaagtgc 960

cccaagtacg tgaagagcac caagctgaga ctggcgaccg gactgagaaa cgtgcccagc 1020

atccagtcaa gaggcctgtt cggcgccatc gccggcttca tcgagggcgg ctggacaggc 1080

atggtggacg gctggtacgg ctaccaccac cagaacgagc agggcagcgg atacgccgcc 1140

gacctgaaga gcacacaaaa cgccatcgac aagatcacca acaaggtgaa cagcgtgatc 1200

gaaaagatga acacccagtt caccgccgtg ggcaaggagt tcaaccacct ggagaagaga 1260

atcgagaacc tgaacaagaa agtggacgac ggcttcctgg acatctggac ctacaacgcc 1320

gagctgctgg tcctgctgga gaacgagaga accctggact accatgacag caacgtgaag 1380

aacctgtacg agaaggtgag aagccagctg aagaacaacg ccaaggagat aggcaacggc 1440

tgcttcgagt tctaccacaa gtgcgacaac acctgcatgg agagcgtgaa gaacggcacc 1500

tacgactacc caaagtatag cgaggaggca aagctgaaca gagaggagat cgacggcgtg 1560

aagctggaga gcaccaggat ctatcaa 1587

<210> 30
<211> 1695
<212> DNA
<213> Artificial Sequence

<220>
<223> MRK_sH1_Con

<400> 30
   atgaaggtga agctgctggt gctgctgtgt accttcactg ccacttacgc cgacaccatt 60
   tgcatcggct accacgccaa caacagcacc gataccgtgg acaccgtgct ggagaagaac 120
   gtcaccgtga cccacagcgt gaacctgctg gaggatagcc ataacggcaa gctgtgcctg 180
   ctgaagggaa tcgcccccct gcagctcggc aactgcagcg tggccggctg gattctgggc 240
   aaccccgagt gcgaactgct gattagcaaa gagtcctgga gctacatcgt ggaaaccccg 300
   aatcccgaga acggcacctg ctaccccggc tacttcgccg actacgaaga gctaagagag 360
   cagctgagta gcgtgagctc attcgagaga ttcgagatct ttcccaagga gtctagctgg 420
   cccaatcaca ccgtgaccgg cgtgagcgcc agctgtagcc acaacggcaa gagcagcttc 480
   tacagaaacc tgctgtggct gaccggcaag aacggactgt accctaacct gagcaagagc 540
   tacgcgaaca ataaggagaa ggaggtgcta gtgctgtggg gcgtgcacca cccccccaac 600
   atcggcgacc agagagccct gtaccacacc gagaacgcct acgtgagcgt ggtgagcagc 660
   cactatagca gaagattcac ccccgagatc gccaagagac caaaggtgag agatcaggaa 720
   ggaagaataa actactactg gaccctcctg gagcccggcg acaccatcat cttcgaggct 780
   aacggcaacc tgatcgcccc cagatacgcc ttcgccctga gcagaggctt cggcagcggc 840
   atcatcacca gcaatgcccc catggatgag tgcgacgcca agtgccagac cccccagggc 900
   gccatcaact cgagcctgcc cttccagaat gtgcaccccg tgaccatcgg cgagtgcccc 960
   aagtacgtga gaagcgccaa gctgagaatg gtgaccggcc tgagaaacat cccaagcatc 1020
   cagagcagag ggctgttcgg cgccatcgct ggcttcatcg agggcggctg gaccggcatg 1080
   gtggacggct ggtacggtta tcaccaccag aacgagcagg gcagcggcta cgccgccgac 1140
   cagaagagca cccagaacgc catcaacggc attacaaaca aggtgaacag cgttatcgag 1200
   aagatgaaca cccaattcac cgccgtgggc aaggagttca acaagctgga gagaagaatg 1260
   gagaacctga acaagaaggt ggacgacggc ttcctggaca tctggaccta caacgccgag 1320
   ctgctggtgc tgctggagaa cgagagaacc ctggacttcc acgactccaa cgtgaagaac 1380
   ttatacgaga aggtgaagag ccagctgaag aacaacgcca aagaaatcgg aaacggctgc 1440
   ttcgaattct accacaagtg caacgacgaa tgcatggaga gcgtgaagaa cggaacctac 1500
   gactacccca agtacagcga ggaaagcaaa ctgaacagag agaagatcga cggcgtgaag 1560
   ttagagagca tgggcgtgta tcagatcctg gccatttata gcacggtggc cagcagcctg 1620
   gtgctgctgg tgagcctggg cgccatcagc ttctggatgt gcagcaacgg cagcctgcag 1680
   tgcagaatct gcatc 1695

<210> 31
<211> 1584
<212> DNA
<213> Artificial Sequence

<220>
<223> MRK_sH1_Con

<400> 31
atgaaggtga agctgctggt gctgctgtgt accttcactg ccacttacgc cgacaccatt 60

tgcatcggct accacgccaa caacagcacc gataccgtgg acaccgtgct ggagaagaac 120

gtcaccgtga cccacagcgt gaacctgctg gaggatagcc ataacggcaa gctgtgcctg 180

ctgaagggaa tcgcccccct gcagctcggc aactgcagcg tggccggctg gattctgggc 240

aaccccgagt gcgaactgct gattagcaaa gagtcctgga gctacatcgt ggaaaccccg 300

aatcccgaga acggcacctg ctaccccggc tacttcgccg actacgaaga gctaagagag 360

cagctgagta gcgtgagctc attcgagaga ttcgagatct ttcccaagga gtctagctgg 420

cccaatcaca ccgtgaccgg cgtgagcgcc agctgtagcc acaacggcaa gagcagcttc 480

tacagaaacc tgctgtggct gaccggcaag aacggactgt accctaacct gagcaagagc 540

tacgcgaaca ataaggagaa ggaggtgcta gtgctgtggg gcgtgcacca cccccccaac 600

atcggcgacc agagagccct gtaccacacc gagaacgcct acgtgagcgt ggtgagcagc 660

cactatagca gaagattcac ccccgagatc gccaagagac caaaggtgag agatcaggaa 720

ggaagaataa actactactg gaccctcctg gagcccggcg acaccatcat cttcgaggct 780

aacggcaacc tgatcgcccc cagatacgcc ttcgccctga gcagaggctt cggcagcggc 840

atcatcacca gcaatgcccc catggatgag tgcgacgcca agtgccagac cccccagggc 900

gccatcaact cgagcctgcc cttccagaat gtgcaccccg tgaccatcgg cgagtgcccc 960

aagtacgtga gaagcgccaa gctgagaatg gtgaccggcc tgagaaacat cccaagcatc 1020

cagagcagag ggctgttcgg cgccatcgct ggcttcatcg agggcggctg gaccggcatg 1080

gtggacggct ggtacggtta tcaccaccag aacgagcagg gcagcggcta cgccgccgac 1140

cagaagagca cccagaacgc catcaacggc attacaaaca aggtgaacag cgttatcgag 1200

aagatgaaca cccaattcac cgccgtgggc aaggagttca acaagctgga gagaagaatg 1260

gagaacctga acaagaaggt ggacgacggc ttcctggaca tctggaccta caacgccgag 1320

ctgctggtgc tgctggagaa cgagagaacc ctggacttcc acgactccaa cgtgaagaac 1380

ttatacgaga aggtgaagag ccagctgaag aacaacgcca aagaaatcgg aaacggctgc 1440

ttcgaattct accacaagtg caacgacgaa tgcatggaga gcgtgaagaa cggaacctac 1500

gactacccca agtacagcga ggaaagcaaa ctgaacagag agaagatcga cggcgtgaag 1560

ttagagagca tgggcgtgta tcag 1584

<210> 32
<211> 1698
<212> DNA
<213> Artificial Sequence

<220>
<223> Cobra_P1

<400> 32
atgaaggccc gcctcttggt gctgctgtgc gccctggcgg ccacagacgc cgacacaatc 60

tgtatcggct accacgccaa taatagcacc gataccgtgg ataccgtgct cgagaagaac 120

gtcaccgtta cacactccgt gaatttactg gaggacagcc acaatggcaa gctctgcaaa 180

ctgaagggta tcgccccact ccaactgggc aagtgcaaca tcgcaggctg gctgctgggc 240

aaccctgagt gtgagagcct gctgagcgct agaagctgga gctacatagt ggagacacct 300

aacagcgaaa acggcacatg ctaccccggc gacttcatcg attacgagga actgcgggag 360

cagctgagta gcgtgagctc ctttgagaga tttgagatct tccccaaaga gagcagctgg 420

cccaaccata ataccaccaa aggcgtgacc gccgcttgca gtcatgcagg gaaaagtagc 480

ttctaccgga acctgctctg gttgaccaag aagggaggga gctacccaaa gttgagcaaa 540

agctacgtga ataacaaggg caaggaggtg ctcgtgctgt ggggagtcca ccatcccagc 600

acatccactg atcagcagtc cctgtatcag aacgaaaacg cctacgtgag tgtggtgagc 660

tctaactaca acagacggtt cacccctgaa attgctgaga ggccaaaggt gagaggccag 720

gccggcagaa tgaactacta ttggaccctc ctggagcctg gggacacgat catcttcgag 780

gcgaccggga acctcatcgc tccctggtat gccttcgccc tgagcagggg cagtggcagc 840

ggaatcatca ccagcaacgc cagcatgcat gagtgcaaca ctaaatgcca gaccccccag 900

ggggccatca acagctccct gcccttccag aacatccatc ctgtgaccat tggggagtgc 960

cccaagtacg tgaggtccac caagctgagg atggtgactg gactgaggaa catccccagc 1020

atccagagcc gggggctgtt tggcgccatt gccggcttta tcgagggtgg ctggacaggt 1080

atgattgatg gctggtacgg ataccaccac cagaacgagc aggggagtgg gtatgctgcc 1140

gaccagaaat ctactcagaa cgccatcaat ggcatcacca ataaggtgaa cagcgtcatc 1200

gagaagatga acacccagtt caccgctgtg ggcaaggagt tcaacaacct ggaaaagcgc 1260

atggagaacc tgaacaagaa ggtggacgac ggcttcctgg acatctggac ctacaacgcc 1320

gagctgcttg tcctcctgga gaacgagagg accttggact tccatgacag caatgtgaag 1380

aacctctacg agaaagtgaa gagccagctg agaaacaatg ctaaggagat cggcaacggc 1440

tgcttcgagt tttaccacaa gtgcgacaat gagtgcatgg agagcgtgaa gaatggcact 1500

tatgactacc ccaagtactc agaggagtcc aaactgaata gagagaagat tgatggcgtc 1560

aagctagagt ccatgggcgt ttaccagatc ctggcaatct atagcaccgt ggccagctcc 1620

ctggtgctgc tggtgtcact gggagccata tccttctgga tgtgctccaa cggcagcctt 1680

cagtgtagaa tctgcatc 1698

<210> 33
<211> 1698
<212> DNA
<213> Artificial Sequence

<220>
<223> Cobra_X3

<400> 33
atggaggccc gcctgctcgt gcttctgtgc gcctttgccg ccactaacgc cgacaccatc 60

tgtatcggct accacgccaa caatagtaca gataccgtgg acactgtgct ggagaagaac 120

gtaacagtga cacattctgt caacctgctc gaggactctc ataatggcaa gctgtgccgc 180

ctgaagggca tcgcccctct gcagctggga aattgctccg tggccggctg gatcctgggc 240

aatccggaat gcgaaagcct gttcagcaag gagagctgga gctacatcgc cgagacacct 300

aaccctgaga acgggacctg ctaccctgga tacttcgccg actacgaaga gctgcgggag 360

cagctcagct cagtgtcatc cttcgagcgg ttcgagatct tccccaagga gagctcttgg 420

cccaaccaca ccgtgaccaa gggcgtcaca gcaagctgta gccacaacgg caagagctcc 480

ttctatagaa acctgctgtg gctgaccgag aagaacggcc tgtaccccaa tctgagtaag 540

tcctacgtga acaacaagga aaaggaagtg ctggtgctgt ggggcgtgca ccacccctcc 600

aacatcggcg accagcgcgc catctaccac actgagaatg catacgtaag cgttgtcagc 660

tcccactata gtaggagatt cacacccgag atcgctaaga ggcccaaggt gagagaccag 720

gagggcagaa tcaattatta ctggaccctg ctggagcccg gagacaccat tatcttcgaa 780

gctaacggca atttgatcgc cccttggtat gcctttgccc tctcaagggg tttcgggagc 840

ggaattatca cctccaatgc cagcatggat gagtgcgacg ccaagtgcca gacgcctcag 900

ggcgccatta attcctccct gcccttccag aacgtgcacc ccgtgaccat cggggagtgc 960

cccaagtatg ttagatccac taagctcagg atggtgacag gactgcgcaa catcccgagc 1020

attcagagca ggggcctctt cggggccatt gctgggttca tcgagggcgg gtggaccggc 1080

atgatcgacg gctggtatgg ctaccaccac cagaacgagc agggcagcgg gtacgctgct 1140

gaccaaaagt ccacccaaaa tgctatcaac ggcatcacca acaaggttaa tagcgtcatc 1200

gaaaagatga atacccagtt cacagccgtg ggaaaggaat tcaacaagct ggaacgacgg 1260

atggagaacc tgaataagaa ggtggacgac gggttcctgg acatctggac ttataacgct 1320

gagctgctcg tgctgttaga gaacgagaga accctggact ttcacgacag caacgtgaag 1380

aacctgtacg agaaggtgaa gtctcagctg aaaaataacg ctaaggaaat tggcaacggg 1440

tgcttcgaat tctatcacaa gtgcaacaac gaatgcatgg agagtgttaa gaacggaacc 1500

tatgactacc ccaagtacag tgaggaaagt aaactgaata gggagaagat cgacggcgtg 1560

aaactggagt ccatgggggt ttaccagatt ctggccatct atagcaccgt ggccagcagc 1620

ttagtgctgc tggtgtccct cggcgctatt agcttctgga tgtgcagcaa cggaagcctg 1680

cagtgtcgga tatgcatc 1698

<210> 34
<211> 2052
<212> DNA
<213> Artificial Sequence

<220>
<223> ConH1_ferritin

<400> 34
atgaaggcga agctccttgt gctgctctgc gcgttcaccg ccaccgacgc agatacaatt 60

tgcatcggat accacgccaa caattccacc gacaccgtgg acaccgttct ggagaaaaac 120

gtgacggtga cccacagcgt gaacctcctg gaggatagcc ataacggcaa gctgtgtaag 180

ctgaaaggca tcgcccccct gcagctggga aagtgcaaca ttgctggatg gatcctggga 240

aatcccgagt gtgaaagcct cattagcaaa cgcagctgga gctacattgt ggagacccca 300

aattctgaga atgggacctg ttaccctggc gactttgccg actacgagga gctgagagag 360

cagttgagca gcgtcagctc cttcgagaga ttcgaaatct ttccaaagga gtcttcgtgg 420

cccaaccaca acgtgactaa gggcgtcacc gcagcttgta gccacgcggg caaatcttcc 480

ttctacagaa acctactgtg gctcaccgag aaaaacggca gctaccccaa gctgagcaag 540

agctacgtga ataacaaaga gaaggaagtg ctggtgctgt ggggcgtcca ccaccccagc 600

aacatcacag accaaagaac actctaccag aacgagaacg cctacgtgag tgtggtgtcc 660

agccattaca accgccgatt cacccccgag atcgccaaac ggcccaaagt gcggggccag 720

gccggaagaa ttaactacta ctggaccctc ctggaaccag gagacaccat tatcttcgaa 780

gccaatggca atctgatcgc tccctggtac gccttcgcac tgtcgagagg gtttggcagc 840

ggcatcatca cctccaacgc cccaatgcat gaatgtgata ccaagtgcca gaccccacag 900

ggcgccatta acagcagcct gccattccag aacgtccatc ccgtgacaat cggcgagtgt 960

cctaagtacg tgcgctcaac gaaactgagg atggtgacag gactgagaaa cattccctca 1020

atccagagca gagggctgtt cggcgccata gccggattca ttgagggcgg atggacaggc 1080

atgattgacg gctggtatgg ctaccaccat cagaacgagc aaggcagtgg ctacgcagcc 1140

gaccagaaga gcacacagaa cgccattaac gggatcacca acaaggtgaa tagcgtgatc 1200

gagaagatga atacccagtt cactgccgtg ggtaaggagt tcaacaagct ggagaagcgg 1260

atggagaacc tcaacaagaa agtcgatgat ggcttcctgg acatctggac ctataatgct 1320

gaactgctcg tgctacttga gaatgagagg acgcttgact ttcacgactc caacgtaaaa 1380

aacctgtacg agaaggtgaa gtcgcagctg aaaaataacg ccaaggaaat cggcaacggc 1440

tgttttgagt tttaccataa atgcaataac gagtgcatgg agagcgtgaa gaatggcacc 1500

tacgactatc ccaaatactc cgaggagagc aagctcaacc gggagaaaat cgatagcggc 1560

ggggatatca ttaagctgct taacgagcag gtcaacaagg agatgcagtc aagcaacctt 1620

tacatgagca tgagcagctg gtgttacaca cacagcctgg acggagccgg actgttcctg 1680

ttcgaccacg ctgcagagga atatgagcac gctaagaagc ttataatttt cctcaacgag 1740

aataacgtgc ccgtccagct gacctccatc agcgcccccg agcacaagtt tgagggcctg 1800

acccagatct tccagaaggc ctacgagcac gagcagcaca tcagcgagtc tatcaacaac 1860

atcgtagacc atgcaatcaa gtctaaggac cacgctacat ttaactttct gcaatggtac 1920

gtggctgaac aacacgagga ggaggtactg ttcaaggata ttctcgacaa gatcgaactc 1980

atcgggaatg agaaccacgg cctgtacctg gccgaccagt acgtgaaagg aattgccaaa 2040

tccagaaagt cc 2052

<210> 35
<211> 2055
<212> DNA
<213> Artificial Sequence

<220>
<223> ConH3_ferritin

<400> 35
atgaagacaa tcattgccct gagctacatt ttttgcttag tgtttgctca gaaactgcca 60

ggcaacgata attcaacagc caccttgtgc ctcggccacc acgctgtgcc taacggcact 120

ctggtgaaga ccatcaccaa cgaccagatc gaggtgacca acgccacgga gctggtgcag 180

tcaagctcca ccggaagaat ctgcgatagc ccccatagga ttctggatgg caccaactgc 240

accctgattg acgccctgct cggcgatccc cactgcgacg gtttccaaaa caaggagtgg 300

gacctgtttg tggagagaag caaggcctat tcaaattgct acccttacga cgtccctgat 360

tacgcctcac tcaggtccct ggtggccagc agcgggaccc tggaattcaa caatgagggg 420

ttcaactgga ccggggtgac ccaaaacggc ggctccagcg cctgtaagag gggcagcgac 480

aagtccttct tctctaggct gaactggttg cacaaactga agtacaagta ccctgcatta 540

aacgtgacca tgcccaacaa cgataaattc gacaagctgt acatctgggg agtgcatcac 600

cccagcacag actcagacca gaccagtctg tatgtgcagg caagcgggag ggtgacggtc 660

tccaccaagc ggagccagca gaccgtgatc cccaacatcg gctccagacc atgggtcagg 720

ggcctgagca gccggatctc catctactgg accatagtga agcctggcga catcctgctg 780

atcaacagca ccggcaacct catcgcccct cgcggttact tcaagatccg tagtggcaaa 840

tcaagcatca tgagatccga cgcacccatc gggacctgca atagcgagtg catcaccccc 900

aacggatcta tccctaatga caagcctttt cagaacgtga accggattac ctatggtgcc 960

tgccccagat acgtgaagca gaacaccctg aagctggcga ccggcatgcg caacgtgccg 1020

gaaaagcaga cccggggcat cttcggcgcc attgccgggt ttattgagaa tggctgggaa 1080

ggcatggtgg atgggtggta cggctttagg catcagaact ctgagggtac tggtcaggcc 1140

gccgacctga aatccaccca ggccgccatt gaccagatta acgggaaact taacagactg 1200

attgagaaga ccaatgagaa gttccaccag atcgaaaagg aattctccga agtggagggc 1260

aggattcagg acttagagaa atatgtggag gataccaaga tcgacctgtg gagctataac 1320

gccgagctgc ttgtggctct ggagaaccag cacaccatcg atctgaccga cagcgagatg 1380

aataagctgt tcgagaggac acgcaagcag ctgagggaga acgccgagga catggggaac 1440

gggtgcttta agatctatca caagtgcgac aatgcctgca tcgggtctat cagaaatggc 1500

acttatgatc atgacgtgta cagagatgag gccctgaata atagatttca aattaagtcc 1560

gggggtgaca tcattaaact gctgaacgaa caagtgaata aagagatgca gagctctaac 1620

ttgtacatga gcatgagcag ttggtgctac acacactctc tggacggggc tggcctgttc 1680

ctgttcgatc acgcagcaga ggagtatgag cacgccaaaa aactgattat cttcctcaac 1740

gagaacaacg tgcccgtcca gctcacctcc atctcagccc ccgagcacaa gttcgagggc 1800

ctcacccaga tcttccagaa agcatatgag catgaacagc atatcagtga aagcatcaac 1860

aatatcgtgg accacgctat taaatcaaag gatcacgcca ccttcaactt tctgcagtgg 1920

tatgtcgccg agcagcatga ggaggaggtg ctttttaaag acatcctgga caagatcgag 1980

ctgatcggca acgaaaacca tggcctgtac ctggctgacc agtatgtgaa gggaattgcc 2040

aagtccagaa aatcc 2055

<210> 36
<211> 2052
<212> DNA
<213> Artificial Sequence

<220>
<223> MRK_pH1_Con_ferritin

<400> 36
atgaaggcga ttctggttgt gctgctgtac accttcgcca ccgccaacgc cgacacactg 60

tgcattggct accacgccaa taacagcacg gacaccgtgg acacggtgct ggagaaaaac 120

gtgaccgtga cccacagcgt gaacctgctg gaggacaagc acaacggtaa gttgtgcaag 180

ctcaggggag tggcaccact gcacttgggc aagtgtaata tcgctggctg gatattggga 240

aatccagagt gcgaaagcct gagtactgcc tccagctgga gctacattgt ggagaccagc 300

agcagcgaca acggcacctg ctaccccggc gacttcatcg actatgagga attgagggaa 360

cagctgagtt cagtttccag cttcgagcga tttgagatat ttcccaagac gtcctcttgg 420

cccaaccacg acagcaacaa gggcgtgaca gccgcctgcc cccacgccgg ggcgaagagc 480

ttctacaaga acctgatctg gctggtgaag aagggcaaca gctacccaaa gctatccaag 540

tcctatatta acgacaaagg caaggaggtg ttggtgctct ggggcattca ccacccctcc 600

acctccgccg accagcaaag tctttaccag aacgcggacg cctacgtctt tgtcggcacc 660

agcagataca gcaagaagtt taagcccgag attgctatca gacccaaggt gagagaccag 720

gaaggcagaa tgaactatta ttggaccctg gtggaacccg gcgacaaaat aacattcgaa 780

gccaccggga atctggtggt gcccagatat gcctttgcca tggagcgcaa tgccggcagc 840

ggcattatta tctctgacac ccccgtgcac gactgcaaca ccacctgtca gacccctaag 900

ggggctatca acaccagcct gcccttccag aatattcacc ccatcactat cggcaagtgc 960

cccaagtacg tcaagagcac aaaactgaga ctggccacag ggctgaggaa tgtacctagc 1020

atccagtcca gagggctgtt cggggccatc gctggcttca tcgaaggagg ctggaccggc 1080

atggtcgatg gatggtacgg atatcaccac caaaacgagc aggggtcagg atacgccgct 1140

gacctgaaga gcacccagaa cgccatcgac aagatcacca acaaggtgaa tagcgtgatc 1200

gagaagatga acacccagtt caccgcagtg ggcaaggagt tcaaccacct ggagaagaga 1260

atcgagaacc tgaacaagaa agtggatgac gggttcctgg acatctggac ctacaacgcc 1320

gagcttctgg tgctcttgga gaatgagaga accctggatt atcatgacag caatgtcaaa 1380

aacctctacg agaaggtgcg gagccagctg aagaacaacg caaaggagat tggcaacggc 1440

tgcttcgagt tttatcacaa gtgcgacaac acttgtatgg agagcgttaa gaatggcact 1500

tacgattacc ccaagtactc cgaggaagcc aagctgaaca gagaagaaat cgactccggc 1560

ggcgacataa tcaagctcct gaacgaacag gtgaacaagg agatgcaaag ctccaacctc 1620

tacatgagca tgagctcatg gtgctacact cacagcctgg acggagctgg actgttcttg 1680

ttcgaccacg cggccgagga gtacgagcac gccaagaagc tcatcatctt ccttaacgag 1740

aataacgtgc cagtgcagct cacctccatc agcgcccccg agcataagtt cgagggtctg 1800

acccaaatct tccagaaggc ttacgagcat gagcagcaca tcagcgagag cattaacaac 1860

atcgtggatc acgctattaa atctaaagac cacgccacct tcaacttcct gcagtggtac 1920

gtggcagaac agcacgagga ggaggtcctg ttcaaggata tactggacaa aatcgagctg 1980

atcggcaacg agaaccacgg cctgtacctg gccgatcagt acgtcaaagg tattgccaag 2040

tctcgcaaga gc 2052

<210> 37
<211> 2049
<212> DNA
<213> Artificial Sequence

<220>
<223> MRK_sH1_Con_ferritin

<400> 37
atgaaggtga agctgcttgt gctgctgtgc accttcaccg ctacctacgc agacacaatc 60

tgtatcggat accacgccaa taactcaacc gatacagtgg acaccgtgct cgagaagaac 120

gtgacagtga cgcacagcgt gaacctgctt gaggattccc ataacggtaa gctctgtctg 180

ctgaagggca tcgcccctct tcagctggga aactgctccg ttgccggctg gatcctgggc 240

aaccccgagt gtgagcttct gatcagcaag gagtcgtggt catatatcgt ggagacccct 300

aatccagaga acggaacctg ttaccccggc tactttgccg actacgagga gctcagagag 360

cagctgagca gcgtgagcag cttcgagaga ttcgagatct tccccaagga gagcagttgg 420

cctaatcaca ccgtgaccgg cgtgagcgcc tcctgcagcc acaacggcaa gtcttccttt 480

tacagaaacc tgctgtggct gacaggcaaa aacgggttgt accctaacct gagcaagtcc 540

tatgctaaca ataaggagaa ggaagtcctg gtgttgtggg gcgttcacca tcccccaaac 600

atcggagacc aacgcgccct atatcacact gagaacgcct acgtgagcgt ggtgtcaagc 660

cactatagca gacggttcac ccccgaaatc gcaaagagac cgaaggtgcg ggaccaggag 720

ggaaggatta actattactg gacactcctg gagcccgggg acactatcat ctttgaagcc 780

aacgggaacc tcatcgcacc caggtacgct ttcgctctgt ccaggggatt cgggagcggt 840

atcattacct cgaacgcccc gatggatgag tgcgacgcca aatgccaaac cccccagggc 900

gctattaact ctagcctccc ttttcagaac gtgcaccccg tgaccatcgg agagtgcccc 960

aagtacgtgc ggagcgctaa gctcaggatg gtgaccggcc tgcggaacat cccctctatc 1020

caatccaggg gtctgttcgg cgccattgcc ggatttatcg agggcgggtg gaccgggatg 1080

gtggatggat ggtatggata ccaccatcag aatgaacaag gcagcggata cgccgccgat 1140

cagaagtcaa cacaaaacgc catcaacgga attaccaaca aagtcaactc cgtgatcgag 1200

aagatgaaca cccagttcac ggccgtgggc aaagagttca acaagctcga gcggcgaatg 1260

gagaacctca acaagaaggt ggacgatgga ttcctggaca tctggacgta caatgccgaa 1320

ctgctcgtgc tgctggaaaa cgagagaaca ctcgatttcc acgacagcaa cgtgaagaat 1380

ctgtatgaga aggtcaaatc ccagttgaag aacaacgcca aggagatcgg caatggctgt 1440

ttcgagttct atcacaagtg taatgacgag tgcatggaga gcgttaagaa cggcacctac 1500

gactacccca aatacagcga agagagcaag ctgaaccgtg agaagatcga cagcggaggc 1560

gatatcatca agctgctgaa cgaacaggtg aacaaggaga tgcagtccag caatctctac 1620

atgagtatgt cctcgtggtg ctacacccac agcctggatg gagccggact gtttctgttc 1680

gaccacgccg ccgaggagta cgagcatgcc aaaaagctga tcatcttcct caatgaaaac 1740

aacgtgcccg tgcagttgac cagcatcagc gcccccgagc ataaattcga gggactgaca 1800

cagatctttc agaaggccta tgagcacgag cagcacatca gcgaaagcat caacaacatc 1860

gtggaccacg ccatcaagtc caaggatcac gccaccttca acttcctgca gtggtacgtt 1920

gccgaacagc acgaggagga ggtgctgttt aaggacatcc tggacaaaat cgaactgatc 1980

ggaaacgaga accatggtct gtacctcgcc gaccagtacg tgaagggaat cgccaagagc 2040

aggaagtcg 2049

<210> 38
<211> 2052
<212> DNA
<213> Artificial Sequence

<220>
<223> Cobra_P1_ferritin

<400> 38
   atgaaggcca gactgttggt gctgctgtgt gcccttgccg ccacagacgc cgacaccatc 60
   tgtatcggct accacgctaa taacagcacc gacaccgtgg acacagtgct tgaaaagaac 120
   gtgacagtga cccacagcgt taacctgctt gaggactctc acaacgggaa gctgtgtaaa 180
   ctgaagggga tcgcccctct gcagctgggc aagtgcaaca tcgctggctg gctgctggga 240
   aatcccgagt gtgagagcct gctgtccgct cgtagctgga gctacatagt tgaaacccct 300
   aacagcgaga atggcacctg ctaccctgga gacttcatcg actacgagga gctcagagag 360
   cagctgagca gcgtgagctc gtttgaaaga tttgagatct ttcccaagga gtcctcatgg 420
   cccaaccaca acactaccaa aggcgtgacc gctgcttgtt cacacgctgg caaatcctcc 480
   ttctaccgga acctgctgtg gctgaccaag aaaggcggat cctaccccaa actgagcaag 540
   tcatacgtga ataacaaggg caaagaggtg ctggtgctgt ggggcgtgca ccacccctcc 600
   accagcaccg atcagcaaag cctgtaccag aacgagaacg cctacgtcag cgttgtgagc 660
   agcaactaca accggagatt cacccccgag attgccgaga gacctaaggt gagagggcag 720
   gctggcagaa tgaactacta ttggactctg ctggagcccg gagacacaat tatcttcgag 780
   gccactggca atctgatcgc accctggtac gccttcgcct taagcagggg cagcgggtct 840
   ggaattatca cttccaatgc cagcatgcac gagtgcaaca ccaagtgcca gaccccccag 900
   ggcgccatta acagcagcct gcccttccag aacatccacc ccgtcactat cggcgagtgc 960
   cccaagtatg tgaggagcac taagctgagg atggtgaccg ggcttagaaa catccccagc 1020
   atccagtcaa gaggcctatt cggcgcaata gccggcttca ttgaaggcgg ctggaccggg 1080
   atgatcgatg gctggtatgg ctatcaccat cagaacgagc aaggctccgg gtacgccgcc 1140
   gaccagaaat ccacacagaa tgccatcaat ggaattacta acaaggttaa ttccgtcatc 1200
   gagaagatga atacccagtt taccgccgtg ggaaaggagt tcaacaatct ggagaagcgg 1260
   atggagaacc tcaacaagaa ggtagatgat ggattcctcg acatctggac atacaatgct 1320
   gaactgctgg tgctgctcga gaacgagaga accttagact tccacgacag caacgtgaag 1380
   aatctgtacg agaaggttaa gtctcaactg agaaataacg ctaaggagat tggcaatggc 1440
   tgtttcgagt tctaccacaa gtgtgacaac gaatgtatgg aatctgtgaa gaacgggacc 1500
   tacgactacc ccaagtacag cgaggagagc aagctgaaca gagagaagat cgactcaggc 1560
   ggcgacatca ttaagctgct gaatgagcag gttaataagg agatgcagag ctccaatctg 1620
   tatatgagta tgagcagctg gtgttacact cactccctgg acggcgccgg actgttcctg 1680
   ttcgaccacg ctgcagagga gtacgaacac gcaaaaaagc tgataatctt tctgaatgaa 1740
   aacaacgtgc ccgtccagct gacctctatt tctgccccag agcacaagtt cgagggcctg 1800
   acacagatct tccaaaaggc ctacgaacac gagcagcaca tcagcgagtc aatcaacaac 1860
   atagtcgatc acgccattaa gtctaaggac cacgccacct tcaacttcct ccagtggtat 1920
   gtggccgagc agcacgagga ggaggttctt tttaaggata ttctcgataa aatcgagttg 1980
   atcggcaacg agaatcatgg cctgtacctg gcagaccaat atgtgaaggg gatcgccaag 2040
   tcaaggaaga gc 2052

<210> 39
<211> 2052

<212>  DNA
<213>  Artificial Sequence

<220>
<223>  Cobra_X3_ferritin

<400>  39
atggaggcca gactgctggt gctgctgtgc gccttcgccg ccaccaacgc agacaccatc      60

tgcattggct accacgccaa caacagcacc gataccgtgg acacagtgct cgaaaagaac     120

gtgacagtga ctcacagcgt gaacctcctg gaggacagcc acaacggcaa gctgtgccgg     180

ctgaagggta tcgccccctt gcagctggga aactgcagcg tggcagggtg gatcttgggc     240

aatcccgagt gcgaaagtct gttttctaag gagtcctggt cctacatcgc cgagacaccg     300

aaccccgaaa acggaacatg ctatcctggc tacttcgctg actacgaaga gctgcgggag     360

cagcttagct ccgtctccag ctttgagcgg tttgagatct tcccgaaaga gtctagctgg     420

cccaatcaca cagtcaccaa gggggtgacc gcatcctgca gccacaacgg caagtcctct     480

ttctacagaa acctgctgtg gctgaccgag aaaaacgggc tgtaccctaa cctttccaag     540

agctatgtca acaacaagga gaaggaggtg ctggtgctgt ggggggttca ccaccccagc     600

aacatcggag accagagagc tatctatcac accgaaaacg cctacgtgag cgtggtgagc     660

agccattata gcagacgctt cacccctgag attgccaaac ggcccaaagt gcgggaccag     720

gagggcagaa tcaactatta ctggaccctc ctggaacctg gcgataccat tatctttgag     780

gccaacggca acctgatcgc cccatggtac gcctttgctc tgagccgggg ctttggctca     840

ggcatcatta ccagcaacgc cagcatggac gagtgcgatg ccaagtgcca gacaccccag     900

ggcgccatca acagctccct gccctttcaa aatgtccatc ccgtgaccat cggcgagtgt     960

cccaagtacg tccggtccac taaactgcgg atggtgaccg gactcagaaa tatcccaagc    1020

atccagagca gaggcctgtt tggcgccatc gctggattta tcgagggagg ctggactggc    1080

atgatcgatg gctggtacgg ctatcatcat cagaacgagc agggcagcgg atatgccgca    1140

gaccagaagt cgacccagaa cgccatcaat ggaattacca acaaggtgaa cagcgtgatc    1200

gagaagatga acacccagtt cactgccgtc ggcaaggaat tcaacaagct ggaacgtcgg    1260

atggaaaacc tcaacaaaaa ggtggatgac ggcttcctgg atatctggac ctacaacgcc    1320

gagctcctgg tgctccttga gaacgagaga accctcgatt tccacgatag caacgtgaaa    1380

aatctctacg aaaaggtgaa gagccagctg aaaaataacg ccaaggagat agggaatggc    1440

tgcttcgagt tctaccataa gtgcaacaac gagtgcatgg agagcgtcaa aaacggcact    1500

tacgattacc ccaagtattc agaagagagc aaactgaaca gggaaaaaat tgactccggc    1560

ggagacatta tcaagctgct gaatgaacag gtgaacaaag agatgcagag ctccaacctt    1620

tacatgagca tgagcagctg gtgctatacc cattccctcg acggggccgg gctgttcctg    1680

ttcgaccatg ccgctgaaga atacgagcac gccaagaaac tgatcatctt cttaaacgag 1740

aacaatgtgc cagtgcagct gacctcaatc agcgcccccg agcacaagtt cgagggactc 1800

actcagattt tccagaaggc ctacgagcac gagcaacaca ttagcgaatc catcaacaat 1860

atcgtggacc acgccataaa gagcaaggac catgccacct ttaacttcct tcaatggtac 1920

gtggccgagc agcacgagga ggaggtcctg ttcaaggaca tcctcgacaa aatcgagctg 1980

atcggcaatg aaaaccatgg cctctacctg gctgaccagt atgtgaaagg tatcgctaag 2040

tcaagaaaaa gc 2052

<210> 40
<211> 1494
<212> DNA
<213> Artificial Sequence

<220>
<223> Wildtype Sequence of Nucleoprotein

<400> 40
atggccagcc agggcaccaa gagaagctac gagcagatgg agaccgacgg cgagagacag 60

aacgccaccg agatcagagc cagcgtgggc aagatgatcg acggcatcgg cagattctac 120

atccagatgt gcaccgagct caagctgagc gactacgagg gcagactgat ccagaacagc 180

ctgaccatcg aaagaatggt tctgagcgcc ttcgacgaga gaagaaacag atacctggag 240

gagcacccca gcgccggcaa ggaccccaag aagaccggcg gccccatcta caagagagtg 300

gacggcagat ggatgagaga gctggtgctg tacgacaagg aggagatcag aagaatctgg 360

agacaggcca acaacggcga cgacgccacc gccggcctga cccacatgat gatctggcac 420

agcaacctga acgacaccac ctaccagaga accagagccc tggtgagaac cggcatggac 480

cccagaatgt gcagcttaat gcagggcagc accctgccca gaagatccgg cgccgctggt 540

gccgccgtca agggcatcgg caccatggtg atggagctga tccgcatgat caagcgcggc 600

atcaacgaca gaaacttctg gagaggcgaa aacggcagaa agaccagaag cgcctacgag 660

agaatgtgca acatcctgaa gggcaagttc cagaccgccg cccaaagagc catgatggac 720

caggtgagag agagcagaaa ccccggcaac gccgagatcg aagacctgat cttcagcgcc 780

agatcggccc tgatcctgag aggcagcgtg gcccacaaga gctgcctgcc cgcctgcgtg 840

tatggccccg ccgtgagcag cggctacaac ttcgagaagg agggctacag cctggtgggc 900

atcgacccct tcaagctgct gcagaactct caggtgtata gcctgatcag acccaacgag 960

aaccccgccc acaagagcca gctggtgtgg atggcctgcc acagcgccgc cttcgaggac 1020

ctgagactgc tgagcttcat cagaggtacc aaggtgtccc ccagaggcaa gctgagcacc 1080

agaggtgtgc agatcgccag caatgagaac atggacaata tggagagcag caccctggag 1140

ctaagaagca ggtactgggc catccggacc agaagcggcg gcaataccaa ccagcagaga 1200

gccagcgccg gccagatcag cgtgcagccc accttcagcg tgcagagaaa cctgcccttt     1260

gagaagagca ccgtgatggc cgccttcacc ggcaacaccg agggcagaac cagcgacatg     1320

agagccgaga tcatcagaat gatggagggc gccaagcccg aggaggtgag ctttagaggc     1380

agaggcgtgt tcgagctgag cgacgagaag gccaccaacc caattgtgcc cagcttcgac     1440

atgtcgaacg agggcagcta cttcttcggc gacaacgccg aggagtacga caac           1494

<210>  41
<211>  566
<212>  PRT
<213>  Artificial Sequence

<220>
<223>  MRK_H3_consUnique

<400>  41

Met Lys Thr Ile Ile Ala Leu Ser Tyr Ile Leu Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Ile Val Lys Thr Ile Thr Asn Asp
        35                  40                  45

Gln Ile Glu Val Thr Asn Ala Thr Glu Leu Val Gln Ser Ser Ser Thr
    50                  55                  60

Gly Glu Ile Cys Asp Ser Pro His Gln Ile Leu Asp Gly Glu Asn Cys
65                  70                  75                  80

Thr Leu Ile Asp Ala Leu Leu Gly Asp Pro Gln Cys Asp Gly Phe Gln
                85                  90                  95

Asn Lys Lys Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Ser Phe Asn Trp Thr
    130                 135                 140

Gly Val Thr Gln Asn Gly Thr Ser Ser Ala Cys Ile Arg Arg Ser Asn
145                 150                 155                 160

Ser Ser Phe Phe Ser Arg Leu Asn Trp Leu Thr His Leu Asn Phe Lys

                165                 170                 175

Tyr Pro Ala Leu Asn Val Thr Met Pro Asn Asn Glu Gln Phe Asp Lys
            180                 185                 190

Leu Tyr Ile Trp Gly Val His His Pro Gly Thr Asp Lys Asp Gln Ile
        195                 200                 205

Phe Leu Tyr Ala Gln Ala Ser Gly Arg Ile Thr Val Ser Thr Lys Arg
    210                 215                 220

Ser Gln Gln Ala Val Ile Pro Asn Ile Gly Ser Arg Pro Arg Val Arg
225                 230                 235                 240

Asn Ile Pro Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Lys Cys Asn Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Asn Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Arg Gly Gln Ala Ala Asp Leu Lys
    370                 375                 380

Ser Thr Gln Ala Ala Ile Asp Gln Ile Asn Gly Lys Leu Asn Arg Leu
385                 390                 395                 400

Ile Gly Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Lys Thr Lys Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Ala Leu Leu Gly Phe Ile Met Trp Ala Cys Gln Lys Gly Asn Ile
545                 550                 555                 560

Arg Cys Asn Ile Cys Ile
                565

<210>  42
<211>  566
<212>  PRT
<213>  Artificial Sequence

<220>
<223>  MRK_H3_ConsensusA

<400>  42

Met Lys Thr Ile Ile Ala Leu Ser Tyr Ile Leu Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Leu Val Lys Thr Ile Thr Asn Asp
        35                  40                  45

Gln Ile Glu Val Thr Asn Ala Thr Glu Leu Val Gln Ser Ser Ser Thr

50                  55                  60

Gly Arg Ile Cys Asp Ser Pro His Arg Ile Leu Asp Gly Glu Asn Cys
65                  70                  75                  80

Thr Leu Ile Asp Ala Leu Leu Gly Asp Pro His Cys Asp Gly Phe Gln
                85                  90                  95

Asn Lys Glu Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Ser Phe Asn Trp Thr
    130                 135                 140

Gly Val Ala Gln Asn Gly Thr Ser Tyr Ala Cys Lys Arg Gly Ser Val
145                 150                 155                 160

Lys Ser Phe Phe Ser Arg Leu Asn Trp Leu His Gln Leu Lys Tyr Lys
                165                 170                 175

Tyr Pro Ala Leu Asn Val Thr Met Pro Asn Asn Asp Lys Phe Asp Lys
            180                 185                 190

Leu Tyr Ile Trp Gly Val His His Pro Ser Thr Asp Ser Asp Gln Thr
        195                 200                 205

Ser Leu Tyr Val Gln Ala Ser Gly Arg Val Thr Val Ser Thr Lys Arg
    210                 215                 220

Ser Gln Gln Thr Val Ile Pro Asn Ile Gly Ser Arg Pro Trp Val Arg
225                 230                 235                 240

Gly Val Ser Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Lys Cys Asn Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Asn Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Thr Gly Gln Ala Ala Asp Leu Lys
    370                 375                 380

Ser Thr Gln Ala Ala Ile Asn Gln Ile Asn Gly Lys Leu Asn Arg Leu
385                 390                 395                 400

Ile Glu Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Arg Thr Arg Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Val Leu Leu Gly Phe Ile Met Trp Ala Cys Gln Lys Gly Asn Ile
545                 550                 555                 560

Arg Cys Asn Ile Cys Ile
                565

<210>  43
<211>  566
<212>  PRT
<213>  Artificial Sequence

<220>
<223>  MRK_H3_ConsensusB

<400>  43

Met Lys Thr Ile Ile Ala Leu Ser Tyr Ile Leu Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Ile Val Lys Thr Ile Thr Asn Asp
        35                  40                  45

Gln Ile Glu Val Thr Asn Ala Thr Glu Leu Val Gln Asn Ser Ser Thr
    50                  55                  60

Gly Glu Ile Cys Asp Ser Pro His Gln Ile Leu Asp Gly Glu Asn Cys
65                  70                  75                  80

Thr Leu Ile Asp Ala Leu Leu Gly Asp Pro Gln Cys Asp Gly Phe Gln
                85                  90                  95

Asn Lys Lys Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Ser Phe Asn Trp Thr
    130                 135                 140

Gly Val Thr Gln Asn Gly Thr Ser Ser Ala Cys Ile Arg Arg Ser Asn
145                 150                 155                 160

Ser Ser Phe Phe Ser Arg Leu Asn Trp Leu Thr His Leu Asn Phe Lys
                165                 170                 175

Tyr Pro Ala Leu Asn Val Thr Met Pro Asn Asn Glu Gln Phe Asp Lys
            180                 185                 190

Leu Tyr Ile Trp Gly Val His His Pro Gly Thr Asp Lys Asp Gln Ile
        195                 200                 205

Phe Leu Tyr Ala Gln Ser Ser Gly Arg Ile Thr Val Ser Thr Lys Arg
    210                 215                 220

Ser Gln Gln Ala Val Ile Pro Asn Ile Gly Ser Arg Pro Arg Ile Arg
225                 230                 235                 240

Asn Ile Pro Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Lys Cys Asn Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Ser Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Arg Gly Gln Ala Ala Asp Leu Lys
    370                 375                 380

Ser Thr Gln Ala Ala Ile Asp Gln Ile Asn Gly Lys Leu Asn Arg Leu
385                 390                 395                 400

Ile Gly Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Lys Thr Lys Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Ala Leu Leu Gly Phe Ile Met Trp Ala Cys Gln Lys Gly Asn Ile
545                 550                 555                 560

Arg Cys Asn Ile Cys Ile
                565

<210>  44
<211>  566
<212>  PRT
<213>  Artificial Sequence

<220>
<223>  MRK_H1_cot_all

<400>  44

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Ser Ser Ser Asp Asn Gly Thr Cys Tyr Pro Gly Asp Phe
            100                 105                 110

Ile Asn Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Thr Ala Asp Gln Gln Ser Leu
        195                 200                 205

Tyr Gln Asn Ala Asp Ala Tyr Val Phe Val Gly Thr Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Val Pro Ser Ile Gln Ser Arg Gly Leu Phe Gly Ala Ile Ala Gly
        340                 345                 350

Phe Ile Glu Gly Gly Trp Thr Gly Met Val Asp Gly Trp Tyr Gly Tyr
            355                 360                 365

His His Gln Asn Glu Gln Gly Ser Gly Tyr Ala Ala Asp Leu Lys Ser
    370                 375                 380

Thr Gln Asn Ala Ile Asp Lys Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
        420                 425                 430

Leu Asp Ile Trp Thr Tyr Asn Ala Glu Leu Leu Val Leu Leu Glu Asn
            435                 440                 445

Glu Arg Thr Leu Asp Tyr His Asp Ser Asn Val Lys Asn Leu Tyr Glu
    450                 455                 460

Lys Val Arg Asn Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
        500                 505                 510

Asn Arg Glu Lys Ile Asp Gly Val Lys Leu Glu Ser Thr Arg Ile Tyr
            515                 520                 525

Gln Ile Leu Ala Ile Tyr Ser Thr Val Ala Ser Ser Leu Val Leu Val
    530                 535                 540

Val Ser Leu Gly Ala Ile Ser Phe Trp Met Cys Ser Asn Gly Ser Leu
545                 550                 555                 560

Gln Cys Arg Ile Cys Ile
                565

<210>  45
<211>  566

<212>  PRT
<213>  Artificial Sequence

<220>
<223>  MRK_H3_cot_all

<400>  45

Met Lys Thr Ile Ile Ala Leu Ser Tyr Ile Leu Cys Leu Val Phe Ala
1               5                   10                  15

Gln Lys Leu Pro Gly Asn Asp Asn Ser Thr Ala Thr Leu Cys Leu Gly
            20                  25                  30

His His Ala Val Pro Asn Gly Thr Ile Val Lys Thr Ile Thr Asn Asp
        35                  40                  45

Arg Ile Glu Val Thr Asn Ala Thr Glu Leu Val Gln Asn Ser Ser Ile
    50                  55                  60

Gly Glu Ile Cys Asp Ser Pro His Gln Ile Leu Asp Gly Glu Asn Cys
65                  70                  75                  80

Thr Leu Ile Asp Ala Leu Leu Gly Asp Pro Gln Cys Asp Gly Phe Gln
                85                  90                  95

Asn Lys Lys Trp Asp Leu Phe Val Glu Arg Ser Lys Ala Tyr Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Asn Asn Glu Ser Phe Asn Trp Thr
    130                 135                 140

Gly Val Thr Gln Asn Gly Thr Ser Ser Ala Cys Ile Arg Arg Ser Asn
145                 150                 155                 160

Ser Ser Phe Phe Ser Arg Leu Asn Trp Leu Thr His Leu Asn Phe Lys
                165                 170                 175

Tyr Pro Ala Leu Asn Val Thr Met Pro Asn Asn Glu Gln Phe Asp Lys
            180                 185                 190

Leu Tyr Ile Trp Gly Val His His Pro Gly Thr Asp Lys Asp Gln Ile
        195                 200                 205

Phe Leu Tyr Ala Gln Ser Ser Gly Arg Ile Thr Val Ser Thr Lys Arg
    210                 215                 220

Ser Gln Gln Ala Val Ile Pro Asn Ile Gly Ser Arg Pro Arg Ile Arg
225                 230                 235                 240

Asn Ile Pro Ser Arg Ile Ser Ile Tyr Trp Thr Ile Val Lys Pro Gly
                245                 250                 255

Asp Ile Leu Leu Ile Asn Ser Thr Gly Asn Leu Ile Ala Pro Arg Gly
            260                 265                 270

Tyr Phe Lys Ile Arg Ser Gly Lys Ser Ser Ile Met Arg Ser Asp Ala
        275                 280                 285

Pro Ile Gly Lys Cys Lys Ser Glu Cys Ile Thr Pro Asn Gly Ser Ile
    290                 295                 300

Pro Asn Asp Lys Pro Phe Gln Asn Val Asn Arg Ile Thr Tyr Gly Ala
305                 310                 315                 320

Cys Pro Arg Tyr Val Lys Gln Ser Thr Leu Lys Leu Ala Thr Gly Met
                325                 330                 335

Arg Asn Val Pro Glu Lys Gln Thr Arg Gly Ile Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Arg Gly Gln Ala Ala Asp Leu Lys
    370                 375                 380

Ser Thr Gln Ala Ala Ile Asp Gln Ile Asn Gly Lys Leu Asn Arg Leu
385                 390                 395                 400

Ile Gly Lys Thr Asn Glu Lys Phe His Gln Ile Glu Lys Glu Phe Ser
                405                 410                 415

Glu Val Glu Gly Arg Ile Gln Asp Leu Glu Lys Tyr Val Glu Asp Thr
            420                 425                 430

Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460

Glu Lys Thr Lys Lys Gln Leu Arg Glu Asn Ala Glu Asp Met Gly Asn
465                 470                 475                 480

Gly Cys Phe Lys Ile Tyr His Lys Cys Asp Asn Ala Cys Ile Gly Ser
                485                 490                 495

Ile Arg Asn Gly Thr Tyr Asp His Asp Val Tyr Arg Asp Glu Ala Leu
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Ala Leu Leu Gly Phe Ile Met Trp Ala Cys Gln Lys Gly Asn Ile
545                 550                 555                 560

Arg Cys Asn Ile Cys Ile
                565

<210>  46
<211>  565
<212>  PRT
<213>  Artificial Sequence

<220>
<223>  MRK_sH1_Con_v2

<400>  46

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asn Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Lys Pro Asn Pro Glu Asn Gly Thr Cys Tyr Pro Gly His Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Glu Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Lys Ala Leu Tyr
        195                 200                 205

His Thr Glu Asn Ala Tyr Val Ser Val Val Ser Ser His Tyr Ser Arg
    210                 215                 220

Lys Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala
            260                 265                 270

Leu Ser Arg Gly Phe Gly Ser Gly Ile Ile Asn Ser Asn Ala Pro Met
        275                 280                 285

Asp Lys Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Val Asp Gly Trp Tyr Gly Tyr His
        355                 360                 365

His Gln Asn Glu Gln Gly Ser Gly Tyr Ala Ala Asp Gln Lys Ser Thr
    370                 375                 380

Gln Asn Ala Ile Asn Gly Ile Thr Asn Lys Val Asn Ser Val Ile Glu
385                 390                 395                 400

Lys Met Asn Thr Gln Phe Thr Ala Val Gly Lys Glu Phe Asn Lys Leu
                405                 410                 415

Glu Arg Arg Met Glu Asn Leu Asn Lys Lys Val Asp Asp Gly Phe Ile
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asn Asp Glu Cys Met Glu Ser Val Lys
                485                 490                 495

Asn Gly Thr Tyr Asp Tyr Pro Lys Tyr Ser Glu Glu Ser Lys Leu Asn
            500                 505                 510

Arg Glu Lys Ile Asp Gly Val Lys Leu Glu Ser Met Gly Val Tyr Gln
        515                 520                 525

Ile Leu Ala Ile Tyr Ser Thr Val Ala Ser Ser Leu Val Leu Leu Val
    530                 535                 540

Ser Leu Gly Ala Ile Ser Phe Trp Met Cys Ser Asn Gly Ser Leu Gln
545                 550                 555                 560

Cys Arg Ile Cys Ile
                565

<210>  47
<211>  558
<212>  PRT
<213>  Artificial Sequence

<220>
<223>  MRK_sH1_Con_ecto

<400>  47

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Thr Pro Asn Pro Glu Asn Gly Thr Cys Tyr Pro Gly Tyr Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Lys Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Arg Ala Leu Tyr
        195                 200                 205

His Thr Glu Asn Ala Tyr Val Ser Val Val Ser Ser His Tyr Ser Arg
    210                 215                 220

Arg Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala
            260                 265                 270

Leu Ser Arg Gly Phe Gly Ser Gly Ile Ile Thr Ser Asn Ala Pro Met
        275                 280                 285

Asp Glu Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Val Asp Gly Trp Tyr Gly Tyr His
        355                 360                 365

His Gln Asn Glu Gln Gly Ser Gly Tyr Ala Ala Asp Gln Lys Ser Thr
    370                 375                 380

Gln Asn Ala Ile Asn Gly Ile Thr Asn Lys Val Asn Ser Val Ile Glu
385                 390                 395                 400

Lys Met Asn Thr Gln Phe Thr Ala Val Gly Lys Glu Phe Asn Lys Leu
                405                 410                 415

Glu Arg Arg Met Glu Asn Leu Asn Lys Lys Val Asp Asp Gly Phe Leu
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asn Asp Glu Cys Met Glu Ser Val Lys
                485                 490                 495

Asn Gly Thr Tyr Asp Tyr Pro Lys Tyr Ser Glu Glu Ser Lys Leu Asn

500                 505                 510

Arg Glu Lys Ile Asp Gly Val Lys Leu Glu Ser Met Gly Val Gly Ser
        515                 520                 525

Ala Gly Ser Ala Gly Tyr Ile Pro Glu Ala Pro Arg Asp Gly Gln Ala
    530                 535                 540

Tyr Val Arg Lys Asp Gly Glu Trp Val Leu Ser Thr Phe Leu
545                 550                 555

<210>  48
<211>  231
<212>  PRT
<213>  Artificial Sequence

<220>
<223>  MRK_sH1_Con_RBD

<400>  48

Met Lys Val Lys Leu Leu Val Leu Leu Cys Thr Phe Thr Ala Thr Tyr
1               5                   10                  15

Ala Gly Ile Ala Pro Leu Gln Leu Gly Asn Cys Ser Val Ala Gly Trp
            20                  25                  30

Ile Leu Gly Asn Pro Glu Cys Glu Leu Leu Ile Ser Lys Glu Ser Trp
        35                  40                  45

Ser Tyr Ile Val Glu Thr Pro Asn Pro Glu Asn Gly Thr Cys Tyr Pro
    50                  55                  60

Gly Tyr Phe Ala Asp Tyr Glu Glu Leu Arg Glu Gln Leu Ser Ser Val
65                  70                  75                  80

Ser Ser Phe Glu Arg Phe Glu Ile Phe Pro Lys Glu Ser Ser Trp Pro
                85                  90                  95

Asn His Thr Val Thr Gly Val Ser Ala Ser Cys Ser His Asn Gly Lys
            100                 105                 110

Ser Ser Phe Tyr Arg Asn Leu Leu Trp Leu Thr Gly Lys Asn Gly Leu
        115                 120                 125

Tyr Pro Asn Leu Ser Lys Ser Tyr Ala Asn Asn Lys Glu Lys Glu Val
    130                 135                 140

Leu Val Leu Trp Gly Val His His Pro Pro Asn Ile Gly Asp Gln Arg
145                 150                 155                 160

Ala Leu Tyr His Thr Glu Asn Ala Tyr Val Ser Val Val Ser Ser His
                165                 170                 175

Tyr Ser Arg Arg Phe Thr Pro Glu Ile Ala Lys Arg Pro Lys Val Arg
            180                 185                 190

Asp Gln Glu Gly Arg Ile Asn Tyr Tyr Trp Thr Leu Leu Glu Pro Gly
        195                 200                 205

Asp Thr Ile Ile Phe Glu Ala Asn Gly Asn Leu Ile Ala Pro Arg Tyr
    210                 215                 220

Ala Phe Ala Leu Ser Arg Gly
225                 230

<210>  49
<211>  560
<212>  PRT
<213>  Artificial Sequence

<220>
<223>  MRK_pH1_Con_ecto

<400>  49

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Ser Ser Ser Asp Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Ala Tyr Val Phe Val Gly Thr Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Val Pro Ser Ile Gln Ser Arg Gly Leu Phe Gly Ala Ile Ala Gly
            340                 345                 350

Phe Ile Glu Gly Gly Trp Thr Gly Met Val Asp Gly Trp Tyr Gly Tyr
        355                 360                 365

His His Gln Asn Glu Gln Gly Ser Gly Tyr Ala Ala Asp Leu Lys Ser
    370                 375                 380

Thr Gln Asn Ala Ile Asp Lys Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Tyr His Asp Ser Asn Val Lys Asn Leu Tyr Glu
    450                 455                 460

Lys Val Arg Ser Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
            500                 505                 510

Asn Arg Glu Glu Ile Asp Gly Val Lys Leu Glu Ser Thr Arg Ile Gly
        515                 520                 525

Ser Ala Gly Ser Ala Gly Tyr Ile Pro Glu Ala Pro Arg Asp Gly Gln
    530                 535                 540

Ala Tyr Val Arg Lys Asp Gly Glu Trp Val Leu Leu Ser Thr Phe Leu
545                 550                 555                 560

<210>  50
<211>  233
<212>  PRT
<213>  Artificial Sequence

<220>
<223>  MRK_pH1_Con_RBD

<400>  50

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Leu Ser Thr Ala Ser Ser Trp
        35                  40                  45

Ser Tyr Ile Val Glu Thr Ser Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Asp Ser Asn Lys Gly Val Thr Ala Ala Cys Pro His Ala Gly
            100                 105                 110

Ala Lys Ser Phe Tyr Lys Asn Leu Ile Trp Leu Val Lys Lys Gly Asn
        115                 120                 125

Ser Tyr Pro Lys Leu Ser Lys Ser Tyr Ile Asn Asp Lys Gly Lys Glu
    130                 135                 140

Val Leu Val Leu Trp Gly Ile His His Pro Ser Thr Ser Ala Asp Gln
145                 150                 155                 160

Gln Ser Leu Tyr Gln Asn Ala Asp Ala Tyr Val Phe Val Gly Thr Ser
                165                 170                 175

Arg Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  51
<211>  562
<212>  PRT
<213>  Artificial Sequence

<220>
<223>  eH1HA_d5v1

<400>  51

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Leu Glu Asp Ser His Asn Gly Lys Leu Cys Arg Leu
    50                  55                  60

Lys Gly Ile Ala Pro Leu Gln Leu Gly Lys Cys Asn Ile Ala Gly Trp
65                  70                  75                  80

Leu Leu Gly Asn Pro Glu Cys Asp Pro Leu Pro Pro Met Lys Ser Trp
                85                  90                  95

Ser Tyr Ile Val Glu Thr Pro Asn Ser Glu Asn Gly Ile Cys Tyr Pro
            100                 105                 110

Gly Asp Phe Ile Asp Tyr Glu Glu Leu Arg Glu Gln Leu Ser Ser Val
        115                 120                 125

Ser Ser Phe Glu Arg Phe Glu Ile Phe Pro Lys Gly Ser Ser Trp Pro
    130                 135                 140

Asn His Asn Thr Asn Gly Val Thr Ala Ala Cys Ser His Glu Gly Lys
145                 150                 155                 160

Asn Ser Phe Tyr Arg Asn Leu Leu Trp Leu Thr Lys Lys Glu Gly Leu
                165                 170                 175

Tyr Pro Asn Leu Glu Asn Ser Tyr Val Asn Lys Lys Glu Lys Glu Val
            180                 185                 190

Leu Val Leu Trp Gly Ile His His Pro Ser Asn Asn Lys Glu Gln Gln
        195                 200                 205

Asn Leu Tyr Gln Asn Glu Asn Ala Tyr Val Ser Val Val Thr Ser Asn
    210                 215                 220

Tyr Asn Arg Arg Phe Thr Pro Glu Ile Ala Glu Arg Pro Lys Val Arg
225                 230                 235                 240

Asp Gln Ala Gly Arg Met Asn Tyr Tyr Trp Thr Leu Leu Lys Pro Gly
                245                 250                 255

Asp Thr Ile Ile Phe Glu Ala Asn Gly Asn Leu Ile Ala Pro Met Tyr
        260                 265                 270

Ala Phe Ala Leu Ser Arg Gly Phe Gly Ser Gly Ile Ile Thr Ser Asn
    275                 280                 285

Ala Ser Met His Glu Cys Asn Thr Lys Cys Gln Thr Pro Leu Gly Ala
    290                 295                 300

Ile Asn Ser Ser Leu Pro Tyr Gln Asn Ile His Pro Val Thr Ile Gly
305                 310                 315                 320

Glu Cys Pro Lys Tyr Val Arg Ser Ala Lys Leu Arg Met Val Thr Gly
                325                 330                 335

Leu Arg Asn Ile Pro Ser Ile Gln Ser Arg Gly Leu Phe Gly Ala Ile
        340                 345                 350

Ala Gly Phe Ile Glu Gly Gly Trp Thr Gly Met Ile Asp Gly Trp Tyr
    355                 360                 365

Gly Tyr His His Gln Asn Glu Gln Gly Ser Gly Tyr Ala Ala Asp Gln
    370                 375                 380

Lys Ser Thr Gln Asn Ala Ile Asn Gly Ile Thr Asn Lys Val Asn Thr
385                 390                 395                 400

Val Ile Glu Lys Met Asn Ile Gln Phe Thr Ala Val Gly Lys Glu Phe
                405                 410                 415

Asn Lys Leu Glu Lys Arg Met Glu Asn Leu Asn Lys Lys Val Asp Asp
        420                 425                 430

Gly Phe Leu Asp Ile Trp Thr Tyr Asn Ala Glu Leu Leu Val Leu Leu
    435                 440                 445

Glu Asn Glu Arg Thr Leu Asp Phe His Asp Ser Asn Val Lys Asn Leu
    450                 455                 460

Tyr Glu Lys Val Lys Ser Gln Leu Lys Asn Asn Ala Lys Glu Ile Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Lys Tyr Ser Glu Glu Ser
        500                 505                 510

Lys Leu Asn Arg Glu Lys Val Asp Gly Val Lys Leu Glu Ser Met Gly
        515                 520                 525

Ile Gly Ser Ala Gly Ser Ala Gly Tyr Ile Pro Glu Ala Pro Arg Asp
    530                 535                 540

Gly Gln Ala Tyr Val Arg Lys Asp Gly Glu Trp Val Leu Leu Ser Thr
545                 550                 555                 560

Phe Leu

<210> 52
<211> 562
<212> PRT
<213> Artificial Sequence

<220>
<223> eH1HA_d5v2

<400> 52

Met Glu Thr Pro Ala Gln Leu Leu Phe Leu Leu Leu Leu Trp Leu Pro
1               5                   10                  15

Asp Arg Arg Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Leu Glu Asp Ser His Asn Gly Lys Leu Cys Arg Leu
    50                  55                  60

Lys Gly Ile Ala Pro Leu Gln Leu Gly Lys Cys Asn Ile Ala Gly Trp
65                  70                  75                  80

Leu Leu Gly Asn Pro Glu Cys Asp Pro Leu Pro Pro Met Lys Ser Trp
                85                  90                  95

Ser Tyr Ile Val Glu Thr Pro Asn Ser Glu Asn Gly Ile Cys Tyr Pro
            100                 105                 110

Gly Asp Phe Ile Asp Tyr Glu Glu Leu Arg Glu Gln Leu Ser Ser Val
        115                 120                 125

Ser Ser Phe Glu Arg Phe Glu Ile Phe Pro Lys Gly Ser Ser Trp Pro
    130                 135                 140

Asn His Thr Thr Asn Gly Val Thr Ala Ala Cys Ser His Glu Gly Lys
145                 150                 155                 160

Asn Ser Phe Tyr Arg Asn Leu Leu Trp Leu Thr Lys Lys Glu Gly Ser
                165                 170                 175

Tyr Pro Asn Leu Lys Asn Ser Tyr Val Asn Lys Lys Glu Lys Glu Val
            180                 185                 190

Leu Val Leu Trp Gly Ile His His Pro Ser Asn Ser Lys Glu Gln Gln
        195                 200                 205

Asn Leu Tyr Gln Asn Glu Asn Ala His Val Ser Val Val Thr Ser Asn
    210                 215                 220

Tyr Asn Arg Arg Phe Thr Pro Glu Ile Ala Glu Arg Pro Lys Val Arg
225                 230                 235                 240

Asp Gln Ala Gly Arg Met Asn Tyr Tyr Trp Thr Leu Leu Lys Pro Gly
                245                 250                 255

Asp Thr Ile Ile Phe Glu Ala Asp Gly Asn Leu Ile Ala Pro Met Tyr
            260                 265                 270

Ala Phe Ala Leu Ser Arg Gly Phe Gly Ser Gly Ile Ile Thr Ser Asn
        275                 280                 285

Ala Ser Met His Glu Cys Asn Thr Lys Cys Gln Thr Pro Leu Gly Ala
    290                 295                 300

Ile Asn Ser Ser Leu Pro Tyr Gln Asn Ile His Pro Val Thr Ile Gly
305                 310                 315                 320

Glu Cys Pro Lys Tyr Val Arg Ser Ala Lys Leu Arg Met Val Thr Gly
                325                 330                 335

Leu Arg Asn Ile Pro Ser Ile Gln Ser Arg Gly Leu Phe Gly Ala Ile
            340                 345                 350

Ala Gly Phe Ile Glu Gly Gly Trp Thr Gly Met Ile Asp Gly Trp Tyr
        355                 360                 365

Gly Tyr His His Gln Asn Glu Gln Gly Ser Gly Tyr Ala Ala Asp Gln
    370                 375                 380

Lys Ser Thr Gln Asn Ala Ile Asn Gly Ile Thr Asn Lys Val Asn Thr
385                 390                 395                 400

Val Ile Glu Lys Met Asn Ile Gln Phe Thr Ala Val Gly Lys Glu Phe
                405                 410                 415

Asn Lys Leu Glu Lys Arg Met Glu Asn Leu Asn Lys Lys Val Asp Asp
            420                 425                 430

Gly Phe Leu Asp Ile Trp Thr Tyr Asn Ala Glu Leu Leu Val Leu Leu
        435                 440                 445

Glu Asn Glu Arg Thr Leu Asp Phe His Asp Ser Asn Val Lys Asn Leu
    450                 455                 460

Tyr Glu Lys Val Lys Ser Gln Leu Lys Asn Asn Ala Lys Glu Ile Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Lys Tyr Ser Glu Glu Ser
            500                 505                 510

Lys Leu Asn Arg Glu Lys Val Asp Gly Val Lys Leu Glu Ser Met Gly
        515                 520                 525

Ile Gly Ser Ala Gly Ser Ala Gly Tyr Ile Pro Glu Ala Pro Arg Asp
    530                 535                 540

Gly Gln Ala Tyr Val Arg Lys Asp Gly Glu Trp Val Leu Leu Ser Thr
545                 550                 555                 560

Phe Leu

<210>  53
<211>  562
<212>  PRT
<213>  Artificial Sequence

<220>
<223>  eH1HA_d5v3

<400>  53

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Leu Glu Asp Ser His Asn Gly Lys Leu Cys Arg Leu
    50                  55                  60

Lys Gly Ile Ala Pro Leu Gln Leu Gly Lys Cys Asn Ile Ala Gly Trp
65                  70                  75                  80

Leu Leu Gly Asn Pro Glu Cys Asp Pro Leu Pro Pro Met Lys Ser Trp
                85                  90                  95

Ser Tyr Ile Val Glu Thr Pro Asn Ser Glu Asn Gly Ile Cys Tyr Pro
            100                 105                 110

Gly Asp Phe Ile Asp Tyr Glu Glu Leu Arg Glu Gln Leu Ser Ser Val
        115                 120                 125

Ser Ser Phe Glu Arg Phe Glu Ile Phe Pro Lys Gly Ser Ser Trp Pro
    130                 135                 140

Asp His Asn Thr Asn Gly Val Thr Ala Ala Cys Ser His Glu Gly Lys
145                 150                 155                 160

Asn Ser Phe Tyr Arg Asn Leu Leu Trp Leu Thr Glu Lys Lys Gly Ser
                165                 170                 175

Tyr Pro Asn Leu Lys Asn Pro Tyr Val Asn Lys Lys Glu Lys Glu Val
            180                 185                 190

Leu Val Leu Trp Gly Ile His His Pro Ser Asn Ser Lys Glu Gln Gln
        195                 200                 205

Asn Leu Tyr Arg Asn Glu Asn Ala Tyr Val Ser Val Val Thr Ser Asn
    210                 215                 220

Tyr Asn Arg Arg Phe Thr Pro Glu Ile Ala Glu Arg Pro Lys Val Arg
225                 230                 235                 240

Asp Gln Ala Gly Arg Met Asn Tyr Tyr Trp Thr Leu Leu Lys Pro Gly
                245                 250                 255

Asp Thr Ile Ile Phe Glu Ala Asn Gly Asn Leu Ile Ala Pro Met Tyr
            260                 265                 270

Ala Phe Ala Leu Ser Arg Gly Phe Gly Ser Gly Ile Ile Thr Ser Asn
        275                 280                 285

Ala Ser Met His Glu Cys Asn Thr Lys Cys Gln Thr Pro Leu Gly Ala
    290                 295                 300

Ile Asn Ser Ser Leu Pro Tyr Gln Asn Ile His Pro Val Thr Ile Gly
305                 310                 315                 320

Glu Cys Pro Lys Tyr Val Arg Ser Ala Lys Leu Arg Met Val Thr Gly
                325                 330                 335

Leu Arg Asn Ile Pro Ser Ile Gln Ser Arg Gly Leu Phe Gly Ala Ile
            340                 345                 350

Ala Gly Phe Ile Glu Gly Gly Trp Thr Gly Met Ile Asp Gly Trp Tyr
        355                 360                 365

Gly Tyr His His Gln Asn Glu Gln Gly Ser Gly Tyr Ala Ala Asp Gln
    370                 375                 380

Lys Ser Thr Gln Asn Ala Ile Asn Gly Ile Thr Asn Lys Val Asn Thr
385                 390                 395                 400

Val Ile Glu Lys Met Asn Ile Gln Phe Thr Ala Val Gly Lys Glu Phe
                405                 410                 415

Asn Lys Leu Glu Lys Arg Met Glu Asn Leu Asn Lys Lys Val Asp Asp
            420                 425                 430

Gly Phe Leu Asp Ile Trp Thr Tyr Asn Ala Glu Leu Leu Val Leu Leu
        435                 440                 445

Glu Asn Glu Arg Thr Leu Asp Phe His Asp Ser Asn Val Lys Asn Leu
    450                 455                 460

Tyr Glu Lys Val Lys Ser Gln Leu Lys Asn Asn Ala Lys Glu Ile Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Lys Tyr Ser Glu Glu Ser
            500                 505                 510

Lys Leu Asn Arg Glu Lys Val Asp Gly Val Lys Leu Glu Ser Met Gly
        515                 520                 525

Ile Gly Ser Ala Gly Ser Ala Gly Tyr Ile Pro Glu Ala Pro Arg Asp

530                 535                 540

Gly Gln Ala Tyr Val Arg Lys Asp Gly Glu Trp Val Leu Leu Ser Thr
545                 550                 555                 560

Phe Leu

<210>  54
<211>  562
<212>  PRT
<213>  Artificial Sequence

<220>
<223>  eH1HA_d5v4

<400>  54

Met Glu Thr Pro Ala Gln Leu Leu Phe Leu Leu Leu Leu Trp Leu Pro
1               5                   10                  15

Asp Thr Thr Gly Asp Thr Ile Cys Ile Gly Tyr His Ala Asn Asn Ser
            20                  25                  30

Thr Asp Thr Val Asp Thr Val Leu Glu Lys Asn Val Thr Val Thr His
        35                  40                  45

Ser Val Asn Leu Leu Glu Asp Ser His Asn Gly Lys Leu Cys Lys Leu
    50                  55                  60

Lys Gly Ile Ala Pro Leu Gln Leu Gly Lys Cys Asn Ile Ala Gly Trp
65                  70                  75                  80

Leu Leu Gly Asn Pro Gly Cys Asp Pro Leu Leu Pro Val Gly Ser Trp
                85                  90                  95

Ser Tyr Ile Val Glu Thr Pro Asn Ser Glu Asn Gly Ile Cys Tyr Pro
            100                 105                 110

Gly Asp Phe Ile Asp Tyr Glu Glu Leu Arg Glu Gln Leu Ser Ser Val
        115                 120                 125

Ser Ser Phe Glu Arg Phe Lys Ile Phe Pro Lys Glu Ser Ser Trp Pro
    130                 135                 140

Asp His Asn Thr Asn Gly Val Thr Ala Ala Cys Ser His Glu Gly Lys
145                 150                 155                 160

Asn Ser Phe Tyr Arg Asn Leu Leu Trp Leu Thr Lys Lys Glu Ser Ser
                165                 170                 175

Tyr Pro Asn Leu Glu Asn Ser Tyr Val Asn Lys Lys Arg Lys Glu Val
        180                 185                 190

Leu Val Leu Trp Gly Ile His His Pro Ser Asn Ser Lys Glu Gln Gln
    195                 200                 205

Asn Leu Tyr Gln Asn Glu Asn Ala Tyr Val Ser Val Val Thr Ser Asn
    210                 215                 220

Tyr Asn Arg Arg Phe Thr Pro Glu Ile Ala Glu Arg Pro Lys Val Lys
225                 230                 235                 240

Gly Gln Ala Gly Arg Met Asn Tyr Tyr Trp Thr Leu Leu Lys Pro Gly
                245                 250                 255

Asp Thr Ile Ile Phe Glu Ala Asn Gly Asn Leu Ile Ala Pro Met Tyr
        260                 265                 270

Ala Phe Ala Leu Ser Arg Gly Phe Gly Ser Gly Ile Ile Thr Ser Asn
    275                 280                 285

Ala Ser Met His Glu Cys Asn Thr Lys Cys Gln Thr Pro Leu Gly Ala
    290                 295                 300

Ile Asn Ser Ser Leu Pro Tyr Gln Asn Ile His Pro Val Thr Ile Gly
305                 310                 315                 320

Glu Cys Pro Lys Tyr Val Arg Ser Ala Lys Leu Arg Met Val Thr Gly
                325                 330                 335

Leu Arg Asn Ile Pro Ser Ile Gln Ser Arg Gly Leu Phe Gly Ala Ile
        340                 345                 350

Ala Gly Phe Ile Glu Gly Gly Trp Thr Gly Met Ile Asp Gly Trp Tyr
    355                 360                 365

Gly Tyr His His Gln Asn Glu Gln Gly Ser Gly Tyr Ala Ala Asp Gln
    370                 375                 380

Lys Ser Thr Gln Asn Ala Ile Asn Gly Ile Thr Asn Lys Val Asn Thr
385                 390                 395                 400

Val Ile Glu Lys Met Asn Ile Gln Phe Thr Ala Val Gly Lys Glu Phe
                405                 410                 415

Asn Lys Leu Glu Lys Arg Met Glu Asn Leu Asn Lys Lys Val Asp Asp

            420                 425                 430

Gly Phe Leu Asp Ile Trp Thr Tyr Asn Ala Glu Leu Leu Val Leu Leu
        435                 440                 445

Glu Asn Glu Arg Thr Leu Asp Phe His Asp Ser Asn Val Lys Asn Leu
    450                 455                 460

Tyr Glu Lys Val Lys Ser Gln Leu Lys Asn Asn Ala Lys Glu Ile Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Lys Tyr Ser Glu Glu Ser
            500                 505                 510

Lys Leu Asn Arg Glu Lys Val Asp Gly Val Lys Leu Glu Ser Met Gly
        515                 520                 525

Ile Gly Ser Ala Gly Ser Ala Gly Tyr Ile Pro Glu Ala Pro Arg Asp
    530                 535                 540

Gly Gln Ala Tyr Val Arg Lys Asp Gly Glu Trp Val Leu Leu Ser Thr
545                 550                 555                 560

Phe Leu

<210> 55
<211> 233
<212> PRT
<213> Artificial Sequence

<220>
<223> MRK_RBS_HA129

<400> 55

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Leu Leu Gly Asn Pro Glu Cys Glu Leu Leu Leu Thr Val Ser Ser Trp
        35                  40                  45

Ser Tyr Ile Val Glu Thr Ser Asn Ser Asp Asn Gly Thr Cys Tyr Pro
    50                  55                  60

Gly Asp Phe Ile Asn Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asp His Glu Thr Asn Arg Gly Val Thr Ala Ala Cys Pro Tyr Ala Gly
            100                 105                 110

Ala Asn Ser Phe Tyr Arg Asn Leu Ile Trp Leu Val Lys Lys Gly Asn
        115                 120                 125

Ser Tyr Pro Lys Leu Ser Lys Ser Tyr Val Asn Asn Lys Gly Lys Glu
    130                 135                 140

Val Leu Val Leu Trp Gly Ile His His Pro Pro Thr Ser Thr Asp Gln
145                 150                 155                 160

Gln Ser Leu Tyr Gln Asn Ala Asp Ala Tyr Val Phe Val Gly Ser Ser
                165                 170                 175

Arg Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  56
<211>  233
<212>  PRT
<213>  Artificial Sequence

<220>
<223>  RBD1-Cal09-PC-Cb

<400>  56

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Leu Ser Thr Ala Ser Ser Trp
        35                  40                  45

Ser Asn Ile Thr Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Ser Ser Asn Lys Gly Val Thr Ala Ala Cys Pro His Ala Gly
            100                 105                 110

Ala Lys Ser Phe Tyr Lys Asn Leu Ile Trp Leu Val Lys Lys Asn Gly
        115                 120                 125

Ser Tyr Pro Lys Leu Asn Lys Ser Tyr Ile Asn Asp Ser Gly Lys Glu
    130                 135                 140

Val Leu Val Leu Trp Gly Ile His His Pro Ser Asn Ser Thr Asp Gln
145                 150                 155                 160

Gln Ser Leu Tyr Gln Asn Ala Asp Thr Tyr Val Phe Val Gly Ser Ser
                165                 170                 175

Asn Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  57
<211>  233
<212>  PRT
<213>  Artificial Sequence

<220>
<223>  RBD1-Cal09-PC

<400>  57

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Asn Ser Thr Ala Ser Ser Trp
        35                  40                  45

Ser Asn Ile Thr Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Ser Ser Asn Lys Gly Val Thr Ala Ala Cys Pro His Ala Gly
            100                 105                 110

Ala Lys Ser Phe Tyr Lys Asn Leu Ile Trp Leu Val Lys Lys Asn Gly
        115                 120                 125

Ser Tyr Pro Lys Leu Asn Lys Ser Tyr Ile Asn Asp Ser Gly Lys Glu
    130                 135                 140

Val Leu Val Leu Trp Gly Ile His His Pro Ser Asn Ser Thr Asp Gln
145                 150                 155                 160

Gln Ser Leu Tyr Gln Asn Ala Asp Thr Tyr Val Phe Val Gly Ser Ser
                165                 170                 175

Asn Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  58
<211>  233
<212>  PRT

<213>  Artificial Sequence

<220>
<223>  RBD1-Cal09

<400>  58

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Leu Ser Thr Ala Ser Ser Trp
        35                  40                  45

Ser Asn Ile Thr Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Asp Ser Asn Lys Gly Val Thr Ala Ala Cys Pro His Ala Gly
            100                 105                 110

Ala Lys Ser Phe Tyr Lys Asn Leu Ile Trp Leu Val Lys Lys Gly Asn
        115                 120                 125

Ser Tyr Pro Lys Leu Ser Lys Ser Tyr Ile Asn Asp Lys Gly Lys Glu
    130                 135                 140

Val Leu Val Leu Trp Gly Ile His His Pro Ser Thr Ser Ala Asp Gln
145                 150                 155                 160

Gln Ser Leu Tyr Gln Asn Ala Asp Thr Tyr Val Phe Val Gly Ser Ser
                165                 170                 175

Arg Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  59
<211>  233
<212>  PRT
<213>  Artificial Sequence

<220>
<223>  MRK_RBD-Cal09-PC-Cb

<400>  59

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Leu Ser Thr Ala Ser Ser Trp
        35                  40                  45

Ser Tyr Ile Val Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Ser Ser Asn Lys Gly Val Thr Ala Ala Cys Pro His Ala Gly
            100                 105                 110

Ala Lys Ser Phe Tyr Lys Asn Leu Ile Trp Leu Val Lys Lys Asn Gly
        115                 120                 125

Ser Tyr Pro Lys Leu Asn Lys Ser Tyr Ile Asn Asp Ser Gly Lys Glu
    130                 135                 140

Val Leu Val Leu Trp Gly Ile His His Pro Ser Asn Ser Thr Asp Gln
145                 150                 155                 160

Gln Ser Leu Tyr Gln Asn Ala Asp Thr Tyr Val Phe Val Gly Ser Ser
                165                 170                 175

Asn Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro

195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  60
<211>  233
<212>  PRT
<213>  Artificial Sequence

<220>
<223>  MRK_RBD-Cal09-PC

<400>  60

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Asn Ser Thr Ala Ser Ser Trp
        35                  40                  45

Ser Tyr Ile Val Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Ser Ser Asn Lys Gly Val Thr Ala Ala Cys Pro His Ala Gly
            100                 105                 110

Ala Lys Ser Phe Tyr Lys Asn Leu Ile Trp Leu Val Lys Lys Asn Gly
        115                 120                 125

Ser Tyr Pro Lys Leu Asn Lys Ser Tyr Ile Asn Asp Ser Gly Lys Glu
    130                 135                 140

Val Leu Val Leu Trp Gly Ile His His Pro Ser Asn Ser Thr Asp Gln
145                 150                 155                 160

Gln Ser Leu Tyr Gln Asn Ala Asp Thr Tyr Val Phe Val Gly Ser Ser
                165                 170                 175

Asn Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  61
<211>  233
<212>  PRT
<213>  Artificial Sequence

<220>
<223>  MRKRBD-Cal09

<400>  61

Met Lys Val Lys Leu Leu Val Leu Leu Cys Thr Phe Thr Ala Thr Tyr
1               5                   10                  15

Ala Gly Val Ala Pro Leu His Leu Gly Lys Cys Asn Ile Ala Gly Trp
            20                  25                  30

Ile Leu Gly Asn Pro Glu Cys Glu Ser Leu Ser Thr Ala Ser Ser Trp
        35                  40                  45

Ser Tyr Ile Val Glu Thr Pro Ser Ser Asp Asn Gly Thr Cys Tyr Pro
    50                  55                  60

Gly Asp Phe Ile Asp Tyr Glu Glu Leu Arg Glu Gln Leu Ser Ser Val
65                  70                  75                  80

Ser Ser Phe Glu Arg Phe Glu Ile Phe Pro Lys Thr Ser Ser Trp Pro
                85                  90                  95

Asn His Asp Ser Asn Lys Gly Val Thr Ala Ala Cys Pro His Ala Gly
            100                 105                 110

Ala Lys Ser Phe Tyr Lys Asn Leu Ile Trp Leu Val Lys Lys Gly Asn
        115                 120                 125

Ser Tyr Pro Lys Leu Ser Lys Ser Tyr Ile Asn Asp Lys Gly Lys Glu
    130                 135                 140

Val Leu Val Leu Trp Gly Ile His His Pro Ser Thr Ser Ala Asp Gln
145                 150                 155                 160

Gln Ser Leu Tyr Gln Asn Ala Asp Thr Tyr Val Phe Val Gly Ser Ser
                165                 170                 175

Arg Tyr Ser Lys Lys Phe Lys Pro Glu Ile Ala Ile Arg Pro Lys Val
            180                 185                 190

Arg Asp Gln Glu Gly Arg Met Asn Tyr Tyr Trp Thr Leu Val Glu Pro
        195                 200                 205

Gly Asp Lys Ile Thr Phe Glu Ala Thr Gly Asn Leu Val Val Pro Arg
    210                 215                 220

Tyr Ala Phe Ala Met Glu Arg Asn Ala
225                 230

<210>  62
<211>  565
<212>  PRT
<213>  Artificial Sequence

<220>
<223>  FLHA_PR8

<400>  62

Met Lys Ala Asn Leu Leu Val Leu Leu Cys Ala Leu Ala Ala Ala Asp
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Ser His Asn Gly Lys Leu Cys Arg Leu Lys Gly Ile
    50                  55                  60

Ala Pro Leu Gln Leu Gly Lys Cys Asn Ile Ala Gly Trp Leu Leu Gly
65                  70                  75                  80

Asn Pro Glu Cys Asp Pro Leu Leu Pro Val Arg Ser Trp Ser Tyr Ile
                85                  90                  95

Val Glu Thr Pro Asn Ser Glu Asn Gly Ile Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Asn
    130                 135                 140

Thr Asn Gly Val Thr Ala Ala Cys Ser His Glu Gly Lys Ser Ser Phe
145                 150                 155                 160

Tyr Arg Asn Leu Leu Trp Leu Thr Glu Lys Glu Gly Ser Tyr Pro Lys
                165                 170                 175

Leu Lys Asn Ser Tyr Val Asn Lys Lys Gly Lys Glu Val Leu Val Leu
            180                 185                 190

Trp Gly Ile His His Pro Pro Asn Ser Lys Glu Gln Gln Asn Leu Tyr
        195                 200                 205

Gln Asn Glu Asn Ala Tyr Val Ser Val Val Thr Ser Asn Tyr Asn Arg
    210                 215                 220

Arg Phe Thr Pro Glu Ile Ala Glu Arg Pro Lys Val Arg Asp Gln Ala
225                 230                 235                 240

Gly Arg Met Asn Tyr Tyr Trp Thr Leu Leu Lys Pro Gly Asp Thr Ile
                245                 250                 255

Ile Phe Glu Ala Asn Gly Asn Leu Ile Ala Pro Met Tyr Ala Phe Ala
            260                 265                 270

Leu Ser Arg Gly Phe Gly Ser Gly Ile Ile Thr Ser Asn Ala Ser Met
        275                 280                 285

His Glu Cys Asn Thr Lys Cys Gln Thr Pro Leu Gly Ala Ile Asn Ser
    290                 295                 300

Ser Leu Pro Tyr Gln Asn Ile His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg Gly Leu Phe Gly Ala Ile Ala Gly Phe
            340                 345                 350

Ile Glu Gly Gly Trp Thr Gly Met Ile Asp Gly Trp Tyr Gly Tyr His
        355                 360                 365

His Gln Asn Glu Gln Gly Ser Gly Tyr Ala Ala Asp Gln Lys Ser Thr
    370                 375                 380

Gln Asn Ala Ile Asn Gly Ile Thr Asn Lys Val Asn Thr Val Ile Glu
385                 390                 395                 400

Lys Met Asn Ile Gln Phe Thr Ala Val Gly Lys Glu Phe Asn Lys Leu
                405                 410                 415

Glu Lys Arg Met Glu Asn Leu Asn Lys Lys Val Asp Asp Gly Phe Leu
            420                 425                 430

Asp Ile Trp Thr Tyr Asn Ala Glu Leu Leu Val Leu Leu Glu Asn Glu
        435                 440                 445

Arg Thr Leu Asp Phe His Asp Ser Asn Val Lys Asn Leu Tyr Glu Lys
    450                 455                 460

Val Lys Ser Gln Leu Lys Asn Asn Ala Lys Glu Ile Gly Asn Gly Cys
465                 470                 475                 480

Phe Glu Phe Tyr His Lys Cys Asp Asn Glu Cys Met Glu Ser Val Arg
                485                 490                 495

Asn Gly Thr Tyr Asp Tyr Pro Lys Tyr Ser Glu Glu Ser Lys Leu Asn
            500                 505                 510

Arg Glu Lys Val Asp Gly Val Lys Leu Glu Ser Met Gly Ile Tyr Gln
        515                 520                 525

Ile Leu Ala Ile Tyr Ser Thr Val Ala Ser Ser Leu Val Leu Leu Val
    530                 535                 540

Ser Leu Gly Ala Ile Ser Phe Trp Met Cys Ser Asn Gly Ser Leu Gln
545                 550                 555                 560

Cys Arg Ile Cys Ile
                565

<210>  63
<211>  566
<212>  PRT
<213>  Artificial Sequence

<220>
<223>  FLHA_Cal09

<400>  63

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Pro Ser Ser Asp Asn Gly Thr Cys Tyr Pro Gly Asp Phe
            100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Thr Tyr Val Phe Val Gly Ser Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
        260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Ile Pro Ser Ile Gln Ser Arg Gly Leu Phe Gly Ala Ile Ala Gly
            340                 345                 350

Phe Ile Glu Gly Gly Trp Thr Gly Met Val Asp Gly Trp Tyr Gly Tyr
        355                 360                 365

His His Gln Asn Glu Gln Gly Ser Gly Tyr Ala Ala Asp Leu Lys Ser
    370                 375                 380

Thr Gln Asn Ala Ile Asp Glu Ile Thr Asn Lys Val Asn Ser Val Ile
385                 390                 395                 400

Glu Lys Met Asn Thr Gln Phe Thr Ala Val Gly Lys Glu Phe Asn His
                405                 410                 415

Leu Glu Lys Arg Ile Glu Asn Leu Asn Lys Lys Val Asp Asp Gly Phe
            420                 425                 430

Leu Asp Ile Trp Thr Tyr Asn Ala Glu Leu Leu Val Leu Leu Glu Asn
        435                 440                 445

Glu Arg Thr Leu Asp Tyr His Asp Ser Asn Val Lys Asn Leu Tyr Glu
    450                 455                 460

Lys Val Arg Ser Gln Leu Lys Asn Asn Ala Lys Glu Ile Gly Asn Gly
465                 470                 475                 480

Cys Phe Glu Phe Tyr His Lys Cys Asp Asn Thr Cys Met Glu Ser Val
                485                 490                 495

Lys Asn Gly Thr Tyr Asp Tyr Pro Lys Tyr Ser Glu Glu Ala Lys Leu
            500                 505                 510

Asn Arg Glu Glu Ile Asp Gly Val Lys Leu Glu Ser Thr Arg Ile Tyr
        515                 520                 525

Gln Ile Leu Ala Ile Tyr Ser Thr Val Ala Ser Ser Leu Val Leu Val
    530                 535                 540

Val Ser Leu Gly Ala Ile Ser Phe Trp Met Cys Ser Asn Gly Ser Leu
545                 550                 555                 560

Gln Cys Arg Ile Cys Ile
                565

<210>  64
<211>  699
<212>  DNA
<213>  Artificial Sequence

<220>
<223>  MRK_pH1_Con_RBD

<400>  64
atgaaggtga agctgctggt gctgctgtgc accttcaccg ccacctacgc cggcgtggcc      60

cctctgcacc tgggcaagtg caacatcgcc ggctggatcc tgggcaaccc tgagtgcgag     120

agccttagca cagcctcctc ctggagctac atcgtggaga cgagcagcag cgataacggg     180

acctgctacc ctggcgactt catcgactac gaggagctga gagagcagct gagcagcgtg     240

agcagcttcg agagattcga gatcttccct aagaccagca gctggcctaa ccacgacagc     300

aacaagggcg tgaccgccgc ctgcccacac gccggggcca agagcttcta caagaacctg     360

atctggctgg tgaagaaggg caacagctac cctaaactga gcaagtccta catcaacgac     420

aaaggcaagg aggtcctcgt gctctggggc atccaccacc ctagcaccag cgccgatcag     480

cagagcctgt accagaacgc cgacgcgtac gtgttcgtgg gcaccagcag atacagcaag     540

aagttcaagc ctgagatcgc catcagacct aaggtgaggg accaggaggg cagaatgaac     600

tactactgga ccctggtgga gcccggagat aagatcacat ttgaggccac cggcaacctg     660

gtggtgccta gatacgcctt cgccatggag agaaacgcc                            699

<210>  65
<211>  1680
<212>  DNA
<213>  Artificial Sequence

<220>
<223>  MRK_pH1_Con_ecto

<400>  65
atgaaggcca tcctcgtggt gctgctgtac acctttgcca ccgccaacgc cgataccctg      60

tgtatcggct accacgccaa caacagcacc gacaccgtgg atactgtcct ggagaagaac 120

gtgaccgtga cccacagcgt gaacctgctg gaggacaagc acaacggcaa gctgtgcaag 180

ctgagaggcg tggcccctct gcacctgggc aagtgcaaca tcgccggctg gatcctgggc 240

aaccctgagt gcgagagcct tagcacagcc tcctcctgga gctacatcgt ggagacgagc 300

agcagcgata acgggacctg ctaccctggc gacttcatcg actacgagga gctgagagag 360

cagctgagca gcgtgagcag cttcgagaga ttcgagatct tccctaagac cagcagctgg 420

cctaaccacg acagcaacaa gggcgtgacc gccgcctgcc cacacgccgg ggccaagagc 480

ttctacaaga acctgatctg gctggtgaag aagggcaaca gctaccctaa actgagcaag 540

tcctacatca acgacaaagg caaggaggtc ctcgtgctct ggggcatcca ccaccctagc 600

accagcgccg atcagcagag cctgtaccag aacgccgacg cgtacgtgtt cgtgggcacc 660

agcagataca gcaagaagtt caagcctgag atcgccatca gacctaaggt gagggaccag 720

gagggcagaa tgaactacta ctggaccctg gtggagcccg gagataagat cacatttgag 780

gccaccggca acctggtggt gcctagatac gccttcgcca tggagagaaa cgccggcagc 840

ggcatcatca tcagcgacac ccctgtgcac gactgcaaca ccacctgcca gacccctaag 900

ggcgccatca acacgagcct gcctttccag aacatccacc ctatcaccat cggcaagtgc 960

cctaagtacg tgaagtcaac caaactgaga ctcgccaccg gcctcagaaa cgtgcctagc 1020

atccagagca gaggcctctt cggcgccatc gcgggattca tcgagggcgg ctggaccggc 1080

atggtggacg gctggtacgg ctaccaccat cagaacgagc agggcagcgg gtacgcggcc 1140

gacctcaaga gcacccagaa cgccatcgac aagatcacca acaaggtgaa cagcgtgatc 1200

gagaagatga acacccagtt caccgccgtg ggcaaggagt tcaaccacct ggagaagaga 1260

atcgagaacc tgaacaagaa ggtggacgac ggcttcctgg acatctggac ctacaacgca 1320

gaactgctcg tgcttctgga gaacgagaga accctggact accacgactc caacgtgaag 1380

aacctgtacg agaaggtgag aagccagctg aagaacaacg ccaaggagat cggcaacggc 1440

tgcttcgagt tctaccacaa gtgcgacaac acctgcatgg agagcgtgaa gaacggcacc 1500

tacgactacc ctaagtacag cgaggaggcc aagctgaaca gagaggagat cgacggcgtg 1560

aagctggaga gcaccagaat cggctcagcc gggagcgccg gctacatccc tgaggcccct 1620

agagacggcc aggcctacgt gagaaaggac ggcgagtggg tgctgctgag caccttcctg 1680

<210> 66
<211> 693
<212> DNA
<213> Artificial Sequence

<220>
<223> MRK_sH1_Con_RBD

<400>  66
atgaaggtga agctgctggt gctgctgtgc accttcaccg ccacctacgc cggaatcgct      60

cccctgcagc tcggcaactg cagcgtggcc ggctggattc tgggcaaccc cgagtgcgaa     120

ctgctgatta gcaaagagtc ctggagctac atcgtggaaa ccccgaatcc cgagaacggc     180

acctgctacc ccggctactt cgccgactac gaggagctaa gagagcagct gagtagcgtg     240

agctcattcg agagattcga gatctttccc aaggagtcta gctggcccaa tcacaccgtc     300

accggcgtgt ccgccagctg tagccacaac ggcaagagca gcttctacag aaacctgctg     360

tggctgaccg gcaagaacgg actgtaccct aacctgagca agagctacgc gaacaataag     420

gagaaggagg tgctagtgct gtggggcgtg caccatccgc ccaacatcgg cgaccagaga     480

gccctgtacc acaccgagaa cgcctacgtg agcgtggtga gcagccacta tagcagaaga     540

ttcacccctg agatcgccaa gaggccaaag gtgagagatc aggaaggaag aataaactac     600

tactggaccc tcctggagcc cggcgacacc atcatcttcg aggctaacgg caacctgatc     660

gcccctagat acgccttcgc cctgagcaga ggc                                  693


<210>  67
<211>  1677
<212>  DNA
<213>  Artificial Sequence

<220>
<223>  MRK_sH1_Con_ecto

<400>  67
atgaaggtga agctgctggt gctgctgtgt accttcactg ccacttacgc cgacaccatt      60

tgcatcggct accacgccaa caacagcacc gataccgtgg acaccgtgct ggagaagaac     120

gtcaccgtga cccacagcgt gaacctgctg gaggatagcc ataacggcaa gctgtgcctg     180

ctgaagggaa tcgctcccct gcagctcggc aactgcagcg tggccggctg gattctgggc     240

aaccccgagt gcgaactgct gattagcaaa gagtcctgga gctacatcgt ggaaaccccg     300

aatcccgaga acggcacctg ctaccccggc tacttcgccg actacgagga gctaagagag     360

cagctgagta gcgtgagctc attcgagaga ttcgagatct ttcccaagga gtctagctgg     420

cccaatcaca ccgtcaccgg cgtgtccgcc agctgtagcc acaacggcaa gagcagcttc     480

tacagaaacc tgctgtggct gaccggcaag aacggactgt accctaacct gagcaagagc     540

tacgcgaaca ataaggagaa ggaggtgcta gtgctgtggg gcgtgcacca tccgcccaac     600

atcggcgacc agagagccct gtaccacacc gagaacgcct acgtgagcgt ggtgagcagc     660

cactatagca gaagattcac ccctgagatc gccaagaggc caaaggtgag agatcaggaa     720

ggaagaataa actactactg gaccctcctg gagcccggcg acaccatcat cttcgaggct     780

aacggcaacc tgatcgcccc tagatacgcc ttcgccctga gcagaggctt cggcagcggc     840

atcatcacca gcaacgctcc catggacgag tgcgacgcca agtgccagac cccgcagggc 900

gccatcaact cgagcctgcc cttccagaac gtgcaccccg tgaccatcgg cgagtgcccc 960

aagtacgtga gaagcgccaa gctgagaatg gtgaccggcc tgagaaacat cccaagcatc 1020

cagagcagag ggctgttcgg cgccatcgct ggcttcatcg agggcggctg gaccggcatg 1080

gtggacggct ggtacggtta tcaccaccag aacgagcagg gcagcggcta cgccgccgac 1140

cagaagtcca cccagaacgc catcaacggc attacaaaca aggtgaacag cgttatcgag 1200

aagatgaaca cccaattcac cgccgtgggc aaggagttca acaagctgga gagaagaatg 1260

gagaacctga acaagaaggt ggacgacggc ttcctggaca tctggaccta caacgccgaa 1320

ctgctggtcc tgctggagaa cgagagaacc ctggacttcc acgactccaa cgtgaagaac 1380

ttatacgaga aggtcaaatc ccagctgaag aacaacgcca aagaaatcgg aaacggctgc 1440

ttcgaattct accacaagtg caacgacgag tgcatggaga gcgtgaagaa cggaacctac 1500

gactacccca agtacagcga ggaaagcaaa ctgaacagag agaagatcga cggcgtgaag 1560

ttagagagca tgggcgtggg cagcgccggc tctgctggat acatccctga ggcccctaga 1620

gacggccagg cctacgtgag aaaggacggc gagtgggtgc tgctgagcac cttcctg 1677

<210> 68
<211> 1695
<212> DNA
<213> Artificial Sequence

<220>
<223> MRK_sH1_Con_v2

<400> 68
atgaaggtga aactcctcgt cctgctgtgc accttcaccg ccacctacgc cgataccatc 60

tgtattggct accacgccaa caactccacc gacaccgtgg ataccgtgct cgagaagaac 120

gtgaccgtga cccacagcgt gaacctgctg gagaacagcc acaacggcaa gctgtgcctg 180

ctgaagggca tcgcgcccct gcagttgggt aactgctccg tggccggctg gatcctgggc 240

aaccctgagt gcgagctgct gatcagcaag gagagctgga gctacatcgt ggagaagcct 300

aaccccgaga acggcacctg ctaccctggc cacttcgccg actacgagga gctgagagag 360

caactcagca gcgtgagcag cttcgagaga ttcgagatct tccctaagga gagcagctgg 420

cccaatcaca ctgtgaccgg cgtgtccgct tcttgcagcc ataacgggga aagctccttc 480

tacagaaatc tcctttggct gacggggaag aacggcctgt accctaacct gagcaagagc 540

tacgccaaca acaaggagaa ggaggtgctg gtgctgtggg gcgtgcacca ccctcctaac 600

atcggcgacc agaaggccct gtaccacacc gagaacgcct acgtcagcgt ggtgtccagc 660

cactacagca gaaagttcac ccctgagatc gccaagaggc ctaaggtgcg ggaccaggag 720

ggcagaatca actactactg gaccctgctg gagcctggcg acaccatcat cttcgaggcc 780

aacggcaacc tgatcgcccc tagatacgcc ttcgccctga gcagaggctt cggcagcggc 840

atcatcaaca gcaacgcccc tatggacaag tgcgacgcca agtgccagac tccgcagggc 900

gctatcaaca gctccctgcc tttccagaac gtgcaccctg tgaccatcgg cgagtgccct 960

aagtacgtga gaagcgccaa gctgagaatg gtgaccggcc tgagaaacat ccctagcatc 1020

cagagcagag gcctgttcgg cgccatcgcc gggtttatcg agggcggctg gaccggcatg 1080

gtggacggct ggtacggcta ccaccaccag aacgagcagg gctccggcta cgccgccgac 1140

cagaaatcca cccagaacgc catcaacggc atcaccaaca aggtgaacag cgtcatcgag 1200

aagatgaaca cccagttcac cgccgtgggc aaggagttca acaagctgga gagaagaatg 1260

gagaacctga acaagaaggt ggacgacggc ttcatcgaca tctggaccta caacgccgag 1320

cttctggtgc tcctggagaa cgagagaacc ctggacttcc acgacagcaa cgtgaagaac 1380

ctgtacgaga aggtgaagtc ccagctgaag aacaacgcca aggagatcgg caacggctgc 1440

ttcgagttct accacaagtg caacgacgag tgcatggaga gcgtgaagaa cggcacctac 1500

gattacccca agtacagcga ggagagcaag ctgaacagag agaagatcga cggcgtgaag 1560

ctggagagca tgggcgtgta ccagatcctg gccatctact ccaccgtggc cagtagcctg 1620

gtgctgctgg tgagcctggg cgcaatcagc ttctggatgt gcagcaacgg cagcctgcag 1680

tgcagaatct gcatc 1695

<210> 69
<211> 699
<212> DNA
<213> Artificial Sequence

<220>
<223> MRK_RBS_HA129

<400> 69
atgaaggtca aacttctcgt gctcctgtgc accttcaccg ccacctacgc gggcgtggct 60

ccgcttcacc tgggcaagtg caacatcgcc ggttggctgc tgggtaaccc agagtgcgag 120

ctactgctga ccgtgagcag ctggagctac atcgtggaaa ccagcaacag cgacaacggc 180

acctgctacc ctggcgactt catcaactac gaggagctga gagagcagct cagcagcgtg 240

tccagcttcg agagattcga gatcttccct aagactagca gctggcccga ccacgaaaca 300

aacagaggcg tgaccgccgc ttgtccatac gccggcgcca acagcttcta cagaaacctg 360

atctggctgg tgaagaaggg caacagctac cctaagctga gcaagagcta cgtgaacaac 420

aagggcaagg aggtgcttgt gctgtggggc atccaccacc ctcctaccag caccgaccag 480

cagagcctgt accagaacgc cgacgcctac gtgttcgtgg gcagcagcag atacagcaag 540

aagttcaagc ctgagatcgc catcagacct aaggtgaggg accaggaggg cagaatgaac 600

tactactgga ctctggtgga gcccggcgac aagatcacct tcgaggccac cggcaacctg 660

gtggtgccta gatacgcctt cgccatggag agaaacgcc 699

<210> 70
<211> 1698
<212> DNA
<213> Artificial Sequence

<220>
<223> MRK_H1_cot_all

<400> 70
atgaaggcca tcctggtcgt gctgctctac acattcgcca ccgccaacgc agacactctg 60

tgcatcggct accacgccaa caacagcacc gacaccgtgg ataccgtgct ggagaagaac 120

gtgaccgtga cccacagcgt gaacctgctg gaggacaagc acaacggcaa gctgtgcaag 180

ctgagaggcg tggcccctct gcacctgggc aagtgcaaca tcgccggctg gatcctggga 240

aaccccgagt gcgagagcct gtcaaccgcc tcgagctggt cctacatcgt ggaaaccagc 300

agcagcgata acgggacgtg ctacccgggc gacttcatca actacgagga gctgagagaa 360

cagctgagca gcgtcagtag cttcgagaga ttcgagatct tccctaagac cagcagctgg 420

cctaaccacg acagcaacaa gggcgtgacc gccgcttgcc cgcacgcagg cgccaagagc 480

ttctacaaga acctgatctg gctggtgaag aagggcaaca gctaccctaa gctgagcaag 540

agctacatca acgacaaggg gaaggaggtg ctagtcctgt ggggcatcca tcaccctagc 600

accacagccg accagcaaag cctgtaccag aacgcggacg cctacgtgtt cgtcggcacc 660

agcagataca gcaagaagtt caagcctgag atcgccatca gacctaaggt gcgagatcag 720

gagggcagaa tgaactacta ctggaccctg gtggagcccg gagacaagat tactttcgaa 780

gcgaccggca acctggtggt gcctagatac gccttcgcca tggagagaaa cgccggcagc 840

ggcatcatca tcagcgacac ccctgtgcac gactgcaaca ccacctgcca gacccctaaa 900

ggcgccatca acacaagcct gccttttcag aacatccacc ctatcaccat cggcaagtgc 960

cctaagtacg tgaagtccac caagctccgc ctggcaaccg gcctcaggaa cgtgcctagc 1020

atccagagca gaggcctgtt cggggccata gccggcttca tagagggtgg ctggaccggc 1080

atggttgacg ggtggtacgg ataccatcac cagaacgagc aaggcagcgg ctacgccgca 1140

gacctgaagt caacccagaa cgccatcgac aagatcacca acaaggtgaa cagcgtgatc 1200

gagaagatga acacccagtt caccgccgtg ggcaaggagt tcaaccacct agagaagagg 1260

atcgagaacc tgaataagaa ggtggacgac ggcttcctgg acatctggac ctacaacgcc 1320

gagctgctcg tcctcctgga gaacgagaga accctggact accacgatag caacgtgaag 1380

aacctgtacg agaaggtgag aaaccagctg aagaataacg ccaaggagat cggcaacggc 1440

tgcttcgagt tctaccacaa gtgcgacaac acctgcatgg agagcgtgaa gaacggcacc 1500

tacgactacc ctaagtacag cgaggaggcc aagctgaaca gagagaagat cgacggcgtg 1560

aagctggaga gcaccagaat ctaccagatc ctggccatct acagcaccgt ggccagcagc 1620

ctcgtgctcg tggtgagcct gggcgccatc tccttctgga tgtgcagcaa cggcagcctg 1680

cagtgcagaa tctgcatc 1698

<210> 71
<211> 1698
<212> DNA
<213> Artificial Sequence

<220>
<223> MRK_H3_cot_all

<400> 71
atgaagacca tcatcgccct gagctacatc ctgtgcctgg tgttcgcgca gaaactcccc 60

ggcaacgaca atagcactgc caccctgtgt ctgggccatc acgccgtgcc taacggaacc 120

atcgtgaaga cgatcaccaa cgacagaatc gaggtgacca acgccaccga gctggtccag 180

aattcgagca tcggcgaaat ctgcgacagc cctcaccaga tcctggacgg cgagaactgc 240

accctgattg acgcactgct aggcgaccca cagtgtgacg gcttccagaa caagaagtgg 300

gacctgttcg tggagagaag caaggcctac agcaactgct acccttacga cgtgcctgac 360

tacgccagcc tgagatccct cgtggcctcc agcggcaccc tcgagttcaa taacgagagc 420

ttcaactgga ccggagtcac ccagaacggg acatccagcg cctgcatcag aagaagcaac 480

agcagcttct tcagcagact gaactggctg acccacctga acttcaagta ccctgccctg 540

aacgtgacca tgcctaacaa cgagcagttc gacaagctgt acatctgggg cgtgcaccat 600

cccggcaccg acaaggacca gatcttcctg tacgcccaga gctccggcag gatcaccgtg 660

agcaccaaga gaagccagca ggccgtgatc cctaacatcg gcagcagacc tagaatcaga 720

aacatcccta gcagaatcag catctactgg accatagtga agcccggcga catcctgctg 780

atcaactcga ccggcaacct gatcgctcct aggggctact tcaagatcag aagcggcaag 840

agcagcatca tgagaagcga cgcgcccatc gggaagtgca agtccgagtg catcacccct 900

aacggcagca tccccaacga caagcctttc cagaacgtga acagaatcac ctacggcgcc 960

tgccctagat acgtgaagca gagcacactg aagctggcca ccggcatgag gaacgtgcct 1020

gagaagcaga ccagaggcat cttcggggct attgccggct tcatcgagaa cggttgggag 1080

ggaatggtcg acgggtggta cggcttcaga caccagaaca gcgaaggcag gggacaggcc 1140

gccgacctca agtccaccca ggctgccatc gatcagatca acgggaagct gaacagactg 1200

atcggcaaga ccaacgagaa gttccaccag atcgagaagg agttcagcga ggtggagggc 1260

agaatccagg acctggagaa gtacgtggag gacacgaaga tcgacctgtg gagctacaac 1320

gcagagctgt tggtggcact ggagaaccag cacaccatcg acctgaccga cagcgagatg 1380

aacaagctgt tcgagaagac caagaagcag ttacgagaga acgccgagga catgggaaac 1440

ggctgtttta agatctacca caagtgcgac aacgcctgca tcgggagcat caggaacggg 1500

acctacgacc acgacgtgta cagagacgag gccctgaaca acagattcca gatcaagggc 1560

gtggagctga agtccggcta caaggactgg atcctgtgga tcagcttcgc catcagctgc 1620

ttcctgctgt gcgtggccct cctgggcttt ataatgtggg cctgccagaa gggcaacatc 1680

aggtgcaaca tctgcatc 1698

<210> 72
<211> 1698
<212> DNA
<213> Artificial Sequence

<220>
<223> MRK_H3_ConsensusA

<400> 72
atgaagacca tcatcgccct gagctacatc ctgtgcctgg tgttcgcgca gaaactcccc 60

ggcaacgaca atagcactgc caccctgtgt ctgggccatc acgccgtgcc taacggaacc 120

ctcgtgaaga cgatcaccaa cgaccagatc gaggtgacca acgccaccga gctggtccag 180

agttcgagca ccggcagaat ctgcgacagc cctcaccgga tcctggacgg cgagaactgc 240

accctgattg acgcactgct aggcgaccca cactgtgacg gcttccagaa caaggagtgg 300

gacctgttcg tggagagaag caaggcctac agcaactgct acccttacga cgtgcctgac 360

tacgccagcc tgagatccct cgtggcctcc agcggcaccc tcgagttcaa taacgagagc 420

ttcaactgga ccggagtcgc ccagaacggg acatcctacg cctgcaagag aggaagcgtc 480

aagagcttct tcagcagact gaactggctg caccagctga agtacaagta ccctgccctg 540

aacgtgacca tgcctaacaa cgacaagttc gacaagctgt acatctgggg cgtgcaccat 600

cccagcaccg acagcgacca gacctccctg tacgtccagg catccggcag ggtcaccgtg 660

agcaccaaga gaagccagca gaccgtgatc cctaacatcg gcagcagacc ttgggtcaga 720

ggcgtctcta gcagaatcag catctactgg accatagtga agcccggcga catcctgctg 780

atcaactcga ccggcaacct gatcgctcct aggggctact tcaagatcag aagcggcaag 840

agcagcatca tgagaagcga cgcgcccatc gggaagtgca actccgagtg catcacccct 900

aacggcagca tccccaacga caagcctttc cagaacgtga acagaatcac ctacggcgcc 960

tgccctagat acgtgaagca gaacacactg aagctggcca ccggcatgag gaacgtgcct 1020

gagaagcaga ccagaggcat cttcggggct attgccggct tcatcgagaa cggttgggag 1080

ggaatggtcg acgggtggta cggcttcaga caccagaaca gcgaaggcac gggacaggcc 1140

gccgacctca agtccaccca ggctgccatc aatcagatca acgggaagct gaacagactg 1200

atcgagaaga ccaacgagaa gttccaccag atcgagaagg agttcagcga ggtggagggc 1260

agaatccagg acctggagaa gtacgtggag gacacgaaga tcgacctgtg gagctacaac 1320

gcagagctgt tggtggcact ggagaaccag cacaccatcg acctgaccga cagcgagatg 1380

aacaagctgt tcgagaggac caggaagcag ttacgagaga acgccgagga catgggaaac 1440

ggctgtttta agatctacca caagtgcgac aacgcctgca tcgggagcat caggaacggg 1500

acctacgacc acgacgtgta cagagacgag gccctgaaca acagattcca gatcaagggc 1560

gtggagctga agtccggcta caaggactgg atcctgtgga tcagcttcgc catcagctgc 1620

ttcctgctgt gcgtggtcct cctgggcttt ataatgtggg cctgccagaa gggcaacatc 1680

aggtgcaaca tctgcatc 1698

<210> 73
<211> 1698
<212> DNA
<213> Artificial Sequence

<220>
<223> MRK_H3_ConsensusB

<400> 73
atgaagacca tcatcgccct gagctacatc ctgtgcctgg tgttcgcgca gaaactcccc 60

ggcaacgaca atagcactgc caccctgtgt ctgggccatc acgccgtgcc taacggaacc 120

atcgtgaaga cgatcaccaa cgaccagatc gaggtgacca acgccaccga gctggtccag 180

aattcgagca ccggcgaaat ctgcgacagc cctcaccaga tcctggacgg cgagaactgc 240

accctgattg acgcactgct aggcgaccca cagtgtgacg gcttccagaa caagaagtgg 300

gacctgttcg tggagagaag caaggcctac agcaactgct acccttacga cgtgcctgac 360

tacgccagcc tgagatccct cgtggcctcc agcggcaccc tcgagttcaa taacgagagc 420

ttcaactgga ccggagtcac ccagaacggg acatccagcg cctgcatcag aagaagcaac 480

agcagcttct tcagcagact gaactggctg acccacctga acttcaagta ccctgccctg 540

aacgtgacca tgcctaacaa cgagcagttc gacaagctgt acatctgggg cgtgcaccat 600

cccggcaccg acaaggacca gatcttcctg tacgcccaga gctccggcag gatcaccgtg 660

agcaccaaga gaagccagca ggccgtgatc cctaacatcg gcagcagacc tagaatcaga 720

aacatcccta gcagaatcag catctactgg accatagtga agcccggcga catcctgctg 780

atcaactcga ccggcaacct gatcgctcct aggggctact tcaagatcag aagcggcaag 840

agcagcatca tgagaagcga cgcgcccatc gggaagtgca actccgagtg catcacccct 900

aacggcagca tccccaacga caagcctttc cagaacgtga acagaatcac ctacggcgcc 960

tgccctagat acgtgaagca gagcacactg aagctggcca ccggcatgag gaacgtgcct 1020

gagaagcaga ccagaggcat cttcggggct attgccggct tcatcgagaa cggttgggag 1080

ggaatggtcg acgggtggta cggcttcaga caccagaaca gcgaaggcag gggacaggcc 1140

gccgacctca agtccaccca ggctgccatc gatcagatca acgggaagct gaacagactg 1200

atcggcaaga ccaacgagaa gttccaccag atcgagaagg agttcagcga ggtggagggc 1260

agaatccagg acctggagaa gtacgtggag gacacgaaga tcgacctgtg gagctacaac 1320

gcagagctgt tggtggcact ggagaaccag cacaccatcg acctgaccga cagcgagatg 1380

aacaagctgt tcgagaagac caagaagcag ttacgagaga acgccgagga catgggaaac 1440

ggctgtttta agatctacca caagtgcgac aacgcctgca tcgggagcat caggaacggg 1500

acctacgacc acgacgtgta cagagacgag gccctgaaca acagattcca gatcaagggc 1560

gtggagctga agtccggcta caaggactgg atcctgtgga tcagcttcgc catcagctgc 1620

ttcctgctgt gcgtggccct cctgggcttt ataatgtggg cctgccagaa gggcaacatc 1680

aggtgcaaca tctgcatc 1698

<210> 74
<211> 1698
<212> DNA
<213> Artificial Sequence

<220>
<223> MRK_H3_consUnique

<400> 74
atgaagacca tcatcgccct gagctacatc ctgtgcctgg tgttcgcgca gaaactcccc 60

ggcaacgaca atagcactgc caccctgtgt ctgggccatc acgccgtgcc taacggaacc 120

atcgtgaaga cgatcaccaa cgaccagatc gaggtgacca acgccaccga gctggtccag 180

agttcgagca ccggcgaaat ctgcgacagc cctcaccaga tcctggacgg cgagaactgc 240

accctgattg acgcactgct aggcgaccca cagtgtgacg gcttccagaa caagaagtgg 300

gacctgttcg tggagagaag caaggcctac agcaactgct acccttacga cgtgcctgac 360

tacgccagcc tgagatccct cgtggcctcc agcggcaccc tcgagttcaa taacgagagc 420

ttcaactgga ccggagtcac ccagaacggg acatccagcg cctgcatcag aagaagcaac 480

agcagcttct tcagcagact gaactggctg acccacctga acttcaagta ccctgccctg 540

aacgtgacca tgcctaacaa cgagcagttc gacaagctgt acatctgggg cgtgcaccat 600

cccggcaccg acaaggacca gatcttcctg tacgcccagg catccggcag gatcaccgtg 660

agcaccaaga gaagccagca ggccgtgatc cctaacatcg gcagcagacc tagagtcaga 720

aacatcccta gcagaatcag catctactgg accatagtga agcccggcga catcctgctg 780

atcaactcga ccggcaacct gatcgctcct aggggctact tcaagatcag aagcggcaag 840

agcagcatca tgagaagcga cgcgcccatc gggaagtgca actccgagtg catcacccct 900

aacggcagca tccccaacga caagcctttc cagaacgtga acagaatcac ctacggcgcc 960

tgccctagat acgtgaagca gaacacactg aagctggcca ccggcatgag gaacgtgcct 1020

gagaagcaga ccagaggcat cttcggggct attgccggct tcatcgagaa cggttgggag 1080

ggaatggtcg acgggtggta cggcttcaga caccagaaca gcgaaggcag gggacaggcc 1140

gccgacctca agtccaccca ggctgccatc gatcagatca acgggaagct gaacagactg 1200

atcggcaaga ccaacgagaa gttccaccag atcgagaagg agttcagcga ggtggagggc 1260

agaatccagg acctggagaa gtacgtggag gacacgaaga tcgacctgtg gagctacaac 1320

gcagagctgt tggtggcact ggagaaccag cacaccatcg acctgaccga cagcgagatg 1380

aacaagctgt tcgagaagac caagaagcag ttacgagaga acgccgagga catgggaaac 1440

ggctgtttta agatctacca caagtgcgac aacgcctgca tcgggagcat caggaacggg 1500

acctacgacc acgacgtgta cagagacgag gccctgaaca acagattcca gatcaagggc 1560

gtggagctga agtccggcta caaggactgg atcctgtgga tcagcttcgc catcagctgc 1620

ttcctgctgt gcgtggccct cctgggcttt ataatgtggg cctgccagaa gggcaacatc 1680

aggtgcaaca tctgcatc 1698

<210> 75
<211> 699
<212> DNA
<213> Artificial Sequence

<220>
<223> RBD1-Cal09-PC-Cb

<400> 75
atgaaggtga agcttctcgt gctcttatgc accttcaccg ccacctacgc cggcgtggct 60

ccgcttcacc ttggcaagtg caacatcgcc ggctggatct tgggaaaccc cgagtgcgag 120

agcttgagca ccgccagcag ctggagcaac atcacggaaa cccctagcag cgacaacggc 180

acctgctacc ccggcgactt catcgactac gaggagctgc gggagcagct gagcagcgtg 240

agcagcttcg agcggttcga gatcttcccc aagaccagct cttggcccaa ccacagcagc 300

aacaagggcg tgaccgccgc ctgccctcac gctggcgcca agagcttcta caagaacctg 360

atctggctgg tgaagaagaa cggcagctac cccaagctga acaagtctta cattaacgac 420

tcaggcaagg aggtgctggt cctgtggggc atccaccacc ccagcaacag caccgaccaa 480

cagagcctgt accagaacgc cgacacctac gtgttcgtgg gcagcagcaa ctacagcaag 540

aagttcaagc ccgagatcgc catccggccc aaggtgcggg accaggaggg ccggatgaac 600

tactactgga ccctggtgga gcctggcgac aagatcacct tcgaggccac cggcaacctg 660

gtggtgcccc ggtacgcctt cgccatggag cggaacgcc 699

<210>  76
<211>  699
<212>  DNA
<213>  Artificial Sequence

<220>
<223>  RBD1-Cal09-PC

<400>  76
atgaaggtga agcttctcgt gctcttatgc accttcaccg ccacctacgc cggcgtggct      60

ccgcttcacc ttggcaagtg caacatcgcc ggctggatct tgggaaaccc cgagtgcgag     120

agcaacagca ccgccagcag ctggagcaac atcacggaaa cccctagcag cgacaacggc     180

acctgctacc ccggcgactt catcgactac gaggagctgc gggagcagct gagcagcgtg     240

agcagcttcg agcggttcga gatcttcccc aagaccagct cttggcccaa ccacagcagc     300

aacaagggcg tgaccgccgc ctgccctcac gctggcgcca agagcttcta caagaacctg     360

atctggctgg tgaagaagaa cggcagctac cccaagctga acaagtctta cattaacgac     420

tcaggcaagg aggtgctggt cctgtggggc atccaccacc ccagcaacag caccgaccaa     480

cagagcctgt accagaacgc cgacacctac gtgttcgtgg gcagcagcaa ctacagcaag     540

aagttcaagc ccgagatcgc catccggccc aaggtgcggg accaggaggg ccggatgaac     600

tactactgga ccctggtgga gcctggcgac aagatcacct tcgaggccac cggcaacctg     660

gtggtgcccc ggtacgcctt cgccatggag cggaacgcc                            699


<210>  77
<211>  699
<212>  DNA
<213>  Artificial Sequence

<220>
<223>  RBD1-Cal09

<400>  77
atgaaggtga agcttctcgt gctcttatgc accttcaccg ccacctacgc cggcgtggct      60

ccgcttcacc ttggcaagtg caacatcgcc ggctggatct tgggaaaccc cgagtgcgag     120

agcttgagca ccgccagcag ctggagcaac atcacggaaa cccctagcag cgacaacggc     180

acctgctacc ccggcgactt catcgactac gaggagctgc gggagcagct gagcagcgtg     240

agcagcttcg agcggttcga gatcttcccc aagaccagct cttggcccaa ccacgacagc     300

aacaagggcg tgaccgccgc ctgccctcac gctggcgcca agagcttcta caagaacctg     360

atctggctgg tgaagaaggg caacagctac cccaagctgt ccaagtctta cattaacgac     420

aagggcaagg aggtgctggt cctgtggggc atccaccacc ccagcaccag cgccgaccaa     480

cagagcctgt accagaacgc cgacacctac gtgttcgtgg gcagcagccg gtacagcaag     540

aagttcaagc ccgagatcgc catccggccc aaggtgcggg accaggaggg ccggatgaac     600

tactactgga ccctggtgga gcctggcgac aagatcacct tcgaggccac cggcaacctg 660

gtggtgcccc ggtacgcctt cgccatggag cggaacgcc 699

<210> 78
<211> 699
<212> DNA
<213> Artificial Sequence

<220>
<223> MRK_RBD-Cal09-PC-Cb

<400> 78
atgaaggtga agcttctcgt gctcttatgc accttcaccg ccacctacgc cggcgtggct 60

ccgcttcacc ttggcaagtg caacatcgcc ggctggatct tgggaaaccc cgagtgcgag 120

agcttgagca ccgccagcag ctggagctac atcgtggaaa cccctagcag cgacaacggc 180

acctgctacc ccggcgactt catcgactac gaggagctgc gggagcagct gagcagcgtg 240

agcagcttcg agcggttcga gatcttcccc aagaccagct cttggcccaa ccacagcagc 300

aacaagggcg tgaccgccgc ctgccctcac gctggcgcca agagcttcta caagaacctg 360

atctggctgg tgaagaagaa cggcagctac cccaagctga acaagtctta cattaacgac 420

tcaggcaagg aggtgctggt cctgtggggc atccaccacc ccagcaacag caccgaccaa 480

cagagcctgt accagaacgc cgacacctac gtgttcgtgg gcagcagcaa ctacagcaag 540

aagttcaagc ccgagatcgc catccggccc aaggtgcggg accaggaggg ccggatgaac 600

tactactgga ccctggtgga gcctggcgac aagatcacct tcgaggccac cggcaacctg 660

gtggtgcccc ggtacgcctt cgccatggag cggaacgcc 699

<210> 79
<211> 699
<212> DNA
<213> Artificial Sequence

<220>
<223> MRK_RBD-Cal09-PC

<400> 79
atgaaggtga agcttctcgt gctcttatgc accttcaccg ccacctacgc cggcgtggct 60

ccgcttcacc ttggcaagtg caacatcgcc ggctggatct tgggaaaccc cgagtgcgag 120

agcaacagca ccgccagcag ctggagctac atcgtggaaa cccctagcag cgacaacggc 180

acctgctacc ccggcgactt catcgactac gaggagctgc gggagcagct gagcagcgtg 240

agcagcttcg agcggttcga gatcttcccc aagaccagct cttggcccaa ccacagcagc 300

aacaagggcg tgaccgccgc ctgccctcac gctggcgcca agagcttcta caagaacctg 360

atctggctgg tgaagaagaa cggcagctac cccaagctga acaagtctta cattaacgac 420

tcaggcaagg aggtgctggt cctgtggggc atccaccacc ccagcaacag caccgaccaa 480

cagagcctgt accagaacgc cgacacctac gtgttcgtgg gcagcagcaa ctacagcaag 540

aagttcaagc ccgagatcgc catccggccc aaggtgcggg accaggaggg ccggatgaac 600

tactactgga ccctggtgga gcctggcgac aagatcacct tcgaggccac cggcaacctg 660

gtggtgcccc ggtacgcctt cgccatggag cggaacgcc 699

<210> 80
<211> 699
<212> DNA
<213> Artificial Sequence

<220>
<223> MRKRBD-Cal09

<400> 80
atgaaggtga agcttctcgt gctcttatgc accttcaccg ccacctacgc cggcgtggct 60

ccgcttcacc ttggcaagtg caacatcgcc ggctggatct tgggaaaccc cgagtgcgag 120

agcttgagca ccgccagcag ctggagctac atcgtggaaa cccctagcag cgacaacggc 180

acctgctacc ccggcgactt catcgactac gaggagctgc gggagcagct gagcagcgtg 240

agcagcttcg agcggttcga gatcttcccc aagaccagct cttggcccaa ccacgacagc 300

aacaagggcg tgaccgccgc ctgccctcac gctggcgcca agagcttcta caagaacctg 360

atctggctgg tgaagaaggg caacagctac cccaagctgt ccaagtctta cattaacgac 420

aagggcaagg aggtgctggt cctgtggggc atccaccacc ccagcaccag cgccgaccaa 480

cagagcctgt accagaacgc cgacacctac gtgttcgtgg gcagcagccg gtacagcaag 540

aagttcaagc ccgagatcgc catccggccc aaggtgcggg accaggaggg ccggatgaac 600

tactactgga ccctggtgga gcctggcgac aagatcacct tcgaggccac cggcaacctg 660

gtggtgcccc ggtacgcctt cgccatggag cggaacgcc 699

<210> 81
<211> 1695
<212> DNA
<213> Artificial Sequence

<220>
<223> FLHA_PR8

<400> 81
atgaaggcca atttgttggt ccttctatgt gccctagccg ccgccgacgc cgacacaatc 60

tgcatcggat atcacgcaaa caacagcacc gacaccgtgg atacggtctt ggagaagaac 120

gtgaccgtga cccattccgt gaaccttctc gaggatagcc acaatggcaa gctgtgtaga 180

ctcaagggca ttgccccgct gcagctggga aagtgcaata ttgctggctg gctgttgggc 240

aaccctgagt gtgaccctct gttaccagtg agatcttgga gctatatcgt cgaaacccct 300

aacagcgaga acggcatatg ctacccaggc gacttcatcg actacgagga actgcgcgag 360

cagctgagct ctgtgtcgag cttcgagcgg ttcgagatct tccctaagga atctagctgg 420

cctaatcata acacaaatgg cgttactgct gcctgtagcc acgagggaaa gagcagtttc 480

taccggaatc tgctgtggct gacagagaag gagggctcct accctaagct gaagaatagc 540

tatgtgaaca agaagggcaa ggaggtgctg gtgctgtggg gaatacacca cccacctaac 600

tcgaaggagc agcagaatct gtaccagaat gagaatgcct acgtgtccgt cgtgacctcc 660

aactacaacc ggcggttcac gcctgagatc gccgagaggc ctaaggtgag ggaccaggcc 720

ggacgcatga actactactg gaccctgctg aagcctggcg atacaatcat cttcgaggct 780

aatggaaacc tgatcgcgcc aatgtacgcc ttcgccctgt ccagaggatt cggcagcggc 840

atcatcacat ccaacgcctc catgcacgaa tgcaacacca agtgccagac gcctctggga 900

gctatcaata gcagcttgcc ttaccagaat atccaccctg tgaccattgg agagtgtcca 960

aagtacgtgc gcagcgcaaa gctgcggatg gtcacaggcc tgcggaatat accttctatc 1020

cagagccgag gcctgttcgg tgccattgcc ggcttcatcg agggtggctg gaccggaatg 1080

atcgacggct ggtatggata ccaccaccag aatgaacagg gcagcggcta cgccgccgat 1140

cagaagtcca cccagaacgc aatcaatggt atcacaaaca aggtgaacac tgtaatcgag 1200

aagatgaaca tccaattcac agccgtgggc aaggagttca ataagctgga gaagcggatg 1260

gagaacctca acaagaaggt ggacgacggc ttcctggata tctggaccta caacgcagag 1320

ctgctggtgt tgctggagaa cgagagaacc ctcgacttcc atgatagcaa cgttaagaac 1380

ctatacgaga aggtgaagtc acagctgaag aataacgcca aggagattgg caacggctgc 1440

ttcgaattct accacaagtg cgacaacgag tgtatggaga gcgtccggaa tggcacctac 1500

gactatccta agtatagcga ggagagcaag cttaatagag agaaggtcga tggcgtgaag 1560

ctggagtcaa tgggaatcta ccagatcctg gctatttatt caaccgtggc atcaagtctg 1620

gtgcttctgg tcagcctggg cgccatcagc ttctggatgt gctccaatgg cagcctgcaa 1680

tgccgcatct gcata 1695

<210> 82
<211> 1698
<212> DNA
<213> Artificial Sequence

<220>
<223> FLHA_Cal09

<400> 82
atgaaggcta tcttggtggt gttgttgtac acattcgcca ccgccaacgc cgacaccctc 60

tgcatcggct accacgcgaa caattcaacc gacaccgttg acaccgtcct cgagaagaac 120

gtgaccgtga ctcatagcgt caacctcctc gaggacaagc ataacggcaa gctctgtaag 180

cttagaggag tggcccctct ccacctgggc aagtgtaaca ttgcaggctg gatcctgggc 240

aaccctgagt gcgagagcct gtcaaccgct agcagctgga gctacatcgt ggaaacccca 300

tccagcgata acggcacctg ctaccctggc gatttcatcg actacgagga gctgcgcgag 360

cagttgagca gcgtctccag cttcgagaga ttcgagatct tccctaagac tagcagctgg 420

cctaatcatg actccaataa gggcgtgacg gccgcctgtc ctcacgctgg agccaagtcg 480

ttctacaaga acctgatctg gctggtaaag aagggcaaca gctacccaaa gctgagcaag 540

tcctacatca acgacaaggg caaggaagtg ctggtgctgt ggggaatcca tcacccaagc 600

acctctgcgg accagcagtc tctgtatcag aacgccgaca cctatgtgtt cgtaggctcc 660

tccagatact ccaagaagtt caagccagag attgctatcc gcccaaaggt gcgggatcaa 720

gagggtcgca tgaattatta ctggaccctg gtcgagccag gcgataagat cacattcgaa 780

gccacgggaa atctggtggt gcctagatac gctttcgcca tggagagaaa cgccggcagc 840

ggcatcatca tatccgacac acctgtgcac gactgcaaca caacatgcca gacgccaaag 900

ggagccatca acacatctct tccattccag aacattcacc caatcacaat cggcaagtgt 960

ccaaagtacg tgaagtccac caagcttaga ctggccaccg gcctgcgtaa catccctagc 1020

atccagtcga gaggcctctt cggcgccatc gccggattca ttgaaggtgg ctggaccggc 1080

atggtggacg gttggtatgg ctaccaccac cagaacgagc agggcagcgg ctacgccgcg 1140

gacctgaagt ccacccagaa cgctattgac gagatcacca acaaggtgaa cagcgtgatc 1200

gagaagatga atacccagtt caccgccgtc ggcaaggagt tcaaccatct ggagaagaga 1260

atcgagaacc tcaacaagaa ggtcgacgac ggcttcctgg acatttggac ttacaacgct 1320

gagttgttgg tgcttcttga gaatgagcgg accctggact atcacgactc aaatgtgaag 1380

aacctgtacg agaaggtgag atcccagctg aagaacaatg ctaaggaaat cggcaacggc 1440

tgcttcgagt tctatcataa gtgtgacaac acctgcatgg agtctgttaa gaacggcaca 1500

tacgactacc cgaagtactc tgaggaggcc aagctgaacc gagaggagat agacggcgtt 1560

aagctagaaa gtacaaggat ctaccagatc cttgccatct actccaccgt ggcctccagc 1620

ctggtgttgg tggtgagcct gggcgccatc agcttctgga tgtgcagtaa cggaagccta 1680

cagtgccgaa tctgcatc 1698

<210> 83
<211> 585
<212> PRT
<213> Artificial Sequence

<220>
<223> MRK_B_consUnique

<400>  83

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
            20                  25                  30

Thr Ala Thr Gln Gly Glu Val Asn Val Thr Gly Val Ile Pro Leu Thr
        35                  40                  45

Thr Thr Pro Thr Lys Ser His Phe Ala Asn Leu Lys Gly Thr Arg Thr
    50                  55                  60

Arg Gly Lys Leu Cys Pro Asp Cys Leu Asn Cys Thr Asp Leu Asp Val
65                  70                  75                  80

Ala Leu Gly Arg Pro Met Cys Val Gly Thr Thr Pro Ser Ala Lys Ala
                85                  90                  95

Ser Ile Leu His Glu Val Arg Pro Val Thr Ser Gly Cys Phe Pro Ile
            100                 105                 110

Met His Asp Arg Thr Lys Ile Arg Gln Leu Pro Asn Leu Leu Arg Gly
        115                 120                 125

Tyr Glu His Ile Arg Leu Ser Thr Gln Asn Val Ile Asp Ala Glu Lys
    130                 135                 140

Ala Pro Gly Gly Pro Tyr Arg Leu Gly Thr Ser Gly Ser Cys Pro Asn
145                 150                 155                 160

Ala Thr Ser Lys Asn Gly Phe Phe Ala Thr Met Ala Trp Ala Val Pro
                165                 170                 175

Lys Asn Asp Asn Asn Lys Asn Ala Thr Asn Pro Leu Thr Val Glu Val
            180                 185                 190

Pro Tyr Ile Cys Thr Glu Gly Glu Asp Gln Ile Thr Val Trp Gly Phe
        195                 200                 205

His Ser Asp Asn Lys Thr Gln Met Lys Lys Leu Tyr Gly Asp Ser Asn
    210                 215                 220

Pro Gln Lys Phe Thr Ser Ser Ala Asn Gly Val Thr Thr His Tyr Val
225                 230                 235                 240

Ser Gln Ile Gly Gly Phe Pro Asp Gln Thr Glu Asp Gly Gly Leu Pro

245                 250                 255

Gln Ser Gly Arg Ile Val Val Asp Tyr Met Val Gln Lys Pro Gly Lys
            260                 265                 270

Thr Gly Thr Ile Val Tyr Gln Arg Gly Val Leu Leu Pro Gln Lys Val
        275                 280                 285

Trp Cys Ala Ser Gly Arg Ser Lys Val Ile Lys Gly Ser Leu Pro Leu
    290                 295                 300

Ile Gly Glu Ala Asp Cys Leu His Glu Lys Tyr Gly Gly Leu Asn Lys
305                 310                 315                 320

Ser Lys Pro Tyr Tyr Thr Gly Glu His Ala Lys Ala Ile Gly Asn Cys
                325                 330                 335

Pro Ile Trp Val Lys Thr Pro Leu Lys Leu Ala Asn Gly Thr Lys Tyr
            340                 345                 350

Arg Pro Pro Ala Lys Leu Leu Lys Glu Arg Gly Phe Phe Gly Ala Ile
        355                 360                 365

Ala Gly Phe Leu Glu Gly Gly Trp Glu Gly Met Ile Ala Gly Trp His
    370                 375                 380

Gly Tyr Thr Ser His Gly Ala His Gly Val Ala Val Ala Ala Asp Leu
385                 390                 395                 400

Lys Ser Thr Gln Glu Ala Ile Asn Lys Ile Thr Lys Asn Leu Asn Ser
                405                 410                 415

Leu Ser Glu Leu Glu Val Lys Asn Leu Gln Arg Leu Ser Gly Ala Met
            420                 425                 430

Asp Glu Leu His Asn Glu Ile Leu Glu Leu Asp Glu Lys Val Asp Asp
        435                 440                 445

Leu Arg Ala Asp Thr Ile Ser Ser Gln Ile Glu Leu Ala Val Leu Leu
    450                 455                 460

Ser Asn Glu Gly Ile Ile Asn Ser Glu Asp Glu His Leu Leu Ala Leu
465                 470                 475                 480

Glu Arg Lys Leu Lys Lys Met Leu Gly Pro Ser Ala Val Asp Ile Gly
                485                 490                 495

Asn Gly Cys Phe Glu Thr Lys His Lys Cys Asn Gln Thr Cys Leu Asp
            500                 505                 510

Arg Ile Ala Ala Gly Thr Phe Asn Ala Gly Glu Phe Ser Leu Pro Thr
        515                 520                 525

Phe Asp Ser Leu Asn Ile Thr Ala Ala Ser Leu Asn Asp Asp Gly Leu
    530                 535                 540

Asp Asn His Thr Ile Leu Leu Tyr Tyr Ser Thr Ala Ala Ser Ser Leu
545                 550                 555                 560

Ala Val Thr Leu Met Ile Ala Ile Phe Ile Val Tyr Met Val Ser Arg
                565                 570                 575

Asp Asn Val Ser Cys Ser Ile Cys Leu
            580                 585

<210>  84
<211>  584
<212>  PRT
<213>  Artificial Sequence

<220>
<223>  MRK_B_ConsensusA

<400>  84

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
            20                  25                  30

Thr Ala Thr Gln Gly Glu Val Asn Val Thr Gly Val Ile Pro Leu Thr
        35                  40                  45

Thr Thr Pro Thr Lys Ser Tyr Phe Ala Asn Leu Lys Gly Thr Arg Thr
    50                  55                  60

Arg Gly Lys Leu Cys Pro Asp Cys Leu Asn Cys Thr Asp Leu Asp Val
65                  70                  75                  80

Ala Leu Gly Arg Pro Met Cys Val Gly Thr Thr Pro Ser Ala Lys Ala
                85                  90                  95

Ser Ile Leu His Glu Val Arg Pro Val Thr Ser Gly Cys Phe Pro Ile
            100                 105                 110

Met His Asp Arg Thr Lys Ile Arg Gln Leu Pro Asn Leu Leu Arg Gly

        115                 120                 125

Tyr Glu Asn Ile Arg Leu Ser Thr Gln Asn Val Ile Asp Ala Glu Lys
    130                 135                 140

Ala Pro Gly Gly Pro Tyr Arg Leu Gly Thr Ser Gly Ser Cys Pro Asn
145                 150                 155                 160

Ala Thr Ser Lys Ile Gly Phe Phe Ala Thr Met Ala Trp Ala Val Pro
                165                 170                 175

Lys Asp Asn Tyr Lys Asn Ala Thr Asn Pro Leu Thr Val Glu Val Pro
            180                 185                 190

Tyr Ile Cys Thr Glu Gly Glu Asp Gln Ile Thr Val Trp Gly Phe His
        195                 200                 205

Ser Asp Asn Lys Thr Gln Met Lys Asn Leu Tyr Gly Asp Ser Asn Pro
    210                 215                 220

Gln Lys Phe Thr Ser Ser Ala Asn Gly Val Thr Thr His Tyr Val Ser
225                 230                 235                 240

Gln Ile Gly Asp Phe Pro Asp Gln Thr Glu Asp Gly Gly Leu Pro Gln
                245                 250                 255

Ser Gly Arg Ile Val Val Asp Tyr Met Met Gln Lys Pro Gly Lys Thr
            260                 265                 270

Gly Thr Ile Val Tyr Gln Arg Gly Val Leu Leu Pro Gln Lys Val Trp
        275                 280                 285

Cys Ala Ser Gly Arg Ser Lys Val Ile Lys Gly Ser Leu Pro Leu Ile
    290                 295                 300

Gly Glu Ala Asp Cys Leu His Glu Lys Tyr Gly Gly Leu Asn Lys Ser
305                 310                 315                 320

Lys Pro Tyr Tyr Thr Gly Glu His Ala Lys Ala Ile Gly Asn Cys Pro
                325                 330                 335

Ile Trp Val Lys Thr Pro Leu Lys Leu Ala Asn Gly Thr Lys Tyr Arg
            340                 345                 350

Pro Pro Ala Lys Leu Leu Lys Glu Arg Gly Phe Phe Gly Ala Ile Ala
        355                 360                 365

Gly Phe Leu Glu Gly Gly Trp Glu Gly Met Ile Ala Gly Trp His Gly
    370                 375                 380

Tyr Thr Ser His Gly Ala His Gly Val Ala Val Ala Ala Asp Leu Lys
385                 390                 395                 400

Ser Thr Gln Glu Ala Ile Asn Lys Ile Thr Lys Asn Leu Asn Ser Leu
                405                 410                 415

Ser Glu Leu Glu Val Lys Asn Leu Gln Arg Leu Ser Gly Ala Met Asp
            420                 425                 430

Glu Leu His Asn Glu Ile Leu Glu Leu Asp Glu Lys Val Asp Asp Leu
        435                 440                 445

Arg Ala Asp Thr Ile Ser Ser Gln Ile Glu Leu Ala Val Leu Leu Ser
    450                 455                 460

Asn Glu Gly Ile Ile Asn Ser Glu Asp Glu His Leu Leu Ala Leu Glu
465                 470                 475                 480

Arg Lys Leu Lys Lys Met Leu Gly Pro Ser Ala Val Asp Ile Gly Asn
                485                 490                 495

Gly Cys Phe Glu Thr Lys His Lys Cys Asn Gln Thr Cys Leu Asp Arg
            500                 505                 510

Ile Ala Ala Gly Thr Phe Asn Ala Gly Glu Phe Ser Leu Pro Thr Phe
        515                 520                 525

Asp Ser Leu Asn Ile Thr Ala Ala Ser Leu Asn Asp Asp Gly Leu Asp
    530                 535                 540

Asn His Thr Ile Leu Leu Tyr Tyr Ser Thr Ala Ala Ser Ser Leu Ala
545                 550                 555                 560

Val Thr Leu Met Leu Ala Ile Phe Ile Val Tyr Met Val Ser Arg Asp
                565                 570                 575

Asn Val Ser Cys Ser Ile Cys Leu
            580

<210>  85
<211>  585
<212>  PRT
<213>  Artificial Sequence

<220>
<223>  MRK_B_ConsensusB

<400>  85

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
            20                  25                  30

Thr Ala Thr Gln Gly Glu Val Asn Val Thr Gly Val Ile Pro Leu Thr
        35                  40                  45

Thr Thr Pro Thr Lys Ser His Phe Ala Asn Leu Lys Gly Thr Glu Thr
    50                  55                  60

Arg Gly Lys Leu Cys Pro Lys Cys Leu Asn Cys Thr Asp Leu Asp Val
65                  70                  75                  80

Ala Leu Gly Arg Pro Lys Cys Thr Gly Lys Ile Pro Ser Ala Arg Val
                85                  90                  95

Ser Ile Leu His Glu Val Arg Pro Val Thr Ser Gly Cys Phe Pro Ile
            100                 105                 110

Met His Asp Arg Thr Lys Ile Arg Gln Leu Pro Asn Leu Leu Arg Gly
        115                 120                 125

Tyr Glu His Ile Arg Leu Ser Thr His Asn Val Ile Asn Ala Glu Asn
    130                 135                 140

Ala Pro Gly Gly Pro Tyr Lys Ile Gly Thr Ser Gly Ser Cys Pro Asn
145                 150                 155                 160

Val Thr Asn Gly Asn Gly Phe Phe Ala Thr Met Ala Trp Ala Val Pro
                165                 170                 175

Lys Asn Asp Lys Asn Lys Thr Ala Thr Asn Pro Leu Thr Ile Glu Val
            180                 185                 190

Pro Tyr Ile Cys Thr Glu Gly Glu Asp Gln Ile Thr Val Trp Gly Phe
        195                 200                 205

His Ser Asp Asn Glu Thr Gln Met Ala Lys Leu Tyr Gly Asp Ser Lys
    210                 215                 220

Pro Gln Lys Phe Thr Ser Ser Ala Asn Gly Val Thr Thr His Tyr Val
225                 230                 235                 240

Ser Gln Ile Gly Gly Phe Pro Asn Gln Thr Glu Asp Gly Gly Leu Pro
                245                 250                 255

Gln Ser Gly Arg Ile Val Val Asp Tyr Met Val Gln Lys Ser Gly Lys
            260                 265                 270

Thr Gly Thr Ile Thr Tyr Gln Arg Gly Ile Leu Leu Pro Gln Lys Val
        275                 280                 285

Trp Cys Ala Ser Gly Arg Ser Lys Val Ile Lys Gly Ser Leu Pro Leu
    290                 295                 300

Ile Gly Glu Ala Asp Cys Leu His Glu Lys Tyr Gly Gly Leu Asn Lys
305                 310                 315                 320

Ser Lys Pro Tyr Tyr Thr Gly Glu His Ala Lys Ala Ile Gly Asn Cys
                325                 330                 335

Pro Ile Trp Val Lys Thr Pro Leu Lys Leu Ala Asn Gly Thr Lys Tyr
            340                 345                 350

Arg Pro Pro Ala Lys Leu Leu Lys Glu Arg Gly Phe Phe Gly Ala Ile
        355                 360                 365

Ala Gly Phe Leu Glu Gly Gly Trp Glu Gly Met Ile Ala Gly Trp His
    370                 375                 380

Gly Tyr Thr Ser His Gly Ala His Gly Val Ala Val Ala Ala Asp Leu
385                 390                 395                 400

Lys Ser Thr Gln Glu Ala Ile Asn Lys Ile Thr Lys Asn Leu Asn Ser
                405                 410                 415

Leu Ser Glu Leu Glu Val Lys Asn Leu Gln Arg Leu Ser Gly Ala Met
            420                 425                 430

Asp Glu Leu His Asn Glu Ile Leu Glu Leu Asp Glu Lys Val Asp Asp
        435                 440                 445

Leu Arg Ala Asp Thr Ile Ser Ser Gln Ile Glu Leu Ala Val Leu Leu
    450                 455                 460

Ser Asn Glu Gly Ile Ile Asn Ser Glu Asp Glu His Leu Leu Ala Leu
465                 470                 475                 480

Glu Arg Lys Leu Lys Lys Met Leu Gly Pro Ser Ala Val Glu Ile Gly
                485                 490                 495

Asn Gly Cys Phe Glu Thr Lys His Lys Cys Asn Gln Thr Cys Leu Asp
                500                 505                 510

Arg Ile Ala Ala Gly Thr Phe Asp Ala Gly Glu Phe Ser Leu Pro Thr
        515                 520                 525

Phe Asp Ser Leu Asn Ile Thr Ala Ala Ser Leu Asn Asp Asp Gly Leu
    530                 535                 540

Asp Asn His Thr Ile Leu Leu Tyr Tyr Ser Thr Ala Ala Ser Ser Leu
545                 550                 555                 560

Ala Val Thr Leu Met Ile Ala Ile Phe Val Val Tyr Met Val Ser Arg
                565                 570                 575

Asp Asn Val Ser Cys Ser Ile Cys Leu
                580                 585

<210>  86
<211>  585
<212>  PRT
<213>  Artificial Sequence

<220>
<223>  MRK_B_cot_AA

<400>  86

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
                20                  25                  30

Thr Ala Thr Gln Gly Glu Val Asn Val Thr Gly Val Ile Pro Leu Thr
        35                  40                  45

Thr Thr Pro Thr Lys Ser Tyr Phe Ala Asn Leu Lys Gly Thr Arg Thr
    50                  55                  60

Arg Gly Lys Leu Cys Pro Asp Cys Leu Asn Cys Thr Asp Leu Asp Val
65                  70                  75                  80

Ala Leu Gly Arg Pro Met Cys Val Gly Thr Thr Pro Ser Ala Lys Ala
                85                  90                  95

Ser Ile Leu His Glu Val Arg Pro Val Thr Ser Gly Cys Phe Pro Ile
                100                 105                 110

Met His Asp Arg Thr Lys Ile Arg Gln Leu Pro Asn Leu Leu Arg Gly
        115                 120                 125

Tyr Glu Asn Ile Arg Leu Ser Thr Gln Asn Val Ile Asp Ala Glu Lys
    130                 135                 140

Ala Pro Gly Gly Pro Tyr Arg Leu Gly Thr Ser Gly Ser Cys Pro Asn
145                 150                 155                 160

Ala Thr Ser Lys Ser Gly Phe Phe Ala Thr Met Ala Trp Ala Val Pro
                165                 170                 175

Lys Asn Asp Asn Asn Lys Asn Ala Thr Asn Pro Leu Thr Val Glu Val
            180                 185                 190

Pro Tyr Ile Cys Thr Glu Gly Glu Asp Gln Ile Thr Val Trp Gly Phe
        195                 200                 205

His Ser Asp Asn Lys Thr Gln Met Lys Asn Leu Tyr Gly Asp Ser Asn
    210                 215                 220

Pro Gln Lys Phe Thr Ser Ser Ala Asn Gly Val Thr Thr His Tyr Val
225                 230                 235                 240

Ser Gln Ile Gly Gly Phe Pro Asp Gln Thr Glu Asp Gly Gly Leu Pro
                245                 250                 255

Gln Ser Gly Arg Ile Val Val Asp Tyr Met Met Gln Lys Pro Gly Lys
            260                 265                 270

Thr Gly Thr Ile Val Tyr Gln Arg Gly Val Leu Leu Pro Gln Lys Val
        275                 280                 285

Trp Cys Ala Ser Gly Arg Ser Lys Val Ile Lys Gly Ser Leu Pro Leu
    290                 295                 300

Ile Gly Glu Ala Asp Cys Leu His Glu Lys Tyr Gly Gly Leu Asn Lys
305                 310                 315                 320

Ser Lys Pro Tyr Tyr Thr Gly Glu His Ala Lys Ala Ile Gly Asn Cys
                325                 330                 335

Pro Ile Trp Val Lys Thr Pro Leu Lys Leu Ala Asn Gly Thr Lys Tyr
            340                 345                 350

Arg Pro Pro Ala Lys Leu Leu Lys Glu Arg Gly Phe Phe Gly Ala Ile
        355                 360                 365

Ala Gly Phe Leu Glu Gly Gly Trp Glu Gly Met Ile Ala Gly Trp His
    370                 375                 380

Gly Tyr Thr Ser His Gly Ala His Gly Val Ala Val Ala Ala Asp Leu
385                 390                 395                 400

Lys Ser Thr Gln Glu Ala Ile Asn Lys Ile Thr Lys Asn Leu Asn Ser
                405                 410                 415

Leu Ser Glu Leu Glu Val Lys Asn Leu Gln Arg Leu Ser Gly Ala Met
            420                 425                 430

Asp Glu Leu His Asn Glu Ile Leu Glu Leu Asp Glu Lys Val Asp Asp
        435                 440                 445

Leu Arg Ala Asp Thr Ile Ser Ser Gln Ile Glu Leu Ala Val Leu Leu
    450                 455                 460

Ser Asn Glu Gly Ile Ile Asn Ser Glu Asp Glu His Leu Leu Ala Leu
465                 470                 475                 480

Glu Arg Lys Leu Lys Lys Met Leu Gly Pro Ser Ala Val Asp Ile Gly
                485                 490                 495

Asn Gly Cys Phe Glu Thr Lys His Lys Cys Asn Gln Thr Cys Leu Asp
            500                 505                 510

Arg Ile Ala Ala Gly Thr Phe Asn Ala Gly Glu Phe Ser Leu Pro Thr
        515                 520                 525

Phe Asp Ser Leu Asn Ile Thr Ala Ala Ser Leu Asn Asp Asp Gly Leu
    530                 535                 540

Asp Asn His Thr Ile Leu Leu Tyr Tyr Ser Thr Ala Ala Ser Ser Leu
545                 550                 555                 560

Ala Val Thr Leu Met Leu Ala Ile Phe Ile Val Tyr Met Val Ser Arg
                565                 570                 575

Asp Asn Val Ser Cys Ser Ile Cys Leu
            580                 585

<210>  87
<211>  585
<212>  PRT
<213>  Artificial Sequence

<220>

<223>  MRK_B_COT_A

<400>  87

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
            20                  25                  30

Thr Ala Thr Gln Gly Glu Val Asn Val Thr Gly Val Ile Pro Leu Thr
        35                  40                  45

Thr Thr Pro Thr Lys Ser His Phe Ala Asn Leu Lys Gly Thr Glu Thr
    50                  55                  60

Arg Gly Lys Leu Cys Pro Lys Cys Leu Asn Cys Thr Asp Leu Asp Val
65                  70                  75                  80

Ala Leu Gly Arg Pro Lys Cys Thr Gly Lys Ile Pro Ser Ala Arg Val
                85                  90                  95

Ser Ile Leu His Glu Val Arg Pro Val Thr Ser Gly Cys Phe Pro Ile
            100                 105                 110

Met His Asp Arg Thr Lys Ile Arg Gln Leu Pro Asn Leu Leu Arg Gly
        115                 120                 125

Tyr Glu His Ile Arg Leu Ser Thr His Asn Val Ile Asn Ala Glu Asn
    130                 135                 140

Ala Pro Gly Gly Pro Tyr Lys Ile Gly Thr Ser Gly Ser Cys Pro Asn
145                 150                 155                 160

Val Thr Asn Gly Asn Gly Phe Phe Ala Thr Met Ala Trp Ala Val Pro
                165                 170                 175

Lys Asn Asp Lys Asn Lys Thr Ala Thr Asn Pro Leu Thr Ile Glu Val
            180                 185                 190

Pro Tyr Ile Cys Thr Glu Gly Glu Asp Gln Ile Thr Val Trp Gly Phe
        195                 200                 205

His Ser Asp Asn Glu Thr Gln Met Ala Lys Leu Tyr Gly Asp Ser Lys
    210                 215                 220

Pro Gln Lys Phe Thr Ser Ser Ala Asn Gly Val Thr Thr His Tyr Val
225                 230                 235                 240

Ser Gln Ile Gly Gly Phe Pro Asn Gln Thr Glu Asp Gly Gly Leu Pro
                245                 250                 255

Gln Ser Gly Arg Ile Val Val Asp Tyr Met Val Gln Lys Ser Gly Lys
            260                 265                 270

Thr Gly Thr Ile Thr Tyr Gln Arg Gly Ile Leu Leu Pro Gln Lys Val
        275                 280                 285

Trp Cys Ala Ser Gly Arg Ser Lys Val Ile Lys Gly Ser Leu Pro Leu
    290                 295                 300

Ile Gly Glu Ala Asp Cys Leu His Glu Lys Tyr Gly Gly Leu Asn Lys
305                 310                 315                 320

Ser Lys Pro Tyr Tyr Thr Gly Glu His Ala Lys Ala Ile Gly Asn Cys
                325                 330                 335

Pro Ile Trp Val Lys Thr Pro Leu Lys Leu Ala Asn Gly Thr Lys Tyr
            340                 345                 350

Arg Pro Pro Ala Lys Leu Leu Lys Glu Arg Gly Phe Phe Gly Ala Ile
        355                 360                 365

Ala Gly Phe Leu Glu Gly Gly Trp Glu Gly Met Ile Ala Gly Trp His
    370                 375                 380

Gly Tyr Thr Ser His Gly Ala His Gly Val Ala Val Ala Ala Asp Leu
385                 390                 395                 400

Lys Ser Thr Gln Glu Ala Ile Asn Lys Ile Thr Lys Asn Leu Asn Ser
                405                 410                 415

Leu Ser Glu Leu Glu Val Lys Asn Leu Gln Arg Leu Ser Gly Ala Met
            420                 425                 430

Asp Glu Leu His Asn Glu Ile Leu Glu Leu Asp Glu Lys Val Asp Asp
        435                 440                 445

Leu Arg Ala Asp Thr Ile Ser Ser Gln Ile Glu Leu Ala Val Leu Leu
    450                 455                 460

Ser Asn Glu Gly Ile Ile Asn Ser Glu Asp Glu His Leu Leu Ala Leu
465                 470                 475                 480

Glu Arg Lys Leu Lys Lys Met Leu Gly Pro Ser Ala Val Glu Ile Gly
                485                 490                 495

Asn Gly Cys Phe Glu Thr Lys His Lys Cys Asn Gln Thr Cys Leu Asp
            500                 505                 510

Arg Ile Ala Ala Gly Thr Phe Asp Ala Gly Glu Phe Ser Leu Pro Thr
        515                 520                 525

Phe Asp Ser Leu Asn Ile Thr Ala Ala Ser Leu Asn Asp Asp Gly Leu
    530                 535                 540

Asp Asn His Thr Ile Leu Leu Tyr Tyr Ser Thr Ala Ala Ser Ser Leu
545                 550                 555                 560

Ala Val Thr Leu Met Ile Ala Ile Phe Val Val Tyr Met Val Ser Arg
                565                 570                 575

Asp Asn Val Ser Cys Ser Ile Cys Leu
            580                 585

<210> 88
<211> 584
<212> PRT
<213> Artificial Sequence

<220>
<223> MRK_B_COT_B

<400> 88

Met Lys Ala Ile Ile Val Leu Leu Met Val Val Thr Ser Asn Ala Asp
1               5                   10                  15

Arg Ile Cys Thr Gly Ile Thr Ser Ser Asn Ser Pro His Val Val Lys
            20                  25                  30

Thr Ala Thr Gln Gly Glu Val Asn Val Thr Gly Val Ile Pro Leu Thr
        35                  40                  45

Thr Thr Pro Thr Lys Ser Tyr Phe Ala Asn Leu Lys Gly Thr Arg Thr
    50                  55                  60

Arg Gly Lys Leu Cys Pro Asp Cys Leu Asn Cys Thr Asp Leu Asp Val
65                  70                  75                  80

Ala Leu Gly Arg Pro Met Cys Val Gly Thr Thr Pro Ser Ala Lys Ala
                85                  90                  95

Ser Ile Leu His Glu Val Arg Pro Val Thr Ser Gly Cys Phe Pro Ile
            100                 105                 110

Met His Asp Arg Thr Lys Ile Arg Gln Leu Pro Asn Leu Leu Arg Gly
        115                 120                 125

Tyr Glu Asn Ile Arg Leu Ser Thr Gln Asn Val Ile Asp Ala Glu Lys
    130                 135                 140

Ala Pro Gly Gly Pro Tyr Arg Leu Gly Thr Ser Gly Ser Cys Pro Asn
145                 150                 155                 160

Ala Thr Ser Lys Ile Gly Phe Phe Ala Thr Met Ala Trp Ala Val Pro
                165                 170                 175

Lys Asp Asn Tyr Lys Asn Ala Thr Asn Pro Leu Thr Val Glu Val Pro
            180                 185                 190

Tyr Ile Cys Thr Glu Gly Glu Asp Gln Ile Thr Val Trp Gly Phe His
        195                 200                 205

Ser Asp Asn Lys Thr Gln Met Lys Ser Leu Tyr Gly Asp Ser Asn Pro
    210                 215                 220

Gln Lys Phe Thr Ser Ser Ala Asn Gly Val Thr Thr His Tyr Val Ser
225                 230                 235                 240

Gln Ile Gly Asp Phe Pro Asp Gln Thr Glu Asp Gly Gly Leu Pro Gln
                245                 250                 255

Ser Gly Arg Ile Val Val Asp Tyr Met Met Gln Lys Pro Gly Lys Thr
            260                 265                 270

Gly Thr Ile Val Tyr Gln Arg Gly Val Leu Leu Pro Gln Lys Val Trp
        275                 280                 285

Cys Ala Ser Gly Arg Ser Lys Val Ile Lys Gly Ser Leu Pro Leu Ile
    290                 295                 300

Gly Glu Ala Asp Cys Leu His Glu Lys Tyr Gly Gly Leu Asn Lys Ser
305                 310                 315                 320

Lys Pro Tyr Tyr Thr Gly Glu His Ala Lys Ala Ile Gly Asn Cys Pro
                325                 330                 335

Ile Trp Val Lys Thr Pro Leu Lys Leu Ala Asn Gly Thr Lys Tyr Arg
            340                 345                 350

Pro Pro Ala Lys Leu Leu Lys Glu Arg Gly Phe Phe Gly Ala Ile Ala
        355                 360                 365

Gly Phe Leu Glu Gly Gly Trp Glu Gly Met Ile Ala Gly Trp His Gly
    370                 375                 380

Tyr Thr Ser His Gly Ala His Gly Val Ala Val Ala Ala Asp Leu Lys
385                 390                 395                 400

Ser Thr Gln Glu Ala Ile Asn Lys Ile Thr Lys Asn Leu Asn Ser Leu
                405                 410                 415

Ser Glu Leu Glu Val Lys Asn Leu Gln Arg Leu Ser Gly Ala Met Asp
            420                 425                 430

Glu Leu His Asn Glu Ile Leu Glu Leu Asp Glu Lys Val Asp Asp Leu
        435                 440                 445

Arg Ala Asp Thr Ile Ser Ser Gln Ile Glu Leu Ala Val Leu Leu Ser
    450                 455                 460

Asn Glu Gly Ile Ile Asn Ser Glu Asp Glu His Leu Leu Ala Leu Glu
465                 470                 475                 480

Arg Lys Leu Lys Lys Met Leu Gly Pro Ser Ala Val Asp Ile Gly Asn
                485                 490                 495

Gly Cys Phe Glu Thr Lys His Lys Cys Asn Gln Thr Cys Leu Asp Arg
            500                 505                 510

Ile Ala Ala Gly Thr Phe Asn Ala Gly Glu Phe Ser Leu Pro Thr Phe
        515                 520                 525

Asp Ser Leu Asn Ile Thr Ala Ala Ser Leu Asn Asp Asp Gly Leu Asp
    530                 535                 540

Asn His Thr Ile Leu Leu Tyr Tyr Ser Thr Ala Ala Ser Ser Leu Ala
545                 550                 555                 560

Val Thr Leu Met Leu Ala Ile Phe Ile Val Tyr Met Val Ser Arg Asp
                565                 570                 575

Asn Val Ser Cys Ser Ile Cys Leu
            580

## Description

### SEQUENCE LISTING

The instant application contains a Sequence Listing which has been submitted electronically in ASCII format and is hereby incorporated by reference in its entirety. Said ASCII copy, created October 10, 2017, is named 24378-US-PCT_SL.txt and is 277 kilobytes in size.

### FIELD OF THE INVENTION

The present invention relates to influenza virus hemagglutinin protein and DNA vaccines, as well as methods of using the vaccines and compositions comprising the vaccines.

### BACKGROUND OF INVENTION

Influenza viruses are members of the orthomyxoviridae family, and are classified into three distinct types (A, B, and C), based on antigenic differences between their nucleoprotein (NP) and matrix (M) protein. The orthomyxoviruses are enveloped animal viruses of approximately 100 nm in diameter. The influenza virions consist of an internal ribonucleoprotein core (a helical nucleocapsid) containing a single-stranded RNA genome, and an outer lipoprotein envelope lined inside by a matrix protein (M1). The segmented genome of influenza A and B viruses consists of eight molecules (seven for influenza C virus) of linear, negative polarity, single-stranded RNAs, which encode several polypeptides including: the RNA-directed RNA polymerase proteins (PB2, PB1 and PA) and nucleoprotein (NP), which form the nucleocapsid; the matrix proteins (M1, M2, which is also a surface-exposed protein embedded in the virus membrane); two surface glycoproteins, which project from the lipoprotein envelope: hemagglutinin (HA) and neuraminidase (NA); and nonstructural proteins (NS1 and NS2). Transcription and replication of the genome takes place in the nucleus and assembly takes place at the plasma membrane.

Hemagglutinin is the major envelope glycoprotein of influenza A and B viruses, and hemagglutinin-esterase (HE) of influenza C viruses is a protein homologous to HA. The rapid evolution of the HA protein of the influenza virus results in the constant emergence of new strains, rendering the adaptive immune response of the host only partially protective to new infections. The amino acid sequence of the stem region of the hemagglutinin protein is highly conserved across types, sub-types and strains of influenza viruses and contains a site of vulnerability for group 1 viruses. Thus, an immune response directed to this region of the HA protein may protect individuals against influenza viruses from several types, sub-types and/or strains.

Ferritin, an iron storage protein found in almost all living organisms, is an example which has been extensively studied and engineered for a number of potential biochemical/biomedical purposes [Iwahori, K. U.S. Patent 2009/0233377 (2009); Meldrum, F. C. et al. Science 257, 522-523 (1992); Naitou, M. et al. U.S. Patent 2011/0038025 (2011); Yamashita, I. Biochim Biophys Acta 1800, 846-857 (2010)]. Further, the molecular architecture of ferritin, which consists of 24 subunits assembling into an octahedral cage with 432 symmetry, has the potential to display multimeric antigens on its surface.

There are eighteen known HA serotypes and nine known NA serotypes for Influenza A viruses. The identity of the different serotypes present in a viral particle typically is used to describe a virus. For example, H1N1 is an influenza virus with HA serotype H1 and NA serotype N1; H5N1 is an influenza virus with HA serotype H5 and NA serotype N1. Only H1, H2 and H3 serotypes, and N1 and N2 serotypes usually infect humans.

Influenza strains are generally species or genus specific; i.e. an influenza strain which can infect pigs (a swine influenza virus) typically does not infect humans or birds; an influenza strain which can infect birds (an avian influenza virus) typically does not infect humans or pigs; and an influenza strain which can infect humans (a human influenza virus) does not infect birds or pigs. Influenza strains, however, can mutate and become infective from one species to another. For example, a strain which only infects pigs, a swine influenza, can mutate or recombine to become a strain that can infect humans only or both pigs and humans. A flu virus commonly referred to as "swine flu" is an influenza virus strain, such as an H1N1 strain, which can infect humans and which was derived from a strain that was previously specific for pigs (i.e. a swine flu virus is a swine origin human influenza or swine derived human influenza). A flu virus commonly referred to as "bird flu" is an influenza virus strain, such as an H5N1 strain, which can infect humans and which was derived from a strain that was previously specific for birds (i.e. a bird flu virus avian origin human influenza or avian derived human influenza).

The biggest challenge for therapy and prophylaxis against influenza and other infections using traditional vaccines is the limitation of vaccines in breadth, providing protection only against closely related subtypes. In addition, the length of time required to complete current standard influenza virus vaccine production processes inhibits the rapid development and production of an adapted vaccine in a pandemic situation.

### SUMMARY OF INVENTION

The present invention provides influenza vaccines comprising at least one isolated antigenic hemagglutinin (HA) protein (SEQ ID NOs: 1-4, 8-11, 14-19, 41-61, and 83-88). In certain embodiments, the protein has at least 90%, 95%, 96%, 97.5%, 98%, 99%, 95-99% identity to a mature amino acid sequence in any one of SEQ ID NOs: 1-4, 8-11, 14-19, 41-61, and 83-88. In one embodiment, the polypeptide comprises a mature amino acid sequence that is the extracellular domain of SEQ ID NO: 8 or 10. The exact sequence of the extracellular domain can vary based on the truncation site. In one embodiment, the truncation site is any of the italic residues of SEQ ID NO:8 or 10 in Table 1. In another embodiment, the extracellular domain of SEQ ID NO: 8 or 10 can be linked to a foldon domain ( GYIPEAPRDGQAYVRKDGEWVLLSTFL) through a linker.

The present invention also provides an influenza virus vaccine comprising at least two isolated antigenic polypeptides, wherein the first polypeptide comprises the mature amino acid sequence of any one of SEQ ID NOS: 1-5, 8-11, 14-19, 41-63, and 83-88, and the second polypeptide comprises the mature amino acid sequence of SEQ ID NO: 20. In certain embodiments, the protein has at least 90%, 95%, 96%, 97.5%, 98%, 99%, or 95-99% identity to a mature amino acid sequence in any one of SEQ ID NOs: 1-5, 8-11, 14-19, 41-63, and 83-88. The present invention also provides an influenza virus vaccine comprising at least two isolated antigenic polypeptides, wherein the first polypeptide comprises the mature amino acid sequence of any one of SEQ ID NO: 4 or 5, and the second polypeptide comprises the mature amino acid sequence of SEQ ID NO: 20.

The present invention also provides an influenza virus vaccine comprising an isolated polynucleotide sequence comprising SEQ ID NOs: 21-24, 28-31 34-39, or 64-81. In certain embodiments, the polynucleotide has at least 90%, 95%, 96%, 97%, 98%, 99%, 95-99% identity to any one of SEQ ID NOs: 21-24, 28-31 34-39, or 64-81. Also provided is an influenza virus vaccine comprising an isolated polynucleotide sequence which encodes an antigenic peptide having the mature amino acid sequence of any one of SEQ ID NO: 1-4, 8-11, 14-19, 41-61, and 83-88.

The present invention also provides an influenza virus vaccine comprising a first isolated polynucleotide sequence comprising SEQ ID NOs: 21-24, 28-31, 34-39, or 64-81, and a second isolated polynucleotide sequence comprising SEQ ID NO: 40. In certain embodiments, the polynucleotide has at least 90%, 95%, 96%, 97.5%, 98%, 99%, or 95-99% identity to any one of SEQ ID NOs: 21-24, 28-31, 34-39, 40, and 64-81. The present invention also provides an influenza virus vaccine comprising a first isolated polynucleotide sequence comprising SEQ ID NO: 24 or 25 and a second isolated polynucleotide sequence comprising SEQ ID NO: 40.

The present disclosure also provides antibody molecules, including full length antibodies and antibody derivatives, directed against the novel influenza virus sequences.

In some embodiments, the vaccine further comprises an aluminum adjuvant such as MMA or APA.

In some embodiments, the antigenic polypeptide comprises G430C, E438C, Q457L mutations of the Influenza B/Brisbane/60/2008 HA sequence to improve trimerization (amino acid residue number one is from the first amino acid of the N-terminal Methionine of the signal peptide for the HA sequence).

In some embodiments, the antigenic polypeptide comprises mutations I333T, M429S and L432T of the HA sequence of the Influenza B/Brisbane/60/2008 (amino acid residue number one is from the first amino acid of the N-terminal Methionine of the signal peptide for the HA sequence).

In some embodiments, the antigenic polypeptide comprises ferritin sequence of HA protein for nanoparticle formation.

In some embodiments, the antigenic polypeptide comprises the transmembrane domain of the HA sequence.

In some embodiments, the antigenic polypeptide comprises the consensus HA sequence for pandemic H1 strains.

In some embodiments, the antigenic polypeptide comprises the consensus HA sequence for seasonal H1 strains.

In some embodiments, the antigenic polypeptide is formulated with a lipid nanoparticle comprising a cationic lipid, a PEG-modified lipid, a sterol and a non-cationic lipid.

In some embodiments, at least one influenza antigenic polypeptide comprises a mutated N-linked glycosylation site.

In some embodiments, the vaccine is multivalent, and comprises at least two to ten, two, three, four or five or ten of the above antigenic polypeptides.

Some embodiments of the present disclosure provide methods of inducing an antigen specific immune response in a subject, comprising administering to the subject any of the vaccine as provided herein in an amount effective to produce an antigen-specific immune response. In some embodiments, the vaccine is a combination vaccine comprising a combination of influenza vaccines.

In some embodiments, an antigen-specific immune response comprises a T cell response or a B cell response.

In some embodiments, a method of producing an antigen-specific immune response comprises administering to a subject a single dose (no booster dose) of an influenza vaccine of the present disclosure.

In some embodiments, a method further comprises administering to the subject a second (booster) dose of an influenza vaccine. Additional doses of an influenza vaccine may be administered.

In some embodiments, an influenza vaccine is administered to a subject by intradermal injection, intramuscular injection, or by intranasal administration. In some embodiments, an influenza vaccine is administered to a subject by intramuscular injection.

Some embodiments of the present disclosure provide methods of inducing an antigen specific immune response in a subject, including administering to a subject an influenza vaccine in an effective amount to produce an antigen specific immune response in a subject. Antigen-specific immune responses in a subject may be determined, in some embodiments, by assaying for antibody titer (for titer of an antibody that binds to an influenza antigenic polypeptide) following administration to the subject of any of the influenza vaccines of the present disclosure.

In some embodiments, the vaccine is formulated in an effective amount to produce an antigen specific immune response in a subject.

In some embodiments, the vaccine immunizes the subject against Influenza for up to 1 or 2 years. In some embodiments, the vaccine immunizes the subject against Influenza for more than 2 years, more than 3 years, more than 4 years, or for 5-10 years. In one embodiment, the vaccination is yearly.

In some embodiments, the subject is about 5 years old or younger. For example, the subject may be between the ages of about 1 year and about 5 years (e.g., about 1, 2, 3, 5 or 5 years), or between the ages of about 6 months and about 1 year (e.g., about 6, 7, 8, 9, 10, 11 or 12 months). In some embodiments, the subject is about 12 months or younger *(e.g.,* 12, 11, 10, 9, 8, 7, 6, 5, 4, 3, 2 months or 1 month). In some embodiments, the subject is about 6 months or younger.

In some embodiments, the subject was born full term (e.g., about 37-42 weeks). In some embodiments, the subject was born prematurely, for example, at about 36 weeks of gestation or earlier (e.g., about 36, 35, 34, 33, 32, 31, 30, 29, 28, 27, 26 or 25 weeks). For example, the subject may have been born at about 32 weeks of gestation or earlier. In some embodiments, the subject was born prematurely between about 32 weeks and about 36 weeks of gestation. In such subjects, a vaccine may be administered later in life, for example, at the age of about 6 months to about 5 years, or older.

In some embodiments, the subject is a young adult between the ages of about 20 years and about 50 years (e.g., about 20, 25, 30, 35, 40, 45 or 50 years old).

In some embodiments, the subject is an elderly subject about 50-60 years old, 60 years old, about 70 years old, or older, 80 years or older, 90 years or older *(e.g.,* about 60, 65, 70, 75, 80, 85 or 90 years old).

In some embodiments, the subject has been exposed to influenza; the subject is infected with influenza; or subject is at risk of infection by influenza.

In some embodiments, the subject is immunocompromised (has an impaired immune system, e.g., has an immune disorder or autoimmune disorder).

The details of various embodiments of the disclosure are set forth in the description below. Other features, objects, and advantages of the disclosure will be apparent from the description and from the claims.

### BRIEF DESCRIPTION OF THE DRAWINGS

The foregoing and other objects, features and advantages will be apparent from the following description of particular embodiments of the invention, as illustrated in the accompanying drawings in which like reference characters refer to the same parts throughout the different views. The drawings are not necessarily to scale, emphasis instead being placed upon illustrating the principles of various embodiments of the invention.

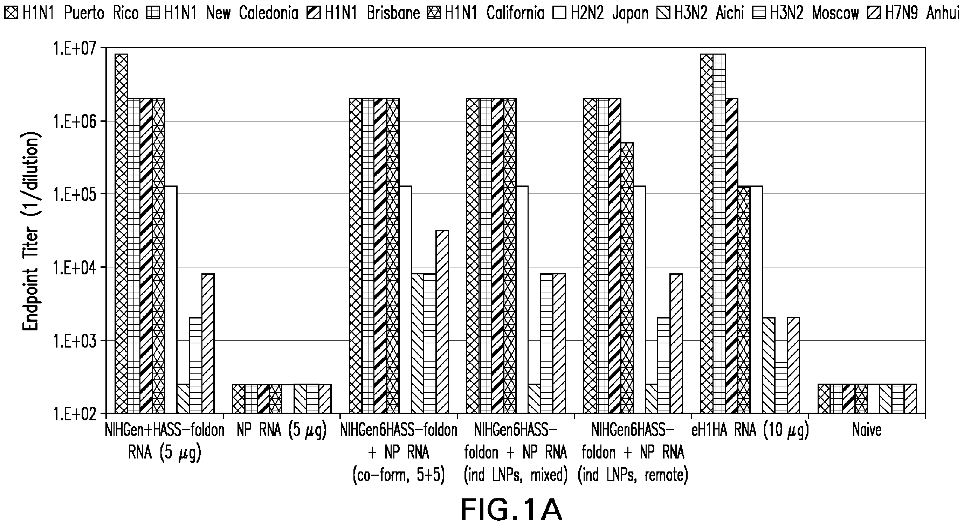

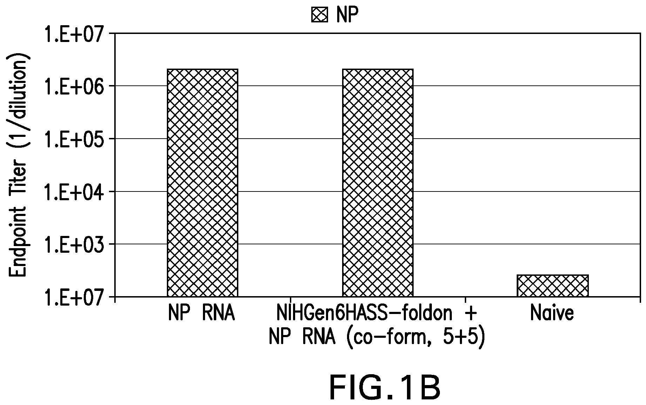

Figs. 1A-1B depict endpoint titers of pooled serum from animals vaccinated with the test vaccines. In Fig. 1A, the vaccines tested are shown on the x-axis and the binding to HA from each of the different strains of influenza is plotted as an endpoint titer. In Fig. 1B, the vaccines tested are shown on the x-axis, and the endpoint titer to NP protein is plotted as an endpoint titer.

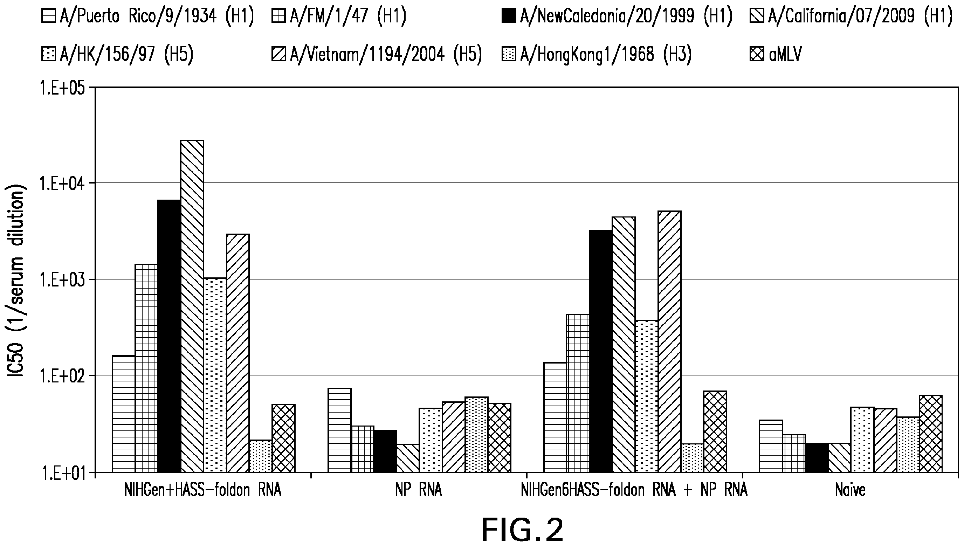

Fig. 2 shows an examination of functional antibody response through an assessment of the ability of serum to neutralize a panel of HA-pseudotyped viruses.

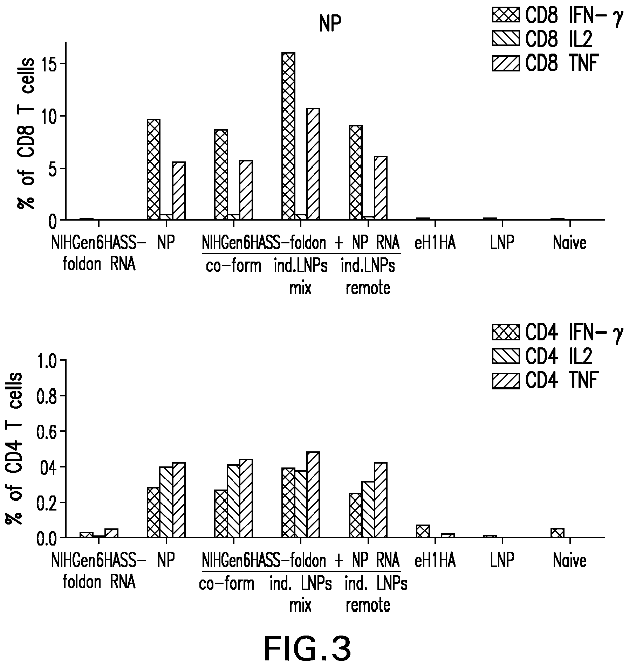

Fig. 3 is a representation of cell-mediated immune responses following mRNA vaccination. Splenocytes were harvested from vaccinated mice and stimulated with a pool of overlapping NP peptides. The % of CD4 or CD8 T cells secreting one of the three cytokines (IFN-γ, IL-2, or TNF-α) is plotted.

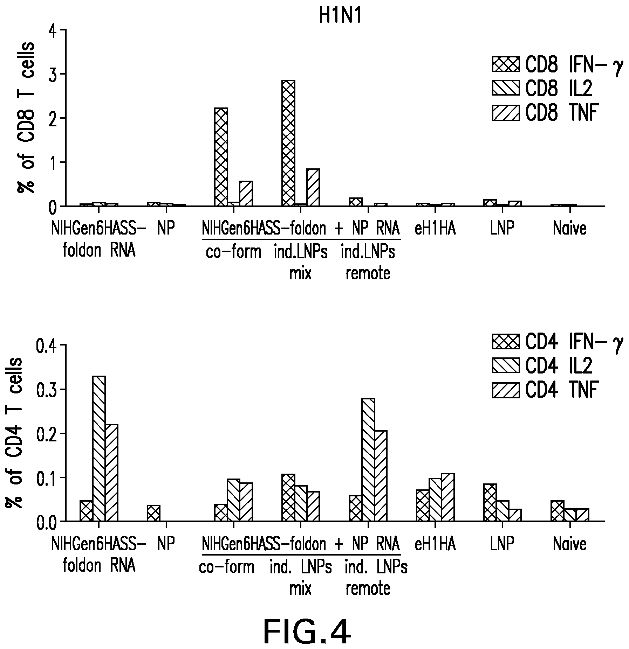

Fig. 4 is a representation of cell-mediated immune responses following mRNA vaccination. Splenocytes were harvested from vaccinated mice and stimulated with a pool of overlapping HA peptides. The % of CD4 or CD8 T cells secreting one of the three cytokines (IFN-γ, IL-2, or TNF-α) is plotted.

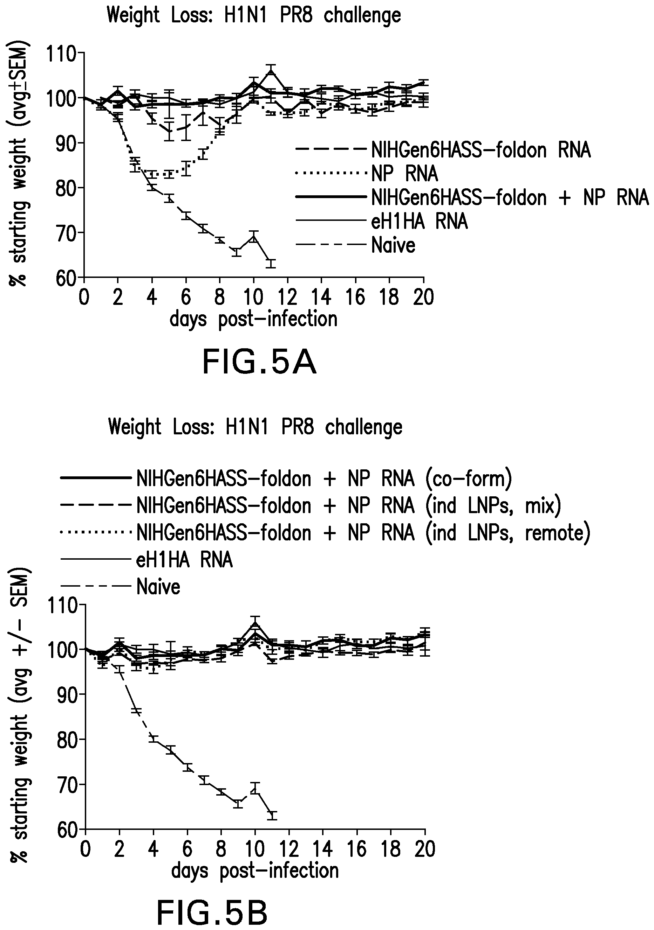

Fig. 5 shows murine weight loss following challenge with a lethal dose of mouse-adapted H1N1 A/Puerto Rico/8/1934. The percentage of weight lost as compared to baseline was calculated for each animal and was averaged across the group. The group average was plotted over time in days. Error bars represent standard error of the mean. Efficacy of the NIHGen6HASS-foldon + NP combination vaccines was better than that of either the NIHGen6HASS-foldon or NP mRNA vaccine alone, regardless of antigen co-formulation or co-delivery method.

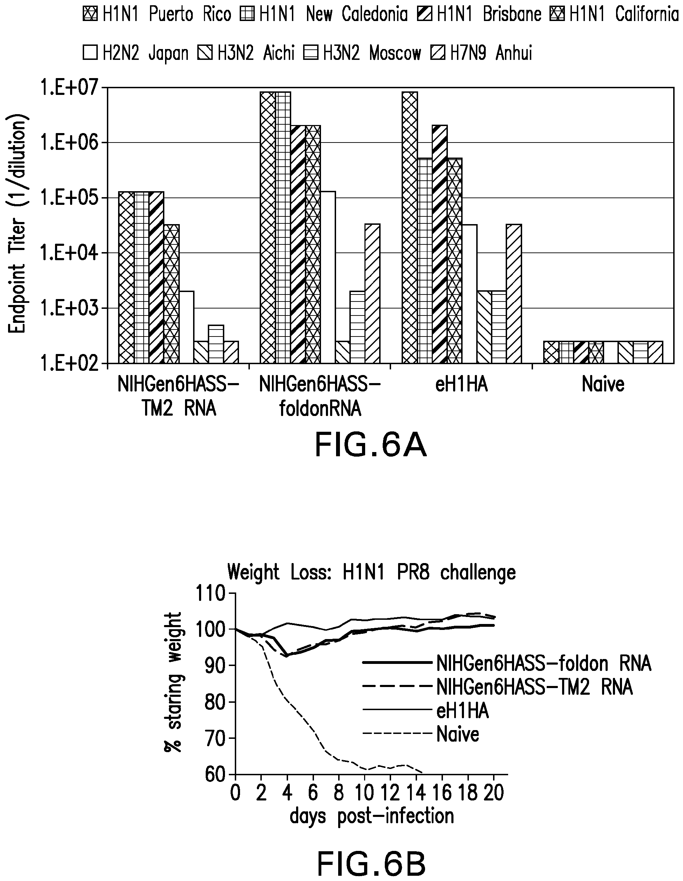

Fig. 6A depicts the endpoint titers of the pooled serum from animals vaccinated with the test vaccines. Fig. 6B shows efficacy of the test vaccines (NIHGen6HASS-foldon and NIHGen6HASS-TM2) is similar. Following challenge with a lethal dose of mouse-adapted H1N1 A/Puerto Rico/8/1934, the percentage of group weight lost as compared to baseline was calculated and plotted over time in days.

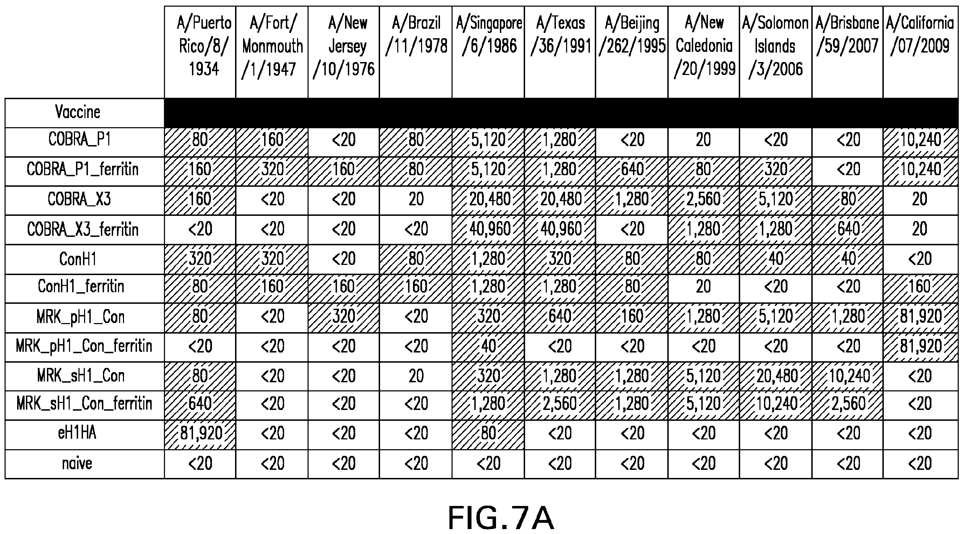

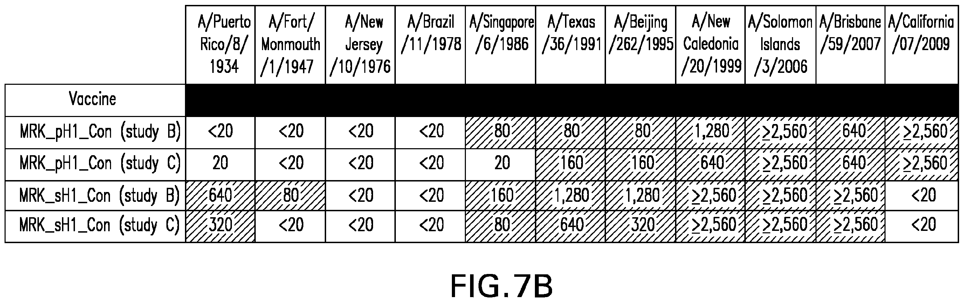

Figs. 7A and 7B shows the endpoint neutralization titers detected in serum from vaccinated animals against a panel of 11 H1N1 influenza viruses. For each sample, the highest dilution of serum that resulted in an OD greater than the cutoff was assigned as the microneutralization titer. A value of <20 indicates lack of neutralization, even at the lowest dilution tested.

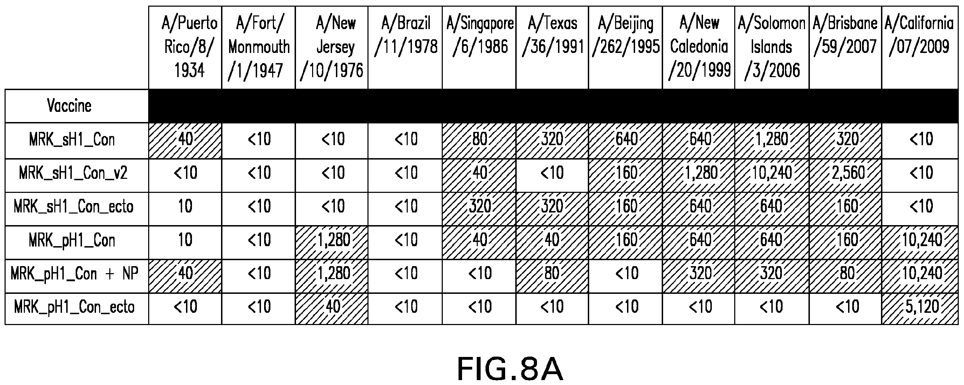

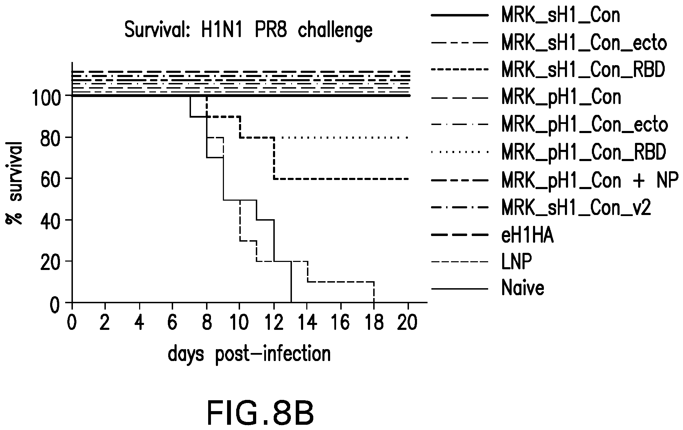

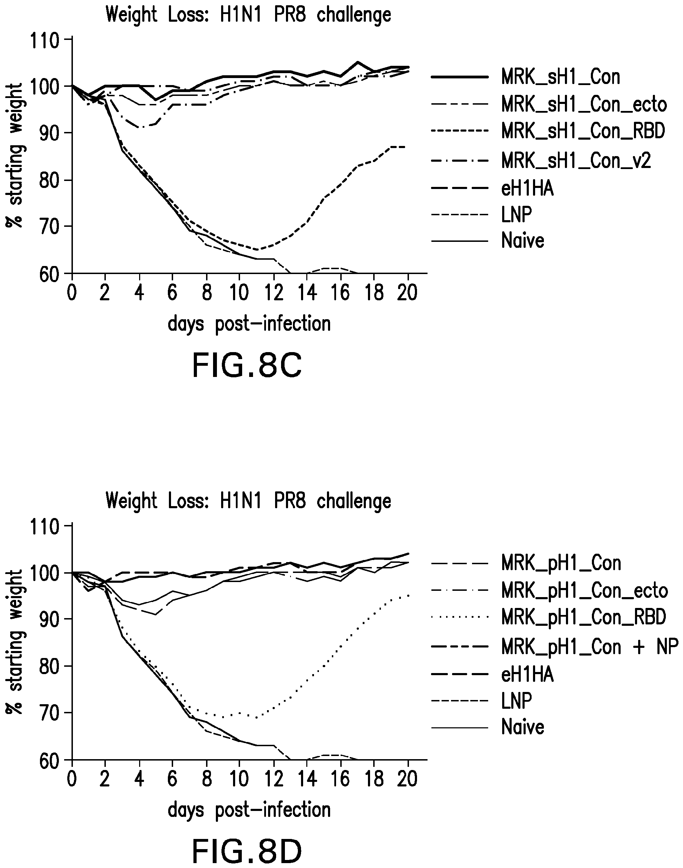

Figs. 8A and 8B show hemagglutination inhibition (HAI) titers of each serum sample (indicated in the first column) against a panel of 11 H1N1 influenza viruses. Antisera from individual animals exposed to the same vaccine regimen were pooled and tested for their ability to inhibit agglutination of turkey red blood cells induced by multiple influenza A H1 virus strains. The highest dilution with no visible agglutination was assigned as the serum titer represented in the table. A value of <10 indicates a lack of HAI, even at the lowest serum dilution tested. Titers ≥40 (correlate of protection for influenza) are highlighted in gray. Fig. 8C and 8D shows murine weight loss following challenge with a lethal dose of mouse adapted H1N1 A/Puerto Rico/8/1934. The percentage of group weight lost as compared to baseline was calculated and plotted over time in days.

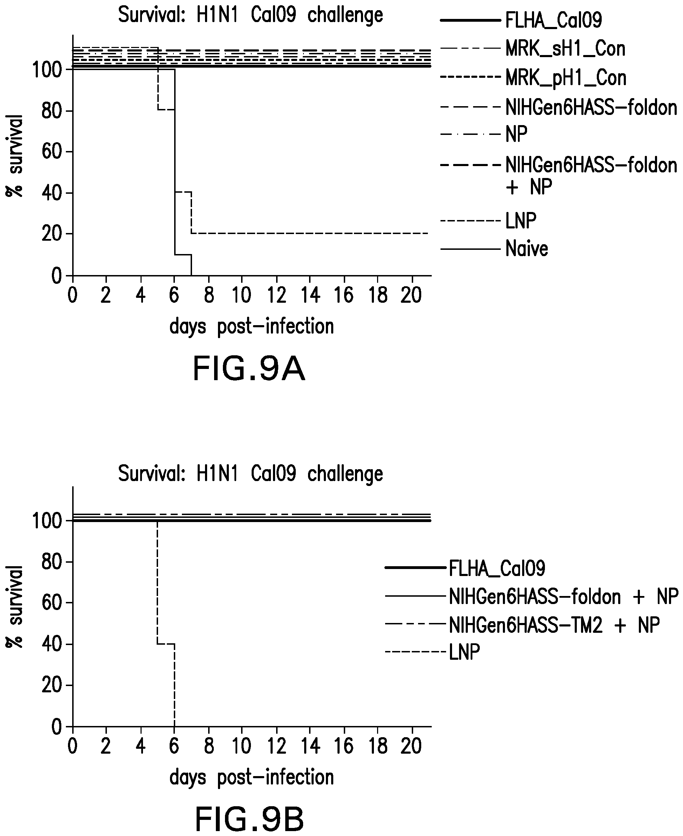

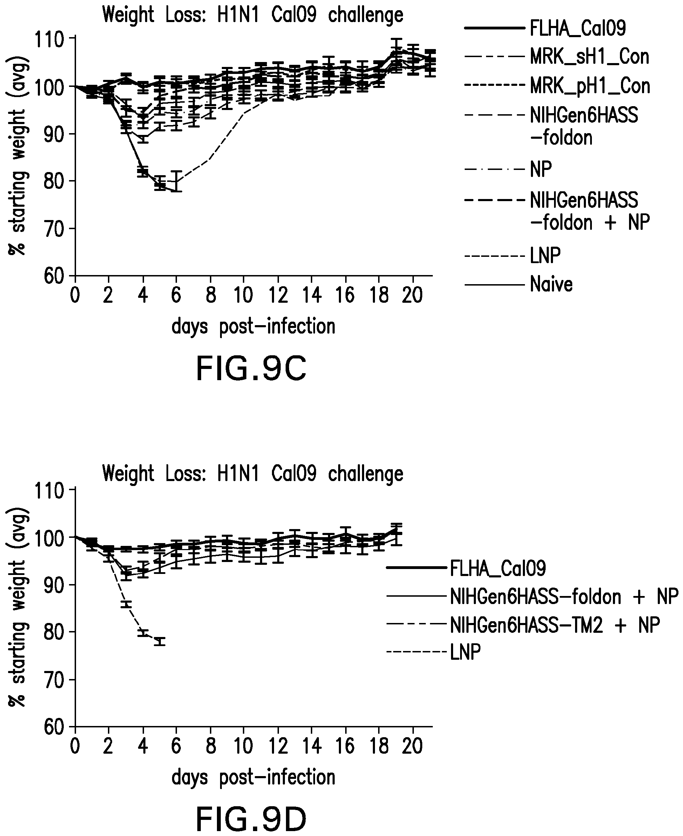

Fig. 9A and 9B show the murine survival following challenge with H1N1 Cal09. The percentage of group survival was calculated and plotted over time in days. Fig. 9C and 9D shows murine weight loss following challenge with H1N1 Cal09. The percentage of group weight lost as compared to baseline was calculated and plotted over time in days.

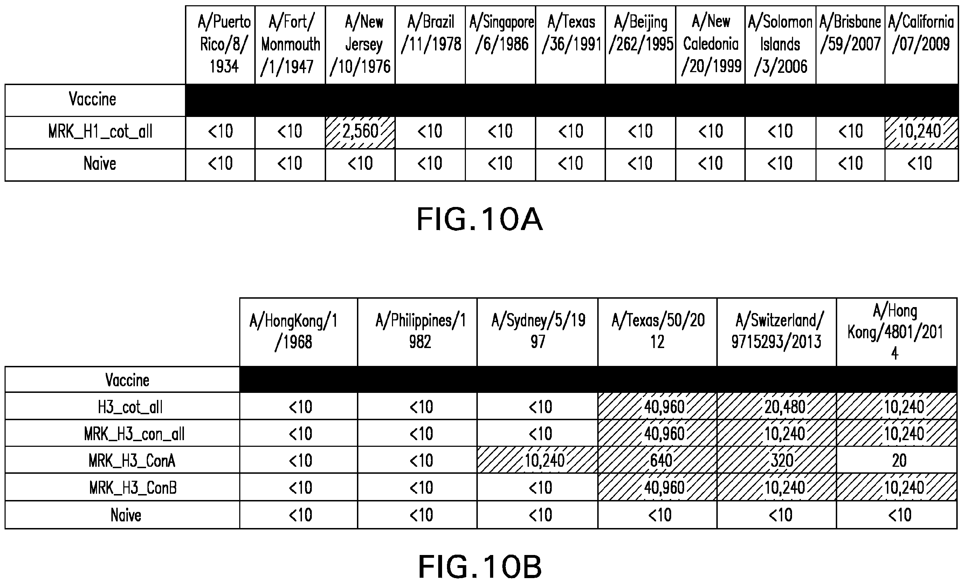

Fig. 10A and 10B show hemagglutination inhibition (HAI) titers of each serum sample (indicated in the first column) against a panel of 11 H1N1 influenza viruses. Antisera from individual animals exposed to the same vaccine regimen were pooled and tested for their ability to inhibit agglutination of turkey red blood cells induced by multiple influenza A h1 (Fig. 10A) and H3 (Fig. 10B) virus strains. The highest dilution with no visible agglutination was assigned as the serum titer represented in the table. A value of <10 indicates a lack of HAI, even at the lowest serum dilution tested. Titers ≥40 (correlate of protection for influenza) are highlighted in gray.

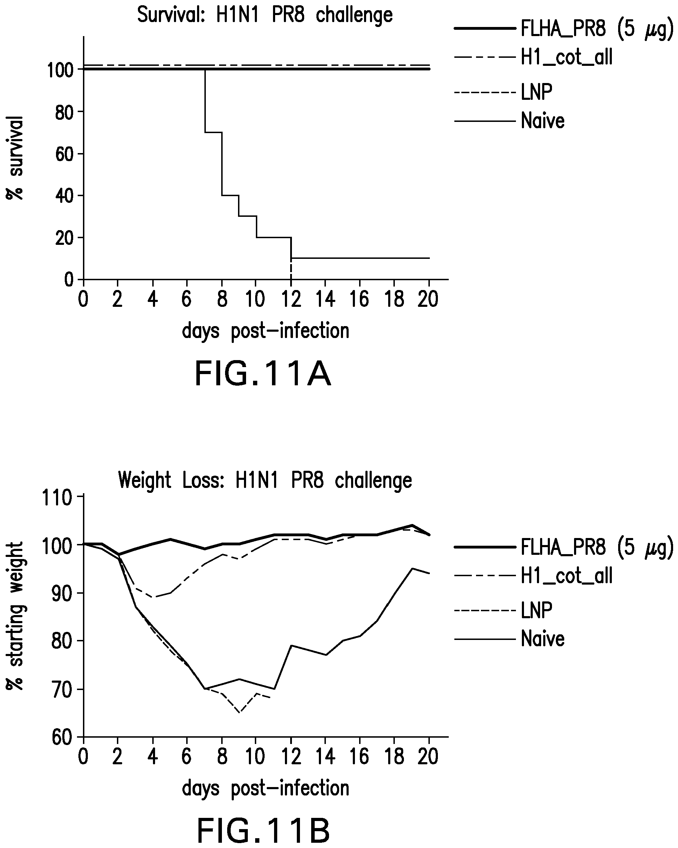

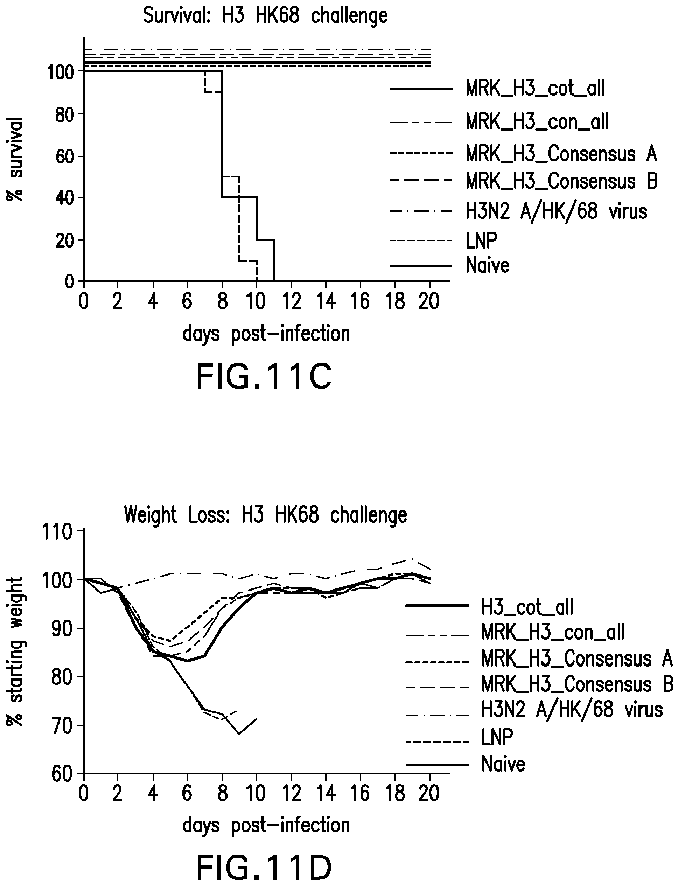

Fig. 11A shows the murine survival following challenge with H1N1 PR8. The percentage of group survival was calculated and plotted over time in days. Fig. 11B shows murine weight loss following challenge with H1N1 PR8. The percentage of group weight lost as compared to baseline was calculated and plotted over time in days. Fig. 11C shows murine survival following challenge with H3 HK68. The percentage of group survival was calculated and plotted over time in days. Fig. 11D shows murine weight loss following challenge with H3 HK68. The percentage of group weight lost as compared to baseline was calculated and plotted over time in days.

### DETAILED DESCRIPTION

"Consensus" or "consensus sequence" as used herein means a polypeptide sequence based on analysis of an alignment of multiple subtypes of a particular influenza antigen. DNA sequences that encode a consensus polypeptide sequence may be prepared. Vaccines comprising isolated proteins that comprise consensus sequences and/or DNA molecules that encode such proteins can be used to induce broad immunity against multiple subtypes or serotypes of a particular influenza antigen. Consensus influenza antigens can include influenza A consensus hemagglutinin amino acid sequences, including for example consensus H1, consensus H2, consensus H3, or influenza B consensus hemagglutinin amino acid sequences.

"RBD" as used herein means receptor binding domain.

"Isolated" polypeptides or polynucleotides are at least partially free of other biological molecules from the cells or cell cultures in which they are produced. Such biological molecules include other nucleic acids, proteins, lipids, carbohydrates, or other material such as cellular debris and growth medium. It may further be at least partially free of expression system components such as biological molecules from a host cell or of the growth medium thereof. Generally, the term "isolated" is not intended to refer to a complete absence of such biological molecules or to an absence of water, buffers, or salts or to components of a pharmaceutical formulation that includes the polypeptides or polynucleotides.

In some embodiments, the virus is a strain of Influenza A or Influenza B or combinations thereof. In some embodiments, the strain of Influenza A or Influenza B is associated with birds, pigs, horses, dogs, humans or non-human primates.

Some embodiments provide methods of preventing or treating influenza viral infection comprising administering to a subject any of the vaccines described herein. In some embodiments, the antigen specific immune response comprises a T cell response. In some embodiments, the antigen specific immune response comprises a B cell response. In some embodiments, the antigen specific immune response comprises both a T cell response and a B cell response. In some embodiments, the method of producing an antigen specific immune response involves a single administration of the vaccine. In some embodiments, the vaccine is administered to the subject by intradermal, intramuscular injection, subcutaneous injection, intranasal inoculation, or oral administration.

In some embodiments, the vaccine comprises at least one of the aforementioned antigenic polypeptides of the invention and at least one protein, or immunogenic fragment or variant or homolog thereof, selected from a NP protein, a NA protein, a M1 protein, a M2 protein, a NS1 protein and a NS2 protein obtained from influenza virus.

The influenza antigens provided herein can be arranged as a vaccine that causes seroconversion in vaccinated mammals and provides cross-reactivity against a broad range of seasonal strains of influenza and also pandemic strains of influenza. The seroconversion and broad cross-reactivity can be determined by measuring inhibiting titers against different hemagglutinin strains of influenza. Preferred combinations include at least two antigens from each of the influenza antigens described herein.

### Lipid Nanoparticles

As used herein, "lipid nanoparticle" or "LNP" refers to any lipid composition that can be used to deliver a product, including, but not limited to, liposomes or vesicles, wherein an aqueous volume is encapsulated by amphipathic lipid bilayers (e.g., single; unilamellar or multiple; multilamellar), or, in other embodiments, wherein the lipids coat an interior comprising a prophylactic product, or lipid aggregates or micelles, wherein the lipid encapsulated therapeutic product is contained within a relatively disordered lipid mixture. Except where noted, the lipid nanoparticle does not need to have the antigenic polypeptide incorporated therein and may be used to deliver a product when in the same formulation.

As used herein, "polyamine" means compounds having two or more amino groups. Examples include putrescine, cadaverine, spermidine, and spermine.

Unless otherwise specified, mole % refers to a mole percent of total lipids. Generally, the LNPs of the compositions of the invention are composed of one or more cationic lipids (including ionizable cationic lipids) and one or more poly(ethyleneglycol)-lipids (PEG-lipids). In certain embodiments, the LNPs further comprise one or more non-cationic lipids. The one or more non-cationic lipids can include a phospholipid, phospholipid derivative, a sterol, a fatty acid, or a combination thereof.

Cationic lipids and ionizable cationic lipids suitable for the LNPs are described herein. Ionizable cationic lipids are characterized by the weak basicity of their lipid head groups, which affects the surface charge of the lipid in a pH-dependent manner, rendering them positively charged at acidic pH but close to charge-neutral at physiologic pH. Cationic lipids are characterized by monovalent or multivalent cationic charge on their headgroups, which renders them positively charged at neutral pH. In certain embodiments, the cationic and ionizable lipid is capable of complexing with hydrophilic bioactive molecules to produce a hydrophobic complex that partitions into the organic phase of a two-phase aqueous/organic system. It is contemplated that both monovalent and polyvalent cationic lipids may be utilized to form hydrophobic complexes with bioactive molecules.
Preferred cationic and ionizable cationic lipids for use in forming the LNPs include, but are not limited to, N,N-dioleyl-N,N-dimethylammonium chloride ("DODAC"); N-(2,3dioleyloxy)propyl)-N,N,Ntrimethylammonium chloride ("DOTMA"); N,NdistearylN,N-dimethylammonium bromide ("DDAB"); N-(2,3dioleoyloxy)propyl)-N,N,N-trimethylamntonium chloride ("DODAP"); 1,2 bis (oleoyloxy)-3-(trimethylammonio) propane (DOTAP); 3-(N-(N,N-dimethylaminoethane)-carbam-oyl)cholesterol ('DC-Chol"); diheptadecylamidoglycylspermidine ("DHGS") and N-(1,2-dimyristyloxyprop-3-yl)-N,N-dimethyl-N-hydoxyethyl ammonium bromide ("DMRIE"). Additionally, a number of commercial preparations of cationic lipids, as well as other components, are available which can be used in the present invention. These include, for example, LIPOFECTIN^{®} (commercially available cationic lipid nanoparticles comprising DOTMA and 1,2dioleoyl-sn-3-phosphoethanolamine ("DOPE"), from GIBCOBRL, Grand Island, N.Y., USA); and LIPOFECTAMINE^{®} (commercially available cationic lipid nanoparticles comprising N-(1-(2,3dioleyloxy)propyl)N-(2-(sperminecarboxamido)ethyl)-N,N-dimethylammonium trifluoroacetate ("DOSPA') and ("DOPE"), from (GIBCOBRL). The following lipids are cationic and have a positive charge at below physiological pH: DODAP, DODMA, DMDMA, 1,2-DiLinoleyloxy-N,N-dimethylaminopropane (DLinDMA), 4-(2,2-diocta-9,12-dienyl-[1,3]dioxolan-4-ylmethyl)-dimethylamine, DLinKDMA (WO 2009/132131 A1), DLin-K-C2-DMA (WO2010/042877), DLin-M-C3-DMA (WO2010/146740 and/or WO2010/105209), DLin-MC3-DMA (heptatriaconta-6,9,28,3 1-tetraen-19-yl 4-(dimethylamino)butanoate; Jayaraman et al., 2012, Angew. Chem. Int. Ed. Engl. 51:8529-8533), 2-{4-[(3β)-cholest-5-en-3-yloxy]butoxy}-N,N-dimethyl-3-[(9Z,12Z)-octadeca-9,12-dienlyloxyl]propan-1-amine) (CLinDMA), and the like. Other cationic lipids suitable for use in the invention include, e.g., the cationic lipids described in U.S. Pat. Nos. 5,208,036, 5,264,618, 5,279,833 and 5,283,185, and U.S. Patent Application Publication Nos. 2008/0085870 and 2008/0057080. Other cationic lipids suitable for use in the invention include, e.g., Lipids E0001-E0118 or E0119-E0180 as disclosed in Table 6 (pages 112 - 139) of International Patent Application Publication No. WO2011/076807 (which also discloses methods of making, and methods of using these cationic lipids).

In certain aspects of this embodiment of the invention, the LNPs comprise one or more of the following ionizable cationic lipids: DLinDMA, DlinKC2DMA DLin-MC3-DMA, CLinDMA, or S-Octyl CLinDMA (See International Patent Application Publication No. WO2010/021865).

In certain aspects of this embodiment of the invention, LNPs comprise one or more ionizable cationic lipids described in International Patent Application Publication No. WO2011/022460 A1, or any pharmaceutically acceptable salt thereof, or a stereoisomer of any of the compounds or salts therein.

When structures of the same constitution differ in respect to the spatial arrangement of certain atoms or groups, they are stereoisomers, and the considerations that are significant in analyzing their interrelationships are topological. If the relationship between two stereoisomers is that of an object and its nonsuperimposable mirror image, the two structures are enantiomeric, and each structure is said to be chiral. Stereoisomers also include diastereomers, cis-trans isomers and conformational isomers. Diastereoisomers can be chiral or achiral, and are not mirror images of one another. Cis-trans isomers differ only in the positions of atoms relative to a specified plane in cases where these atoms are, or are considered as if they were, parts of a rigid structure. Conformational isomers are isomers that can be interconverted by rotations about formally single bonds. Examples of such conformational isomers include cyclohexane conformations with chair and boat conformers, carbohydrates, linear alkane conformations with staggered, eclipsed and gauche conformers, etc. See J. Org. Chem. 35, 2849 (1970).

Many organic compounds exist in optically active forms having the ability to rotate the plane of plane-polarized light. In describing an optically active compound, the prefixes D and L or R and S are used to denote the absolute configuration of the molecule about its chiral center(s). The prefixes d and 1 or (+) and (-) are employed to designate the sign of rotation of plane-polarized light by the compound, with (-) or meaning that the compound is levorotatory. A compound prefixed with (+) or d is dextrorotatory. For a given chemical structure, enantiomers are identical except that they are non-superimposable mirror images of one another. A mixture of enantiomers is often called an enantiomeric mixture. A 50:50 mixture of enantiomers is referred to as a racemic mixture. Many of the compounds described herein can have one or more chiral centers and therefore can exist in different enantiomeric forms. If desired, a chiral carbon can be designated with an asterisk (^{∗}). When bonds to the chiral carbon are depicted as straight lines in the Formulas of the invention, it is understood that both the (R) and (S) configurations of the chiral carbon, and hence both enantiomers and mixtures thereof, are embraced within the Formula. As is used in the art, when it is desired to specify the absolute configuration about a chiral carbon, one of the bonds to the chiral carbon can be depicted as a wedge (bonds to atoms above the plane) and the other can be depicted as a series or wedge of short parallel lines (bonds to atoms below the plane). The Cahn-Inglod-Prelog system can be used to assign the (R) or (S) configuration to a chiral carbon.

When the compounds of the present invention contain one chiral center, the compounds exist in two enantiomeric forms and the present invention includes both enantiomers and mixtures of enantiomers, such as the specific 50:50 mixture referred to as a racemic mixtures. The enantiomers can be resolved by methods known to those skilled in the art, such as formation of diastereoisomeric salts which may be separated, for example, by crystallization (see, CRC Handbook of Optical Resolutions via Diastereomeric Salt Formation by David Kozma (CRC Press, 2001)); formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. It will be appreciated that where the desired enantiomer is converted into another chemical entity by one of the separation procedures described above, a further step is required to liberate the desired enantiomeric form. Alternatively, specific enantiomers may be synthesized by asymmetric synthesis using optically active reagents, substrates, catalysts or solvents, or by converting one enantiomer into the other by asymmetric transformation.

Designation of a specific absolute configuration at a chiral carbon of the compounds of the invention is understood to mean that the designated enantiomeric form of the compounds is in enantiomeric excess (ee) or in other words is substantially free from the other enantiomer. For example, the "R" forms of the compounds are substantially free from the "S" forms of the compounds and are, thus, in enantiomeric excess of the "S" forms. Conversely, "S" forms of the compounds are substantially free of "R" forms of the compounds and are, thus, in enantiomeric excess of the "R" forms. Enantiomeric excess, as used herein, is the presence of a particular enantiomer at greater than 50%. In a particular embodiment when a specific absolute configuration is designated, the enantiomeric excess of depicted compounds is at least about 90%.

When a compound of the present invention has two or more chiral carbons it can have more than two optical isomers and can exist in diastereoisomeric forms. For example, when there are two chiral carbons, the compound can have up to 4 optical isomers and 2 pairs of enantiomers ((S,S)/(R,R) and (R,S)/(S,R)). The pairs of enantiomers (e.g., (S,S)/(R,R)) are mirror image stereoisomers of one another. The stereoisomers that are not mirror-images (e.g., (S,S) and (R,S)) are diastereomers. The diastereoisomeric pairs may be separated by methods known to those skilled in the art, for example chromatography or crystallization and the individual enantiomers within each pair may be separated as described above. The present invention includes each diastereoisomer of such compounds and mixtures thereof.

The LNPs may also comprise any combination of two or more of the cationic lipids described herein. In certain aspects, the cationic lipid typically comprises from about 0.1 to about 99.9 mole % of the total lipid present in said particle. In certain aspects, the cationic lipid can comprise from about 80 to about 99.9% mole %. In other aspects, the cationic lipid comprises from about 2% to about 70%, from about 5% to about 50%, from about 10% to about 45%, from about 20% to about 99.8%, from about 30% to about 70%, from about 34% to about 59%, from about 20% to about 40%, or from about 30% to about 40% (mole %) of the total lipid present in said particle.

The LNPs described herein can further comprise a noncationic lipid, which can be any of a variety of neutral uncharged, zwitterionic or anionic lipids capable of producing a stable complex. They are preferably neutral, although they can be negatively charged. Examples of noncationic lipids useful in the present invention include phospholipid-related materials, such as natural phospholipids, synthetic phospholipid derivatives, fatty acids, sterols, and combinations thereof. Natural phospholipids include phosphatidylcholine (PC), phosphatidylethanolamine (PE), and phosphatidylglycerol (PG), phosphatidylserine (PS), phosphatidylinositol (PI), Phosphatidic acid (phosphatidate) (PA), dipalmitoylphosphatidylcholine, monoacyl-phosphatidylcholine (lyso PC), 1-palmitoyl-2-oleoyl-sn-glycero-3-phosphocholine (POPC), N-Acyl-PE, phosphoinositides, and phosphosphingolipids. Phospholipid derivatives include phosphatidic acid (DMPA, DPPA, DSPA), phosphatidylcholine (DDPC, DLPC, DMPC, DPPC, DSPC, DOPC, POPC, DEPC), phosphatidylglycerol (DMPG, DPPG, DSPG, POPG), phosphatidylethanolamine (DMPE, DPPE, DSPE DOPE), and phosphatidylserine (DOPS). Fatty acids include C14:0, palmitic acid (C16:0), stearic acid (C18:0), oleic acid (C18:1), linoleic acid (C18:2), linolenic acid (C18:3), and arachidonic acid (C20:4), C20:0, C22:0 and lethicin.

In certain embodiments of the invention the non-cationic lipid is selected from lecithin, phosphatidylethanolamine, lysolecithin, lysophosphatidylethanolamine, phosphatidylserine, phosphatidylinositol, sphingomyelin, cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG) , dipalmitoylphosphatidylglycerol (DPPG), dioleoyl-phosphatidylethanolamine (DOPE), palmitoyloleoylphosphatidylcholine (POPC) , palmitoyloleoyl-phosphatidylet-hanolamine (POPE) and dioleoyl-phosphatidylethanolamine 4-(N-maleimidomethyl)-cyclohexane-1-carboxylate (DOPE-mal). Noncationic lipids also include sterols such as cholesterol, stigmasterol or stigmastanol. Cholesterol is known in the art. See U.S. Patent Application Publication Nos: U.S. 2006/0240554 and U.S. 2008/0020058. In certain embodiments, the LNP comprise a combination of a phospholipid and a sterol.

Where present, the non-cationic lipid typically comprises from about 0.1% to about 65%, about 2% to about 65%, about 10% to about 65%, or about 25% to about 65% expressed as mole percent of the total lipid present in the LNP. The LNPs described herein further include a polyethyleneglycol (PEG) lipid conjugate ("PEG-lipid") which may aid as a bilayer stabilizing component. The lipid component of the PEG lipid may be any non-cationic lipid described above including natural phospholipids, synthetic phospholipid derivatives, fatty acids, sterols, and combinations thereof. In certain embodiments of the invention, the PEG-lipids include, PEG coupled to dialkyloxypropyls (PEG-DAA) as described in, e.g., International Patent Application Publication No. WO 05/026372, PEG coupled to diacylglycerol (PEG-DAG) as described in, e.g., U.S. Patent Publication Nos. 20030077829 and 2005008689; PEG coupled to phosphatidylethanolamine (PE) (PEG-PE), or PEG conjugated to 1,2-Di-O-hexadecyl-sn-glyceride (PEG-DSG), or any mixture thereof (see, e.g., U.S. Pat. No. 5,885,613).

In one embodiment, the PEG-DAG conjugate is a dilaurylglycerol (C 12)-PEG conjugate, a PEG dimyristylglycerol (C14)conjugate, a PEG-dipalmitoylglycerol (C16) conjugate, a PEG-dilaurylglycamide (C12) conjugate, a PEG-dimyristylglycamide (C14) conjugate, a PEG-dipalmitoylglycamide (C16) conjugate, or a PEG-disterylglycamide (C18). Those of skill in the art will readily appreciate that other diacylglycerols can be used in the PEG-DAG conjugates.

In certain embodiments, PEG-lipids include, but are not limited to, PEG-dimyristolglycerol (PEG-DMG), PEG-disteryl glycerol (PEG-DSG), PEG-dipalmetoleyl, PEG-dioleyl, PEG-distearyl, PEG-diacylglycamide (PEG-DAG), PEG- *dipalmitoyl* phosphatidylethanolamine (PEG-DPPE), and PEG-1,2-dimyristyloxlpropyl-3-amine (PEG-c-DMA).

In certain embodiments, the PEG-lipid is PEG coupled to dimyristoylglycerol (PEG-DMG), e.g., as described in Abrams et al., 2010, Molecular Therapy 18(1): 171, and U.S. Patent Application Publication Nos. US 2006/0240554 and US 2008/0020058.

In certain embodiments, the PEG-lipid, such as a PEG-DAG, PEG-cholesterol, PEG-DMB, comprises a polyethylene glycol having an average molecular weight ranging of about 500 daltons to about 10,000 daltons, of about 750 daltons to about 5,000 daltons, of about 1,000 daltons to about 5,000 daltons, of about 1,500 daltons to about 3,000 daltons or of about 2,000 daltons. In certain embodiments, the PEG-lipid comprises PEG400, PEG1500, PEG2000 or PEG5000.

The acyl groups in any of the lipids described above are preferably acyl groups derived from fatty acids having about C10 to about C24 carbon chains. In one embodiment, the acyl group is lauroyl, myristoyl, palmitoyl, stearoyl or oleoyl.

The PEG-lipid conjugate typically comprises from about 0.1% to about 15%, from about 0.5% to about 20%, from about 1.5% to about 18%, from about 4% to about 15%, from about 5% to about 12%, from about 1% to about 4%, or about 2% expressed as a mole % of the total lipid present in said particle.

In certain embodiments of the invention, the LNPs comprise one or more cationic lipids, cholesterol and 1,2-Dimyristoyl-sn-glycerol methoxypolyethylene glycol (PEG-DMG).

In certain embodiments the invention, the LNPs comprise one or more cationic lipids, cholesterol, 1,2-Distearoyl-sn-glycero-3-phosphocholine (DSPC), and 1,2-Dimyristoyl-sn-glycerol methoxypolyethylene glycol (PEG-DMG).

In certain embodiments of the invention, the LNPs comprise lipid compounds assembled within the following molar ratios:
Cationic Lipid (20-99.8 mole %)
Non-cationic lipid (0.1-65 mole %) and
PEG-DMG (0.1-20 mole %).

In certain embodiments of the invention, the LNPs comprise lipid compounds assembled within the following molar ratios:
Cationic Lipid (30-70 mole %)
Non-cationic lipid (20-65 mole %) and
PEG-DMG (1-15 mole %).

In certain aspects of this embodiment, the non-cationic lipid is cholesterol. Exemplary LNPs may include cationic lipid/cholesterol/PEG-DMG at about the following molar ratios: 58/30/10.

In certain aspects of this embodiment, the non-cationic lipid is cholesterol and DSPC. Exemplary LNPs may include cationic lipid/cholesterol/DSPC/PEG-DMG at about the following molar ratios: 59/30/10/1; 58/30/10/2; 43/41/15/1; 42/41/15/2; 40/48/10/2; 39/41/19/1; 38/41/19/2; 34/41/24/1; and 33/41/24/2.

### Preparation of LNPs

LNPs can be formed, for example, by a rapid precipitation process which entails micro-mixing the lipid components dissolved in ethanol with an aqueous solution using a confined volume mixing apparatus such as a confined volume T-mixer, a multi-inlet vortex mixer (MIVM), or a microfluidics mixer device as described below. The lipid solution contains one or more cationic lipids, one or more noncationic lipids (e.g., DSPC), PEG-DMG, and optionally cholesterol, at specific molar ratios in ethanol. The aqueous solution consists of a sodium citrate or sodium acetate buffered salt solution with pH in the range of 2-6, preferably 3.5-5.5. The two solutions are heated to a temperature in the range of 25°C-45°C, preferably 30°C-40°C, and then mixed in a confined volume mixer thereby instantly forming the LNP. When a confined volume T-mixer is used, the T-mixer has an internal diameter (ID) range from 0.25 to 1.0 mm. The alcohol and aqueous solutions are delivered to the inlet of the T-mixer using programmable syringe pumps, and with a total flow rate from 10-600 mL/minute. The alcohol and aqueous solutions are combined in the confined-volume mixer with a ratio in the range of 1:1 to 1:3 vol:vol, but targeting 1:1.1 to 1:2.3. The combination of ethanol volume fraction, reagent solution flow rates and t-mixer tubing ID utilized at this mixing stage has the effect of controlling the particle size of the LNPs between 30 and 300 nm. The resulting LNP suspension is twice diluted into higher pH buffers in the range of 6-8 in a sequential, multi-stage in-line mixing process. For the first dilution, the LNP suspension is mixed with a buffered solution at a higher pH (pH 6-7.5) with a mixing ratio in the range of 1:1 to 1:3 vol:vol, but targeting 1:2 vol:vol. This buffered solution is at a temperature in the range of 15-40°C, targeting 30-40°C. The resulting LNP suspension is further mixed with a buffered solution at a higher pH, e.g., 6-8 and with a mixing ratio in the range of 1:1 to 1:3 vol:vol, but targeting 1:2 vol:vol. This later buffered solution is at a temperature in the range of 15-40°C, targeting 16-25°C. The mixed LNPs are held from 30 minutes to 2 hours prior to an anion exchange filtration step. The temperature during incubation period is in the range of 15-40°C, targeting 30-40°C. After incubation, the LNP suspension is filtered through a 0.8 µm filter containing an anion exchange separation step. This process uses tubing IDs ranging from 1 mm ID to 5 mm ID and a flow rate from 10 to 2000 mL/minute. The LNPs are concentrated and diafiltered via an ultrafiltration process where the alcohol is removed and the buffer is exchanged for the final buffer solution such as phosphate buffered saline or a buffer system suitable for cryopreservation (for example containing sucrose, trehalose or combinations thereof). The ultrafiltration process uses a tangential flow filtration format (TFF). This process uses a membrane nominal molecular weight cutoff range from 30-500 KD, targeting 100 KD. The membrane format can be hollow fiber or flat sheet cassette. The TFF processes with the proper molecular weight cutoff retains the LNP in the retentate and the filtrate or permeate contains the alcohol and final buffer wastes. The TFF process is a multiple step process with an initial concentration to a lipid concentration of 20-30 mg/mL. Following concentration, the LNP suspension is diafiltered against the final buffer (for example, phosphate buffered saline (PBS) with pH 7-8, 10 mM Tris, 140 mM NaCl with pH 7-8, or 10 mM Tris, 70 mM NaCl, 5 wt% sucrose, with pH 7-8) for 5-20 volumes to remove the alcohol and perform buffer exchange. The material is then concentrated an additional 1-3 fold via ultrafiltration. The final steps of the LNP manufacturing process are to sterile filter the concentrated LNP solution into a suitable container under aseptic conditions. Sterile filtration is accomplished by passing the LNP solution through a pre-filter (Acropak 500 PES 0.45/0.8 µm capsule) and a bioburden reduction filter (Acropak 500 PES 0.2/0.8 µm capsule). Following filtration, the vialed LNP product is stored under suitable storage conditions (2°C-8°C, or -20°C if frozen formulation).

In some embodiments, the LNPs of the compositions provided herein have a mean geometric diameter that is less than 1000 nm. In some embodiments, the LNPs have mean geometric diameter that is greater than 50 nm but less than 500 nm. In some embodiments, the mean geometric diameter of a population of LNPs is about 60 nm, 75 nm, 100 nm, 125 nm, 150 nm, 175 nm, 200 nm, 225 nm, 250 nm, 275 nm, 300 nm, 325 nm, 350 nm, 375 nm, 400 nm, 425 nm, 450 nm, or 475 nm. In some embodiments, the mean geometric diameter is between 100-400 nm, 100-300 nm, 100-250 nm, or 100-200 nm. In some embodiments, the mean geometric diameter is between 60-400 nm, 60-350 nm, 60-300 nm, 60-250 nm, or 60-200 nm. In some embodiments, the mean geometric diameter is between 75-250 nm. In some embodiments, 30%, 40%, 50%, 60%, 70%, 80%, 90%, or more of the LNPs of a population of LNPs have a diameter that is less than 500 nm. In some embodiments, 10%, 20%, 30%, 40%, 50%, 60%, 70%, 80%, 90%, or more of the LNPs of a population of LNPs have a diameter that is greater than 50 nm but less than 500 nm. In some embodiments, 10%, 20%, 30%, 40%, 50%, 60%, 70%, 80%, 90%, or more of the LNPs of a population of LNPs have a diameter of about 60 nm, 75 nm, 100 nm, 125 nm, 150 nm, 175 nm, 200 nm, 225 nm, 250 nm, 275 nm, 300 nm, 325 nm, 350 nm, 375 nm, 400 nm, 425 nm, 450 nm, or 475 nm. In some embodiments, 10%, 20%, 30%, 40%, 50%, 60%, 70%, 80%, 90%, or more of the LNPs of a population of LNPs have a diameter that is between 100-400 nm, 100-300 nm, 100-250 nm, or 100-200 nm. In some embodiments, 10%, 20%, 30%, 40%, 50%, 60%, 70%, 80%, 90%, or more of the LNPs of a population of LNPs have a diameter that is between 60-400 nm, 60-350 nm, 60-300 nm, 60-250 nm, or 60-200 nm.

In a particular embodiment, the size of the LNPs ranges between about 1 and 1000 nm, preferably between about 10 and 500 nm, more preferably between about 100 to 300 nm, and preferably 100 nm.

### Nucleic Acids/Polynucleotides

DNA of the present disclosure, in some embodiments, are codon optimized. Codon optimization methods are known in the art and may be used as provided herein. Codon optimization, in some embodiments, may be used to match codon frequencies in target and host organisms to ensure proper folding; bias GC content to increase mRNA stability or reduce secondary structures; minimize tandem repeat codons or base runs that may impair gene construction or expression; customize transcriptional and translational control regions; insert or remove protein trafficking sequences; remove/add post translation modification sites in encoded protein (e.g. glycosylation sites); add, remove or shuffle protein domains; insert or delete restriction sites; modify ribosome binding sites and mRNA degradation sites; adjust translational rates to allow the various domains of the protein to fold properly; or to reduce or eliminate problem secondary structures within the polynucleotide. Codon optimization tools, algorithms and services are known in the art ― non-limiting examples include services from GeneArt (Life Technologies), DNA2.0 (Menlo Park CA) and/or proprietary methods. In some embodiments, the open reading frame (ORF) sequence is optimized using optimization algorithms.

In some embodiments, a codon optimized sequence shares less than 95% sequence identity, less than 90% sequence identity, less than 85% sequence identity, less than 80% sequence identity, or less than 75% sequence identity to a naturally-occurring or wild-type sequence.

In some embodiments, a codon-optimized sequence shares between 65% and 85% *(e.g.,* between about 67% and about 85%, or between about 67% and about 80%) sequence identity to a naturally-occurring sequence or a wild-type sequence. In some embodiments, a codon-optimized sequence shares between 65% and 75%, or about 80% sequence identity to a naturally-occurring sequence or wild-type sequence.

### Antigens/Antigenic Polypeptides

In some embodiments, an antigenic polypeptide includes gene products, naturally occurring polypeptides, synthetic polypeptides, homologs, orthologs, paralogs, fragments and other equivalents, variants, and analogs of the foregoing. A polypeptide may be a single molecule or may be a multi-molecular complex such as a dimer, trimer or tetramer. Polypeptides may also comprise single chain polypeptides or multichain polypeptides, such as antibodies or insulin, and may be associated or linked to each other. Most commonly, disulfide linkages are found in multichain polypeptides. The term "polypeptide" may also apply to amino acid polymers in which at least one amino acid residue is an artificial chemical analogue of a corresponding naturally-occurring amino acid.

A "polypeptide variant" is a molecule that differs in its amino acid sequence relative to a native sequence or a reference sequence. Amino acid sequence variants may possess substitutions, deletions, insertions, or a combination of any two or three of the foregoing, at certain positions within the amino acid sequence, as compared to a native sequence or a reference sequence. Ordinarily, variants possess at least 50% identity to a native sequence or a reference sequence. In some embodiments, variants share at least 80% identity or at least 90% identity with a native sequence or a reference sequence.

"Analogs" is meant to include polypeptide variants that differ by one or more amino acid alterations, for example, substitutions, additions or deletions of amino acid residues that still maintain one or more of the properties of the parent or starting polypeptide.

The present disclosure provides several types of compositions that are polynucleotide or polypeptide based, including variants and derivatives. These include, for example, substitutional, insertional, deletion and covalent variants and derivatives. The term "derivative" is synonymous with the term "variant" and generally refers to a molecule that has been modified and/or changed in any way relative to a reference molecule or a starting molecule.

As such, polynucleotides encoding peptides or polypeptides containing substitutions, insertions and/or additions, deletions and covalent modifications with respect to reference sequences, in particular the polypeptide sequences disclosed herein, are included within the scope of this disclosure. For example, sequence tags or amino acids, such as one or more lysines, can be added to peptide sequences (e.g., at the N-terminal or C-terminal ends). Sequence tags can be used for peptide detection, purification or localization. Lysines can be used to increase peptide solubility or to allow for biotinylation. Alternatively, amino acid residues located at the carboxy and amino terminal regions of the amino acid sequence of a peptide or protein may optionally be deleted providing for truncated sequences. Certain amino acids *(e.g.,* C-terminal residues or N-terminal residues) alternatively may be deleted depending on the use of the sequence, as for example, expression of the sequence as part of a larger sequence that is soluble, or linked to a solid support.

"Substitutional variants" when referring to polypeptides are those that have at least one amino acid residue in a native or starting sequence removed and a different amino acid inserted in its place at the same position. Substitutions may be single, where only one amino acid in the molecule has been substituted, or they may be multiple, where two or more *(e.g.,* 3, 4 or 5) amino acids have been substituted in the same molecule.

As used herein the term "conservative amino acid substitution" refers to the substitution of an amino acid that is normally present in the sequence with a different amino acid of similar size, charge, or polarity. Examples of conservative substitutions include the substitution of a non-polar (hydrophobic) residue such as isoleucine, valine and leucine for another non-polar residue. Likewise, examples of conservative substitutions include the substitution of one polar (hydrophilic) residue for another such as between arginine and lysine, between glutamine and asparagine, and between glycine and serine. Additionally, the substitution of a basic residue such as lysine, arginine or histidine for another, or the substitution of one acidic residue such as aspartic acid or glutamic acid for another acidic residue are additional examples of conservative substitutions. Examples of non-conservative substitutions include the substitution of a non-polar (hydrophobic) amino acid residue such as isoleucine, valine, leucine, alanine, methionine for a polar (hydrophilic) residue such as cysteine, glutamine, glutamic acid or lysine and/or a polar residue for a non-polar residue.

As used herein when referring to polypeptides the term "domain" refers to a motif of a polypeptide having one or more identifiable structural or functional characteristics or properties (e.g., binding capacity, serving as a site for protein-protein interactions).

As used herein when referring to polypeptides the terms "site" as it pertains to amino acid based embodiments is used synonymously with "amino acid residue" and "amino acid side chain." As used herein when referring to polynucleotides the terms "site" as it pertains to nucleotide based embodiments is used synonymously with "nucleotide." A site represents a position within a peptide or polypeptide or polynucleotide that may be modified, manipulated, altered, derivatized or varied within the polypeptide-based or polynucleotide-based molecules.

As used herein the terms "termini" or "terminus" when referring to polypeptides or polynucleotides refers to an extremity of a polypeptide or polynucleotide respectively. Such extremity is not limited only to the first or final site of the polypeptide or polynucleotide but may include additional amino acids or nucleotides in the terminal regions. Polypeptide-based molecules may be characterized as having both an N-terminus (terminated by an amino acid with a free amino group (NH2)) and a C-terminus (terminated by an amino acid with a free carboxyl group (COOH)). Proteins are in some cases made up of multiple polypeptide chains brought together by disulfide bonds or by non-covalent forces (multimers, oligomers). These proteins have multiple N- and C-termini. Alternatively, the termini of the polypeptides may be modified such that they begin or end, as the case may be, with a non-polypeptide based moiety such as an organic conjugate.

As recognized by those skilled in the art, protein fragments, functional protein domains, and homologous proteins are also considered to be within the scope of polypeptides of interest. For example, provided herein is any protein fragment (meaning a polypeptide sequence at least one amino acid residue shorter than a reference polypeptide sequence but otherwise identical) of a reference protein having a length of 10, 20, 30, 40, 50, 60, 70, 80, 90, 100 or longer than 100 amino acids. In another example, any protein that includes a stretch of 20, 30, 40, 50, or 100 (contiguous) amino acids that are 40%, 50%, 60%, 70%, 80%, 90%, 95%, 96%, 97%, 98%, 99% or 100% identical to any of the sequences described herein can be utilized in accordance with the disclosure. In some embodiments, a polypeptide includes 2, 3, 4, 5, 6, 7, 8, 9, 10, or more mutations as shown in any of the sequences provided herein or referenced herein. In another example, any protein that includes a stretch of 20, 30, 40, 50, or 100 amino acids that are greater than 80%, 90%, 95%, or 100% identical to any of the sequences described herein, wherein the protein has a stretch of 5, 10, 15, 20, 25, or 30 amino acids that are less than 80%, 75%, 70%, 65% to 60% identical to any of the sequences described herein can be utilized in accordance with the disclosure.

Polypeptide or polynucleotide molecules of the present disclosure may share a certain degree of sequence similarity or identity with the reference molecules (e.g., reference polypeptides or reference polynucleotides), for example, with art-described molecules (e.g., engineered or designed molecules or wild-type molecules). The term "identity," as known in the art, refers to a relationship between the sequences of two or more polypeptides or polynucleotides, as determined by comparing the sequences. In the art, identity also means the degree of sequence relatedness between two sequences as determined by the number of matches between strings of two or more amino acid residues or nucleic acid residues. Identity measures the percent of identical matches between the smaller of two or more sequences with gap alignments (if any) addressed by a particular mathematical model or computer program (e.g., "algorithms"). Identity of related peptides can be readily calculated by known methods. "% identity" as it applies to polypeptide or polynucleotide sequences is defined as the percentage of residues (amino acid residues or nucleic acid residues) in the candidate amino acid or nucleic acid sequence that are identical with the residues in the amino acid sequence or nucleic acid sequence of a second sequence after aligning the sequences and introducing gaps, if necessary, to achieve the maximum percent identity. Methods and computer programs for the alignment are well known in the art. Identity depends on a calculation of percent identity but may differ in value due to gaps and penalties introduced in the calculation. Generally, variants of a particular polynucleotide or polypeptide have at least 40%, 45%, 50%, 55%, 60%, 65%, 70%, 75%, 80%, 85%, 90%, 91%, 92%, 93%, 94%, 95%, 96%, 97%, 98%, 99% but less than 100% sequence identity to that particular reference polynucleotide or polypeptide as determined by sequence alignment programs and parameters described herein and known to those skilled in the art. Such tools for alignment include those of the BLAST suite (Stephen F. Altschul, et al. (1997)." Gapped BLAST and PSI-BLAST: a new generation of protein database search programs," Nucleic Acids Res. 25:3389-3402). Another popular local alignment technique is based on the Smith-Waterman algorithm (Smith, T.F. & Waterman, M.S. (1981) "Identification of common molecular subsequences." J. Mol. Biol. 147:195-197). A general global alignment technique based on dynamic programming is the Needleman-Wunsch algorithm (Needleman, S.B. & Wunsch, C.D. (1970) "A general method applicable to the search for similarities in the amino acid sequences of two proteins." J. Mol. Biol. 48:443-453). More recently, a Fast Optimal Global Sequence Alignment Algorithm (FOGSAA) was developed that purportedly produces global alignment of nucleotide and protein sequences faster than other optimal global alignment methods, including the Needleman-Wunsch algorithm. Other tools are described herein, specifically in the definition of "identity" below.

As used herein, the term "homology" refers to the overall relatedness between polymeric molecules, *e.g.* between nucleic acid molecules *(e.g.* DNA molecules) and/or between polypeptide molecules. Polymeric molecules *(e.g.* nucleic acid molecules *(e.g.* DNA molecules) and/or polypeptide molecules) that share a threshold level of similarity or identity determined by alignment of matching residues are termed homologous. Homology is a qualitative term that describes a relationship between molecules and can be based upon the quantitative similarity or identity. Similarity or identity is a quantitative term that defines the degree of sequence match between two compared sequences. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, 96%, 97%, 98% or 99% identical or similar. The term "homologous" necessarily refers to a comparison between at least two sequences (polynucleotide or polypeptide sequences). Two polynucleotide sequences are considered homologous if the polypeptides they encode are at least 50%, 60%, 70%, 80%, 90%, 95%, or even 99% for at least one stretch of at least 20 amino acids. In some embodiments, homologous polynucleotide sequences are characterized by the ability to encode a stretch of at least 4-5 uniquely specified amino acids. For polynucleotide sequences less than 60 nucleotides in length, homology is determined by the ability to encode a stretch of at least 4-5 uniquely specified amino acids. Two protein sequences are considered homologous if the proteins are at least 50%, 60%, 70%, 80%, or 90% identical for at least one stretch of at least 20 amino acids.

Homology implies that the compared sequences diverged in evolution from a common origin. The term "homolog" refers to a first amino acid sequence or nucleic acid sequence (e.g., gene (DNA or RNA) or protein sequence) that is related to a second amino acid sequence or nucleic acid sequence by descent from a common ancestral sequence. The term "homolog" may apply to the relationship between genes and/or proteins separated by the event of speciation or to the relationship between genes and/or proteins separated by the event of genetic duplication. "Orthologs" are genes (or proteins) in different species that evolved from a common ancestral gene (or protein) by speciation. Typically, orthologs retain the same function in the course of evolution. "Paralogs" are genes (or proteins) related by duplication within a genome. Orthologs retain the same function in the course of evolution, whereas paralogs evolve new functions, even if these are related to the original one.

The term "identity" refers to the overall relatedness between polymeric molecules, for example, between polynucleotide molecules (*e*.*g*. DNA molecules) and/or between polypeptide molecules. Calculation of the percent identity of two polynucleic acid sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleic acid sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991; each of which is incorporated herein by reference. For example, the percent identity between two nucleic acid sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleic acid sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988); incorporated herein by reference. Techniques for determining identity are codified in publicly available computer programs. Exemplary computer software to determine homology between two sequences include, but are not limited to, GCG program package, Devereux, J., et al., Nucleic Acids Research, 12, 387 (1984)), BLASTP, BLASTN, and FASTA Altschul, S. F. et al., J. Molec. Biol., 215, 403 (1990)).

### Signal peptides

In some embodiments, antigenic polypeptides comprise a signal peptide. Signal peptides, comprising the N-terminal 15-60 amino acids of proteins, are typically needed for the translocation across the membrane on the secretory pathway and, thus, universally control the entry of most proteins both in eukaryotes and prokaryotes to the secretory pathway. ER processing produces mature proteins, wherein the signal peptide is cleaved from precursor proteins, typically by a ER-resident signal peptidase of the host cell, or they remain uncleaved and function as a membrane anchor. As referred herein, "mature amino acid sequence" does not contain the signal peptide sequence.

### Methods of Treatment

Provided herein are compositions (e.g., pharmaceutical compositions), methods, kits and reagents for prevention and/or treatment of influenza virus in humans and other mammals. Influenza virus vaccines can be used as therapeutic or prophylactic agents. They may be used in medicine to prevent and/or treat infectious disease. In exemplary aspects, the influenza virus vaccines of the present disclosure are used to provide prophylactic protection from influenza virus. Prophylactic protection from influenza virus can be achieved following administration of an influenza virus vaccine of the present disclosure. Vaccines can be administered once, twice, three times, four times or more. It is possible, although less desirable, to administer the vaccine to an infected individual to achieve a therapeutic response. Dosing may need to be adjusted accordingly.

In some embodiments, the influenza virus vaccines of the present disclosure can be used as a method of preventing an influenza virus infection in a subject, the method comprising administering to said subject at least one influenza virus vaccine as provided herein. In some embodiments, the influenza virus vaccines of the present disclosure can be used as a method of inhibiting a primary influenza virus infection in a subject, the method comprising administering to said subject at least one influenza virus vaccine as provided herein. In some embodiments, the influenza virus vaccines of the present disclosure can be used as a method of treating an influenza virus infection in a subject, the method comprising administering to said subject at least one influenza virus vaccine as provided herein. In some embodiments, the influenza virus vaccines of the present disclosure can be used as a method of reducing an incidence of influenza virus infection in a subject, the method comprising administering to said subject at least one influenza virus vaccine as provided herein. In some embodiments, the influenza virus vaccines of the present disclosure can be used as a method of inhibiting spread of influenza virus from a first subject infected with influenza virus to a second subject not infected with influenza virus, the method comprising administering to at least one of said first subject sand said second subject at least one influenza virus vaccine as provided herein.

A method of eliciting an immune response in a subject against an influenza virus is provided in aspects of the invention. The method involves administering to the subject an influenza virus vaccine described herein, thereby inducing in the subject an immune response specific to influenza virus antigenic polypeptide or an immunogenic fragment thereof.

A prophylactically effective dose is a therapeutically effective dose that prevents infection with the virus at a clinically acceptable level. In some embodiments the therapeutically effective dose is a dose listed in a package insert for the vaccine.

### Therapeutic and Prophylactic Compositions

Provided herein are compositions (e.g., pharmaceutical compositions), methods, kits and reagents for prevention, treatment or diagnosis of influenza in humans and other mammals, for example. Influenza virus vaccines can be used as therapeutic or prophylactic agents. They may be used in medicine to prevent and/or treat infectious disease. In some embodiments, the respiratory vaccines of the present disclosure are used for the priming of immune effector cells, for example, to activate peripheral blood mononuclear cells (PBMCs) *ex vivo,* which are then infused (re-infused) into a subject. In some embodiments, vaccines in accordance with the present disclosure may be used for treatment of Influenza.

Influenza virus vaccines may be administered prophylactically or therapeutically as part of an active immunization scheme to healthy individuals or early in infection during the incubation phase or during active infection after onset of symptoms. In some embodiments, the amount of vaccine of the present disclosure provided to a cell, a tissue or a subject may be an amount effective for immune prophylaxis.

Influenza virus vaccines may be administrated with other prophylactic or therapeutic compounds. As a non-limiting example, a prophylactic or therapeutic compound may be an adjuvant or a booster. As used herein, when referring to a prophylactic composition, such as a vaccine, the term "booster" refers to an extra administration of the prophylactic (vaccine) composition. A booster (or booster vaccine) may be given after an earlier administration of the prophylactic composition. The time of administration between the initial administration of the prophylactic composition and the booster may be, but is not limited to, 1 minute, 2 minutes, 3 minutes, 4 minutes, 5 minutes, 6 minutes, 7 minutes, 8 minutes, 9 minutes, 10 minutes, 15 minutes, 20 minutes 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 36 hours, 2 days, 3 days, 4 days, 5 days, 6 days, 1 week, 10 days, 2 weeks, 3 weeks, 1 month, 2 months, 3 months, 4 months, 5 months, 6 months, 7 months, 8 months, 9 months, 10 months, 11 months, 1 year, 18 months, 2 years, 3 years, 4 years, 5 years, 6 years, 7 years, 8 years, 9 years, 10 years, 11 years, 12 years, 13 years, 14 years, 15 years, 16 years, 17 years, 18 years, 19 years, 20 years, 25 years, 30 years, 35 years, 40 years, 45 years, 50 years, 55 years, 60 years, 65 years, 70 years, 75 years, 80 years, 85 years, 90 years, 95 years or more than 99 years. In some embodiments, the time of administration between the initial administration of the prophylactic composition and the booster may be, but is not limited to, 1 week, 2 weeks, 3 weeks, 1 month, 2 months, 3 months, 6 months or 1 year.

In some embodiments, influenza virus vaccines may be administered intramuscularly, intradermally, or intranasally, similarly to the administration of inactivated vaccines known in the art. In some embodiments, influenza virus vaccines are administered intramuscularly.

Influenza virus vaccines may be utilized in various settings depending on the prevalence of the infection or the degree or level of unmet medical need. As a non-limiting example, the Vaccines may be utilized to treat and/or prevent a variety of influenzas. Vaccines have superior properties in that they produce much larger antibody titers and produce responses early than commercially available anti-viral agents/compositions.

Provided herein are pharmaceutical compositions including influenza virus vaccines optionally in combination with one or more pharmaceutically acceptable excipients.

Influenza virus vaccines may be formulated or administered alone or in conjunction with one or more other components. For instance, Influenza virus vaccines (vaccine compositions) may comprise other components including, but not limited to, adjuvants.

In some embodiments, influenza vaccines do not include an adjuvant (they are adjuvant free).

Aluminium has long been shown to stimulate the immune response against co-administered antigens, primarily by stimulating a TH2 response. It is preferred that the aluminium adjuvant of the compositions provided herein is not in the form of an aluminium precipitate. Aluminium-precipitated vaccines may increase the immune response to a target antigen, but have been shown to be highly heterogeneous preparations and have had inconsistent results {see Lindblad E.B. Immunology and Cell Biology 82: 497-505 (2004)). Aluminium-adsorbed vaccines, in contrast, can be preformed in a standardized manner, which is an essential characteristic of vaccine preparations for administration into humans. Moreover, it is thought that physical adsorption of a desired antigen onto the aluminium adjuvant has an important role in adjuvant function, perhaps in part by allowing a slower clearing from the injection site or by allowing a more efficient uptake of antigen by antigen presenting cells.

The aluminium adjuvant of the present invention may be in the form of aluminium hydroxide (Al(OH)₃), aluminium phosphate (AlPO₄), aluminium hydroxyphosphate, amorphous aluminium hydroxyphosphate sulfate (AAHS) or so-called "alum" (KA1(S04)-12H20) {see Klein et al, Analysis of aluminium hydroxyphosphate vaccine adjuvants by (27)A1 MAS NMR., J. Pharm. Sci. 89(3): 311-21 (2000)). In exemplary embodiments of the invention provided herein, the aluminium adjuvant is aluminium hydroxyphosphate or AAHS. The ratio of phosphate to aluminium in the aluminium adjuvant can range from 0 to 1.3. In preferred embodiments of this aspect of the invention, the phosphate to aluminium ratio is within the range of 0.1 to 0.70. In particularly preferred embodiments, the phosphate to aluminium ratio is within the range of 0.2 to 0.50. APA is an aqueous suspension of aluminum hydroxyphosphate. APA is manufactured by blending aluminum chloride and sodium phosphate in a 1:1 volumetric ratio to precipitate aluminum hydroxyphosphate. After the blending process, the material is size-reduced with a high-shear mixer to achieve a target aggregate particle size in the range of 2-8 µm. The product is then diafiltered against physiological saline and steam sterilized. See, e.g., International Patent Application Publication No. WO2013/078102.

In some embodiments of the invention, the aluminium adjuvant is in the form of AAHS (referred to interchangeably herein as Merck aluminium adjuvant (MAA)). MAA carries zero charge at neutral pH, while AIOH carries a net positive charge and AIPO₄ typically carries a net negative charge at neutral pH.

One of skill in the art will be able to determine an optimal dosage of aluminium adjuvant that is both safe and effective at increasing the immune response to the targeted antigenic polypeptides. For a discussion of the safety profile of aluminium, as well as amounts of aluminium included in FDA-licensed vaccines, see Baylor et al., Vaccine 20: S18-S23 (2002). Generally, an effective and safe dose of aluminium adjuvant varies from 150 to 600 µg/dose (300 to 1200 µg/mL concentration). In specific embodiments of the formulations and compositions of the present invention, there is between 200 and 300 µg aluminium adjuvant per dose of vaccine. In alternative embodiments of the formulations and compositions of the present invention, there is between 300 and 500 µg aluminium adjuvant per dose of vaccine.

Influenza virus vaccines may be formulated or administered in combination with one or more pharmaceutically-acceptable excipients. In some embodiments, vaccine compositions comprise at least one additional active substances, such as, for example, a therapeutically-active substance, a prophylactically-active substance, or a combination of both. Vaccine compositions may be sterile, pyrogen-free or both sterile and pyrogen-free. General considerations in the formulation and/or manufacture of pharmaceutical agents, such as vaccine compositions, may be found, for example, in Remington: The Science and Practice of Pharmacy 21st ed., Lippincott Williams & Wilkins, 2005 (incorporated herein by reference in its entirety). In some embodiments, influenza virus vaccines are administered to humans, human patients or subjects.

Formulations of the influenza vaccine compositions described herein may be prepared by any method known or hereafter developed in the art of pharmacology. In general, such preparatory methods include the step of bringing the active ingredient (e.g., polypeptide or polynucleotide) into association with an excipient and/or one or more other accessory ingredients, and then, if necessary and/or desirable, dividing, shaping and/or packaging the product into a desired single- or multi-dose unit.

Relative amounts of the active ingredient, the pharmaceutically acceptable excipient, and/or any additional ingredients in a pharmaceutical composition in accordance with the disclosure will vary, depending upon the identity, size, and/or condition of the subject treated and further depending upon the route by which the composition is to be administered. By way of example, the composition may comprise between 0.1% and 100%, e.g., between 0.5 and 50%, between 1-30%, between 5-80%, at least 80% (w/w) active ingredient.

### Modes of Vaccine Administration

Influenza vaccines may be administered by any route which results in a therapeutically effective outcome. These include, but are not limited, to intradermal, intramuscular, intranasal and/or subcutaneous administration. The present disclosure provides methods comprising administering vaccines to a subject in need thereof. The exact amount required will vary from subject to subject, depending on the species, age, and general condition of the subject, the severity of the disease, the particular composition, its mode of administration, its mode of activity, and the like. Influenza vaccines compositions are typically formulated in dosage unit form for ease of administration and uniformity of dosage. It will be understood, however, that the total daily usage of vaccine compositions may be decided by the attending physician within the scope of sound medical judgment. The specific therapeutically effective, prophylactically effective, or appropriate imaging dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

In some embodiments, influenza vaccines compositions may be administered at dosage levels sufficient to deliver 0.0001 mg/kg to 100 mg/kg, 0.001 mg/kg to 0.05 mg/kg, 0.005 mg/kg to 0.05 mg/kg, 0.001 mg/kg to 0.005 mg/kg, 0.05 mg/kg to 0.5 mg/kg, 0.01 mg/kg to 50 mg/kg, 0.1 mg/kg to 40 mg/kg, 0.5 mg/kg to 30 mg/kg, 0.01 mg/kg to 10 mg/kg, 0.1 mg/kg to 10 mg/kg, or 1 mg/kg to 25 mg/kg, of subject body weight per day, one or more times a day, per week, per month, etc. to obtain the desired therapeutic, diagnostic, prophylactic, or imaging effect (*see, e.g.,* the range of unit doses described in International Publication No WO2013078199, the contents of which are herein incorporated by reference in their entirety). The desired dosage may be delivered three times a day, two times a day, once a day, every other day, every third day, every week, every two weeks, every three weeks, every four weeks, every 2 months, every three months, every 6 months, *etc.* In some embodiments, the desired dosage may be delivered using multiple administrations (e.g., two, three, four, five, six, seven, eight, nine, ten, eleven, twelve, thirteen, fourteen, or more administrations). When multiple administrations are employed, split dosing regimens such as those described herein may be used. In exemplary embodiments, influenza vaccines compositions may be administered at dosage levels sufficient to deliver 0.0005 mg/kg to 0.01 mg/kg, *e.g.,* about 0.0005 mg/kg to about 0.0075 mg/kg, *e.g.,* about 0.0005 mg/kg, about 0.001 mg/kg, about 0.002 mg/kg, about 0.003 mg/kg, about 0.004 mg/kg or about 0.005 mg/kg.

In some embodiments, influenza vaccine compositions may be administered once or twice (or more) at dosage levels sufficient to deliver 0.025 mg/kg to 0.250 mg/kg, 0.025 mg/kg to 0.500 mg/kg, 0.025 mg/kg to 0.750 mg/kg, or 0.025 mg/kg to 1.0 mg/kg.

An influenza vaccine pharmaceutical composition described herein can be formulated into a dosage form described herein, such as an intranasal, intratracheal, or injectable (e.g., intravenous, intraocular, intravitreal, intramuscular, intradermal, intracardiac, intraperitoneal, intranasal and subcutaneous).

### Influenza Virus vaccine formulations and methods of use

Some aspects of the present disclosure provide formulations of the influenza vaccine, wherein the vaccine is formulated in an effective amount to produce an antigen specific immune response in a subject (e.g., production of antibodies specific to an influenza antigenic polypeptide). "An effective amount" is a dose of a vaccine effective to produce an antigen-specific immune response. Also provided herein are methods of inducing an antigen-specific immune response in a subject.

In some embodiments, the antigen-specific immune response is characterized by measuring an anti- influenza antigenic polypeptide antibody titer produced in a subject administered an influenza vaccine as provided herein. An antibody titer is a measurement of the amount of antibodies within a subject, for example, antibodies that are specific to a particular antigen *(e.g.,* an influenza antigenic polypeptide) or epitope of an antigen. Antibody titer is typically expressed as the inverse of the greatest dilution that provides a positive result. Enzyme-linked immunosorbent assay (ELISA) is a common assay for determining antibody titers, for example.

In some embodiments, an antibody titer is used to assess whether a subject has had an infection or to determine whether immunizations are required. In some embodiments, an antibody titer is used to determine the strength of an autoimmune response, to determine whether a booster immunization is needed, to determine whether a previous vaccine was effective, and to identify any recent or prior infections. In accordance with the present disclosure, an antibody titer may be used to determine the strength of an immune response induced in a subject by the influenza vaccine.

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

### EXAMPLES

The first underlined sequence for each of the amino acid sequences listed in Table 1, indicates a signal or secretory sequence, which may be substituted by an alternative sequence that achieves the same or similar function, or the signal or secretory sequence may be deleted. Other underlined sequences for the amino acid sequences listed in Table 1, indicates a foldon sequence, which is a heterologous sequence that naturally trimerizes, to bring 3 HA stems together in a trimer; a ferritin; or transmembrane sequence. Such foldon , ferritin or transmembrane sequence may be substituted by an alternative sequence, which achieves the same or similar function.

**Table 1: Antigenic Polypeptide Sequences**

| Name | **Sequence** | **SEQ ID NO:** |
|---|---|---|
| BHA10-2: HA10 version for Influenza B/Bnsbane/60/2008 strain, with exposed hydrophobic residues mutated I333T, M432S, L435T (bold/underlined) and foldon sequence (second underlined) | METPAQLLFLLLLWLPDTTGHVVKTATQGEVNVT GVIPLTTTPTGSANKSKPYYTGEHAKATGNCPIWV KTPLKLANGTKYGSAGSATQEAINKITKNLNSLSEL EVKNLQRLSGASDETHNEILELDEKVDDLRADTISS QIELAVLLSNEGIINSEDEGTGGGYIPEAPRDGQAY VRKDGEWVLLSTFL | 1 |
| BHA10-3:BHA10-2 without GTGG linker or foldon domain, with G430C, E438C, Q457L mutations (bold) for trimerization | METPAQLLFLLLLWLPDTTGHVVKTATQGEVNVT GVIPLTTTPTGSANKSKPYYTGEHAKATGNCPIWV KTPLKLANGTKYGSAGSATQEAINKITKNLNSLSEL EVKNLQRLSCASDETHNCILELDEKVDDLRADTIS SLIELAVLLSNEGIINSEDE | 2 |
| NIHGen6HASS-TM: Gen6 HA SS construct without foldon or ferritin, linker (bold) with transmembrane domain (second underlined), version 1 | METPAQLLFLLLLWLPDTTGDTICIGYHANNSTDT VDTVLEKNVTVTHSVNLGSGLRMVTGLRNIPQRET RGLFGAIAGFIEGGWTGMVDGWYGYHHQNEQGS GYAADQKSTQNAINGITNMVNSVIEKMGSGGSGT DLAELLVLLLNERTLDFHDSNVKNLYEKVKSQLK NNAKEIGNGCFEFYHKCNNECMESVKNGTYDYPK YSEESKLNREKIDQGTGGILAIYSTVASSLVLLVSL GAISFWMCSNGSLQCRICI | 3 |
| NIHGen6HASS-TM2: Gen6 HA SS construct without foldon or ferritin, linker (bold), with transmembrane domain (second underlined), version 2 | METPAQLLFLLLLWLPDTTGDTICIGYHANNSTDT VDTVLEKNVTVTHSVNLGSGLRMVTGLRNIPQRET RGLFGAIAGFIEGGWTGMVDGWYGYHHQNEQGS GYAADQKSTQNAINGITNMVNSVIEKMGSGGSGT DLAELLVLLLNERTLDFHDSNVKNLYEKVKSQLK NNAKEIGNGCFEFYHKCNNECMESVKNGTYDYPK YSEESKLNREKIDGVKLESMGVVQILAIYSTVASS LVLLVSLGAISFWMCSNGSLQCRICI | 4 |
| NIHGen6HASS-foldon: Gen6 HA SS construct with foldon sequence (second underlined) | METPAQLLFLLLLWLPDTTGDTICIGYHANNSTDT VDTVLEKNVTVTHSVNLGSGLRMVTGLRNIPQRET RGLFGAIAGFIEGGWTGMVDGWYGYHHQNEQGS GYAADQKSTQNAINGITNMVNSVIEKMGSGGSGT DLAELLVLLLNERTLDFHDSNVKNLYEKVKSQLK NNAKEIGNGCFEFYHKCNNECMESVKNGTYDYPK YSEESKLNREKIDPGSGYIPEAPRDGQAYVRKDGE WVLLSTFL | 5 |
| ConH1: consensus HA sequence for subtype H1 with transmembrane domain (second underlined) | MKAKLLVLLCAFTATDADTICIGYHANNSTDTVDT VLEKNVTVTHSVNLLEDSHNGKLCKLKGIAPLQLG KCNIAGWILGNPECESLISKRSWSYIVETPNSENGT CYPGDFADYEELREQLSSVSSFERFEIFPKESSWPN HNVTKGVTAACSHAGKSSFYRNLLWLTEKNGSYP KLSKSYVNNKEKEVLVLWGVHHPSNITDQRTLYQ NENAYVSVVSSHYNRRFTPEIAKRPKVRGQAGRIN YYWTLLEPGDTIIFEANGNLIAPWYAFALSRGFGSG IITSNAPMHECDTKCQTPQGAINSSLPFQNVHPVTI GECPKYVRSTKLRMVTGLRNIPSIQSRGLFGAIAGF IEGGWTGMIDGWYGYHHQNEQGSGYAADQKSTQ NAINGITNKVNSVIEKMNTQFTAVGKEFNKLEKRM ENLNKKVDDGFLDIWTYNAELLVLLENERTLDFH DSNVKNLYEKVKSQLKNNAKEIGNGCFEFYHKCN NECMESVKNGTYDYPKYSEESKLNREKIDGVKLES MGVYQILAIYSTVASSLVLLVSLGAISFWMCSNGS LQCRICI | 6 |
| ConH3: consensus HA sequence for subtype H3 with transmembrane domain (second underlined) | MKTIIALSYIFCLVFAQKLPGNDNSTATLCLGHHAV PNGTLVKTITNDQIEVTNATELVQSSSTGRICDSPH RILDGTNCTLIDALLGDPHCDGFQNKEWDLFVERS KAYSNCYPYDVPDYASLRSLVASSGTLEFNNEGFN WTGVTQNGGSSACKRGSDKSFFSRLNWLHKLKYK YPALNVTMPNNDKFDKLYIWGVHHPSTDSDQTSL | 7 |
| | YVQASGRVTVSTKRSQQTVIPNIGSRPWVRGLSSRI SIYWTIVKPGDILLINSTGNLIAPRGYFKIRSGKSSIM RSDAPIGTCNSECITPNGSIPNDKPFQNVNRITYGAC PRYVKQNTLKLATGMRNVPEKQTRGIFGAIAGFIE NGWEGMVDGWYGFRHQNSEGTGQAADLKSTQA AIDQINGKLNRLIEKTNEKFHQIEKEFSEVEGRIQDL EKYVEDTKIDLWSYNAELLVALENQHTIDLTDSEM NKLFERTRKQLRENAEDMGNGCFKIYHKCDNACI GSIRNGTYDHDVYRDEALNNRFQIKGVELKSGYK DWILWISFAISCFLLCVVLLGFIMWACQKGNIRCNI CI | |
| MRK_pH1_Con: consensus HA sequence for pandemic H1 strains, includes transmembrane sequence (second underlined) | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETSSSDNG TCYPGDFIDYEELREQLSSVSSFERFEIFPKTSSWPN HDSNKGVTAACPHAGAKSFYKNLIWLVKKGNSYP KLSKSYINDKGKEVLVLWGIHHPSTSADQQSLYQN ADAYVFVGTSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG KCPKYVKSTKLRLATGLRNVPSIQSRGLFGAIAGFI EGGWTGMVDGWYGYHHQNEQGSGYAADLKSTQ NAIDKITNKVNSVIEKMNTQFTAVGKEFNHLEKRIE NLNKKVDDGFLDIWTYNAELLVLLENERTLDYHD SNVKNLYEKVRSQLKNNAKEIGNGCFEFYHKCDN TCMESVKNGTYDYPKYSEEAKLNREEIDGVKLEST RIYQILAIYSTVASSLVLVVSLGAISFWMCSNGSLQC RICI | 8 |
| MRK_pH1_Con: consensus HA sequence for pandemic H1 strains, extracellular domain (italics indicate other truncation sites for extracellular domain) | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETSSSDNG TCYPGDFIDYEELREQLSSVSSFERFEIFPKTSSWPN HDSNKGVTAACPHAGAKSFYKNLIWLVKKGNSYP KLSKSYINDKGKEVLVLWGIHHPSTSADQQSLYQN ADAYVFVGTSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG KCPKYVKSTKLRLATGLRNVPSIQSRGLFGAIAGFI EGGWTGMVDGWYGYHHQNEQGSGYAADLKSTQ NAIDKITNKVNSVIEKMNTQFTAVGKEFNHLEKRIE NLNKKVDDGFLDIWTYNAELLVLLENERTLDYHD SNVKNLYEKVRSQLKNNAKEIGNGCFEFYHKCDN TCMESVKNGTYDYPKYSEEAKLNREEIDGVKLEST RIYQ | 9 |
| MRK_sH1_Con: consensus HA sequence for seasonal H1 strains, includes transmembrane sequence (second underlined) | MKVKLLVLLCTFATYADTICIGYHANNSTDTVDT VLEKNVTVTHSVNLLEDSHNGKLCLLKGIAPLQLG NCSVAGWILGNPECELLISKESWSYIVETPNPENGT CYPGYFADYEELREQLSSVSSFERFEIFPKESSWPN HTVTGVSASCSHNGKSSFYRNLLWLTGKNGLYPN LSKSYANNKEKEVLVLWGVHHPPNIGDQRALYHT ENAYVSVVSSHYSRRFTPEIAKRPKVRDQEGRINY YWTLLEPGDTIIFEANGNLIAPRYAFALSRGFGSGII TSNAPMDECDAKCQTPQGAINSSLPFQNVHPVTIG ECPKYVRSAKLRMVTGLRNIPSIQSRGLFGAIAGFI EGGWTGMVDGWYGYHHQNEQGSGYAADQKSTQ | 10 |
| | NAINGITNKVNSVIEKMNTQFTAVGKEFNKLERRM ENLNKKVDDGFLDIWTYNAELLVLLENERTLDFH DSNVKNLYEKVKSQLKNNAKEIGNGCFEFYHKCN DECMESVKNGTYDYPKYSEESKLNREKIDGVKLES MGVYQILAIYSTVASSLVLLVSLGAISFWMCSNGSL QCRICI | |
| MRK_sH1_Con: consensus HA sequence for seasonal H1 strains, extracellular domain (italics indicate other truncation sites for extracellular domain) | MKVKLLVLLCTFATYADTICIGYHANNSTDTVDT VLEKNVTVTHSVNLLEDSHNGKLCLLKGIAPLQLG NCSVAGWILGNPECELLISKESWSYIVETPNPENGT CYPGYFADYEELREQLSSVSSFERFEIFPKESSWPN HTVTGVSASCSHNGKSSFYRNLLWLTGKNGLYPN LSKSYANNKEKEVLVLWGVHHPPNIGDQRALYHT ENAYVSVVSSHYSRRFTPEIAKRPKVRDQEGRINY YWTLLEPGDTIIFEANGNLIAPRYAFALSRGFGSGII TSNAPMDECDAKCQTPQGAINSSLPFQNVHPVTIG ECPKYVRSAKLRMVTGLRNIPSIQSRGLFGAIAGFI EGGWTGMVDGWYGYHHQNEQGSGYAADQKSTQ NAINGITNKVNSVIEKMNTQFTAVGKEFNKLERRM ENLNKKVDDGFLDIWTYNAELLVLLENERTLDFH DSNVKNLYEKVKSQLKNNAKEIGNGCFEFYHKCN DECMESVKNGTYDYPKYSEESKLNREKIDGVKLES MGVYQ | 11 |
| Cobra P1: consensus HA sequence P1 for H1 subtype with transmembrane domain (second underlined) | MKARLLVLLCALAATDADTICIGYHANNSTDTVDT VLEKNVTVTHSVNLLEDSHNGKLCKLKGIAPLQLG KCNIAGWLLGNPECESLLSARSWSYIVETPNSENG TCYPGDFIDYEELREQLSSVSSFERFEIFPKESSWPN HNTTKGVTAACSHAGKSSFYRNLLWLTKKGGSYP KLSKSYVNNKGKEVLVLWGVHHPSTSTDQQSLYQ NENAYVSVVSSNYNRRFTPEIAERPKVRGQAGRM NYYWTLLEPGDTIIFEATGNLIAPWYAFALSRGSGS GIITSNASMHECNTKCQTPQGAINSSLPFQNIHPVTI GECPKYVRSTKLRMVTGLRNIPSIQSRGLFGAIAGF IEGGWTGMIDGWYGYHHQNEQGSGYAADQKSTQ NAINGITNKVNSVIEKMNTQFTAVGKEFNNLEKRM ENLNKKVDDGFLDIWTYNAELLVLLENERTLDFH DSNVKNLYEKVKSQLRNNAKEIGNGCFEFYHKCD NECMESVKNGTYDYPKYSEESKLNREKIDGVKLES MGVYQILAIYSTVASSLVLLVSLGAISFWMCSNGS LQCRICI | 12 |
| Cobra_X3: consensus HA sequence X3 for H1 subtype with transmembrane domain (second underlined) | MEARLLVLLCAFAATNADTICIGYHANNSTDTVDT VLEKNVTVTHSVNLLEDSHNGKLCRLKGIAPLQLG NCSVAGWILGNPECESLFSKESWSYIAETPNPENGT CYPGYFADYEELREQLSSVSSFERFEIFPKESSWPN HTVTKGVTASCSHNGKSSFYRNLLWLTEKNGLYP NLSKSYVNNKEKEVLVLWGVHHPSNIGDQRAIYH TENAYVSVVSSHYSRRFTPEIAKRPKVRDQEGRIN YYWTLLEPGDTIIFEANGNLIAPWYAFALSRGFGSG IITSNASMDECDAKCQTPQGAINSSLPFQNVHPVTI GECPKYVRSTKLRMVTGLRNIPSIQSRGLFGAIAGF IEGGWTGMIDGWYGYHHQNEQGSGYAADQKSTQ NAINGITNKVNSVIEKMNTQFTAVGKEFNKLERRM ENLNKKVDDGFLDIWTYNAELLVLLENERTLDFH DSNVKNLYEKVKSQLKNNAKEIGNGCFEFYHKCN NECMESVKNGTYDYPKYSEESKLNREKIDGVKLES MGVYQILAIYSTVASSLVLLVSLGAISFWMCSNGS LQCRICI | 13 |
| ConH1 ferritin: consensus HA sequence for subtype H1, linker (bold), with ferritin for particle formation (second underlined) | MKAKLLVLLCAFTATDADTICIGYHANNSTDTVDT VLEKNVTVTHSVNLLEDSHNGKLCKLKGIAPLQLG KCNIAGWILGNPECESLISKRSWSYIVETPNSENGT CYPGDFADYEELREQLSSVSSFERFEIFPKESSWPN HNVTKGVTAACSHAGKSSFYRNLLWLTEKNGSYP KLSKSYVNNKEKEVLVLWGVHHPSNITDQRTLYQ NENAYVSVVSSHYNRRFTPEIAKRPKVRGQAGRIN YYWTLLEPGDTIIFEANGNLIAPWYAFALSRGFGSG IITSNAPMHECDTKCQTPQGAINSSLPFQNVHPVTI GECPKYVRSTKLRMVTGLRNIPSIQSRGLFGAIAGF IEGGWTGMIDGWYGYHHQNEQGSGYAADQKSTQ NAINGITNKVNSVIEKMNTQFTAVGKEFNKLEKRM ENLNKKVDDGFLDIWTYNAELLVLLENERTLDFH DSNVKNLYEKVKSQLKNNAKEIGNGCFEFYHKCN NECMESVKNGTYDYPKYSEESKLNREKIDSGGDII KLLNEQVNKEMQSSNLYMSMSSWCYTHSLDGAG LFLFDHAAEEYEHAKKLIIFLNENNVPVQLTSISAPE HKFEGLTQIFQKAYEHEQHISESINNIVDHAIKSKD HATFNFLQWYVAEQHEEEVLFKDILDKIELIGNEN HGLYLADQYVKGIAKSRKS | 14 |
| ConH3_ferritin: consensus HA sequence for subtype H3, linker (bold), with ferritin for particle formation (second underlined) | MKTIIALSYIFCLVFAQKLPGNDNSTATLCLGHHAV PNGTLVKTITNDQIEVTNATELVQSSSTGRICDSPH RILDGTNCTLIDALLGDPHCDGFQNKEWDLFVERS KAYSNCYPYDVPDYASLRSLVASSGTLEFNNEGFN WTGVTQNGGSSACKRGSDKSFFSRLNWLHKLKYK YPALNVTMPNNDKFDKLYIWGVHHPSTDSDQTSL YVQASGRVTVSTKRSQQTVIPNIGSRPWVRGLSSRI SIYWTIVKPGDILLINSTGNLIAPRGYFKIRSGKSSIM RSDAPIGTCNSECITPNGSIPNDKPFQNVNRITYGAC PRYVKQNTLKLATGMRNVPEKQTRGIFGAIAGFIE NGWEGMVDGWYGFRHQNSEGTGQAADLKSTQA AIDQINGKLNRLIEKTNEKFHQIEKEFSEVEGRIQDL EKYVEDTKIDLWSYNAELLVALENQHTIDLTDSEM NKLFERTRKQLRENAEDMGNGCFKIYHKCDNACI GSIRNGTYDHDVYRDEALNNRFQIKSGGDIIKLLN EQVNKEMQSSNLYMSMSSWCYTHSLDGAGLFLFD HAAEEYEHAKKLIIFLNENNVPVQLTSISAPEHKFE GLTQIFQKAYEHEQHISESINNIVDHAIKSKDHATF NFLQWYVAEQHEEEVLFKDILDKIELIGNENHGLY LADQYVKGIAKSRKS | 15 |
| Merck_pH1_Con_ferritin: consensus HA sequence for pandemic H1 strains, linker (bold), with ferritin for particle formation (second underlined) | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETSSSDNG TCYPGDFIDYEELREQLSSVSSFERFEIFPKTSSWPN HDSNKGVTAACPHAGAKSFYKNLIWLVKKGNSYP KLSKSYINDKGKEVLVLWGIHHPSTSADQQSLYQN ADAYVFVGTSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG KCPKYVKSTKLRLATGLRNVPSIQSRGLFGAIAGFI EGGWTGMVDGWYGYHHQNEQGSGYAADLKSTQ NAIDKITNKVNSVIEKMNTQFTAVGKEFNHLEKRIE NLNKKVDDGFLDIWTYNAELLVLLENERTLDYHD SNVKNLYEKVRSQLKNNAKEIGNGCFEFYHKCDN TCMESVKNGTYDYPKYSEEAKLNREEIDSGGDIIK LLNEQVNKEMQSSNLYMSMSSWCYTHSLDGAGLF | 16 |
| | LFDHAAEEYEHAKKLIIFLNENNVPVQLTSISAPEH KFEGLTQIFQKAYEHEQHISESINNIVDHAIKSKDH ATFNFLQWYVAEQHEEEVLFKDILDKIELIGNENH GLYLADQYVKGIAKSRKS | |
| Merck_sH1_Con_ferritin: consensus HA sequence for seasonal H1 strains, linker (bold), with ferritin for particle formation (second underlined) | MKVKLLVLLCTFATYADTICIGYHANNSTDTVDT VLEKNVTVTHSVNLLEDSHNGKLCLLKGIAPLQLG NCSVAGWILGNPECELLISKESWSYIVETPNPENGT CYPGYFADYEELREQLSSVSSFERFEIFPKESSWPN HTVTGVSASCSHNGKSSFYRNLLWLTGKNGLYPN LSKSYANNKEKEVLVLWGVHHPPNIGDQRALYHT ENAYVSVVSSHYSRRFTPEIAKRPKVRDQEGRINY YWTLLEPGDTIIFEANGNLIAPRYAFALSRGFGSGII TSNAPMDECDAKCQTPQGAINSSLPFQNVHPVTIG ECPKYVRSAKLRMVTGLRNIPSIQSRGLFGAIAGFI EGGWTGMVDGWYGYHHQNEQGSGYAADQKSTQ NAINGITNKVNSVIEKMNTQFTAVGKEFNKLERRM ENLNKKVDDGFLDIWTYNAELLVLLENERTLDFH DSNVKNLYEKVKSQLKNNAKEIGNGCFEFYHKCN DECMESVKNGTYDYPKYSEESKLNREKIDSGGDII KLLNEQVNKEMQSSNLYMSMSSWCYTHSLDGAG LFLFDHAAEEYEHAKKLIIFLNENNVPVQLTSISAPE HKFEGLTQIFQKAYEHEQHISESINNIVDHAIKSKD HATFNFLQWYVAEQHEEEVLFKDILDKIELIGNEN HGLYLADQYVKGIAKSRKS | 17 |
| Cobra_P1_ferritin: consensus HA sequence P1 for H1 subtype, linker (bold), with ferritin for particle formation (second underlined) | MKARLLVLLCALAATDADTICIGYHANNSTDTVDT VLEKNVTVTHSVNLLEDSHNGKLCKLKGIAPLQLG KCNIAGWLLGNPECESLLSARSWSYIVETPNSENG TCYPGDFIDYEELREQLSSVSSFERFEIFPKESSWPN HNTTKGVTAACSHAGKSSFYRNLLWLTKKGGSYP KLSKSYVNNKGKEVLVLWGVHHPSTSTDQQSLYQ NENAYVSVVSSNYNRRFTPEIAERPKVRGQAGRM NYYWTLLEPGDTIIFEATGNLIAPWYAFALSRGSGS GIITSNASMHECNTKCQTPQGAINSSLPFQNIHPVTI GECPKYVRSTKLRMVTGLRNIPSIQSRGLFGAIAGF IEGGWTGMIDGWYGYHHQNEQGSGYAADQKSTQ NAINGITNKVNSVIEKMNTQFTAVGKEFNNLEKRM ENLNKKVDDGFLDIWTYNAELLVLLENERTLDFH DSNVKNLYEKVKSQLRNNAKEIGNGCFEFYHKCD NECMESVKNGTYDYPKYSEESKLNREKIDSGGDII KLLNEQVNKEMQSSNLYMSMSSWCYTHSLDGAG LFLFDHAAEEYEHAKKLIIFLNENNVPVQLTSISAPE HKFEGLTQIFQKAYEHEQHISESINNIVDHAIKSKD HATFNFLQWYVAEQHEEEVLFKDILDKIELIGNEN HGLYLADQYVKGIAKSRKS | 18 |
| Cobra_X3_ferritin: consensus HA sequence X3 for H1 subtype, linker (bold), with ferritin for particle formation (second underlined) | MEARLLVLLCAFAATNADTICIGYHANNSTDT VDTVLEKNVTVTHSVNLLEDSHNGKLCRLKG IAPLQLGNCSVAGWILGNPECESLFSKESWSYI AETPNPENGTCYPGYFADYEELREQLSSVSSF ERFEIFPKESSWPNHTVTKGVTASCSHNGKSSF YRNLLWLTEKNGLYPNLSKSYVNNKEKEVLV LWGVHHPSNIGDQRAIYHTENAYVSVVSSHY SRRFTPEIAKRPKVRDQEGRINYYWTLLEPGD TIIFEANGNLIAPWYAFALSRGFGSGIITSNASM DECDAKCQTPQGAINSSLPFQNVHPVTIGECP KYVRSTKLRMVTGLRNIPSIQSRGLFGAIAGFI | 19 |
| | EGGWTGMIDGWYGYHHQNEQGSGYAADQK STQNAINGITNKVNSVIEKMNTQFTAVGKEFN KLERRMENLNKKVDDGFLDIWTYNAELLVLL ENERTLDFHDSNVKNLYEKVKSQLKNNAKEI GNGCFEFYHKCNNECMESVKNGTYDYPKYSE ESKLNREKIDSGGDIIKLLNEQVNKEMQSSNL YMSMSSWCYTHSLDGAGLFLFDHAAEEYEH AKKLIIFLNENNVPVQLTSISAPEHKFEGLTQIF QKAYEHEQHISESINNIVDHAIKSKDHATFNFL QWYVAEQHEEEVLFKDILDKIELIGNENHGLY LADQYVKGIAKSRKS | |
| NP: Wildtype sequence of nucleoprotein | MASQGTKRSYEQMETDGERQNATEIRASVGKMID GIGRFYIQMCTELKLSDYEGRLIQNSLTIERMVLSA FDERRNRYLEEHPSAGKDPKKTGGPIYKRVDGRW MRELVLYDKEEIRRIWRQANNGDDATAGLTHMMI WHSNLNDTTYQRTRALVRTGMDPRMCSLMQGST LPRRSGAAGAAVKGIGTMVMELIRMIKRGINDRNF WRGENGRKTRSAYERMCNILKGKFQTAAQRAMM DQVRESRNPGNAEIEDLIFSARSALILRGSVAHKSC LPACVYGPAVSSGYNFEKEGYSLVGIDPFKLLQNS QVYSLIRPNENPAHKSQLVWMACHSAAFEDLRLLS FIRGTKVSPRGKLSTRGVQIASNENMDNMESSTLE LRSRYWAIRTRSGGNTNQQRASAGQISVQPTFSVQ RNLPFEKSTVMAAFTGNTEGRTSDMRAEIIRMMEG AKPEEVSFRGRGVFELSDEKATNPIVPSFDMSNEGS YFFGDNAEEYDN | 20 |
| MRK_H3_consUnique: consensus sequence for subtype H3 with transmembrane domain (second underlined), italics indicate possible truncation sites for extracellular domain | MKTIIALSYILCLVFAQKLPGNDNSTATLCLGHHA VPNGTIVKTITNDQIEVTNATELVQSSSTGEICDS PHQILDGENCTLIDALLGDPQCDGFQNKKWDLFVE RSKAYSNCYPYDVPDYASLRSLVASSGTLEFNNES FNWTGVTQNGTSSACIRRSNSSFFSRLNWLTHLNF KYPALNVTMPNNEQFDKLYIWGVHHPGTDKDQIF LYAQASGRITVSTKRSQQAVIPNIGSRPRVRNIPSRI SIYWTIVKPGDILLINSTGNLIAPRGYFKIRSGKSSIM RSDAPIGKCNSECITPNGSIPNDKPFQNVNRITYGA CPRYVKQNTLKLATGMRNVPEKQTRGIFGAIAGFI ENGWEGMVDGWYGFRHQNSEGRGQAADLKSTQ AAIDQINGKLNRLIGKTNEKFHQIEKEFSEVEGRIQ DLEKYVEDTKIDLWSYNAELLVALENQHTIDLTDS EMNKLFEKTKKQLRENAEDMGNGCFKIYHKCDN ACIGSIRNGTYDHDVYRDEALNNRFQIKGVELKSGY KDWILWISFAISCFLLCVALLGFIMWACQKGNIRCN ICI | 41 |
| MRK_H3_ConsensusA: consensus sequence for subtype H3, cluster A with transmembrane domain (second underlined), italics indicate possible truncation sites for extracellular domain | MKTIIALSYILCLVFAQKLPGNDNSTATLCLGHHA VPNGTLVKTITNDQIEVTNATELVQSSSTGRICDSP HRILDGENCTLIDALLGDPHCDGFQNKEWDLFVER SKAYSNCYPYDVPDYASLRSLVASSGTLEFNNESF NWTGVAQNGTSYACKRGSVKSFFSRLNWLHQLK YKYPALNVTMPNNDKFDKLYIWGVHHPSTDSDQT SLYVQASGRVTVSTKRSQQTVIPNIGSRPWVRGVS SRISIYWTIVKPGDILLINSTGNLIAPRGYFKIRSGKS SIMRSDAPIGKCNSECITPNGSIPNDKPFQNVNRITY GACPRYVKQNTLKLATGMRNVPEKQTRGIFGAIA GFIENGWEGMVDGWYGFRHQNSEGTGQAADLKS TQAAINQINGKLNRLIEKTNEKFHQIEKEFSEVEGRI | 42 |
| | QDLEKYVEDTKIDLWSYNAELLVALENQHTIDLTD SEMNKLFERTRKQLRENAEDMGNGCFKIYHKCDN ACIGSIRNGTYDHDVYRDEALNNRFQIKGVELKSGY KDWILWISFAISCFLLCVVLLGFIMWACQKGNIRCN ICI | |
| MRK_H3_ConsensusB: consensus sequence for subtype H3, cluster B with transmembrane domain (second underlined), italics indicate possible truncation sites for extracellular domain | MKTIIALSYILCLVFAQKLPGNDNSTATLCLGHHA VPNGTIVKTITNDQIEVTNATELVQNSSTGEICDSPH QILDGENCTLIDALLGDPQCDGFQNKKWDLFVERS KAYSNCYPYDVPDYASLRSLVASSGTLEFNNESFN WTGVTQNGTSSACIRRSNSSFFSRLNWLTHLNFKY PALNVTMPNNEQFDKLYIWGVHHPGTDKDQIFLY AQSSGRITVSTKRSQQAVIPNIGSRPRIRNIPSRISIY WTIVKPGDILLINSTGNLIAPRGYFKIRSGKSSIMRS DAPIGKCNSECITPNGSIPNDKPFQNVNRITYGACP RYVKQSTLKLATGMRNVPEKQTRGIFGAIAGFIEN GWEGMVDGWYGFRHQNSEGRGQAADLKSTQAAI DQINGKLNRLIGKTNEKFHQIEKEFSEVEGRIQDLE KYVEDTKIDLWSYNAELLVALENQHTIDLTDSEMN KLFEKTKKQLRENAEDMGNGCFKIYHKCDNACIG SIRNGTYDHDVYRDEALNNRFQIKGVELKSGYKDWI LWISFAISCFLLCVALLGFIMWACQKGNIRCNICI | 43 |
| MRK_H1_cot_all: "center of tree" sequence for subtype H1 with transmembrane domain (second underlined), italics indicate possible truncation sites for extracellular domain | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETSSSDNG TCYPGDFINYEELREQLSSVSSFERFEIFPKTSSWPN HDSNKGVTAACPHAGAKSFYKNLIWLVKKGNSYP KLSKSYINDKGKEVLVLWGIHHPSTTADQQSLYQN ADAYVFVGTSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG KCPKYVKSTKLRLATGLRNVPSIQSRGLFGAIAGFI EGGWTGMVDGWYGYHHQNEQGSGYAADLKSTQ NAIDKITNKVNSVIEKMNTQFTAVGKEFNHLEKRIE NLNKKVDDGFLDIWTYNAELLVLLENERTLDYHD SNVKNLYEKVRNQLKNNAKEIGNGCFEFYHKCDN TCMESVKNGTYDYPKYSEEAKLNREKIDGVKLEST RIYQILAIYSTVASSLVLVVSLGAISFWMCSNGSLQC RICI | 44 |
| MRK_H3_cot_all: "center of tree" sequence for subtype H3 with transmembrane domain (second underlined), italics indicate possible truncation sites for extracellular domain | MKTIIALSYILCLVFAQKLPGNDNSTATLCLGHHA VPNGTIVKTITNDRIEVTNATELVQNSSIGEICDSPH QILDGENCTLIDALLGDPQCDGFQNKKWDLFVERS KAYSNCYPYDVPDYASLRSLVASSGTLEFNNESFN WTGVTQNGTSSACIRRSNSSFFSRLNWLTHLNFKY PALNVTMPNNEQFDKLYIWGVHHPGTDKDQIFLY AQSSGRITVSTKRSQQAVIPNIGSRPRIRNIPSRISIY WTIVKPGDILLINSTGNLIAPRGYFKIRSGKSSIMRS DAPIGKCKSECITPNGSIPNDKPFQNVNRITYGACP RYVKQSTLKLATGMRNVPEKQTRGIFGAIAGFIEN GWEGMVDGWYGFRHQNSEGRGQAADLKSTQAAI DQINGKLNRLIGKTNEKFHQIEKEFSEVEGRIQDLE KYVEDTKIDLWSYNAELLVALENQHTIDLTDSEMN KLFEKTKKQLRENAEDMGNGCFKIYHKCDNACIG | 45 |
| | SIRNGTYDHDVYRDEALNNRFQIKGVELKSGYKDWI LWISFAISCFLLCVALLGFIMWACQKGNIRCNICI | |
| MRK_sH1_Con_v2: consensus sequence of HA subtype H1, includes transmembrane sequence (second underlined), italics indicate possible truncation sites for extracellular domain | MKVKLLVLLCTFATYADTICIGYHANNSTDTVDT VLEKNVTVTHSVNLLENSHNGKLCLLKGIAPLQLG NCSVAGWILGNPECELLISKESWSYIVEKPNPENGT CYPGHFADYEELREQLSSVSSFERFEIFPKESSWPN HTVTGVSASCSHNGESSFYRNLLWLTGKNGLYPN LSKSYANNKEKEVLVLWGVHHPPNIGDQKALYHT ENAYVSVVSSHYSRKFTPEIAKRPKVRDQEGRINY YWTLLEPGDTIIFEANGNLIAPRYAFALSRGFGSGII NSNAPMDKCDAKCQTPQGAINSSLPFQNVHPVTIG ECPKYVRSAKLRMVTGLRNIPSIQSRGLFGAIAGFI EGGWTGMVDGWYGYHHQNEQGSGYAADQKSTQ NAINGITNKVNSVIEKMNTQFTAVGKEFNKLERRM ENLNKKVDDGFIDIWTYNAELLVLLENERTLDFHD SNVKNLYEKVKSQLKNNAKEIGNGCFEFYHKCND ECMESVKNGTYDYPKYSEESKLNREKIDGVKLESM GVYQILAIYSTVASSLVLLVSLGAISFWMCSNGSLQ CRICI | 46 |
| MRK_sH1_Con_ecto: ecto domain of consensus sH1 sequence (without transmembrane domain) with foldon sequence (second underlined), linker (bold), italics indicate other truncation sites for extracellular domain. | MKVKLLVLLCTFATYADTICIGYHANNSTDTVDT VLEKNVTVTHSVNLLEDSHNGKLCLLKGIAPLQLG NCSVAGWILGNPECELLISKESWSYIVETPNPENGT CYPGYFADYEELREQLSSVSSFERFEIFPKESSWPN HTVTGVSASCSHNGKSSFYRNLLWLTGKNGLYPN LSKSYANNKEKEVLVLWGVHHPPNIGDQRALYHT ENAYVSVVSSHYSRRFTPEIAKRPKVRDQEGRINY YWTLLEPGDTIIFEANGNLIAPRYAFALSRGFGSGII TSNAPMDECDAKCQTPQGAINSSLPFQNVHPVTIG ECPKYVRSAKLRMVTGLRNIPSIQSRGLFGAIAGFI EGGWTGMVDGWYGYHHQNEQGSGYAADQKSTQ NAINGITNKVNSVIEKMNTQFTAVGKEFNKLERRM ENLNKKVDDGFLDIWTYNAELLVLLENERTLDFH DSNVKNLYEKVKSQLKNNAKEIGNGCFEFYHKCN DECMESVKNGTYDYPKYSEESKLNREKIDGVKLES MGVGSAGSAGYIPEAPRDGQAYVRKDGEWVLSTF L | 47 |
| Linker sequence (GSAGSA) in bold | | |
| MRK_sH1_Con_RBD: receptor binding domain (RBD) of consensus sH1 sequence | MKVKLLVLLCTFATYAGIAPLQLGNCSVAGWILG NPECELLISKESWSYIVETPNPENGTCYPGYFADYE ELREQLSSVSSFERFEIFPKESSWPNHTVTGVSASCS HNGKSSFYRNLLWLTGKNGLYPNLSKSYANNKEK EVLVLWGVHHPPNIGDQRALYHTENAYVSVVSSH YSRRFTPEIAKRPKVRDQEGRINYYWTLLEPGDTII FEANGNLIAPRYAFALSRG | 48 |
| MRK_pH1_Con_ecto: ecto domain of consensus pH1 sequence (without transmembrane domain) with foldon sequence (second underlined), linker (bold), italics indicate other truncation sites for extracellular domain. Linker Sequence in bold | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETSSSDNG TCYPGDFIDYEELREQLSSVSSFERFEIFPKTSSWPN HDSNKGVTAACPHAGAKSFYKNLIWLVKKGNSYP KLSKSYINDKGKEVLVLWGIHHPSTSADQQSLYQN ADAYVFVGTSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG | 49 |
| | KCPKYVKSTKLRLATGLRNVPSIQSRGLFGAIAGFI EGGWTGMVDGWYGYHHQNEQGSGYAADLKSTQ NAIDKITNKVNSVIEKMNTQFTAVGKEFNHLEKRIE NLNKKVDDGFLDIWTYNAELLVLLENERTLDYHD SNVKNLYEKVRSQLKNNAKEIGNGCFEFYHKCDN TCMESVKNGTYDYPKYSEEAKLNREEIDGVKLEST RIGSAGSAGYIPEAPRDGQAYVRKDGEWVLLSTFL | |
| MRK_pH1_Con_RBD: | MKVKLLVLLCTFATATYAGVAPLHLGKCNIAGWIL GNPECESLSTASSWSYIVETSSSDNGTCYPGDFIDY EELREQLSSVSSFERFEIFPKTSSWPNHDSNKGVTA ACPHAGAKSFYKNLIWLVKKGNSYPKLSKSYINDK GKEVLVLWGIHHPSTSADQQSLYQNADAYVFVGT SRYSKKFKPEIAIRPKVRDQEGRMNYYWTLVEPGD KITFEATGNLVVPRYAFAMERNA | 50 |
| receptor binding domain (RBD) domain of consensus pH1 sequence | | |
| eH1HA_d5v1: | METPAQLLFLLLLWLPDTTGDTICIGYHANNSTDT VDTVLEKNVTVTHSVNLLEDSHNGKLCRLKGIAPL QLGKCNIAGWLLGNPECDPLPPMKSWSYIVETPNS ENGICYPGDFIDYEELREQLSSVSSFERFEIFPKGSS WPNHNTNGVTAACSHEGKNSFYRNLLWLTKKEG LYPNLENSYVNKKEKEVLVLWGIHHPSNNKEQQN LYQNENAYVSVVTSNYNRRFTPEIAERPKVRDQAG RMNYYWTLLKPGDTIIFEANGNLIAPMYAFALSRG FGSGIITSNASMHECNTKCQTPLGAINSSLPYQNIHP VTIGECPKYVRSAKLRMVTGLRNIPSIQSRGLFGAI AGFIEGGWTGMIDGWYGYHHQNEQGSGYAADQK STQNAINGITNKVNTVIEKMNIQFTAVGKEFNKLEK RMENLNKKVDDGFLDIWTYNAELLVLLENERTLD FHDSNVKNLYEKVKSQLKNNAKEIGNGCFEFYHK CDNECMESVRNGTYDYPKYSEESKLNREKVDGVK LESMGIGSAGSAGYIPEAPRDGQAYVRKDGEWVL LSTFL | 51 |
| | | |
| linker (bold) with foldon sequence (second underlined) | | |
| Includes the following mutations (in bold) | | |
| | | |
| L75P | | |
| V77M | | |
| R78K | | |
| E124G | | |
| G173E | | |
| S145N | | |
| S160L | | |
| K165E | | |
| S188N | | |
| E156K | | |
| **eH1HA_d5v2:** | METPAQLLFLLLLWLPDRRGDTICIGYHANNSTDT VDTVLEKNVTVTHSVNLLEDSHNGKLCRLKGIAPL QLGKCNIAGWLLGNPECDPLPPMKSWSYIVETPNS ENGICYPGDFIDYEELREQLSSVSSFERFEIFPKGSS WPNHTTNGVTAACSHEGKNSFYRNLLWLTKKEGS YPNLKNSYVNKKEKEVLVLWGIHHPSNSKEQQNL YQNENAHVSVVTSNYNRRFTPEIAERPKVRDQAG RMNYYWTLLKPGDTIIFEADGNLIAPMYAFALSRG FGSGIITSNASMHECNTKCQTPLGAINSSLPYQNIHP VTIGECPKYVRSAKLRMVTGLRNIPSIQSRGLFGAI AGFIEGGWTGMIDGWYGYHHQNEQGSGYAADQK STQNAINGITNKVNTVIEKMNIQFTAVGKEFNKLEK RMENLNKKVDDGFLDIWTYNAELLVLLENERTLD FHDSNVKNLYEKVKSQLKNNAKEIGNGCFEFYHK CDNECMESVRNGTYDYPKYSEESKLNREKVDGVK LESMGIGSAGSAGYIPEAPRDGQAYVRKDGEWVL LSTFL | 52 |
| | | |
| linker (bold) with foldon sequence (second underlined) | | |
| Includes the following mutations (in bold) | | |
| | | |
| L75P | | |
| V77M | | |
| R78K | | |
| E124G; | | |
| G173E; | | |
| S145N; | | |
| N248D | | |
| N131 T(glyc) | | |
| Y201H; | | |
| E156K | | |
| **eH1HA_d5v3:** | METPAQLLFLLLLWLPDTTGDTICIGYHANNSTDT VDTVLEKNVTVTHSVNLLEDSHNGKLCRLKGIAPL | 53 |
| linker (bold) with foldon sequence (second underlined) | QLGKCNIAGWLLGNPECDPLPPMKSWSYIVETPNS ENGICYPGDFIDYEELREQLSSVSSFERFEIFPKGSS WPDHNTNGVTAACSHEGKNSFYRNLLWLTEKKG SYPNLKNPYVNKKEKEVLVLWGIHHPSNSKEQQN LYRNENAYVSVVTSNYNRRFTPEIAERPKVRDQAG RMNYYWTLLKPGDTIIFEANGNLIAPMYAFALSRG FGSGIITSNASMHECNTKCQTPLGAINSSLPYQNIHP VTIGECPKYVRSAKLRMVTGLRNIPSIQSRGLFGAI AGFIEGGWTGMIDGWYGYHHQNEQGSGYAADQK STQNAINGITNKVNTVIEKMNIQFTAVGKEFNKLEK RMENLNKKVDDGFLDIWTYNAELLVLLENERTLD FHDSNVKNLYEKVKSQLKNNAKEIGNGCFEFYHK CDNECMESVRNGTYDYPKYSEESKLNREKVDGVK LESMGIGSAGSAGYIPEAPRDGQAYVRKDGEWVL LSTFL | |
| Includes the following mutations (in bold) | | |
| | | |
| L75P | | |
| V77M | | |
| R78K | | |
| E124G | | |
| G173E | | |
| S145N | | |
| N129D | | |
| E158K | | |
| S167P | | |
| Q196R | | |
| **eH1HA_d5v4:** | METPAQLLFLLLLWLPDTTGDTICIGYHANNSTDT VDTVLEKNVTVTHSVNLLEDSHNGKLCKLKGIAPL QLGKCNIAGWLLGNPGCDPLLPVGSWSYIVETPNS ENGICYPGDFIDYEELREQLSSVSSFERFKIFPKESS WPDHNTNGVTAACSHEGKNSFYRNLLWLTKKESS YPNLENSYVNKKRKEVLVLWGIHHPSNSKEQQNL YQNENAYVSVVTSNYNRRFTPEIAERPKVKGQAG RMNYYWTLLKPGDTIIFEANGNLIAPMYAFALSRG FGSGIITSNASMHECNTKCQTPLGAINSSLPYQNIHP VTIGECPKYVRSAKLRMVTGLRNIPSIQSRGLFGAI AGFIEGGWTGMIDGWYGYHHQNEQGSGYAADQK STQNAINGITNKVNTVIEKMNIQFTAVGKEFNKLEK RMENLNKKVDDGFLDIWTYNAELLVLLENERTLD FHDSNVKNLYEKVKSQLKNNAKEIGNGCFEFYHK CDNECMESVRNGTYDYPKYSEESKLNREKVDGVK LESMGIGSAGSAGYIPEAPRDGQAYVRKDGEWVL LSTFL | 54 |
| | | |
| linker (bold) with foldon sequence (second underlined) | | |
| Includes the following mutations (in bold) | | |
| | | |
| R47K | | |
| R78G | | |
| E119K | | |
| G173R | | |
| R224K | | |
| E70G | | |
| S145N | | |
| D225G | | |
| N129D | | |
| K165E | | |
| E156K | | |
| G159S | | |
| **MRK_RBS_HA129** | MKVKLLVLLCTFATYAGVAPLHLGKCNIAGWLL GNPECELLLTVSSWSYIVETSNSDNGTCYPGDFINY EELREQLSSVSSFERFEIFPKTSSWPDHETNRGVTA ACPYAGANSFYRNLIWLVKKGNSYPKLSKSYVNN KGKEVLVLWGIHHPPTSTDQQSLYQNADAYVFVG SSRYSKKFKPEIAIRPKVRDQEGRMNYYWTLVEPG DKITFEATGNLVVPRYAFAMERNA | 55 |
| **RBD1-Cal09-PC-Cb** | MKVKLLVLLCTFATYAGVAPLHLGKCNIAGWIL GNPECESLSTASSWSNITETPSSDNGTCYPGDFIDY EELREQLSSVSSFERFEIFPKTSSWPNHSSNKGVTA ACPHAGAKSFYKNLIWLVKKNGSYPKLNKSYINDS GKEVLVLWGIHHPSNSTDQQSLYQNADTYVFVGS SNYSKKFKPEIAIRPKVRDQEGRMNYYWTLVEPGD KITFEATGNLVVPRYAFAMERNA | 56 |
| | | |
| 6 glycosylation sites to allow access to the Cb epitope | | |
| **RBD1-Cal09-PC** | MKVKLLVLLCTFATYAGVAPLHLGKCNIAGWIL GNPECESNSTASSWSNITETPSSDNGTCYPGDFIDY EELREQLSSVSSFERFEIFPKTSSWPNHSSNKGVTA ACPHAGAKSFYKNLIWLVKKNGSYPKLNKSYINDS GKEVLVLWGIHHPSNSTDQQSLYQNADTYVFVGS SNYSKKFKPEIAIRPKVRDQEGRMNYYWTLVEPGD KITFEATGNLVVPRYAFAMERNA | 57 |
| | | |
| 7 added glycosylation sites | | |
| | | |
| **RBD1-Cal09** | MKVKLLVLLCTFATYAGVAPLHLGKCNIAGWIL GNPECESLSTASSWSNITETPSSDNGTCYPGDFIDY EELREQLSSVSSFERFEIFPKTSSWPNHDSNKGVTA ACPHAGAKSFYKNLIWLVKKGNSYPKLSKSYINDK GKEVLVLWGIHHPSTSADQQSLYQNADTYVFVGS SRYSKKFKPEIAIRPKVRDQEGRMNYYWTLVEPGD KITFEATGNLVVPRYAFAMERNA | 58 |
| **MRK_RBD-Cal09-PC-Cb** | MKVKLLVLLCTFATYAGVAPLHLGKCNIAGWIL GNPECESLSTASSWSYIVETPSSDNGTCYPGDFIDY EELREQLSSVSSFERFEIFPKTSSWPNHSSNKGVTA ACPHAGAKSFYKNLIWLVKKNGSYPKLNKSYINDS GKEVLVLWGIHHPSNSTDQQSLYQNADTYVFVGS SNYSKKFKPEIAIRPKVRDQEGRMNYYWTLVEPGD KITFEATGNLVVPRYAFAMERNA | 59 |
| **MRK_RBD-Cal09-PC** | MKVKLLVLLCTFATYAGVAPLHLGKCNIAGWIL GNPECESNSTASSWSYIVETPSSDNGTCYPGDFIDY EELREQLSSVSSFERFEIFPKTSSWPNHSSNKGVTA ACPHAGAKSFYKNLIWLVKKNGSYPKLNKSYINDS GKEVLVLWGIHHPSNSTDQQSLYQNADTYVFVGS SNYSKKFKPEIAIRPKVRDQEGRMNYYWTLVEPGD KITFEATGNLVVPRYAFAMERNA | 60 |
| **MRKRBD-Cal09** | MKVKLLVLLCTFATYAGVAPLHLGKCNIAGWIL GNPECESLSTASSWSYIVETPSSDNGTCYPGDFIDY EELREQLSSVSSFERFEIFPKTSSWPNHDSNKGVTA ACPHAGAKSFYKNLIWLVKKGNSYPKLSKSYINDK GKEVLVLWGIHHPSTSADQQSLYQNADTYVFVGS SRYSKKFKPEIAIRPKVRDQEGRMNYYWTLVEPGD KITFEATGNLVVPRYAFAMERNA | 61 |
| **FLHA_PR8** | | 62 |
| | | |
| includes transmembrane sequence (second underlined), italics indicate possible truncation sites for extracellular domain | MKANLLVLLCALAAADADTICIGYHANNSTDTVD TVLEKNVTVTHSVNLLEDSHNGKLCRLKGIAPLQL GKCNIAGWLLGNPECDPLLPVRSWSYIVETPNSEN GICYPGDFIDYEELREQLSSVSSFERFEIFPKESSWP NHNTNGVTAACSHEGKSSFYRNLLWLTEKEGSYP KLKNSYVNKKGKEVLVLWGIHHPPNSKEQQNLYQ NENAYVSVVTSNYNRRFTPEIAERPKVRDQAGRM NYYWTLLKPGDTIIFEANGNLIAPMYAFALSRGFG SGIITSNASMHECNTKCQTPLGAINSSLPYQNIHPVT IGECPKYVRSAKLRMVTGLRNIPSIQSRGLFGAIAG FIEGGWTGMIDGWYGYHHQNEQGSGYAADQKST QNAINGITNKVNTVIEKMNIQFTAVGKEFNKLEKR MENLNKKVDDGFLDIWTYNAELLVLLENERTLDF HDSNVKNLYEKVKSQLKNNAKEIGNGCFEFYHKC DNECMESVRNGTYDYPKYSEESKLNREKVDGVKL ESMGIYQILAIYSTVASSLVLLVSLGAISFWMCSNGS LQCRICI | |
| **FLHA_Cal09** | MKAILVVLLYTFATANADTLCIGYHANNSTDTVDT VLEKNVTVTHSVNLLEDKHNGKLCKLRGVAPLHL GKCNIAGWILGNPECESLSTASSWSYIVETPSSDNG TCYPGDFIDYEELREQLSSVSSFERFEIFPKTSSWPN HDSNKGVTAACPHAGAKSFYKNLIWLVKKGNSYP KLSKSYINDKGKEVLVLWGIHHPSTSADQQSLYQN ADTYVFVGSSRYSKKFKPEIAIRPKVRDQEGRMNY YWTLVEPGDKITFEATGNLVVPRYAFAMERNAGS GIIISDTPVHDCNTTCQTPKGAINTSLPFQNIHPITIG KCPKYVKSTKLRLATGLRNIPSIQSRGLFGAIAGFIE GGWTGMVDGWYGYHHQNEQGSGYAADLKSTQN AIDEITNKVNSVIEKMNTQFTAVGKEFNHLEKRIEN LNKKVDDGFLDIWTYNAELLVLLENERTLDYHDS NVKNLYEKVRSQLKNNAKEIGNGCFEFYHKCDNT CMESVKNGTYDYPKYSEEAKLNREEIDGVKLESTRI YQILAIYSTVASSLVLVVSLGAISFWMCSNGSLQCR ICI | 63 |
| | | |
| includes transmembrane sequence (second underlined), italics indicate possible truncation sites for extracellular domain | | |
| **MRK_B_consUnique: consensus sequence for type B** | MKAIIVLLMVVTSNADRICTGITSSNSPHVVKTATQ GEVNVTGVIPLTTTPTKSHFANLKGTRTRGKLCPD CLNCTDLDVALGRPMCVGTTPSAKASILHEVRPVT SGCFPIMHDRTKIRQLPNLLRGYEHIRLSTQNVIDA EKAPGGPYRLGTSGSCPNATSKNGFFATMAWAVP KNDNNKNATNPLTVEVPYICTEGEDQITVWGFHSD NKTQMKKLYGDSNPQKFTSSANGVTTHYVSQIGG FPDQTEDGGLPQSGRIVVDYMVQKPGKTGTIVYQR GVLLPQKVWCASGRSKVIKGSLPLIGEADCLHEKY GGLNKSKPYYTGEHAKAIGNCPIWVKTPLKLANG TKYRPPAKLLKERGFFGAIAGFLEGGWEGMIAGW HGYTSHGAHGVAVAADLKSTQEAINKITKNLNSLS ELEVKNLQRLSGAMDELHNEILELDEKVDDLRADT ISSQIELAVLLSNEGIINSEDEHLLALERKLKKMLGP SAVDIGNGCFETKHKCNQTCLDRIAAGTFNAGEFS LPTFDSLNITAASLNDDGLDNHTILLYYSTAASSLA VTLMIAIFIVYMVSRDNVSCSICL | 83 |
| **MRK_B_ConsensusA**: **consensus sequence for type B, cluster A** | MKAIIVLLMVVTSNADRICTGITSSNSPHVVKTATQ GEVNVTGVIPLTTTPTKSYFANLKGTRTRGKLCPD CLNCTDLDVALGRPMCVGTTPSAKASILHEVRPVT SGCFPIMHDRTKIRQLPNLLRGYENIRLSTQNVIDA EKAPGGPYRLGTSGSCPNATSKIGFFATMAWAVPK DNYKNATNPLTVEVPYICTEGEDQITVWGFHSDNK TQMKNLYGDSNPQKFTSSANGVTTHYVSQIGDFPD QTEDGGLPQSGRIVVDYMMQKPGKTGTIVYQRGV LLPQKVWCASGRSKVIKGSLPLIGEADCLHEKYGG LNKSKPYYTGEHAKAIGNCPIWVKTPLKLANGTK YRPPAKLLKERGFFGAIAGFLEGGWEGMIAGWHG YTSHGAHGVAVAADLKSTQEAINKITKNLNSLSEL EVKNLQRLSGAMDELHNEILELDEKVDDLRADTIS SQIELAVLLSNEGIINSEDEHLLALERKLKKMLGPS AVDIGNGCFETKHKCNQTCLDRIAAGTFNAGEFSL PTFDSLNITAASLNDDGLDNHTILLYYSTAASSLAV TLMLAIFIVYMVSRDNVSCSICL | 84 |
| **MRK_B_ConsensusB: consensus sequence for type B, cluster B** | MKAIIVLLMVVTSNADRICTGITSSNSPHVVKTATQ GEVNVTGVIPLTTTPTKSHFANLKGTETRGKLCPK CLNCTDLDVALGRPKCTGKIPSARVSILHEVRPVTS GCFPIMHDRTKIRQLPNLLRGYEHIRLSTHNVINAE | 85 |
| | NAPGGPYKIGTSGSCPNVTNGNGFFATMAWAVPK NDKNKTATNPLTIEVPYICTEGEDQITVWGFHSDNE TQMAKLYGDSKPQKFTSSANGVTTHYVSQIGGFPN QTEDGGLPQSGRIVVDYMVQKSGKTGTITYQRGIL LPQKVWCASGRSKVIKGSLPLIGEADCLHEKYGGL NKSKPYYTGEHAKAIGNCPIWVKTPLKLANGTKY RPPAKLLKERGFFGAIAGFLEGGWEGMIAGWHGY TSHGAHGVAVAADLKSTQEAINKITKNLNSLSELE VKNLQRLSGAMDELHNEILELDEKVDDLRADTISS QIELAVLLSNEGIINSEDEHLLALERKLKKMLGPSA VEIGNGCFETKHKCNQTCLDRIAAGTFDAGEFSLPT FDSLNITAASLNDDGLDNHTILLYYSTAASSLAVTL MIAIFVVYMVSRDNVSCSICL | |
| **MRK_B_cot_AA** | MKAIIVLLMVVTSNADRICTGITSSNSPHVVKTATQ GEVNVTGVIPLTTTPTKSYFANLKGTRTRGKLCPD CLNCTDLDVALGRPMCVGTTPSAKASILHEVRPVT SGCFPIMHDRTKIRQLPNLLRGYENIRLSTQNVIDA EKAPGGPYRLGTSGSCPNATSKSGFFATMAWAVP KNDNNKNATNPLTVEVPYICTEGEDQITVWGFHSD NKTQMKNLYGDSNPQKFTSSANGVTTHYVSQIGG FPDQTEDGGLPQSGRIVVDYMMQKPGKTGTIVYQ RGVLLPQKVWCASGRSKVIKGSLPLIGEADCLHEK YGGLNKSKPYYTGEHAKAIGNCPIWVKTPLKLAN GTKYRPPAKLLKERGFFGAIAGFLEGGWEGMIAG WHGYTSHGAHGVAVAADLKSTQEAINKITKNLNS LSELEVKNLQRLSGAMDELHNEILELDEKVDDLRA DTISSQIELAVLLSNEGIINSEDEHLLALERKLKKML GPSAVDIGNGCFETKHKCNQTCLDRIAAGTFNAGE FSLPTFDSLNITAASLNDDGLDNHTILLYYSTAASS LAVTLMLAIFIVYMVSRDNVSCSICL | 86 |
| | | |
| **"Center of tree" sequence for type B** | | |
| **MRK_B_COT_A** | MKAIIVLLMVVTSNADRICTGITSSNSPHVVKTATQ GEVNVTGVIPLTTTPTKSHFANLKGTETRGKLCPK CLNCTDLDVALGRPKCTGKIPSARVSILHEVRPVTS GCFPIMHDRTKIRQLPNLLRGYEHIRLSTHNVINAE NAPGGPYKIGTSGSCPNVTNGNGFFATMAWAVPK NDKNKTATNPLTIEVPYICTEGEDQITVWGFHSDNE TQMAKLYGDSKPQKFTSSANGVTTHYVSQIGGFPN QTEDGGLPQSGRIVVDYMVQKSGKTGTITYQRGIL LPQKVWCASGRSKVIKGSLPLIGEADCLHEKYGGL NKSKPYYTGEHAKAIGNCPIWVKTPLKLANGTKY RPPAKLLKERGFFGAIAGFLEGGWEGMIAGWHGY TSHGAHGVAVAADLKSTQEAINKITKNLNSLSELE VKNLQRLSGAMDELHNEILELDEKVDDLRADTISS QIELAVLLSNEGIINSEDEHLLALERKLKKMLGPSA VEIGNGCFETKHKCNQTCLDRIAAGTFDAGEFSLPT FDSLNITAASLNDDGLDNHTILLYYSTAASSLAVTL MIAIFVVYMVSRDNVSCSICL | 87 |
| | | |
| **"center of tree" sequence for type B, cluster A** | | |
| **MRK_B_COT_B** | MKAIIVLLMVVTSNADRICTGITSSNSPHVVKTATQ GEVNVTGVIPLTTTPTKSYFANLKGTRTRGKLCPD CLNCTDLDVALGRPMCVGTTPSAKASILHEVRPVT SGCFPIMHDRTKIRQLPNLLRGYENIRLSTQNVIDA EKAPGGPYRLGTSGSCPNATSKIGFFATMAWAVPK DNYKNATNPLTVEVPYICTEGEDQITVWGFHSDNK | 88 |
| | | |
| **"center of tree" sequence for type B, cluster B** | | |
| | TQMKSLYGDSNPQKFTSSANGVTTHYVSQIGDFPD QTEDGGLPQSGRIVVDYMMQKPGKTGTIVYQRGV LLPQKVWCASGRSKVIKGSLPLIGEADCLHEKYGG LNKSKPYYTGEHAKAIGNCPIWVKTPLKLANGTK YRPPAKLLKERGFFGAIAGFLEGGWEGMIAGWHG YTSHGAHGVAVAADLKSTQEAINKITKNLNSLSEL EVKNLQRLSGAMDELHNEILELDEKVDDLRADTIS SQIELAVLLSNEGIINSEDEHLLALERKLKKMLGPS AVDIGNGCFETKHKCNQTCLDRIAAGTFNAGEFSL PTFDSLNITAASLNDDGLDNHTILLYYSTAASSLAV TLMLAIFIVYMVSRDNVSCSICL | |

**Table 2: DNA Sequences**

| **Name** | **DNA Sequence** | **SEQ ID NO:** |
|---|---|---|
| BHA10-2: HA10 version for Influenza B/Brisbane/60/2008 strain, with exposed hydrophobic residues mutated I333T, M432S, L435T (bold/underlined) and foldon sequence (second underlined) | ATGGAGACCCCCGCCCAGCTGCTGTTCCTGCTGC TGCTGTGGCTGCCCGACACCACCGGCCACGTGGT GAAGACCGCCACCCAGGGCGAGGTGAACGTGAC CGGCGTGATCCCCCTGACCACCACCCCCACCGGC AGCGCCAACAAGAGCAAGCCCTACTACACCGGC GAGCACGCCAAGGCCACCGGCAACTGCCCCATC TGGGTGAAGACCCCCCTGAAGCTGGCCAACGGC ACCAAGTACGGCAGCGCCGGCAGCGCCACCCAG GAGGCCATCAACAAGATCACCAAGAACCTGAAC AGCCTGAGCGAGCTGGAGGTGAAGAACCTGCAG AGGCTGAGCGGCGCCAGCGACGAGACCCACAA CGAGATCCTGGAGCTGGACGAGAAGGTGGACGA CCTGAGGGCCGACACCATCAGCAGCCAGATCGA GCTGGCCGTGCTGCTGAGCAACGAGGGCATCAT CAACAGCGAGGACGAGGGCACCGGCGGCGGCTA CATCCCCGAGGCCCCCAGGGACGGCCAGGCCTA CGTGAGGAAGGACGGCGAGTGGGTGCTGCTGAG CACCTTCCTG | 21 |
| BHA10-3:BHA10-2 without GTGG linker or foldon domain, with G430C, E438C, Q457L mutations (bold) for trimerization | ATGGAGACCCCCGCCCAGCTGCTGTTCCTGCTGC TGCTGTGGCTGCCCGACACCACCGGCCACGTGGT GAAGACCGCCACCCAGGGCGAGGTGAACGTGAC CGGCGTGATCCCCCTGACCACCACCCCCACCGGC AGCGCCAACAAGAGCAAGCCCTACTACACCGGC GAGCACGCCAAGGCCACCGGCAACTGCCCCATC TGGGTGAAGACCCCCCTGAAGCTGGCCAACGGC ACCAAGTACGGCAGCGCCGGCAGCGCCACCCAG GAGGCCATCAACAAGATCACCAAGAACCTGAAC AGCCTGAGCGAGCTGGAGGTGAAGAACCTGCAG AGGCTGAGCTGCGCCAGCGACGAGACCCACAAC TGCATCCTGGAGCTGGACGAGAAGGTGGACGAC CTGAGGGCCGACACCATCAGCAGCCTGATCGAG CTGGCCGTGCTGCTGAGCAACGAGGGCATCATC AACAGCGAGGACGAG | 22 |
| NIHGen6HASS-TM: Gen6 HA SS construct without foldon or ferritin, linker (bold) with transmembrane domain (second underlined), version 1 | ATGGAGACCCCCGCCCAGCTGCTGTTCCTGCTGC TGCTGTGGCTGCCCGACACCACCGGCGACACCA TCTGCATCGGCTACCACGCCAACAACAGCACCG | 23 |
| | ACACCGTGGACACCGTGCTGGAGAAGAACGTGA CCGTGACCCACAGCGTGAACCTGGGCAGCGGCC TGAGGATGGTGACCGGCCTGAGGAACATCCCCC AGAGGGAGACCAGGGGCCTGTTCGGCGCCATCG CCGGCTTCATCGAGGGCGGCTGGACCGGCATGG TGGACGGCTGGTACGGCTACCACCACCAGAACG AGCAGGGCAGCGGCTACGCCGCCGACCAGAAGA GCACCCAGAACGCCATCAACGGCATCACCAACA TGGTGAACAGCGTGATCGAGAAGATGGGCAGCG GCGGCAGCGGCACCGACCTGGCCGAGCTGCTGG TGCTGCTGCTGAACGAGAGGACCCTGGACTTCC ACGACAGCAACGTGAAGAACCTGTACGAGAAGG TGAAGAGCCAGCTGAAGAACAACGCCAAGGAG ATCGGCAACGGCTGCTTCGAGTTCTACCACAAGT GCAACAACGAGTGCATGGAGAGCGTGAAGAACG GCACCTACGACTACCCCAAGTACAGCGAGGAGA GCAAGCTGAACAGGGAGAAGATCGACCAGGGC ACCGGCGGCATCCTGGCCATCTACAGCACCGTG GCCAGCAGCCTGGTGCTGCTGGTGAGCCTGGGC GCCATCAGCTTCTGGATGTGCAGCAACGGCAGC CTGCAGTGCAGAATCTGCATC | |
| NIHGen6HASS-TM2: Gen6 HA SS construct without foldon or ferritin, linker (bold), with transmembrane domain (second underlined), version 2 | ATGGAGACCCCGCCCAGCTGCTGTTCCTGCTGC TGCTGTGGCTGCCCGACACCACCGGCGACACCA TCTGCATCGGCTACCACGCCAACAACAGCACCG ACACCGTGGACACCGTGCTGGAGAAGAACGTGA CCGTGACCCACAGCGTGAACCTGGGCAGCGGCC TGAGGATGGTGACCGGCCTGAGGAACATCCCCC AGAGGGAGACCAGGGGCCTGTTCGGCGCCATCG CCGGCTTCATCGAGGGCGGCTGGACCGGCATGG TGGACGGCTGGTACGGCTACCACCACCAGAACG AGCAGGGCAGCGGCTACGCCGCCGACCAGAAGA GCACCCAGAACGCCATCAACGGCATCACCAACA TGGTGAACAGCGTGATCGAGAAGATGGGCAGCG GCGGCAGCGGCACCGACCTGGCCGAGCTGCTGG TGCTGCTGCTGAACGAGAGGACCCTGGACTTCC ACGACAGCAACGTGAAGAACCTGTACGAGAAGG TGAAGAGCCAGCTGAAGAACAACGCCAAGGAG ATCGGCAACGGCTGCTTCGAGTTCTACCACAAGT GCAACAACGAGTGCATGGAGAGCGTGAAGAACG GCACCTACGACTACCCCAAGTACAGCGAGGAGA GCAAGCTGAACAGGGAGAAGATCGACGGAGTG AAATTGGAATCAATGGGGGTCTATCAGATCCT GGCCATCTACAGCACCGTGGCCAGCAGCCTGGT GCTGCTGGTGAGCCTGGGCGCCATCAGCTTCTGG ATGTGCAGCAACGGCAGCCTGCAGTGCAGAATC TGCATC | 24 |
| NIHGen6HASS-foldon Gen6 HA SS construct with foldon sequence (second underlined) | ATGGAGACCCCGCCCAGCTGCTGTTCCTGCTGC TGCTGTGGCTGCCCGACACCACCGGCGACACCA TCTGCATCGGCTACCACGCCAACAACAGCACCG ACACCGTGGACACCGTGCTGGAGAAGAACGTGA CCGTGACCCACAGCGTGAACCTGGGCAGCGGCC TGAGGATGGTGACCGGCCTGAGGAACATCCCCC AGAGGGAGACCAGGGGCCTGTTCGGCGCCATCG CCGGCTTCATCGAGGGCGGCTGGACCGGCATGG | 25 |
| | TGGACGGCTGGTACGGCTACCACCACCAGAACG AGCAGGGCAGCGGCTACGCCGCCGACCAGAAGA GCACCCAGAACGCCATCAACGGCATCACCAACA TGGTGAACAGCGTGATCGAGAAGATGGGCAGCG GCGGCAGCGGCACCGACCTGGCCGAGCTGCTGG TGCTGCTGCTGAACGAGAGGACCCTGGACTTCC ACGACAGCAACGTGAAGAACCTGTACGAGAAGG TGAAGAGCCAGCTGAAGAACAACGCCAAGGAG ATCGGCAACGGCTGCTTCGAGTTCTACCACAAGT GCAACAACGAGTGCATGGAGAGCGTGAAGAACG GCACCTACGACTACCCCAAGTACAGCGAGGAGA GCAAGCTGAACAGGGAGAAGATCGACCCCGGCA GCGGCTACATCCCCGAGGCCCCCAGGGACGGCC AGGCCTACGTGAGGAAGGACGGCGAGTGGGTGC TGCTGAGCACCTTCCTG | |
| ConH1: consensus HA sequence for subtype H1, with transmembrane domain (second underlined) | ATGAAGGCCAAGCTGCTGGTGCTGCTGTGCGCCT TCACCGCCACCGACGCCGACACCATCTGCATTGG CTACCATGCCAACAACAGCACCGACACCGTGGA CACCGTGCTCGAGAAGAACGTGACCGTGACCCA CTCCGTGAATTTGTTGGAGGACAGCCACAACGG CAAGCTGTGTAAGCTGAAGGGCATCGCCCCCCT GCAGCTGGGCAAGTGCAACATCGCCGGCTGGAT CTTGGGCAATCCCGAGTGCGAAAGCCTGATCTCC AAGAGAAGCTGGAGCTACATCGTGGAGACTCCC AACAGCGAAAACGGCACCTGCTACCCCGGCGAC TTCGCTGACTACGAGGAACTGAGAGAGCAGCTG AGCAGCGTGAGCAGCTTTGAGAGATTCGAGATC TTCCCCAAAGAGAGCAGCTGGCCCAACCACAAC GTAACCAAGGGCGTGACAGCCGCCTGCAGCCAC GCCGGTAAGAGCAGCTTCTACAGAAACCTGCTG TGGCTGACAGAGAAGAACGGCAGCTACCCCAAG CTGAGCAAGAGCTATGTGAACAACAAGGAGAAG GAGGTGCTGGTCCTGTGGGGCGTACACCACCCC AGCAACATTACCGATCAGAGAACCCTGTACCAG AACGAGAATGCCTACGTGAGCGTGGTGAGCAGC CACTACAACAGAAGATTCACCCCCGAGATTGCC AAGAGACCGAAAGTGAGAGGCCAGGCCGGAAG AATCAACTACTACTGGACCCTGCTGGAGCCGG CGACACCATCATCTTCGAGGCCAACGGCAACCT GATCGCCCCCTGGTATGCCTTCGCCCTGAGCAGA GGCTTCGGAAGCGGCATCATCACATCCAACGCC CCCATGCATGAATGCGACACAAAGTGTCAGACC CCCCAGGGCGCCATCAACAGCAGCCTGCCCTTCC AGAACGTGCACCCTGTGACCATCGGCGAGTGCC CCAAGTACGTGAGAAGCACCAAGCTGAGAATGG TGACCGGCCTGAGAAATATCCCCAGTATCCAGA GCAGAGGCCTGTTCGGCGCCATCGCCGGCTTCAT CGAGGGCGGCTGGACCGGCATGATCGACGGCTG GTACGGCTACCACCACCAGAACGAGCAGGGCAG CGGCTACGCCGCCGACCAGAAGAGCACTCAGAA CGCCATCAACGGCATCACCAACAAGGTGAACAG CGTGATCGAGAAGATGAACACACAGTTCACCGC CGTGGGCAAAGAGTTCAATAAGCTCGAGAAGAG AATGGAAAACCTGAACAAGAAGGTTGACGACGG TTTCCTGGATATCTGGACCTACAACGCCGAGCTG | 26 |
| | CTTGTGCTGCTGGAGAACGAGCGTACCCTGGACT TTCACGACTCGAACGTGAAGAACCTGTACGAAA AGGTGAAGTCCCAGCTGAAGAACAACGCCAAAG AAATTGGCAACGGCTGCTTCGAGTTCTACCACAA GTGTAACAACGAGTGCATGGAGAGCGTGAAGAA CGGCACCTACGACTATCCCAAGTACAGCGAGGA GAGCAAGCTAAACAGAGAGAAGATTGACGGCGT GAAACTGGAGTCAATGGGCGTGTACCAGATCCT GGCCATCTACAGCACCGTGGCCAGCAGCCTCGT GCTGCTGGTGAGCCTGGGCGCCATAAGCTTCTGG ATGTGTAGCAACGGCAGCCTGCAGTGCAGAATC TGCATC | |
| ConH3: consensus HA sequence for subtype H3, with transmembrane domain (second underlined) | ATGAAGACCATCATCGCTCTGAGCTACATATTCT GCCTGGTGTTCGCCCAGAAGCTGCCCGGCAACG ACAACAGCACCGCCACCCTGTGCCTGGGCCATC ACGCAGTGCCAAACGGCACCTTAGTTAAGACCA TCACCAACGACCAGATCGAGGTGACCAACGCCA CCGAGCTGGTGCAGAGCAGTAGCACCGGCAGAA TCTGCGACAGCCCCCACCGGATCCTTGACGGCAC TAACTGCACCCTGATCGACGCCCTGCTGGGCGAC CCCCACTGCGACGGGTTCCAGAACAAGGAGTGG GACCTGTTCGTGGAGAGAAGCAAGGCCTACAGC AACTGCTACCCCTACGATGTGCCCGACTACGCCA GCCTGAGATCTCTTGTGGCTAGCAGCGGCACCCT GGAGTTCAACAATGAGGGCTTCAATTGGACAGG CGTGACCCAGAACGGCGGCAGCAGCGCCTGCAA GAGAGGCAGCGACAAGAGCTTCTTCAGCAGACT GAACTGGCTGCACAAGCTGAAGTACAAGTATCC CGCCCTGAACGTGACCATGCCCAATAACGACAA GTTCGATAAGCTGTATATTTGGGGCGTGCACCAC CCCAGCACCGACAGCGACCAGACCTCCCTGTAC GTCCAGGCGAGCGGCAGAGTGACCGTGAGCACC AAACGGAGCCAGCAAACCGTGATCCCCAACATC GGCAGCAGACCTTGGGTCAGAGGACTGAGCAGC AGAATCAGCATCTACTGGACCATCGTGAAGCCT GGCGACATCTTGCTGATCAATAGCACCGGCAAC CTGATCGCCCCCAGAGGCTACTTCAAGATCAGA AGCGGCAAGAGCTCAATCATGAGAAGCGACGCC CCCATAGGCACCTGCAACAGCGAGTGCATCACC CCGAACGGCAGCATCCCCAACGACAAGCCCTTC CAGAACGTGAACAGAATCACATACGGCGCCTGC CCCAGGTACGTAAAGCAAAACACCCTGAAGCTG GCCACCGGCATGAGAAACGTACCCGAGAAGCAG ACCAGAGGCATCTTCGGCGCCATCGCGGGCTTC ATCGAGAATGGCTGGGAAGGCATGGTGGACGGC TGGTACGGCTTCAGACATCAGAACAGCGAGGGC ACCGGCCAGGCCGCCGACCTGAAAAGCACCCAG GCCGCCATCGACCAGATCAACGGCAAGCTGAAC AGACTGATCGAAAAGACCAACGAGAAGTTCCAC CAGATCGAGAAGGAGTTCAGCGAAGTGGAGGGA AGAATACAGGACCTGGAGAAATACGTGGAGGAC ACCAAGATCGACCTGTGGTCGTACAACGCCGAG CTGCTGGTGGCCCTGGAAAACCAGCACACCATT GACCTGACCGATAGCGAGATGAACAAGCTGTTC GAGAGAACCAGAAAACAGCTGAGAGAGAACGC | 27 |
| | CGAGGACATGGGCAACGGCTGCTTTAAGATCTA CCACAAGTGCGACAACGCCTGCATCGGCAGCAT CAGAAACGGCACCTACGACCACGACGTGTACAG AGACGAGGCCCTGAACAACAGATTCCAGATCAA GGGCGTGGAGCTGAAGAGCGGCTACAAGGACTG GATCCTGTGGATCAGCTTCGCCATCAGCTGTTTC CTGCTTTGCGTAGTGCTGCTGGGCTTCATCATGT GGGCCTGCCAGAAGGGCAATATCAGATGCAACA TTTGCATC | |
| MRK_pH1_Con: consensus HA sequence for pandemic H1 strains, includes transmembrane sequence (second underlined) | ATGAAGGCCATCCTGGTGGTGCTGCTGTACACCT TCGCCACGGCCAACGCTGACACCCTGTGCATCG GATACCACGCGAACAACAGCACCGACACCGTTG ACACCGTGCTGGAGAAGAACGTGACCGTGACCC ACAGCGTGAACCTCCTGGAAGACAAGCACAACG GCAAGCTGTGCAAGCTGAGAGGCGTGGCCCCCC TGCACCTGGGCAAGTGCAACATCGCCGGCTGGA TCTTAGGCAACCCCGAGTGCGAGAGCCTGAGCA CCGCCAGCAGCTGGAGCTACATTGTGGAGACCA GCAGCAGCGACAACGGCACCTGCTACCCCGGCG ACTTCATCGACTACGAGGAGCTGAGAGAGCAGC TGAGCAGCGTGAGCAGCTTCGAGAGATTCGAGA TCTTCCCCAAGACCTCAAGCTGGCCCAACCACGA CAGCAATAAGGGCGTGACTGCCGCCTGCCCCCA CGCCGGCGCCAAGAGCTTCTACAAGAACCTGAT CTGGCTGGTGAAGAAAGGCAATAGCTACCCCAA GCTGAGCAAGTCCTATATCAACGACAAGGGCAA GGAGGTGTTGGTTCTGTGGGGCATCCATCACCCC AGCACCAGTGCTGATCAGCAAAGCCTGTACCAA AACGCCGATGCCTACGTGTTCGTGGGCACCTCAA GATACAGCAAGAAGTTCAAGCCCGAGATCGCCA TCAGACCCAAGGTGAGAGACCAGGAGGGTAGAA TGAACTACTACTGGACCCTCGTGGAGCCGGGCG ACAAGATCACCTTCGAGGCCACCGGCAACCTGG TGGTGCCCAGATACGCCTTCGCCATGGAGAGAA ATGCCGGCAGCGGGATCATCATCTCGGACACCC CCGTGCACGATTGTAACACCACCTGCCAGACCCC CAAGGGCGCCATCAACACCTCCCTGCCCTTCCAG AACATCCACCCCATCACCATCGGCAAGTGCCCC AAGTACGTGAAGAGCACCAAGCTGAGACTGGCG ACCGGACTGAGAAACGTGCCCAGCATCCAGTCA AGAGGCCTGTTCGGCGCCATCGCCGGCTTCATCG AGGGCGGCTGGACAGGCATGGTGGACGGCTGGT ACGGCTACCACCACCAGAACGAGCAGGGCAGCG GATACGCCGCCGACCTGAAGAGCACACAAAACG CCATCGACAAGATCACCAACAAGGTGAACAGCG TGATCGAAAAGATGAACACCCAGTTCACCGCCG TGGGCAAGGAGTTCAACCACCTGGAGAAGAGAA TCGAGAACCTGAACAAGAAAGTGGACGACGGCT TCCTGGACATCTGGACCTACAACGCCGAGCTGCT GGTCCTGCTGGAGAACGAGAGAACCCTGGACTA CCATGACAGCAACGTGAAGAACCTGTACGAGAA GGTGAGAAGCCAGCTGAAGAACAACGCCAAGG AGATAGGCAACGGCTGCTTCGAGTTCTACCACA AGTGCGACAACACCTGCATGGAGAGCGTGAAGA ACGGCACCTACGACTACCCAAAGTATAGCGAGG | 28 |
| | AGGCAAAGCTGAACAGAGAGGAGATCGACGGC GTGAAGCTGGAGAGCACCAGGATCTATCAAATC CTGGCCATATACAGCACCGTGGCCAGCAGCCTG GTGTTAGTGGTGAGCCTGGGCGCCATCAGCTTCT GGATGTGTAGCAACGGCAGCCTGCAGTGCAGAA TCTGCATC | |
| MRK_pH1_Con: consensus HA sequence for pandemic H1 strains, extracellular domain (italics indicate other truncation sites for extracellular domain) | ATGAAGGCCATCCTGGTGGTGCTGCTGTACACCT TCGCCACGGCCAACGCTGACACCCTGTGCATCG GATACCACGCGAACAACAGCACCGACACCGTTG ACACCGTGCTGGAGAAGAACGTGACCGTGACCC ACAGCGTGAACCTCCTGGAAGACAAGCACAACG GCAAGCTGTGCAAGCTGAGAGGCGTGGCCCCCC TGCACCTGGGCAAGTGCAACATCGCCGGCTGGA TCTTAGGCAACCCCGAGTGCGAGAGCCTGAGCA CCGCCAGCAGCTGGAGCTACATTGTGGAGACCA GCAGCAGCGACAACGGCACCTGCTACCCCGGCG ACTTCATCGACTACGAGGAGCTGAGAGAGCAGC TGAGCAGCGTGAGCAGCTTCGAGAGATTCGAGA TCTTCCCCAAGACCTCAAGCTGGCCCAACCACGA CAGCAATAAGGGCGTGACTGCCGCCTGCCCCCA CGCCGGCGCCAAGAGCTTCTACAAGAACCTGAT CTGGCTGGTGAAGAAGGCAATAGCTACCCCAA GCTGAGCAAGTCCTATATCAACGACAAGGGCAA GGAGGTGTTGGTTCTGTGGGGCATCCATCACCCC AGCACCAGTGCTGATCAGCAAAGCCTGTACCAA AACGCCGATGCCTACGTGTTCGTGGGCACCTCAA GATACAGCAAGAAGTTCAAGCCCGAGATCGCCA TCAGACCCAAGGTGAGAGACCAGGAGGGTAGAA TGAACTACTACTGGACCCTCGTGGAGCCGGGCG ACAAGATCACCTTCGAGGCCACCGGCAACCTGG TGGTGCCCAGATACGCCTTCGCCATGGAGAGAA ATGCCGGCAGCGGGATCATCATCTCGGACACCC CCGTGCACGATTGTAACACCACCTGCCAGACCCC CAAGGGCGCCATCAACACCTCCCTGCCCTTCCAG AACATCCACCCCATCACCATCGGCAAGTGCCCC AAGTACGTGAAGAGCACCAAGCTGAGACTGGCG ACCGGACTGAGAAACGTGCCCAGCATCCAGTCA AGAGGCCTGTTCGGCGCCATCGCCGGCTTCATCG AGGGCGGCTGGACAGGCATGGTGGACGGCTGGT ACGGCTACCACCACCAGAACGAGCAGGGCAGCG GATACGCCGCCGACCTGAAGAGCACACAAAACG CCATCGACAAGATCACCAACAAGGTGAACAGCG TGATCGAAAAGATGAACACCCAGTTCACCGCCG TGGGCAAGGAGTTCAACCACCTGGAGAAGAGAA TCGAGAACCTGAACAAGAAAGTGGACGACGGCT TCCTGGACATCTGGACCTACAACGCCGAGCTGCT GGTCCTGCTGGAGAACGAGAGAACCCTGGACTA CCATGACAGCAACGTGAAGAACCTGTACGAGAA GGTGAGAAGCCAGCTGAAGAACAACGCCAAGG AGATAGGCAACGGCTGCTTCGAGTTCTACCACA AGTGCGACAACACCTGCATGGAGAGCGTGAAGA ACGGCACCTACGACTACCCAAAGTATAGCGAGG AGGCAAAGCTGAACAGAGAGGAGATCGACGGCG TGAAGCTGGAGAGCACCAGGATCTATCAA | 29 |
| MRK_sH1_Con: consensus HA sequence for seasonal H1 strains, includes transmembrane sequence (second underlined) | ATGAAGGTGAAGCTGCTGGTGCTGCTGTGTACCT TCACTGCCACTTACGCCGACACCATTTGCATCGG CTACCACGCCAACAACAGCACCGATACCGTGGA CACCGTGCTGGAGAAGAACGTCACCGTGACCCA CAGCGTGAACCTGCTGGAGGATAGCCATAACGG CAAGCTGTGCCTGCTGAAGGGAATCGCCCCCCT GCAGCTCGGCAACTGCAGCGTGGCCGGCTGGAT TCTGGGCAACCCCGAGTGCGAACTGCTGATTAG CAAAGAGTCCTGGAGCTACATCGTGGAAACCCC GAATCCCGAGAACGGCACCTGCTACCCCGGCTA CTTCGCCGACTACGAAGAGCTAAGAGAGCAGCT GAGTAGCGTGAGCTCATTCGAGAGATTCGAGAT CTTTCCCAAGGAGTCTAGCTGGCCCAATCACACC GTGACCGGCGTGAGCGCCAGCTGTAGCCACAAC GGCAAGAGCAGCTTCTACAGAAACCTGCTGTGG CTGACCGGCAAGAACGGACTGTACCCTAACCTG AGCAAGAGCTACGCGAACAATAAGGAGAAGGA GGTGCTAGTGCTGTGGGGCGTGCACCACCCCCCC AACATCGGCGACCAGAGAGCCCTGTACCACACC GAGAACGCCTACGTGAGCGTGGTGAGCAGCCAC TATAGCAGAAGATTCACCCCCGAGATCGCCAAG AGACCAAAGGTGAGAGATCAGGAAGGAAGAAT AAACTACTACTGGACCCTCCTGGAGCCCGGCGA CACCATCATCTTCGAGGCTAACGGCAACCTGATC GCCCCCAGATACGCCTTCGCCCTGAGCAGAGGC TTCGGCAGCGGCATCATCACCAGCAATGCCCCC ATGGATGAGTGCGACGCCAAGTGCCAGACCCCC CAGGGCGCCATCAACTCGAGCCTGCCCTTCCAG AATGTGCACCCCGTGACCATCGGCGAGTGCCCC AAGTACGTGAGAAGCGCCAAGCTGAGAATGGTG ACCGGCCTGAGAAACATCCCAAGCATCCAGAGC AGAGGGCTGTTCGGCGCCATCGCTGGCTTCATCG AGGGCGGCTGGACCGGCATGGTGGACGGCTGGT ACGGTTATCACCACCAGAACGAGCAGGGCAGCG GCTACGCCGCCGACCAGAAGAGCACCCAGAACG CCATCAACGGCATTACAAACAAGGTGAACAGCG TTATCGAGAAGATGAACACCCAATTCACCGCCG TGGGCAAGGAGTTCAACAAGCTGGAGAGAAGAA TGGAGAACCTGAACAAGAAGGTGGACGACGGCT TCCTGGACATCTGGACCTACAACGCCGAGCTGCT GGTGCTGCTGGAGAACGAGAGAACCCTGGACTT CCACGACTCCAACGTGAAGAACTTATACGAGAA GGTGAAGAGCCAGCTGAAGAACAACGCCAAAG AAATCGGAAACGGCTGCTTCGAATTCTACCACA AGTGCAACGACGAATGCATGGAGAGCGTGAAGA ACGGAACCTACGACTACCCCAAGTACAGCGAGG AAAGCAAACTGAACAGAGAGAAGATCGACGGC GTGAAGTTAGAGAGCATGGGCGTGTATCAGATC CTGGCCATTTATAGCACGGTGGCCAGCAGCCTG GTGCTGCTGGTGAGCCTGGGCGCCATCAGCTTCT GGATGTGCAGCAACGGCAGCCTGCAGTGCAGAA TCTGCATC | 30 |
| MRK_sH1_Con: consensus HA sequence for seasonal H1 strains, extracellular domain (italics indicate other truncation sites for extracellular domain) | ATGAAGGTGAAGCTGCTGGTGCTGCTGTGTACCT TCACTGCCACTTACGCCGACACCATTTGCATCGG CTACCACGCCAACAACAGCACCGATACCGTGGA | 31 |
| | CACCGTGCTGGAGAAGAACGTCACCGTGACCCA CAGCGTGAACCTGCTGGAGGATAGCCATAACGG CAAGCTGTGCCTGCTGAAGGGAATCGCCCCCCT GCAGCTCGGCAACTGCAGCGTGGCCGGCTGGAT TCTGGGCAACCCCGAGTGCGAACTGCTGATTAG CAAAGAGTCCTGGAGCTACATCGTGGAAACCCC GAATCCCGAGAACGGCACCTGCTACCCCGGCTA CTTCGCCGACTACGAAGAGCTAAGAGAGCAGCT GAGTAGCGTGAGCTCATTCGAGAGATTCGAGAT CTTTCCCAAGGAGTCTAGCTGGCCCAATCACACC GTGACCGGCGTGAGCGCCAGCTGTAGCCACAAC GGCAAGAGCAGCTTCTACAGAAACCTGCTGTGG CTGACCGGCAAGAACGGACTGTACCCTAACCTG AGCAAGAGCTACGCGAACAATAAGGAGAAGGA GGTGCTAGTGCTGTGGGGCGTGCACCACCCCCC AACATCGGCGACCAGAGAGCCCTGTACCACACC GAGAACGCCTACGTGAGCGTGGTGAGCAGCCAC TATAGCAGAAGATTCACCCCCGAGATCGCCAAG AGACCAAAGGTGAGAGATCAGGAAGGAAGAAT AAACTACTACTGGACCCTCCTGGAGCCCGGCGA CACCATCATCTTCGAGGCTAACGGCAACCTGATC GCCCCCAGATACGCCTTCGCCCTGAGCAGAGGC TTCGGCAGCGGCATCATCACCAGCAATGCCCCC ATGGATGAGTGCGACGCCAAGTGCCAGACCCCC CAGGGCGCCATCAACTCGAGCCTGCCCTTCCAG AATGTGCACCCCGTGACCATCGGCGAGTGCCCC AAGTACGTGAGAAGCGCCAAGCTGAGAATGGTG ACCGGCCTGAGAAACATCCCAAGCATCCAGAGC AGAGGGCTGTTCGGCGCCATCGCTGGCTTCATCG AGGGCGGCTGGACCGGCATGGTGGACGGCTGGT ACGGTTATCACCACCAGAACGAGCAGGGCAGCG GCTACGCCGCCGACCAGAAGAGCACCCAGAACG CCATCAACGGCATTACAAACAAGGTGAACAGCG TTATCGAGAAGATGAACACCCAATTCACCGCCG TGGGCAAGGAGTTCAACAAGCTGGAGAGAAGAA TGGAGAACCTGAACAAGAAGGTGGACGACGGCT TCCTGGACATCTGGACCTACAACGCCGAGCTGCT GGTGCTGCTGGAGAACGAGAGAACCCTGGACTT CCACGACTCCAACGTGAAGAACTTATACGAGAA GGTGAAGAGCCAGCTGAAGAACAACGCCAAAG AAATCGGAAACGGCTGCTTCGAATTCTACCACA AGTGCAACGACGAATGCATGGAGAGCGTGAAGA ACGGAACCTACGACTACCCCAAGTACAGCGAGG AAAGCAAACTGAACAGAGAGAAGATCGACGGCG TGAAGTTAGAGAGCATGGGCGTGTATCAG | |
| Cobra P1: consensus HA sequence P1 for H1 subtype, with transmembrane domain (second underlined) | ATGAAGGCCGCCTCTTGGTGCTGCTGTGCGCCC TGGCGGCCACAGACGCCGACACAATCTGTATCG GCTACCACGCCAATAATAGCACCGATACCGTGG ATACCGTGCTCGAGAAGAACGTCACCGTTACAC ACTCCGTGAATTTACTGGAGGACAGCCACAATG GCAAGCTCTGCAAACTGAAGGGTATCGCCCCAC TCCAACTGGGCAAGTGCAACATCGCAGGCTGGC TGCTGGGCAACCCTGAGTGTGAGAGCCTGCTGA GCGCTAGAAGCTGGAGCTACATAGTGGAGACAC CTAACAGCGAAAACGGCACATGCTACCCCGGCG | 32 |
| | ACTTCATCGATTACGAGGAACTGCGGAGCAGC TGAGTAGCGTGAGCTCCTTTGAGAGATTTGAGAT CTTCCCCAAAGAGAGCAGCTGGCCCAACCATAA TACCACCAAAGGCGTGACCGCCGCTTGCAGTCA TGCAGGGAAAAGTAGCTTCTACCGGAACCTGCT CTGGTTGACCAAGAAGGGAGGGAGCTACCCAAA GTTGAGCAAAAGCTACGTGAATAACAAGGGCAA GGAGGTGCTCGTGCTGTGGGGAGTCCACCATCC CAGCACATCCACTGATCAGCAGTCCCTGTATCAG AACGAAAACGCCTACGTGAGTGTGGTGAGCTCT AACTACAACAGACGGTTCACCCCTGAAATTGCT GAGAGGCCAAAGGTGAGAGGCCAGGCCGGCAG AATGAACTACTATTGGACCCTCCTGGAGCCTGGG GACACGATCATCTTCGAGGCGACCGGGAACCTC ATCGCTCCCTGGTATGCCTTCGCCCTGAGCAGGG GCAGTGGCAGCGGAATCATCACCAGCAACGCCA GCATGCATGAGTGCAACACTAAATGCCAGACCC CCCAGGGGGCCATCAACAGCTCCCTGCCCTTCCA GAACATCCATCCTGTGACCATTGGGGAGTGCCCC AAGTACGTGAGGTCCACCAAGCTGAGGATGGTG ACTGGACTGAGGAACATCCCCAGCATCCAGAGC CGGGGGCTGTTTGGCGCCATTGCCGGCTTTATCG AGGGTGGCTGGACAGGTATGATTGATGGCTGGT ACGGATACCACCACCAGAACGAGCAGGGGAGTG GGTATGCTGCCGACCAGAAATCTACTCAGAACG CCATCAATGGCATCACCAATAAGGTGAACAGCG TCATCGAGAAGATGAACACCCAGTTCACCGCTG TGGGCAAGGAGTTCAACAACCTGGAAAAGCGCA TGGAGAACCTGAACAAGAAGGTGGACGACGGCT TCCTGGACATCTGGACCTACAACGCCGAGCTGCT TGTCCTCCTGGAGAACGAGAGGACCTTGGACTTC CATGACAGCAATGTGAAGAACCTCTACGAGAAA GTGAAGAGCCAGCTGAGAAACAATGCTAAGGAG ATCGGCAACGGCTGCTTCGAGTTTTACCACAAGT GCGACAATGAGTGCATGGAGAGCGTGAAGAATG GCACTTATGACTACCCCAAGTACTCAGAGGAGT CCAAACTGAATAGAGAGAAGATTGATGGCGTCA AGCTAGAGTCCATGGGCGTTTACCAGATCCTGGC AATCTATAGCACCGTGGCCAGCTCCCTGGTGCTG CTGGTGTCACTGGGAGCCATATCCTTCTGGATGT GCTCCAACGGCAGCCTTCAGTGTAGAATCTGCAT C | |
| Cobra _X3: consensus HA sequence X3 for H1 subtype, with transmembrane domain (second underlined) | ATGGAGGCCCGCCTGCTCGTGCTTCTGTGCGCCT TTGCCGCCACTAACGCCGACACCATCTGTATCGG CTACCACGCCAACAATAGTACAGATACCGTGGA CACTGTGCTGGAGAAGAACGTAACAGTGACACA TTCTGTCAACCTGCTCGAGGACTCTCATAATGGC AAGCTGTGCCGCCTGAAGGGCATCGCCCCTCTGC AGCTGGGAAATTGCTCCGTGGCCGGCTGGATCCT GGGCAATCCGGAATGCGAAAGCCTGTTCAGCAA GGAGAGCTGGAGCTACATCGCCGAGACACCTAA CCCTGAGAACGGGACCTGCTACCCTGGATACTTC GCCGACTACGAAGAGCTGCGGGAGCAGCTCAGC TCAGTGTCATCCTTCGAGCGGTTCGAGATCTTCC CCAAGGAGAGCTCTTGGCCCAACCACACCGTGA | 33 |
| | CCAAGGGCGTCACAGCAAGCTGTAGCCACAACG GCAAGAGCTCCTTCTATAGAAACCTGCTGTGGCT GACCGAGAAGAACGGCCTGTACCCCAATCTGAG TAAGTCCTACGTGAACAACAAGGAAAAGGAAGT GCTGGTGCTGTGGGGCGTGCACCACCCCTCCAAC ATCGGCGACCAGCGCGCCATCTACCACACTGAG AATGCATACGTAAGCGTTGTCAGCTCCCACTATA GTAGGAGATTCACACCCGAGATCGCTAAGAGGC CCAAGGTGAGAGACCAGGAGGGCAGAATCAATT ATTACTGGACCCTGCTGGAGCCCGGAGACACCA TTATCTTCGAAGCTAACGGCAATTTGATCGCCCC TTGGTATGCCTTTGCCCTCTCAAGGGGTTTCGGG AGCGGAATTATCACCTCCAATGCCAGCATGGAT GAGTGCGACGCCAAGTGCCAGACGCCTCAGGGC GCCATTAATTCCTCCCTGCCCTTCCAGAACGTGC ACCCCGTGACCATCGGGGAGTGCCCCAAGTATG TTAGATCCACTAAGCTCAGGATGGTGACAGGAC TGCGCAACATCCCGAGCATTCAGAGCAGGGGCC TCTTCGGGGCCATTGCTGGGTTCATCGAGGGCGG GTGGACCGGCATGATCGACGGCTGGTATGGCTA CCACCACCAGAACGAGCAGGGCAGCGGGTACGC TGCTGACCAAAAGTCCACCCAAAATGCTATCAA CGGCATCACCAACAAGGTTAATAGCGTCATCGA AAAGATGAATACCCAGTTCACAGCCGTGGGAAA GGAATTCAACAAGCTGGAACGACGGATGGAGAA CCTGAATAAGAAGGTGGACGACGGGTTCCTGGA CATCTGGACTTATAACGCTGAGCTGCTCGTGCTG TTAGAGAACGAGAGAACCCTGGACTTTCACGAC AGCAACGTGAAGAACCTGTACGAGAAGGTGAAG TCTCAGCTGAAAAATAACGCTAAGGAAATTGGC AACGGGTGCTTCGAATTCTATCACAAGTGCAAC AACGAATGCATGGAGAGTGTTAAGAACGGAACC TATGACTACCCCAAGTACAGTGAGGAAAGTAAA CTGAATAGGGAGAAGATCGACGGCGTGAAACTG GAGTCCATGGGGGTTTACCAGATTCTGGCCATCT ATAGCACCGTGGCCAGCAGCTTAGTGCTGCTGGT GTCCCTCGGCGCTATTAGCTTCTGGATGTGCAGC AACGGAAGCCTGCAGTGTCGGATATGCATC | |
| ConH1_ferritin: consensus HA sequence for subtype H1, with ferritin (second underlined) for particle formation | ATGAAGGCGAAGCTCCTTGTGCTGCTCTGCGCGT TCACCGCCACCGACGCAGATACAATTTGCATCG GATACCACGCCAACAATTCCACCGACACCGTGG ACACCGTTCTGGAGAAAAACGTGACGGTGACCC ACAGCGTGAACCTCCTGGAGGATAGCCATAACG GCAAGCTGTGTAAGCTGAAAGGCATCGCCCCCC TGCAGCTGGGAAAGTGCAACATTGCTGGATGGA TCCTGGGAAATCCCGAGTGTGAAAGCCTCATTA GCAAACGCAGCTGGAGCTACATTGTGGAGACCC CAAATTCTGAGAATGGGACCTGTTACCCTGGCG ACTTTGCCGACTACGAGGAGCTGAGAGAGCAGT TGAGCAGCGTCAGCTCCTTCGAGAGATTCGAAA TCTTTCCAAAGGAGTCTTCGTGGCCCAACCACAA CGTGACTAAGGGCGTCACCGCAGCTTGTAGCCA CGCGGGCAAATCTTCCTTCTACAGAAACCTACTG TGGCTCACCGAGAAAAACGGCAGCTACCCCAAG CTGAGCAAGAGCTACGTGAATAACAAAGAGAAG | 34 |
| | GAAGTGCTGGTGCTGTGGGGCGTCCACCACCCC AGCAACATCACAGACCAAAGAACACTCTACCAG AACGAGAACGCCTACGTGAGTGTGGTGTCCAGC CATTACAACCGCCGATTCACCCCCGAGATCGCCA AACGGCCCAAAGTGCGGGGCCAGGCCGGAAGA ATTAACTACTACTGGACCCTCCTGGAACCAGGA GACACCATTATCTTCGAAGCCAATGGCAATCTGA TCGCTCCCTGGTACGCCTTCGCACTGTCGAGAGG GTTTGGCAGCGGCATCATCACCTCCAACGCCCCA ATGCATGAATGTGATACCAAGTGCCAGACCCCA CAGGGCGCCATTAACAGCAGCCTGCCATTCCAG AACGTCCATCCCGTGACAATCGGCGAGTGTCCTA AGTACGTGCGCTCAACGAAACTGAGGATGGTGA CAGGACTGAGAAACATTCCCTCAATCCAGAGCA GAGGGCTGTTCGGCGCCATAGCCGGATTCATTG AGGGCGGATGGACAGGCATGATTGACGGCTGGT ATGGCTACCACCATCAGAACGAGCAAGGCAGTG GCTACGCAGCCGACCAGAAGAGCACACAGAACG CCATTAACGGGATCACCAACAAGGTGAATAGCG TGATCGAGAAGATGAATACCCAGTTCACTGCCG TGGGTAAGGAGTTCAACAAGCTGGAGAAGCGGA TGGAGAACCTCAACAAGAAAGTCGATGATGGCT TCCTGGACATCTGGACCTATAATGCTGAACTGCT CGTGCTACTTGAGAATGAGAGGACGCTTGACTTT CACGACTCCAACGTAAAAAACCTGTACGAGAAG GTGAAGTCGCAGCTGAAAAATAACGCCAAGGAA ATCGGCAACGGCTGTTTTGAGTTTTACCATAAAT GCAATAACGAGTGCATGGAGAGCGTGAAGAATG GCACCTACGACTATCCCAAATACTCCGAGGAGA GCAAGCTCAACCGGGAGAAAATCGATAGCGGCG GGGATATCATTAAGCTGCTTAACGAGCAGGTCA ACAAGGAGATGCAGTCAAGCAACCTTTACATGA GCATGAGCAGCTGGTGTTACACACACAGCCTGG ACGGAGCCGGACTGTTCCTGTTCGACCACGCTGC AGAGGAATATGAGCACGCTAAGAAGCTTATAAT TTTCCTCAACGAGAATAACGTGCCCGTCCAGCTG ACCTCCATCAGCGCCCCCGAGCACAAGTTTGAG GGCCTGACCCAGATCTTCCAGAAGGCCTACGAG CACGAGCAGCACATCAGCGAGTCTATCAACAAC ATCGTAGACCATGCAATCAAGTCTAAGGACCAC GCTACATTTAACTTTCTGCAATGGTACGTGGCTG AACAACACGAGGAGGAGGTACTGTTCAAGGATA TTCTCGACAAGATCGAACTCATCGGGAATGAGA ACCACGGCCTGTACCTGGCCGACCAGTACGTGA AAGGAATTGCCAAATCCAGAAAGTCC | |
| ConH3 _ferritin: consensus HA sequence for subtype H3, with ferritin (second underlined) for particle formation | ATGAAGACAATCATTGCCCTGAGCTACATTTTT GCTTAGTGTTTGCTCAGAAACTGCCAGGCAACG ATAATTCAACAGCCACCTTGTGCCTCGGCCACCA CGCTGTGCCTAACGGCACTCTGGTGAAGACCATC ACCAACGACCAGATCGAGGTGACCAACGCCACG GAGCTGGTGCAGTCAAGCTCCACCGGAAGAATC TGCGATAGCCCCCATAGGATTCTGGATGGCACC AACTGCACCCTGATTGACGCCCTGCTCGGCGATC CCCACTGCGACGGTTTCCAAAACAAGGAGTGGG ACCTGTTTGTGGAGAGAAGCAAGGCCTATTCAA | 35 |
| | ATTGCTACCCTTACGACGTCCCTGATTACGCCTC ACTCAGGTCCCTGGTGGCCAGCAGCGGGACCCT GGAATTCAACAATGAGGGGTTCAACTGGACCGG GGTGACCCAAAACGGCGGCTCCAGCGCCTGTAA GAGGGGCAGCGACAAGTCCTTCTTCTCTAGGCTG AACTGGTTGCACAAACTGAAGTACAAGTACCCT GCATTAAACGTGACCATGCCCAACAACGATAAA TTCGACAAGCTGTACATCTGGGGAGTGCATCACC CCAGCACAGACTCAGACCAGACCAGTCTGTATG TGCAGGCAAGCGGGAGGGTGACGGTCTCCACCA AGCGGAGCCAGCAGACCGTGATCCCCAACATCG GCTCCAGACCATGGGTCAGGGGCCTGAGCAGCC GGATCTCCATCTACTGGACCATAGTGAAGCCTGG CGACATCCTGCTGATCAACAGCACCGGCAACCT CATCGCCCCTCGCGGTTACTTCAAGATCCGTAGT GGCAAATCAAGCATCATGAGATCCGACGCACCC ATCGGGACCTGCAATAGCGAGTGCATCACCCCC AACGGATCTATCCCTAATGACAAGCCTTTTCAGA ACGTGAACCGGATTACCTATGGTGCCTGCCCCAG ATACGTGAAGCAGAACACCCTGAAGCTGGCGAC CGGCATGCGCAACGTGCCGGAAAAGCAGACCCG GGGCATCTTCGGCGCCATTGCCGGGTTTATTGAG AATGGCTGGGAAGGCATGGTGGATGGGTGGTAC GGCTTTAGGCATCAGAACTCTGAGGGTACTGGTC AGGCCGCCGACCTGAAATCCACCCAGGCCGCCA TTGACCAGATTAACGGGAAACTTAACAGACTGA TTGAGAAGACCAATGAGAAGTTCCACCAGATCG AAAAGGAATTCTCCGAAGTGGAGGGCAGGATTC AGGACTTAGAGAAATATGTGGAGGATACCAAGA TCGACCTGTGGAGCTATAACGCCGAGCTGCTTGT GGCTCTGGAGAACCAGCACACCATCGATCTGAC CGACAGCGAGATGAATAAGCTGTTCGAGAGGAC ACGCAAGCAGCTGAGGGAGAACGCCGAGGACAT GGGGAACGGGTGCTTTAAGATCTATCACAAGTG CGACAATGCCTGCATCGGGTCTATCAGAAATGG CACTTATGATCATGACGTGTACAGAGATGAGGC CCTGAATAATAGATTTCAAATTAAGTCCGGGGGT GACATCATTAAACTGCTGAACGAACAAGTGAAT AAAGAGATGCAGAGCTCTAACTTGTACATGAGC ATGAGCAGTTGGTGCTACACACACTCTCTGGACG GGGCTGGCCTGTTCCTGTTCGATCACGCAGCAGA GGAGTATGAGCACGCCAAAAAACTGATTATCTT CCTCAACGAGAACAACGTGCCCGTCCAGCTCAC CTCCATCTCAGCCCCCGAGCACAAGTTCGAGGG CCTCACCCAGATCTTCCAGAAAGCATATGAGCAT GAACAGCATATCAGTGAAAGCATCAACAATATC GTGGACCACGCTATTAAATCAAAGGATCACGCC ACCTTCAACTTTCTGCAGTGGTATGTCGCCGAGC AGCATGAGGAGGAGGTGCTTTTTAAAGACATCC TGGACAAGATCGAGCTGATCGGCAACGAAAACC ATGGCCTGTACCTGGCTGACCAGTATGTGAAGG GAATTGCCAAGTCCAGAAAATCC | |
| MRK_pH1_Con_ferritin: consensus HA sequence for pandemic H1 strains, with ferritin (second underlined) for particle formation | ATGAAGGCGATTCTGGTTGTGCTGCTGTACACCT TCGCCACCGCCAACGCCGACACACTGTGCATTG GCTACCACGCCAATAACAGCACGGACACCGTGG | 36 |
| | ACACGGTGCTGGAGAAAAACGTGACCGTGACCC ACAGCGTGAACCTGCTGGAGGACAAGCACAACG GTAAGTTGTGCAAGCTCAGGGGAGTGGCACCAC TGCACTTGGGCAAGTGTAATATCGCTGGCTGGAT ATTGGGAAATCCAGAGTGCGAAAGCCTGAGTAC TGCCTCCAGCTGGAGCTACATTGTGGAGACCAG CAGCAGCGACAACGGCACCTGCTACCCCGGCGA CTTCATCGACTATGAGGAATTGAGGGAACAGCT GAGTTCAGTTTCCAGCTTCGAGCGATTTGAGATA TTTCCCAAGACGTCCTCTTGGCCCAACCACGACA GCAACAAGGGCGTGACAGCCGCCTGCCCCCACG CCGGGGCGAAGAGCTTCTACAAGAACCTGATCT GGCTGGTGAAGAAGGGCAACAGCTACCCAAAGC TATCCAAGTCCTATATTAACGACAAAGGCAAGG AGGTGTTGGTGCTCTGGGGCATTCACCACCCCTC CACCTCCGCCGACCAGCAAAGTCTTTACCAGAA CGCGGACGCCTACGTCTTTGTCGGCACCAGCAG ATACAGCAAGAAGTTTAAGCCCGAGATTGCTAT CAGACCCAAGGTGAGAGACCAGGAAGGCAGAA TGAACTATTATTGGACCCTGGTGGAACCCGGCG ACAAAATAACATTCGAAGCCACCGGGAATCTGG TGGTGCCCAGATATGCCTTTGCCATGGAGCGCAA TGCCGGCAGCGGCATTATTATCTCTGACACCCCC GTGCACGACTGCAACACCACCTGTCAGACCCCT AAGGGGGCTATCAACACCAGCCTGCCCTTCCAG AATATTCACCCCATCACTATCGGCAAGTGCCCCA AGTACGTCAAGAGCACAAAACTGAGACTGGCCA CAGGGCTGAGGAATGTACCTAGCATCCAGTCCA GAGGGCTGTTCGGGGCCATCGCTGGCTTCATCGA AGGAGGCTGGACCGGCATGGTCGATGGATGGTA CGGATATCACCACCAAAACGAGCAGGGGTCAGG ATACGCCGCTGACCTGAAGAGCACCCAGAACGC CATCGACAAGATCACCAACAAGGTGAATAGCGT GATCGAGAAGATGAACACCCAGTTCACCGCAGT GGGCAAGGAGTTCAACCACCTGGAGAAGAGAAT CGAGAACCTGAACAAGAAAGTGGATGACGGGTT CCTGGACATCTGGACCTACAACGCCGAGCTTCTG GTGCTCTTGGAGAATGAGAGAACCCTGGATTAT CATGACAGCAATGTCAAAAACCTCTACGAGAAG GTGCGGAGCCAGCTGAAGAACAACGCAAAGGA GATTGGCAACGGCTGCTTCGAGTTTATCACAAG TGCGACAACACTTGTATGGAGAGCGTTAAGAAT GGCACTTACGATTACCCCAAGTACTCCGAGGAA GCCAAGCTGAACAGAGAAGAAATCGACTCCGGC GGCGACATAATCAAGCTCCTGAACGAACAGGTG AACAAGGAGATGCAAAGCTCCAACCTCTACATG AGCATGAGCTCATGGTGCTACACTCACAGCCTG GACGGAGCTGGACTGTTCTTGTTCGACCACGCGG CCGAGGAGTACGAGCACGCCAAGAAGCTCATCA TCTTCCTTAACGAGAATAACGTGCCAGTGCAGCT CACCTCCATCAGCGCCCCCGAGCATAAGTTCGA GGGTCTGACCCAAATCTTCCAGAAGGCTTACGA GCATGAGCAGCACATCAGCGAGAGCATTAACAA CATCGTGGATCACGCTATTAAATCTAAAGACCAC GCCACCTTCAACTTCCTGCAGTGGTACGTGGCAG AACAGCACGAGGAGGAGGTCCTGTTCAAGGATA | |
| | TACTGGACAAAATCGAGCTGATCGGCAACGAGA ACCACGGCCTGTACCTGGCCGATCAGTACGTCA AAGGTATTGCCAAGTCTCGCAAGAGC | |
| MRK_sH1_Con_ferritin: consensus HA sequence for seasonal H1 strains, with ferritin (second underlined) for particle formation | ATGAAGGTGAAGCTGCTTGTGCTGCTGTGCACCT TCACCGCTACCTACGCAGACACAATCTGTATCGG ATACCACGCCAATAACTCAACCGATACAGTGGA CACCGTGCTCGAGAAGAACGTGACAGTGACGCA CAGCGTGAACCTGCTTGAGGATTCCCATAACGGT AAGCTCTGTCTGCTGAAGGGCATCGCCCCTCTTC AGCTGGGAAACTGCTCCGTTGCCGGCTGGATCCT GGGCAACCCCGAGTGTGAGCTTCTGATCAGCAA GGAGTCGTGGTCATATATCGTGGAGACCCCTAAT CCAGAGAACGGAACCTGTTACCCCGGCTACTTTG CCGACTACGAGGAGCTCAGAGAGCAGCTGAGCA GCGTGAGCAGCTTCGAGAGATTCGAGATCTTCCC CAAGGAGAGCAGTTGGCCTAATCACACCGTGAC CGGCGTGAGCGCCTCCTGCAGCCACAACGGCAA GTCTTCCTTTTACAGAAACCTGCTGTGGCTGACA GGCAAAAACGGGTTGTACCCTAACCTGAGCAAG TCCTATGCTAACAATAAGGAGAAGGAAGTCCTG GTGTTGTGGGGCGTTCACCATCCCCCAAACATCG GAGACCAACGCGCCCTATATCACACTGAGAACG CCTACGTGAGCGTGGTGTCAAGCCACTATAGCA GACGGTTCACCCCCGAAATCGCAAAGAGACCGA AGGTGCGGGACCAGGAGGGAAGGATTAACTATT ACTGGACACTCCTGGAGCCCGGGGACACTATCA TCTTTGAAGCCAACGGAACCTCATCGCACCCA GGTACGCTTTCGCTCTGTCCAGGGGATTCGGGAG CGGTATCATTACCTCGAACGCCCCGATGGATGA GTGCGACGCCAAATGCCAAACCCCCCAGGGCGC TATTAACTCTAGCCTCCCTTTTCAGAACGTGCAC CCCGTGACCATCGGAGAGTGCCCCAAGTACGTG CGGAGCGCTAAGCTCAGGATGGTGACCGGCCTG CGGAACATCCCCTCTATCCAATCCAGGGGTCTGT TCGGCGCCATTGCCGGATTTATCGAGGGCGGGT GGACCGGGATGGTGGATGGATGGTATGGATACC ACCATCAGAATGAACAAGGCAGCGGATACGCCG CCGATCAGAAGTCAACACAAAACGCCATCAACG GAATTACCAACAAAGTCAACTCCGTGATCGAGA AGATGAACACCCAGTTCACGGCCGTGGGCAAAG AGTTCAACAAGCTCGAGCGGCGAATGGAGAACC TCAACAAGAAGGTGGACGATGGATTCCTGGACA TCTGGACGTACAATGCCGAACTGCTCGTGCTGCT GGAAAACGAGAGAACACTCGATTTCCACGACAG CAACGTGAAGAATCTGTATGAGAAGGTCAAATC CCAGTTGAAGAACAACGCCAAGGAGATCGGCAA TGGCTGTTTCGAGTTCTATCACAAGTGTAATGAC GAGTGCATGGAGAGCGTTAAGAACGGCACCTAC GACTACCCCAAATACAGCGAAGAGAGCAAGCTG AACCGTGAGAAGATCGACAGCGGAGGCGATATC ATCAAGCTGCTGAACGAACAGGTGAACAAGGAG ATGCAGTCCAGCAATCTCTACATGAGTATGTCCT CGTGGTGCTACACCCACAGCCTGGATGGAGCCG GACTGTTTCTGTTCGACCACGCCGCCGAGGAGTA CGAGCATGCCAAAAAGCTGATCATCTTCCTCAAT | 37 |
| | GAAAACAACGTGCCCGTGCAGTTGACCAGCATC AGCGCCCCCGAGCATAAATTCGAGGGACTGACA CAGATCTTTCAGAAGGCCTATGAGCACGAGCAG CACATCAGCGAAAGCATCAACAACATCGTGGAC CACGCCATCAAGTCCAAGGATCACGCCACCTTC AACTTCCTGCAGTGGTACGTTGCCGAACAGCAC GAGGAGGAGGTGCTGTTTAAGGACATCCTGGAC AAAATCGAACTGATCGGAAACGAGAACCATGGT CTGTACCTCGCCGACCAGTACGTGAAGGGAATC GCCAAGAGCAGGAAGTCG | |
| Cobra_P1_ferritin: consensus HA sequence PI for H1 subtype, with ferritin second underlined) for particle formation | ATGAAGGCCAGACTGTTGGTGCTGCTGTGTGCCC TTGCCGCCACAGACGCCGACACCATCTGTATCGG CTACCACGCTAATAACAGCACCGACACCGTGGA CACAGTGCTTGAAAAGAACGTGACAGTGACCCA CAGCGTTAACCTGCTTGAGGACTCTCACAACGG GAAGCTGTGTAAACTGAAGGGGATCGCCCCTCT GCAGCTGGGCAAGTGCAACATCGCTGGCTGGCT GCTGGGAAATCCCGAGTGTGAGAGCCTGCTGTC CGCTCGTAGCTGGAGCTACATAGTTGAAACCCCT AACAGCGAGAATGGCACCTGCTACCCTGGAGAC TTCATCGACTACGAGGAGCTCAGAGAGCAGCTG AGCAGCGTGAGCTCGTTTGAAAGATTTGAGATCT TTCCCAAGGAGTCCTCATGGCCCAACCACAACA CTACCAAAGGCGTGACCGCTGCTTGTTCACACGC TGGCAAATCCTCCTTCTACCGGAACCTGCTGTGG CTGACCAAGAAAGGCGGATCCTACCCCAAACTG AGCAAGTCATACGTGAATAACAAGGGCAAAGAG GTGCTGGTGCTGTGGGGCGTGCACCACCCCTCCA CCAGCACCGATCAGCAAAGCCTGTACCAGAACG AGAACGCCTACGTCAGCGTTGTGAGCAGCAACT ACAACCGGAGATTCACCCCCGAGATTGCCGAGA GACCTAAGGTGAGAGGGCAGGCTGGCAGAATGA ACTACTATTGGACTCTGCTGGAGCCCGGAGACA CAATTATCTTCGAGGCCACTGGCAATCTGATCGC ACCCTGGTACGCCTTCGCCTTAAGCAGGGGCAG CGGGTCTGGAATTATCACTTCCAATGCCAGCATG CACGAGTGCAACACCAAGTGCCAGACCCCCCAG GGCGCCATTAACAGCAGCCTGCCCTTCCAGAAC ATCCACCCCGTCACTATCGGCGAGTGCCCCAAGT ATGTGAGGAGCACTAAGCTGAGGATGGTGACCG GGCTTAGAAACATCCCCAGCATCCAGTCAAGAG GCCTATTCGGCGCAATAGCCGGCTTCATTGAAGG CGGCTGGACCGGGATGATCGATGGCTGGTATGG CTATCACCATCAGAACGAGCAAGGCTCCGGGTA CGCCGCCGACCAGAAATCCACACAGAATGCCAT CAATGGAATTACTAACAAGGTTAATTCCGTCATC GAGAAGATGAATACCCAGTTTACCGCCGTGGGA AAGGAGTTCAACAATCTGGAGAAGCGGATGGAG AACCTCAACAAGAAGGTAGATGATGGATTCCTC GACATCTGGACATACAATGCTGAACTGCTGGTG CTGCTCGAGAACGAGAGAACCTTAGACTTCCAC GACAGCAACGTGAAGAATCTGTACGAGAAGGTT AAGTCTCAACTGAGAAATAACGCTAAGGAGATT GGCAATGGCTGTTTCGAGTTCTACCACAAGTGTG ACAACGAATGTATGGAATCTGTGAAGAACGGGA | 38 |
| | CCTACGACTACCCCAAGTACAGCGAGGAGAGCA AGCTGAACAGAGAGAAGATCGACTCAGGCGGCG ACATCATTAAGCTGCTGAATGAGCAGGTTAATA AGGAGATGCAGAGCTCCAATCTGTATATGAGTA TGAGCAGCTGGTGTTACACTCACTCCCTGGACGG CGCCGGACTGTTCCTGTTCGACCACGCTGCAGAG GAGTACGAACACGCAAAAAAGCTGATAATCTTT CTGAATGAAAACAACGTGCCCGTCCAGCTGACC TCTATTTCTGCCCCAGAGCACAAGTTCGAGGGCC TGACACAGATCTTCCAAAAGGCCTACGAACACG AGCAGCACATCAGCGAGTCAATCAACAACATAG TCGATCACGCCATTAAGTCTAAGGACCACGCCA CCTTCAACTTCCTCCAGTGGTATGTGGCCGAGCA GCACGAGGAGGAGGTTCTTTTTAAGGATATTCTC GATAAAATCGAGTTGATCGGCAACGAGAATCAT GGCCTGTACCTGGCAGACCAATATGTGAAGGGG ATCGCCAAGTCAAGGAAGAGC | |
| Cobra_X3_ferritin: consensus HA sequence X3 for H1 subtype, with ferritin (second underlined) for particle formation | ATGGAGGCCAGACTGCTGGTGCTGCTGTGCGCCT TCGCCGCCACCAACGCAGACACCATCTGCATTG GCTACCACGCCAACAACAGCACCGATACCGTGG ACACAGTGCTCGAAAAGAACGTGACAGTGACTC ACAGCGTGAACCTCCTGGAGGACAGCCACAACG GCAAGCTGTGCCGGCTGAAGGGTATCGCCCCCTT GCAGCTGGGAAACTGCAGCGTGGCAGGGTGGAT CTTGGGCAATCCCGAGTGCGAAAGTCTGTTTTCT AAGGAGTCCTGGTCCTACATCGCCGAGACACCG AACCCCGAAAACGGAACATGCTATCCTGGCTAC TTCGCTGACTACGAAGAGCTGCGGGAGCAGCTT AGCTCCGTCTCCAGCTTTGAGCGGTTTGAGATCT TCCCGAAAGAGTCTAGCTGGCCCAATCACACAG TCACCAAGGGGGTGACCGCATCCTGCAGCCACA ACGGCAAGTCCTCTTTCTACAGAAACCTGCTGTG GCTGACCGAGAAAAACGGGCTGTACCCTAACCT TTCCAAGAGCTATGTCAACAACAAGGAGAAGGA GGTGCTGGTGCTGTGGGGGGTTCACCACCCCAG CAACATCGGAGACCAGAGAGCTATCTATCACAC CGAAAACGCCTACGTGAGCGTGGTGAGCAGCCA TTATAGCAGACGCTTCACCCCTGAGATTGCCAAA CGGCCCAAAGTGCGGGACCAGGAGGGCAGAATC AACTATTACTGGACCCTCCTGGAACCTGGCGATA CCATTATCTTTGAGGCCAACGGCAACCTGATCGC CCCATGGTACGCCTTTGCTCTGAGCCGGGGCTTT GGCTCAGGCATCATTACCAGCAACGCCAGCATG GACGAGTGCGATGCCAAGTGCCAGACACCCCAG GGCGCCATCAACAGCTCCCTGCCCTTTCAAAATG TCCATCCCGTGACCATCGGCGAGTGTCCCAAGTA CGTCCGGTCCACTAAACTGCGGATGGTGACCGG ACTCAGAAATATCCCAAGCATCCAGAGCAGAGG CCTGTTTGGCGCCATCGCTGGATTTATCGAGGGA GGCTGGACTGGCATGATCGATGGCTGGTACGGC TATCATCATCAGAACGAGCAGGGCAGCGGATAT GCCGCAGACCAGAAGTCGACCCAGAACGCCATC AATGGAATTACCAACAAGGTGAACAGCGTGATC GAGAAGATGAACACCCAGTTCACTGCCGTCGGC AAGGAATTCAACAAGCTGGAACGTCGGATGGAA | 39 |
| | AACCTCAACAAAAGGTGGATGACGGCTTCCTG GATATCTGGACCTACAACGCCGAGCTCCTGGTGC TCCTTGAGAACGAGAGAACCCTCGATTTCCACG ATAGCAACGTGAAAAATCTCTACGAAAAGGTGA AGAGCCAGCTGAAAAATAACGCCAAGGAGATAG GGAATGGCTGCTTCGAGTTCTACCATAAGTGCAA CAACGAGTGCATGGAGAGCGTCAAAAACGGCAC TTACGATTACCCCAAGTATTCAGAAGAGAGCAA ACTGAACAGGGAAAAAATTGACTCCGGCGGAGA CATTATCAAGCTGCTGAATGAACAGGTGAACAA AGAGATGCAGAGCTCCAACCTTTACATGAGCAT GAGCAGCTGGTGCTATACCCATTCCCTCGACGGG GCCGGGCTGTTCCTGTTCGACCATGCCGCTGAAG AATACGAGCACGCCAAGAAACTGATCATCTTCTT AAACGAGAACAATGTGCCAGTGCAGCTGACCTC AATCAGCGCCCCCGAGCACAAGTTCGAGGGACT CACTCAGATTTTCCAGAAGGCCTACGAGCACGA GCAACACATTAGCGAATCCATCAACAATATCGT GGACCACGCCATAAAGAGCAAGGACCATGCCAC CTTTAACTTCCTTCAATGGTACGTGGCCGAGCAG CACGAGGAGGAGGTCCTGTTCAAGGACATCCTC GACAAAATCGAGCTGATCGGCAATGAAAACCAT GGCCTCTACCTGGCTGACCAGTATGTGAAAGGT ATCGCTAAGTCAAGAAAAAGC | |
| NP: Wildtype sequence of nucleoprotein | ATGGCCAGCCAGGGCACCAAGAGAAGCTACGAG CAGATGGAGACCGACGGCGAGAGACAGAACGC CACCGAGATCAGAGCCAGCGTGGGCAAGATGAT CGACGGCATCGGCAGATTCTACATCCAGATGTG CACCGAGCTCAAGCTGAGCGACTACGAGGGCAG ACTGATCCAGAACAGCCTGACCATCGAAAGAAT GGTTCTGAGCGCCTTCGACGAGAGAAGAAACAG ATACCTGGAGGAGCACCCCAGCGCCGGCAAGGA CCCCAAGAAGACCGGCGGCCCCATCTACAAGAG AGTGGACGGCAGATGGATGAGAGAGCTGGTGCT GTACGACAAGGAGGAGATCAGAAGAATCTGGAG ACAGGCCAACAACGGCGACGACGCCACCGCCGG CCTGACCCACATGATGATCTGGCACAGCAACCT GAACGACACCACCTACCAGAGAACCAGAGCCCT GGTGAGAACCGGCATGGACCCCAGAATGTGCAG CTTAATGCAGGGCAGCACCCTGCCCAGAAGATC CGGCGCCGCTGGTGCCGCCGTCAAGGGCATCGG CACCATGGTGATGGAGCTGATCCGCATGATCAA GCGCGGCATCAACGACAGAAACTTCTGGAGAGG CGAAAACGGCAGAAAGACCAGAAGCGCCTACG AGAGAATGTGCAACATCCTGAAGGGCAAGTTCC AGACCGCCGCCCAAAGAGCCATGATGGACCAGG TGAGAGAGAGCAGAAACCCCGGCAACGCCGAG ATCGAAGACCTGATCTTCAGCGCCAGATCGGCC CTGATCCTGAGAGGCAGCGTGGCCCACAAGAGC TGCCTGCCCGCCTGCGTGTATGGCCCCGCCGTGA GCAGCGGCTACAACTTCGAGAAGGAGGGCTACA GCCTGGTGGGCATCGACCCCTTCAAGCTGCTGCA GAACTCTCAGGTGTATAGCCTGATCAGACCCAA CGAGAACCCCGCCCACAAGAGCCAGCTGGTGTG GATGGCCTGCCACAGCGCCGCCTTCGAGGACCT | 40 |
| | GAGACTGCTGAGCTTCATCAGAGGTACCAAGGT GTCCCCCAGAGGCAAGCTGAGCACCAGAGGTGT GCAGATCGCCAGCAATGAGAACATGGACAATAT GGAGAGCAGCACCCTGGAGCTAAGAAGCAGGTA CTGGGCCATCCGGACCAGAAGCGGCGGCAATAC CAACCAGCAGAGAGCCAGCGCCGGCCAGATCAG CGTGCAGCCCACCTTCAGCGTGCAGAGAAACCT GCCCTTTGAGAAGAGCACCGTGATGGCCGCCTTC ACCGGCAACACCGAGGGCAGAACCAGCGACATG AGAGCCGAGATCATCAGAATGATGGAGGGCGCC AAGCCCGAGGAGGTGAGCTTTAGAGGCAGAGGC GTGTTCGAGCTGAGCGACGAGAAGGCCACCAAC CCAATTGTGCCCAGCTTCGACATGTCGAACGAG GGCAGCTACTTCTTCGGCGACAACGCCGAGGAG TACGACAAC | |
| **MRK_pH1_Con_RBD** | ATGAAGGTGAAGCTGCTGGTGCTGCTGTGCACCT TCACCGCCACCTACGCCGGCGTGGCCCCTCTGCA CCTGGGCAAGTGCAACATCGCCGGCTGGATCCT GGGCAACCCTGAGTGCGAGAGCCTTAGCACAGC CTCCTCCTGGAGCTACATCGTGGAGACGAGCAG CAGCGATAACGGGACCTGCTACCCTGGCGACTT CATCGACTACGAGGAGCTGAGAGAGCAGCTGAG CAGCGTGAGCAGCTTCGAGAGATTCGAGATCTT CCCTAAGACCAGCAGCTGGCCTAACCACGACAG CAACAAGGGCGTGACCGCCGCCTGCCCACACGC CGGGGCCAAGAGCTTCTACAAGAACCTGATCTG GCTGGTGAAGAAGGGCAACAGCTACCCTAAACT GAGCAAGTCCTACATCAACGACAAAGGCAAGGA GGTCCTCGTGCTCTGGGGCATCCACCACCCTAGC ACCAGCGCCGATCAGCAGAGCCTGTACCAGAAC GCCGACGCGTACGTGTTCGTGGGCACCAGCAGA TACAGCAAGAAGTTCAAGCCTGAGATCGCCATC AGACCTAAGGTGAGGGACCAGGAGGGCAGAAT GAACTACTACTGGACCCTGGTGGAGCCCGGAGA TAAGATCACATTTGAGGCCACCGGCAACCTGGT GGTGCCTAGATACGCCTTCGCCATGGAGAGAAA CGCC | 64 |
| | | |
| Receptor Binding Domain of consensus pH1 sequence. | | |
| **MRK_pH1_Con_ecto:** ecto domain of consensus pH1 sequence (without transmembrane domain) with foldon sequence (second underlined), linker (bold) | ATGAAGGCCATCCTCGTGGTGCTGCTGTACACCT TTGCCACCGCCAACGCCGATACCCTGTGTATCGG CTACCACGCCAACAACAGCACCGACACCGTGGA TACTGTCCTGGAGAAGAACGTGACCGTGACCCA CAGCGTGAACCTGCTGGAGGACAAGCACAACGG CAAGCTGTGCAAGCTGAGAGGCGTGGCCCCTCT GCACCTGGGCAAGTGCAACATCGCCGGCTGGAT CCTGGGCAACCCTGAGTGCGAGAGCCTTAGCAC AGCCTCCTCCTGGAGCTACATCGTGGAGACGAG CAGCAGCGATAACGGGACCTGCTACCCTGGCGA CTTCATCGACTACGAGGAGCTGAGAGAGCAGCT GAGCAGCGTGAGCAGCTTCGAGAGATTCGAGAT CTTCCCTAAGACCAGCAGCTGGCCTAACCACGA CAGCAACAAGGGCGTGACCGCCGCCTGCCCACA CGCCGGGGCCAAGAGCTTCTACAAGAACCTGAT CTGGCTGGTGAAGAAGGGCAACAGCTACCCTAA ACTGAGCAAGTCCTACATCAACGACAAAGGCAA | 65 |
| | GGAGGTCCTCGTGCTCTGGGGCATCCACCACCCT AGCACCAGCGCCGATCAGCAGAGCCTGTACCAG AACGCCGACGCGTACGTGTTCGTGGGCACCAGC AGATACAGCAAGAAGTTCAAGCCTGAGATCGCC ATCAGACCTAAGGTGAGGGACCAGGAGGGCAGA ATGAACTACTACTGGACCCTGGTGGAGCCCGGA GATAAGATCACATTTGAGGCCACCGGCAACCTG GTGGTGCCTAGATACGCCTTCGCCATGGAGAGA AACGCCGGCAGCGGCATCATCATCAGCGACACC CCTGTGCACGACTGCAACACCACCTGCCAGACC CCTAAGGGCGCCATCAACACGAGCCTGCCTTTCC AGAACATCCACCCTATCACCATCGGCAAGTGCC CTAAGTACGTGAAGTCAACCAAACTGAGACTCG CCACCGGCCTCAGAAACGTGCCTAGCATCCAGA GCAGAGGCCTCTTCGGCGCCATCGCGGGATTCAT CGAGGGCGGCTGGACCGGCATGGTGGACGGCTG GTACGGCTACCACCATCAGAACGAGCAGGGCAG CGGGTACGCGGCCGACCTCAAGAGCACCCAGAA CGCCATCGACAAGATCACCAACAAGGTGAACAG CGTGATCGAGAAGATGAACACCCAGTTCACCGC CGTGGGCAAGGAGTTCAACCACCTGGAGAAGAG AATCGAGAACCTGAACAAGAAGGTGGACGACGG CTTCCTGGACATCTGGACCTACAACGCAGAACTG CTCGTGCTTCTGGAGAACGAGAGAACCCTGGAC TACCACGACTCCAACGTGAAGAACCTGTACGAG AAGGTGAGAAGCCAGCTGAAGAACAACGCCAA GGAGATCGGCAACGGCTGCTTCGAGTTCTACCA CAAGTGCGACAACACCTGCATGGAGAGCGTGAA GAACGGCACCTACGACTACCCTAAGTACAGCGA GGAGGCCAAGCTGAACAGAGAGGAGATCGACG GCGTGAAGCTGGAGAGCACCAGAATCGGCTCA GCCGGGAGCGCCGGCTACATCCCTGAGGCCCC TAGAGACGGCCAGGCCTACGTGAGAAAGGACGG CGAGTGGGTGCTGCTGAGCACCTTCCTG | |
| **MRK_sH1_Con_RBD:** | ATGAAGGTGAAGCTGCTGGTGCTGCTGTGCACCT TCACCGCCACCTACGCCGGAATCGCTCCCCTGCA GCTCGGCAACTGCAGCGTGGCCGGCTGGATTCT GGGCAACCCCGAGTGCGAACTGCTGATTAGCAA AGAGTCCTGGAGCTACATCGTGGAAACCCCGAA TCCCGAGAACGGCACCTGCTACCCCGGCTACTTC GCCGACTACGAGGAGCTAAGAGAGCAGCTGAGT AGCGTGAGCTCATTCGAGAGATTCGAGATCTTTC CCAAGGAGTCTAGCTGGCCCAATCACACCGTCA CCGGCGTGTCCGCCAGCTGTAGCCACAACGGCA AGAGCAGCTTCTACAGAAACCTGCTGTGGCTGA CCGGCAAGAACGGACTGTACCCTAACCTGAGCA AGAGCTACGCGAACAATAAGGAGAAGGAGGTG CTAGTGCTGTGGGGCGTGCACCATCCGCCCAAC ATCGGCGACCAGAGAGCCCTGTACCACACCGAG AACGCCTACGTGAGCGTGGTGAGCAGCCACTAT AGCAGAAGATTCACCCCTGAGATCGCCAAGAGG CCAAAGGTGAGAGATCAGGAAGGAAGAATAAA CTACTACTGGACCCTCCTGGAGCCCGGCGACACC | 66 |
| receptor binding domain (RBD) of consensus sH1 sequence | | |
| | ATCATCTTCGAGGCTAACGGCAACCTGATCGCCC CTAGATACGCCTTCGCCCTGAGCAGAGGC | |
| **MRK_sH1_Con_ecto:** ecto domain of consensus sH1 sequence (without transmembrane domain) with foldon sequence (second underlined), linker (bold) | ATGAAGGTGAAGCTGCTGGTGCTGCTGTGTACCT TCACTGCCACTTACGCCGACACCATTTGCATCGG CTACCACGCCAACAACAGCACCGATACCGTGGA CACCGTGCTGGAGAAGAACGTCACCGTGACCCA CAGCGTGAACCTGCTGGAGGATAGCCATAACGG CAAGCTGTGCCTGCTGAAGGGAATCGCTCCCCTG CAGCTCGGCAACTGCAGCGTGGCCGGCTGGATT CTGGGCAACCCCGAGTGCGAACTGCTGATTAGC AAAGAGTCCTGGAGCTACATCGTGGAAACCCCG AATCCCGAGAACGGCACCTGCTACCCCGGCTAC TTCGCCGACTACGAGGAGCTAAGAGAGCAGCTG AGTAGCGTGAGCTCATTCGAGAGATTCGAGATC TTTCCCAAGGAGTCTAGCTGGCCCAATCACACCG TCACCGGCGTGTCCGCCAGCTGTAGCCACAACG GCAAGAGCAGCTTCTACAGAAACCTGCTGTGGC TGACCGGCAAGAACGGACTGTACCCTAACCTGA GCAAGAGCTACGCGAACAATAAGGAGAAGGAG GTGCTAGTGCTGTGGGGCGTGCACCATCCGCCCA ACATCGGCGACCAGAGAGCCCTGTACCACACCG AGAACGCCTACGTGAGCGTGGTGAGCAGCCACT ATAGCAGAAGATTCACCCCTGAGATCGCCAAGA GGCCAAAGGTGAGAGATCAGGAAGGAAGAATA AACTACTACTGGACCCTCCTGGAGCCCGGCGAC ACCATCATCTTCGAGGCTAACGGCAACCTGATCG CCCCTAGATACGCCTTCGCCCTGAGCAGAGGCTT CGGCAGCGGCATCATCACCAGCAACGCTCCCAT GGACGAGTGCGACGCCAAGTGCCAGACCCCGCA GGGCGCCATCAACTCGAGCCTGCCCTTCCAGAA CGTGCACCCCGTGACCATCGGCGAGTGCCCCAA GTACGTGAGAAGCGCCAAGCTGAGAATGGTGAC CGGCCTGAGAAACATCCCAAGCATCCAGAGCAG AGGGCTGTTCGGCGCCATCGCTGGCTTCATCGAG GGCGGCTGGACCGGCATGGTGGACGGCTGGTAC GGTTATCACCACCAGAACGAGCAGGGCAGCGGC TACGCCGCCGACCAGAAGTCCACCCAGAACGCC ATCAACGGCATTACAAACAAGGTGAACAGCGTT ATCGAGAAGATGAACACCCAATTCACCGCCGTG GGCAAGGAGTTCAACAAGCTGGAGAGAAGAATG GAGAACCTGAACAAGAAGGTGGACGACGGCTTC CTGGACATCTGGACCTACAACGCCGAACTGCTG GTCCTGCTGGAGAACGAGAGAACCCTGGACTTC CACGACTCCAACGTGAAGAACTTATACGAGAAG GTCAAATCCCAGCTGAAGAACAACGCCAAAGAA ATCGGAAACGGCTGCTTCGAATTCTACCACAAGT GCAACGACGAGTGCATGGAGAGCGTGAAGAACG GAACCTACGACTACCCCAAGTACAGCGAGGAAA GCAAACTGAACAGAGAGAAGATCGACGGCGTGA AGTTAGAGAGCATGGGCGTGGGCAGCGCCGGC TCTGCTGGATACATCCCTGAGGCCCCTAGAGAC GGCCAGGCCTACGTGAGAAAGGACGGCGAGTGG GTGCTGCTGAGCACCTTCCTG | 67 |
| **MRK_sH1_Con_v2:** | ATGAAGGTGAAACTCCTCGTCCTGCTGTGCACCT TCACCGCCACCTACGCCGATACCATCTGTATTGG CTACCACGCCAACAACTCCACCGACACCGTGGA TACCGTGCTCGAGAAGAACGTGACCGTGACCCA CAGCGTGAACCTGCTGGAGAACAGCCACAACGG CAAGCTGTGCCTGCTGAAGGGCATCGCGCCCCT GCAGTTGGGTAACTGCTCCGTGGCCGGCTGGATC CTGGGCAACCCTGAGTGCGAGCTGCTGATCAGC AAGGAGAGCTGGAGCTACATCGTGGAGAAGCCT AACCCCGAGAACGGCACCTGCTACCCTGGCCAC TTCGCCGACTACGAGGAGCTGAGAGAGCAACTC AGCAGCGTGAGCAGCTTCGAGAGATTCGAGATC TTCCCTAAGGAGAGCAGCTGGCCCAATCACACT GTGACCGGCGTGTCCGCTTCTTGCAGCCATAACG GGGAAAGCTCCTTCTACAGAAATCTCCTTTGGCT GACGGGGAAGAACGGCCTGTACCCTAACCTGAG CAAGAGCTACGCCAACAACAAGGAGAAGGAGG TGCTGGTGCTGTGGGGCGTGCACCACCCTCCTAA CATCGGCGACCAGAAGGCCCTGTACCACACCGA GAACGCCTACGTCAGCGTGGTGTCCAGCCACTA CAGCAGAAAGTTCACCCCTGAGATCGCCAAGAG GCCTAAGGTGCGGGACCAGGAGGGCAGAATCAA CTACTACTGGACCCTGCTGGAGCCTGGCGACACC ATCATCTTCGAGGCCAACGGCAACCTGATCGCCC CTAGATACGCCTTCGCCCTGAGCAGAGGCTTCGG CAGCGGCATCATCAACAGCAACGCCCCTATGGA CAAGTGCGACGCCAAGTGCCAGACTCCGCAGGG CGCTATCAACAGCTCCCTGCCTTTCCAGAACGTG CACCCTGTGACCATCGGCGAGTGCCCTAAGTAC GTGAGAAGCGCCAAGCTGAGAATGGTGACCGGC CTGAGAAACATCCCTAGCATCCAGAGCAGAGGC CTGTTCGGCGCCATCGCCGGGTTTATCGAGGGCG GCTGGACCGGCATGGTGGACGGCTGGTACGGCT ACCACCACCAGAACGAGCAGGGCTCCGGCTACG CCGCCGACCAGAAATCCACCCAGAACGCCATCA ACGGCATCACCAACAAGGTGAACAGCGTCATCG AGAAGATGAACACCCAGTTCACCGCCGTGGGCA AGGAGTTCAACAAGCTGGAGAGAAGAATGGAG AACCTGAACAAGAAGGTGGACGACGGCTTCATC GACATCTGGACCTACAACGCCGAGCTTCTGGTGC TCCTGGAGAACGAGAGAACCCTGGACTTCCACG ACAGCAACGTGAAGAACCTGTACGAGAAGGTGA AGTCCCAGCTGAAGAACAACGCCAAGGAGATCG GCAACGGCTGCTTCGAGTTCTACCACAAGTGCA ACGACGAGTGCATGGAGAGCGTGAAGAACGGCA CCTACGATTACCCCAAGTACAGCGAGGAGAGCA AGCTGAACAGAGAGAAGATCGACGGCGTGAAGC TGGAGAGCATGGGCGTGTACCAGATCCTGGCCA TCTACTCCACCGTGGCCAGTAGCCTGGTGCTGCT GGTGAGCCTGGGCGCAATCAGCTTCTGGATGTG CAGCAACGGCAGCCTGCAGTGCAGAATCTGCAT C | 68 |
| consensus sequence of HA subtype H1, includes transmembrane sequence (second underlined) | | |
| **MRK_RBS_HA129** | ATGAAGGTCAAACTTCTCGTGCTCCTGTGCACCT TCACCGCCACCTACGCGGGCGTGGCTCCGCTTCA CCTGGGCAAGTGCAACATCGCCGGTTGGCTGCT | 69 |
| | GGGTAACCCAGAGTGCGAGCTACTGCTGACCGT GAGCAGCTGGAGCTACATCGTGGAAACCAGCAA CAGCGACAACGGCACCTGCTACCCTGGCGACTT CATCAACTACGAGGAGCTGAGAGAGCAGCTCAG CAGCGTGTCCAGCTTCGAGAGATTCGAGATCTTC CCTAAGACTAGCAGCTGGCCCGACCACGAAACA AACAGAGGCGTGACCGCCGCTTGTCCATACGCC GGCGCCAACAGCTTCTACAGAAACCTGATCTGG CTGGTGAAGAAGGGCAACAGCTACCCTAAGCTG AGCAAGAGCTACGTGAACAACAAGGGCAAGGA GGTGCTTGTGCTGTGGGGCATCCACCACCCTCCT ACCAGCACCGACCAGCAGAGCCTGTACCAGAAC GCCGACGCCTACGTGTTCGTGGGCAGCAGCAGA TACAGCAAGAAGTTCAAGCCTGAGATCGCCATC AGACCTAAGGTGAGGGACCAGGAGGGCAGAAT GAACTACTACTGGACTCTGGTGGAGCCCGGCGA CAAGATCACCTTCGAGGCCACCGGCAACCTGGT GGTGCCTAGATACGCCTTCGCCATGGAGAGAAA CGCC | |
| MRK_H1_cot_all | ATGAAGGCCATCCTGGTCGTGCTGCTCTACACAT TCGCCACCGCCAACGCAGACACTCTGTGCATCG GCTACCACGCCAACAACAGCACCGACACCGTGG ATACCGTGCTGGAGAAGAACGTGACCGTGACCC ACAGCGTGAACCTGCTGGAGGACAAGCACAACG GCAAGCTGTGCAAGCTGAGAGGCGTGGCCCCTC TGCACCTGGGCAAGTGCAACATCGCCGGCTGGA TCCTGGGAAACCCCGAGTGCGAGAGCCTGTCAA CCGCCTCGAGCTGGTCCTACATCGTGGAAACCA GCAGCAGCGATAACGGGACGTGCTACCCGGGCG ACTTCATCAACTACGAGGAGCTGAGAGAACAGC TGAGCAGCGTCAGTAGCTTCGAGAGATTCGAGA TCTTCCCTAAGACCAGCAGCTGGCCTAACCACGA CAGCAACAAGGGCGTGACCGCCGCTTGCCCGCA CGCAGGCGCCAAGAGCTTCTACAAGAACCTGAT CTGGCTGGTGAAGAAGGGCAACAGCTACCCTAA GCTGAGCAAGAGCTACATCAACGACAAGGGGAA GGAGGTGCTAGTCCTGTGGGGCATCCATCACCCT AGCACCACAGCCGACCAGCAAAGCCTGTACCAG AACGCGGACGCCTACGTGTTCGTCGGCACCAGC AGATACAGCAAGAAGTTCAAGCCTGAGATCGCC ATCAGACCTAAGGTGCGAGATCAGGAGGGCAGA ATGAACTACTACTGGACCCTGGTGGAGCCGGA GACAAGATTACTTTCGAAGCGACCGGCAACCTG GTGGTGCCTAGATACGCCTTCGCCATGGAGAGA AACGCCGGCAGCGGCATCATCATCAGCGACACC CCTGTGCACGACTGCAACACCACCTGCCAGACC CCTAAAGGCGCCATCAACACAAGCCTGCCTTTTC AGAACATCCACCCTATCACCATCGGCAAGTGCC CTAAGTACGTGAAGTCCACCAAGCTCCGCCTGG CAACCGGCCTCAGGAACGTGCCTAGCATCCAGA GCAGAGGCCTGTTCGGGGCCATAGCCGGCTTCA TAGAGGGTGGCTGGACCGGCATGGTTGACGGGT GGTACGGATACCATCACCAGAACGAGCAAGGCA GCGGCTACGCCGCAGACCTGAAGTCAACCCAGA ACGCCATCGACAAGATCACCAACAAGGTGAACA GCGTGATCGAGAAGATGAACACCCAGTTCACCG | 70 |
| | | |
| "center of tree" sequence for subtype H1 with transmembrane domain (second underlined) | | |
| | CCGTGGGCAAGGAGTTCAACCACCTAGAGAAGA GGATCGAGAACCTGAATAAGAAGGTGGACGACG GCTTCCTGGACATCTGGACCTACAACGCCGAGCT GCTCGTCCTCCTGGAGAACGAGAGAACCCTGGA CTACCACGATAGCAACGTGAAGAACCTGTACGA GAAGGTGAGAAACCAGCTGAAGAATAACGCCAA GGAGATCGGCAACGGCTGCTTCGAGTTCTACCA CAAGTGCGACAACACCTGCATGGAGAGCGTGAA GAACGGCACCTACGACTACCCTAAGTACAGCGA GGAGGCCAAGCTGAACAGAGAGAAGATCGACG GCGTGAAGCTGGAGAGCACCAGAATCTACCAGA TCCTGGCCATCTACAGCACCGTGGCCAGCAGCCT CGTGCTCGTGGTGAGCCTGGGCGCCATCTCCTTC TGGATGTGCAGCAACGGCAGCCTGCAGTGCAGA ATCTGCATC | |
| MRK_H3_cot_all | ATGAAGACCATCATCGCCCTGAGCTACATCCTGT GCCTGGTGTTCGCGCAGAAACTCCCCGGCAACG ACAATAGCACTGCCACCCTGTGTCTGGGCCATCA CGCCGTGCCTAACGGAACCATCGTGAAGACGAT CACCAACGACAGAATCGAGGTGACCAACGCCAC CGAGCTGGTCCAGAATTCGAGCATCGGCGAAAT CTGCGACAGCCCTCACCAGATCCTGGACGGCGA GAACTGCACCCTGATTGACGCACTGCTAGGCGA CCCACAGTGTGACGGCTTCCAGAACAAGAAGTG GGACCTGTTCGTGGAGAGAAGCAAGGCCTACAG CAACTGCTACCCTTACGACGTGCCTGACTACGCC AGCCTGAGATCCCTCGTGGCCTCCAGCGGCACCC TCGAGTTCAATAACGAGAGCTTCAACTGGACCG GAGTCACCCAGAACGGGACATCCAGCGCCTGCA TCAGAAGAAGCAACAGCAGCTTCTTCAGCAGAC TGAACTGGCTGACCCACCTGAACTTCAAGTACCC TGCCCTGAACGTGACCATGCCTAACAACGAGCA GTTCGACAAGCTGTACATCTGGGGCGTGCACCAT CCCGGCACCGACAAGGACCAGATCTTCCTGTAC GCCCAGAGCTCCGGCAGGATCACCGTGAGCACC AAGAGAAGCCAGCAGGCCGTGATCCCTAACATC GGCAGCAGACCTAGAATCAGAAACATCCCTAGC AGAATCAGCATCTACTGGACCATAGTGAAGCCC GGCGACATCCTGCTGATCAACTCGACCGGCAAC CTGATCGCTCCTAGGGGCTACTTCAAGATCAGAA GCGGCAAGAGCAGCATCATGAGAAGCGACGCGC CCATCGGGAAGTGCAAGTCCGAGTGCATCACCC CTAACGGCAGCATCCCCAACGACAAGCCTTTCC AGAACGTGAACAGAATCACCTACGGCGCCTGCC CTAGATACGTGAAGCAGAGCACACTGAAGCTGG CCACCGGCATGAGGAACGTGCCTGAGAAGCAGA CCAGAGGCATCTTCGGGGCTATTGCCGGCTTCAT CGAGAACGGTTGGGAGGGAATGGTCGACGGGTG GTACGGCTTCAGACACCAGAACAGCGAAGGCAG GGGACAGGCCGCCGACCTCAAGTCCACCCAGGC TGCCATCGATCAGATCAACGGGAAGCTGAACAG ACTGATCGGCAAGACCAACGAGAAGTTCCACCA GATCGAGAAGGAGTTCAGCGAGGTGGAGGGCAG AATCCAGGACCTGGAGAAGTACGTGGAGGACAC | 71 |
| | | |
| "center of tree" sequence for subtype H3 with transmembrane domain (second underlined) | | |
| | GAAGATCGACCTGTGGAGCTACAACGCAGAGCT GTTGGTGGCACTGGAGAACCAGCACACCATCGA CCTGACCGACAGCGAGATGAACAAGCTGTTCGA GAAGACCAAGAAGCAGTTACGAGAGAACGCCG AGGACATGGGAAACGGCTGTTTTAAGATCTACC ACAAGTGCGACAACGCCTGCATCGGGAGCATCA GGAACGGGACCTACGACCACGACGTGTACAGAG ACGAGGCCCTGAACAACAGATTCCAGATCAAGG GCGTGGAGCTGAAGTCCGGCTACAAGGACTGGA TCCTGTGGATCAGCTTCGCCATCAGCTGCTTCCT GCTGTGCGTGGCCCTCCTGGGCTTTATAATGTGG GCCTGCCAGAAGGGCAACATCAGGTGCAACATC TGCATC | |
| MRK_H3_ConsensusA: | ATGAAGACCATCATCGCCCTGAGCTACATCCTGT GCCTGGTGTTCGCGCAGAAACTCCCCGGCAACG ACAATAGCACTGCCACCCTGTGTCTGGGCCATCA CGCCGTGCCTAACGGAACCCTCGTGAAGACGAT CACCAACGACCAGATCGAGGTGACCAACGCCAC CGAGCTGGTCCAGAGTTCGAGCACCGGCAGAAT CTGCGACAGCCCTCACCGGATCCTGGACGGCGA GAACTGCACCCTGATTGACGCACTGCTAGGCGA CCCACACTGTGACGGCTTCCAGAACAAGGAGTG GGACCTGTTCGTGGAGAGAAGCAAGGCCTACAG CAACTGCTACCCTTACGACGTGCCTGACTACGCC AGCCTGAGATCCCTCGTGGCCTCCAGCGGCACCC TCGAGTTCAATAACGAGAGCTTCAACTGGACCG GAGTCGCCCAGAACGGGACATCCTACGCCTGCA AGAGAGGAAGCGTCAAGAGCTTCTTCAGCAGAC TGAACTGGCTGCACCAGCTGAAGTACAAGTACC CTGCCCTGAACGTGACCATGCCTAACAACGACA AGTTCGACAAGCTGTACATCTGGGGCGTGCACC ATCCCAGCACCGACAGCGACCAGACCTCCCTGT ACGTCCAGGCATCCGGCAGGGTCACCGTGAGCA CCAAGAGAAGCCAGCAGACCGTGATCCCTAACA TCGGCAGCAGACCTTGGGTCAGAGGCGTCTCTA GCAGAATCAGCATCTACTGGACCATAGTGAAGC CCGGCGACATCCTGCTGATCAACTCGACCGGCA ACCTGATCGCTCCTAGGGGCTACTTCAAGATCAG AAGCGGCAAGAGCAGCATCATGAGAAGCGACGC GCCCATCGGGAAGTGCAACTCCGAGTGCATCAC CCCTAACGGCAGCATCCCCAACGACAAGCCTTTC CAGAACGTGAACAGAATCACCTACGGCGCCTGC CCTAGATACGTGAAGCAGAACACACTGAAGCTG GCCACCGGCATGAGGAACGTGCCTGAGAAGCAG ACCAGAGGCATCTTCGGGGCTATTGCCGGCTTCA TCGAGAACGGTTGGGAGGGAATGGTCGACGGGT GGTACGGCTTCAGACACCAGAACAGCGAAGGCA CGGGACAGGCCGCCGACCTCAAGTCCACCCAGG CTGCCATCAATCAGATCAACGGGAAGCTGAACA GACTGATCGAGAAGACCAACGAGAAGTTCCACC AGATCGAGAAGGAGTTCAGCGAGGTGGAGGGCA GAATCCAGGACCTGGAGAAGTACGTGGAGGACA CGAAGATCGACCTGTGGAGCTACAACGCAGAGC TGTTGGTGGCACTGGAGAACCAGCACACCATCG | 72 |
| consensus sequence for subtype H3, cluster A with transmembrane domain (second underlined) | | |
| | ACCTGACCGACAGCGAGATGAACAAGCTGTTCG AGAGGACCAGGAAGCAGTTACGAGAGAACGCC GAGGACATGGGAAACGGCTGTTTTAAGATCTAC CACAAGTGCGACAACGCCTGCATCGGGAGCATC AGGAACGGGACCTACGACCACGACGTGTACAGA GACGAGGCCCTGAACAACAGATTCCAGATCAAG GGCGTGGAGCTGAAGTCCGGCTACAAGGACTGG ATCCTGTGGATCAGCTTCGCCATCAGCTGCTTCC TGCTGTGCGTGGTCCTCCTGGGCTTTATAATGTG GGCCTGCCAGAAGGGCAACATCAGGTGCAACAT CTGCATC | |
| **MRK_H3_ConsensusB:** | ATGAAGACCATCATCGCCCTGAGCTACATCCTGT GCCTGGTGTTCGCGCAGAAACTCCCCGGCAACG ACAATAGCACTGCCACCCTGTGTCTGGGCCATCA CGCCGTGCCTAACGGAACCATCGTGAAGACGAT CACCAACGACCAGATCGAGGTGACCAACGCCAC CGAGCTGGTCCAGAATTCGAGCACCGGCGAAAT CTGCGACAGCCCTCACCAGATCCTGGACGGCGA GAACTGCACCCTGATTGACGCACTGCTAGGCGA CCCACAGTGTGACGGCTTCCAGAACAAGAAGTG GGACCTGTTCGTGGAGAGAAGCAAGGCCTACAG CAACTGCTACCCTTACGACGTGCCTGACTACGCC AGCCTGAGATCCCTCGTGGCCTCCAGCGGCACCC TCGAGTTCAATAACGAGAGCTTCAACTGGACCG GAGTCACCCAGAACGGGACATCCAGCGCCTGCA TCAGAAGAAGCAACAGCAGCTTCTTCAGCAGAC TGAACTGGCTGACCCACCTGAACTTCAAGTACCC TGCCCTGAACGTGACCATGCCTAACAACGAGCA GTTCGACAAGCTGTACATCTGGGGCGTGCACCAT CCCGGCACCGACAAGGACCAGATCTTCCTGTAC GCCCAGAGCTCCGGCAGGATCACCGTGAGCACC AAGAGAAGCCAGCAGGCCGTGATCCCTAACATC GGCAGCAGACCTAGAATCAGAAACATCCCTAGC AGAATCAGCATCTACTGGACCATAGTGAAGCCC GGCGACATCCTGCTGATCAACTCGACCGGCAAC CTGATCGCTCCTAGGGGCTACTTCAAGATCAGAA GCGGCAAGAGCAGCATCATGAGAAGCGACGCGC CCATCGGGAAGTGCAACTCCGAGTGCATCACCC CTAACGGCAGCATCCCCAACGACAAGCCTTTCC AGAACGTGAACAGAATCACCTACGGCGCCTGCC CTAGATACGTGAAGCAGAGCACACTGAAGCTGG CCACCGGCATGAGGAACGTGCCTGAGAAGCAGA CCAGAGGCATCTTCGGGGCTATTGCCGGCTTCAT CGAGAACGGTTGGGAGGGAATGGTCGACGGGTG GTACGGCTTCAGACACCAGAACAGCGAAGGCAG GGGACAGGCCGCCGACCTCAAGTCCACCCAGGC TGCCATCGATCAGATCAACGGGAAGCTGAACAG ACTGATCGGCAAGACCAACGAGAAGTTCCACCA GATCGAGAAGGAGTTCAGCGAGGTGGAGGGCAG AATCCAGGACCTGGAGAAGTACGTGGAGGACAC GAAGATCGACCTGTGGAGCTACAACGCAGAGCT GTTGGTGGCACTGGAGAACCAGCACACCATCGA CCTGACCGACAGCGAGATGAACAAGCTGTTCGA GAAGACCAAGAAGCAGTTACGAGAGAACGCCG AGGACATGGGAAACGGCTGTTTTAAGATCTACC ACAAGTGCGACAACGCCTGCATCGGGAGCATCA | 73 |
| consensus sequence for subtype H3, cluster B with transmembrane domain (second underlined) | | |
| | GGAACGGACCTACGACCACGACGTGTACAGAG ACGAGGCCCTGAACAACAGATTCCAGATCAAGG GCGTGGAGCTGAAGTCCGGCTACAAGGACTGGA TCCTGTGGATCAGCTTCGCCATCAGCTGCTTCCT GCTGTGCGTGGCCCTCCTGGGCTTTATAATGTGG GCCTGCCAGAAGGGCAACATCAGGTGCAACATC TGCATC | |
| **MRK_H3_consUnique:** | ATGAAGACCATCATCGCCCTGAGCTACATCCTGT GCCTGGTGTTCGCGCAGAAACTCCCCGGCAACG ACAATAGCACTGCCACCCTGTGTCTGGGCCATCA CGCCGTGCCTAACGGAACCATCGTGAAGACGAT CACCAACGACCAGATCGAGGTGACCAACGCCAC CGAGCTGGTCCAGAGTTCGAGCACCGGCGAAAT CTGCGACAGCCCTCACCAGATCCTGGACGGCGA GAACTGCACCCTGATTGACGCACTGCTAGGCGA CCCACAGTGTGACGGCTTCCAGAACAAGAAGTG GGACCTGTTCGTGGAGAGAAGCAAGGCCTACAG CAACTGCTACCCTTACGACGTGCCTGACTACGCC AGCCTGAGATCCCTCGTGGCCTCCAGCGGCACCC TCGAGTTCAATAACGAGAGCTTCAACTGGACCG GAGTCACCCAGAACGGGACATCCAGCGCCTGCA TCAGAAGAAGCAACAGCAGCTTCTTCAGCAGAC TGAACTGGCTGACCCACCTGAACTTCAAGTACCC TGCCCTGAACGTGACCATGCCTAACAACGAGCA GTTCGACAAGCTGTACATCTGGGGCGTGCACCAT CCCGGCACCGACAAGGACCAGATCTTCCTGTAC GCCCAGGCATCCGGCAGGATCACCGTGAGCACC AAGAGAAGCCAGCAGGCCGTGATCCCTAACATC GGCAGCAGACCTAGAGTCAGAAACATCCCTAGC AGAATCAGCATCTACTGGACCATAGTGAAGCCC GGCGACATCCTGCTGATCAACTCGACCGGCAAC CTGATCGCTCCTAGGGGCTACTTCAAGATCAGAA GCGGCAAGAGCAGCATCATGAGAAGCGACGCGC CCATCGGGAAGTGCAACTCCGAGTGCATCACCC CTAACGGCAGCATCCCCAACGACAAGCCTTTCC AGAACGTGAACAGAATCACCTACGGCGCCTGCC CTAGATACGTGAAGCAGAACACACTGAAGCTGG CCACCGGCATGAGGAACGTGCCTGAGAAGCAGA CCAGAGGCATCTTCGGGGCTATTGCCGGCTTCAT CGAGAACGGTTGGGAGGGAATGGTCGACGGGTG GTACGGCTTCAGACACCAGAACAGCGAAGGCAG GGGACAGGCCGCCGACCTCAAGTCCACCCAGGC TGCCATCGATCAGATCAACGGGAAGCTGAACAG ACTGATCGGCAAGACCAACGAGAAGTTCCACCA GATCGAGAAGGAGTTCAGCGAGGTGGAGGGCAG AATCCAGGACCTGGAGAAGTACGTGGAGGACAC GAAGATCGACCTGTGGAGCTACAACGCAGAGCT GTTGGTGGCACTGGAGAACCAGCACACCATCGA CCTGACCGACAGCGAGATGAACAAGCTGTTCGA GAAGACCAAGAAGCAGTTACGAGAGAACGCCG AGGACATGGGAAACGGCTGTTTTAAGATCTACC ACAAGTGCGACAACGCCTGCATCGGGAGCATCA GGAACGGGACCTACGACCACGACGTGTACAGAG ACGAGGCCCTGAACAACAGATTCCAGATCAAGG | 74 |
| consensus sequence for subtype H3 with transmembrane domain (second underlined) | | |
| | GCGTGGAGCTGAAGTCCGGCTACAAGGACTGGA TCCTGTGGATCAGCTTCGCCATCAGCTGCTTCCT GCTGTGCGTGGCCCTCCTGGGCTTTATAATGTGG GCCTGCCAGAAGGGCAACATCAGGTGCAACATC TGCATC | |
| **RBD1-Cal09-PC-Cb** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCTTGAGCACCGCC AGCAGCTGGAGCAACATCACGGAAACCCCTAGC AGCGACAACGGCACCTGCTACCCCGGCGACTTC ATCGACTACGAGGAGCTGCGGGAGCAGCTGAGC AGCGTGAGCAGCTTCGAGCGGTTCGAGATCTTCC CCAAGACCAGCTCTTGGCCCAACCACAGCAGCA ACAAGGGCGTGACCGCCGCCTGCCCTCACGCTG GCGCCAAGAGCTTCTACAAGAACCTGATCTGGC TGGTGAAGAAGAACGGCAGCTACCCCAAGCTGA ACAAGTCTTACATTAACGACTCAGGCAAGGAGG TGCTGGTCCTGTGGGGCATCCACCACCCCAGCAA CAGCACCGACCAACAGAGCCTGTACCAGAACGC CGACACCTACGTGTTCGTGGGCAGCAGCAACTA CAGCAAGAAGTTCAAGCCCGAGATCGCCATCCG GCCCAAGGTGCGGGACCAGGAGGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | 75 |
| | | |
| 6 glycosylation sites to allow access to the Cb epitope | | |
| **RBD1-Cal09-PC** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCAACAGCACCGCC AGCAGCTGGAGCAACATCACGGAAACCCCTAGC AGCGACAACGGCACCTGCTACCCCGGCGACTTC ATCGACTACGAGGAGCTGCGGGAGCAGCTGAGC AGCGTGAGCAGCTTCGAGCGGTTCGAGATCTTCC CCAAGACCAGCTCTTGGCCCAACCACAGCAGCA ACAAGGGCGTGACCGCCGCCTGCCCTCACGCTG GCGCCAAGAGCTTCTACAAGAACCTGATCTGGC TGGTGAAGAAGAACGGCAGCTACCCCAAGCTGA ACAAGTCTTACATTAACGACTCAGGCAAGGAGG TGCTGGTCCTGTGGGGCATCCACCACCCCAGCAA CAGCACCGACCAACAGAGCCTGTACCAGAACGC CGACACCTACGTGTTCGTGGGCAGCAGCAACTA CAGCAAGAAGTTCAAGCCCGAGATCGCCATCCG GCCCAAGGTGCGGGACCAGGAGGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | 76 |
| | | |
| 7 added glycosylation sites | | |
| **RBD1-Cal09** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCTTGAGCACCGCC AGCAGCTGGAGCAACATCACGGAAACCCCTAGC AGCGACAACGGCACCTGCTACCCCGGCGACTTC ATCGACTACGAGGAGCTGCGGGAGCAGCTGAGC | 77 |
| | | |
| | AGCGTGAGCAGCTTCGAGCGGTTCGAGATCTTCC CCAAGACCAGCTCTTGGCCCAACCACGACAGCA ACAAGGGCGTGACCGCCGCCTGCCCTCACGCTG GCGCCAAGAGCTTCTACAAGAACCTGATCTGGC TGGTGAAGAAGGGCAACAGCTACCCCAAGCTGT CCAAGTCTTACATTAACGACAAGGGCAAGGAGG TGCTGGTCCTGTGGGGCATCCACCACCCCAGCAC CAGCGCCGACCAACAGAGCCTGTACCAGAACGC CGACACCTACGTGTTCGTGGGCAGCAGCCGGTA CAGCAAGAAGTTCAAGCCCGAGATCGCCATCCG GCCCAAGGTGCGGGACCAGGAGGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | |
| **MRK_RBD-Cal09-PC-Cb** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCTTGAGCACCGCC AGCAGCTGGAGCTACATCGTGGAAACCCCTAGC AGCGACAACGGCACCTGCTACCCCGGCGACTTC ATCGACTACGAGGAGCTGCGGGAGCAGCTGAGC AGCGTGAGCAGCTTCGAGCGGTTCGAGATCTTCC CCAAGACCAGCTCTTGGCCCAACCACAGCAGCA ACAAGGGCGTGACCGCCGCCTGCCCTCACGCTG GCGCCAAGAGCTTCTACAAGAACCTGATCTGGC TGGTGAAGAAGAACGGCAGCTACCCCAAGCTGA ACAAGTCTTACATTAACGACTCAGGCAAGGAGG TGCTGGTCCTGTGGGGCATCCACCACCCCAGCAA CAGCACCGACCAACAGAGCCTGTACCAGAACGC CGACACCTACGTGTTCGTGGGCAGCAGCAACTA CAGCAAGAAGTTCAAGCCCGAGATCGCCATCCG GCCCAAGGTGCGGGACCAGGAGGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | 78 |
| **MRK_RBD-Cal09-PC** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCAACAGCACCGCC AGCAGCTGGAGCTACATCGTGGAAACCCCTAGC AGCGACAACGGCACCTGCTACCCCGGCGACTTC ATCGACTACGAGGAGCTGCGGGAGCAGCTGAGC AGCGTGAGCAGCTTCGAGCGGTTCGAGATCTTCC CCAAGACCAGCTCTTGGCCCAACCACAGCAGCA ACAAGGGCGTGACCGCCGCCTGCCCTCACGCTG GCGCCAAGAGCTTCTACAAGAACCTGATCTGGC TGGTGAAGAAGAACGGCAGCTACCCCAAGCTGA ACAAGTCTTACATTAACGACTCAGGCAAGGAGG TGCTGGTCCTGTGGGGCATCCACCACCCCAGCAA CAGCACCGACCAACAGAGCCTGTACCAGAACGC CGACACCTACGTGTTCGTGGGCAGCAGCAACTA CAGCAAGAAGTTCAAGCCCGAGATCGCCATCCG | 79 |
| | GCCCAAGGTGCGGACCAGGAGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | |
| **MRKRBD-Cal09** | ATGAAGGTGAAGCTTCTCGTGCTCTTATGCACCT TCACCGCCACCTACGCCGGCGTGGCTCCGCTTCA CCTTGGCAAGTGCAACATCGCCGGCTGGATCTTG GGAAACCCCGAGTGCGAGAGCTTGAGCACCGCC AGCAGCTGGAGCTACATCGTGGAAACCCCTAGC AGCGACAACGGCACCTGCTACCCCGGCGACTTC ATCGACTACGAGGAGCTGCGGGAGCAGCTGAGC AGCGTGAGCAGCTTCGAGCGGTTCGAGATCTTCC CCAAGACCAGCTCTTGGCCCAACCACGACAGCA ACAAGGGCGTGACCGCCGCCTGCCCTCACGCTG GCGCCAAGAGCTTCTACAAGAACCTGATCTGGC TGGTGAAGAAGGGCAACAGCTACCCCAAGCTGT CCAAGTCTTACATTAACGACAAGGGCAAGGAGG TGCTGGTCCTGTGGGGCATCCACCACCCCAGCAC CAGCGCCGACCAACAGAGCCTGTACCAGAACGC CGACACCTACGTGTTCGTGGGCAGCAGCCGGTA CAGCAAGAAGTTCAAGCCCGAGATCGCCATCCG GCCCAAGGTGCGGGACCAGGAGGGCCGGATGAA CTACTACTGGACCCTGGTGGAGCCTGGCGACAA GATCACCTTCGAGGCCACCGGCAACCTGGTGGT GCCCCGGTACGCCTTCGCCATGGAGCGGAACGC C | 80 |
| **FLHA_PR8** | ATGAAGGCCAATTTGTTGGTCCTTCTATGTGCCC TAGCCGCCGCCGACGCCGACACAATCTGCATCG GATATCACGCAAACAACAGCACCGACACCGTGG ATACGGTCTTGGAGAAGAACGTGACCGTGACCC ATTCCGTGAACCTTCTCGAGGATAGCCACAATGG CAAGCTGTGTAGACTCAAGGGCATTGCCCCGCT GCAGCTGGGAAAGTGCAATATTGCTGGCTGGCT GTTGGGCAACCCTGAGTGTGACCCTCTGTTACCA GTGAGATCTTGGAGCTATATCGTCGAAACCCCTA ACAGCGAGAACGGCATATGCTACCCAGGCGACT TCATCGACTACGAGGAACTGCGCGAGCAGCTGA GCTCTGTGTCGAGCTTCGAGCGGTTCGAGATCTT CCCTAAGGAATCTAGCTGGCCTAATCATAACAC AAATGGCGTTACTGCTGCCTGTAGCCACGAGGG AAAGAGCAGTTTCTACCGGAATCTGCTGTGGCTG ACAGAGAAGGAGGGCTCCTACCCTAAGCTGAAG AATAGCTATGTGAACAAGAAGGGCAAGGAGGTG CTGGTGCTGTGGGGAATACACCACCCACCTAACT CGAAGGAGCAGCAGAATCTGTACCAGAATGAGA ATGCCTACGTGTCCGTCGTGACCTCCAACTACAA CCGGCGGTTCACGCCTGAGATCGCCGAGAGGCC TAAGGTGAGGGACCAGGCCGGACGCATGAACTA CTACTGGACCCTGCTGAAGCCTGGCGATACAATC ATCTTCGAGGCTAATGGAAACCTGATCGCGCCA ATGTACGCCTTCGCCCTGTCCAGAGGATTCGGCA GCGGCATCATCACATCCAACGCCTCCATGCACG AATGCAACACCAAGTGCCAGACGCCTCTGGGAG CTATCAATAGCAGCTTGCCTTACCAGAATATCCA CCCTGTGACCATTGGAGAGTGTCCAAAGTACGT | 81 |
| | | |
| **includes transmembrane sequence (second underlined)** | | |
| | GCGCAGCGCAAAGCTGCGGATGGTCACAGGCCT GCGGAATATACCTTCTATCCAGAGCCGAGGCCT GTTCGGTGCCATTGCCGGCTTCATCGAGGGTGGC TGGACCGGAATGATCGACGGCTGGTATGGATAC CACCACCAGAATGAACAGGGCAGCGGCTACGCC GCCGATCAGAAGTCCACCCAGAACGCAATCAAT GGTATCACAAACAAGGTGAACACTGTAATCGAG AAGATGAACATCCAATTCACAGCCGTGGGCAAG GAGTTCAATAAGCTGGAGAAGCGGATGGAGAAC CTCAACAAGAAGGTGGACGACGGCTTCCTGGAT ATCTGGACCTACAACGCAGAGCTGCTGGTGTTGC TGGAGAACGAGAGAACCCTCGACTTCCATGATA GCAACGTTAAGAACCTATACGAGAAGGTGAAGT CACAGCTGAAGAATAACGCCAAGGAGATTGGCA ACGGCTGCTTCGAATTCTACCACAAGTGCGACA ACGAGTGTATGGAGAGCGTCCGGAATGGCACCT ACGACTATCCTAAGTATAGCGAGGAGAGCAAGC TTAATAGAGAGAAGGTCGATGGCGTGAAGCTGG AGTCAATGGGAATCTACCAGATCCTGGCTATTTA TTCAACCGTGGCATCAAGTCTGGTGCTTCTGGTC AGCCTGGGCGCCATCAGCTTCTGGATGTGCTCCA ATGGCAGCCTGCAATGCCGCATCTGCATA | |
| **FLHA_Cal09** | ATGAAGGCTATCTTGGTGGTGTTGTTGTACACAT TCGCCACCGCCAACGCCGACACCCTCTGCATCGG CTACCACGCGAACAATTCAACCGACACCGTTGA CACCGTCCTCGAGAAGAACGTGACCGTGACTCA TAGCGTCAACCTCCTCGAGGACAAGCATAACGG CAAGCTCTGTAAGCTTAGAGGAGTGGCCCCTCTC CACCTGGGCAAGTGTAACATTGCAGGCTGGATC CTGGGCAACCCTGAGTGCGAGAGCCTGTCAACC GCTAGCAGCTGGAGCTACATCGTGGAAACCCCA TCCAGCGATAACGGCACCTGCTACCCTGGCGATT TCATCGACTACGAGGAGCTGCGCGAGCAGTTGA GCAGCGTCTCCAGCTTCGAGAGATTCGAGATCTT CCCTAAGACTAGCAGCTGGCCTAATCATGACTCC AATAAGGGCGTGACGGCCGCCTGTCCTCACGCT GGAGCCAAGTCGTTCTACAAGAACCTGATCTGG CTGGTAAAGAAGGGCAACAGCTACCCAAAGCTG AGCAAGTCCTACATCAACGACAAGGGCAAGGAA GTGCTGGTGCTGTGGGGAATCCATCACCCAAGC ACCTCTGCGGACCAGCAGTCTCTGTATCAGAACG CCGACACCTATGTGTTCGTAGGCTCCTCCAGATA CTCCAAGAAGTTCAAGCCAGAGATTGCTATCCG CCCAAAGGTGCGGGATCAAGAGGGTCGCATGAA TTATTACTGGACCCTGGTCGAGCCAGGCGATAA GATCACATTCGAAGCCACGGGAAATCTGGTGGT GCCTAGATACGCTTTCGCCATGGAGAGAAACGC CGGCAGCGGCATCATCATATCCGACACACCTGT GCACGACTGCAACACAACATGCCAGACGCCAAA GGGAGCCATCAACACATCTCTTCCATTCCAGAAC ATTCACCCAATCACAATCGGCAAGTGTCCAAAG TACGTGAAGTCCACCAAGCTTAGACTGGCCACC GGCCTGCGTAACATCCCTAGCATCCAGTCGAGA GGCCTCTTCGGCGCCATCGCCGGATTCATTGAAG | 82 |
| | GTGGCTGGACCGGCATGGTGGACGGTTGGTATG GCTACCACCACCAGAACGAGCAGGGCAGCGGCT ACGCCGCGGACCTGAAGTCCACCCAGAACGCTA TTGACGAGATCACCAACAAGGTGAACAGCGTGA TCGAGAAGATGAATACCCAGTTCACCGCCGTCG GCAAGGAGTTCAACCATCTGGAGAAGAGAATCG AGAACCTCAACAAGAAGGTCGACGACGGCTTCC TGGACATTTGGACTTACAACGCTGAGTTGTTGGT GCTTCTTGAGAATGAGCGGACCCTGGACTATCAC GACTCAAATGTGAAGAACCTGTACGAGAAGGTG AGATCCCAGCTGAAGAACAATGCTAAGGAAATC GGCAACGGCTGCTTCGAGTTCTATCATAAGTGTG ACAACACCTGCATGGAGTCTGTTAAGAACGGCA CATACGACTACCCGAAGTACTCTGAGGAGGCCA AGCTGAACCGAGAGGAGATAGACGGCGTTAAGC TAGAAAGTACAAGGATCTACCAGATCCTTGCCA TCTACTCCACCGTGGCCTCCAGCCTGGTGTTGGT GGTGAGCCTGGGCGCCATCAGCTTCTGGATGTGC AGTAACGGAAGCCTACAGTGCCGAATCTGCATC | |

### Example 1: Mouse immunogenicity and efficacy studies

### Study #1

This study was designed to test the immunogenicity and efficacy in mice of a combination of candidate influenza virus vaccines. Animals tested were 6-8 week old female BALB/c mice obtained from Charles River Laboratories. Test vaccines included the following mRNAs formulated in an LNP (comprised of a cationic lipid, a sterol, a phospholipid and a peg-lipid).: NIHGen6HASS-foldon mRNA (based on Yassine et al. Nat. Med. 2015 Sep; 21(9):1065-70), an mRNA encoding the nucleoprotein NP from an H3N2 strain, or one of several combinations of NIHGen6HASS-foldon and NP mRNAs. Several methods of vaccine antigen co-delivery were tested including: mixing individual mRNAs prior to formulation with LNP (co-form), formulation of individual mRNAs prior to mixing (mix ind LNPs), and formulating mRNAs individually and injecting distal sites (opposite legs) (ind LNPs remote). Control animals were vaccinated with an RNA encoding the ectodomain of HA from H1N1 A/Puerto Rico/8/1934 (eH1HA, positive control) or empty LNP (to control for effects of the LNP) or were not vaccinated (naïve).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and are outlined in the table below. Sera were collected from all animals two weeks after the second dose. At week 6, spleens were harvested from a subset of the animals (n=4). The remaining animals (n=6) were challenged intranasally while sedated with a mixture of Ketamine and Xylazine with a lethal dose of mouse-adapted influenza virus strain H1N1 A/Puerto Rico/8/1934. Mortality was recorded and individual mouse weight was assessed daily for 20 days post-infection.

| **Group #** | **Antigen** | **Antiger dose** | **Formulation** | **Volume, Route** |
|---|---|---|---|---|
| 1 | NIHGen6HASS-foldon RNA | 5 ug | LNP | 100 ul, i.m. |
| 2 | NP RNA | 5 ug | LNP | 100 ul, i.m. |
| 3 | NIHGen6HASS-foldon RNA + NP RNA | 5 ug of each RNA mixed, then formulated | LNP | 100 ul, i.m. |
| 4 | NIHGen6HASS-foldon RNA + NP RNA | 5 ug of each RNA formulated, then mixed | LNP | 100 ul, i.m. |
| 5 | NIHGen6HASS-foldon RNA + NP RNA | 5 ug of each RNA formulated and injected into separate legs | LNP | 100 ul, i.m. |
| 6 | eH1HA RNA | 10 ug | LNP | 100 ul, i.m. |
| 7 | LNP | 0 ug | LNP | 100 ul, i.m. |
| 8 | Naive | 0 ug | None | None |

To test the sera for the presence of antibodies capable of binding to hemagglutinin (HA) from a wide variety of influenza strains or nucleoprotein (NP), ELISA plates were coated with 100 ng of the following recombinant proteins obtained from Sino Biological Inc.: Influenza A H1N1 (A/New Caledonia/20/99) HA, cat # 11683-V08H; Influenza A H3N2 (A/Aichi/2/1968) HA, cat # 11707-V08H; Influenza A H1N1 (A/California/04/2009) HA, cat # 11055-V08H; Influenza A H1N1 (A/Puerto Rico/8/34) HA, cat # 11684-V08H; Influenza A H1N1 (A/Brisbane/59/2007) HA, cat # 11052-V08H; Influenza A H2N2 (A/Japan/305/1957) HA, cat # 11088-V08H; Influenza A H7N9 (A/Anhui/1/2013) HA, cat # 40103-V08H, Influenza A H3N2 (A/Moscow/10/99) HA, cat #40154-V08 and Influenza A H3N2 (A/Aichi/2/1968) Nucleoprotein, cat # 40207-V08B. After coating, the plates were washed, blocked with Phosphate Buffered Saline with 0.05% Tween-20 (PBST) + 3% milk, and 100 µL of control antibodies or sera from immunized mice (diluted in PBST + 3% milk) were added to the top well of each plate and serially diluted. Plates were sealed and incubated at room temperature for 2 hours. Plates were washed, and goat anti-mouse IgG (H+L)-HRP conjugate (Novex, diluted 1:2000 in PBST/3% milk) was added to each well containing mouse sera. Plates were incubated at room temperature for 1 hr, washed, and incubated with TMB substrate (Thermo Scientific). The color was allowed to develop for approximately 10 minutes and then quenched with 100 µL of 2N sulfuric acid. The plates were read at 450 nM on a microplate reader. Endpoint titers (2.5-fold above background) were calculated.

Fig. 1 depicts the endpoint titers of the pooled serum from animals vaccinated with the test vaccines. The vaccines tested are shown on the x-axis of Fig. 1A and the binding to HA from each of the different strains of influenza is plotted. The NIHGen6HASS-foldon mRNA vaccine elicited high titers of antibodies that bound all H1, H2 and H7 HAs tested. Combining the NIHGen6HASS-foldon mRNA with one that encodes NP did not negatively affect the observed anti-HA response, regardless of the method of mRNA co-formulation or co-delivery. In serum collected from identical groups from a separate study, a robust antibody response to NP protein was also detected in serum from animals vaccinated with NP mRNA containing vaccines, either NP alone or co-formulated with NIHGen6HASS-foldon mRNA (Fig. 1B).

To probe the functional antibody response, the ability of serum to neutralize a panel of HA-pseudotyped viruses was assessed (Fig. 2). Briefly, 293 cells were co-transfected with a replication-defective retroviral vector containing a firefly luciferase gene, an expression vector encoding a human airway serine protease, and expression vectors encoding influenza hemagglutinin (HA) and neuraminidase (NA) proteins. The resultant pseudoviruses were harvested from the culture supernatant, filtered, and titered. Serial dilutions of serum were incubated in 96 well plates at 37°C for one hour with pseudovirus stocks (30,000 - 300,000 relative light units per well) before 293 cells were added to each well. The cultures were incubated at 37°C for 72 hours, luciferase substrate and cell lysing reagents were added, and relative light units (RLU) were measured on a luminometer. Neutralization titers are expressed as the reciprocal of the serum dilution that inhibited 50% of pseudovirus infection (IC50).

For each sample tested (listed along the x-axis), each bar represents the IC50 for neutralization of a different virus pseudotype. While the serum from naive or NP RNA vaccinated mice was unable to inhibit pseudovirus infection, the serum from mice vaccinated with NIHGen6HASS-foldon mRNA or with a combination of NIHGen6HASS-foldon and NP mRNAs neutralized, to a similar extent, all H1 and H5 virus pseudotypes tested.

Three weeks after the administration of the second vaccine dose, spleens were harvested from a subset of animals in each group and splenocytes from animals in the same group were pooled. Splenic lymphocytes were stimulated with a pool of HA or NP peptides, and IFN-γ, IL-2 or TNF-α production was measured by intracellular staining and flow cytometry. Figure 3 is a representation of responses following stimulation with a pool of NP peptides, and Figure 4 is a representation of responses following stimulation with a pool of H1 HA peptides. Following vaccination with NP mRNA, either in the presence or absence of NIHGen6HASS-foldon mRNA, antigen-specific CD4 and CD8 T cells were found in the spleen. Following vaccination with NIHGen6HASS-foldon RNA or delivery of NIHGen6HASS-foldon and NP RNAs to distal injections sites (ind. LNPs remote), only HA-specific CD4 cells were observed. However, when NIHGen6HASS-foldon and NP RNAs were co-administered to the same injection site (co-form, ind. LNPs mix), an HA-specific CD8 T cell response was detected.

Following lethal challenge with mouse-adapted H1N1 A/Puerto Rico/8/1934, all naive animals succumbed to infection by day 12 post-infection (Fig. 5). In contrast, all animals vaccinated with NIHGen6HASS-foldon mRNA, NP mRNA, any combination of NIHGen6HASS-foldon and NP mRNAs, or eH1HA mRNA survived the challenge. As seen in Fig. 5A, although there was no mortality, mice that were vaccinated with an H3N2 NP mRNA and challenged with H1N1 virus lost a significant amount (~15%) of weight prior to recovery. Those vaccinated with NIHGen6HASS-foldon RNA also lost ~5% body weight. In contrast, mice vaccinated with a combination of NIHGen6HASS-foldon and NP mRNAs appeared to be completely protected from lethal influenza virus challenge, similar to those vaccinated with mRNA expressing an HA antigen homologous to that of the challenge virus (eH1HA). Although the cell-mediated immune responses varied, the vaccine efficacy was similar with all co-formulation and co-delivery methods assessed (Fig. 5B).

### Study #2

This study was designed to test the immunogenicity and efficacy in mice of a candidate influenza virus vaccine. Animals tested were 6-8 week old female BALB/c mice obtained from Charles River Laboratories. Test vaccines included the following mRNAs formulated in an LNP (comprised of a cationic lipid, a sterol, a phospholipid and a peg-lipid). LNP: NIHGen6HASS-foldon mRNA (based on Yassine et al. Nat. Med. 2015 Sep; 21(9):1065-70) and NIHGen6HASS-TM2 mRNA, an RNA expressing HA stem fused to the native influenza transmembrane domain. Control animals were vaccinated with an mRNA encoding the ectodomain of the HA from H1N1 A/Puerto Rico/8/1934 (eH1HA, positive control) or were not vaccinated (naïve).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and outlined in the table below. Sera were collected from all animals two weeks after the second dose. At week 6, all animals were challenged intranasally while sedated with a mixture of Ketamine and Xylazine with a lethal dose of mouse-adapted influenza virus strain H1N1 A/Puerto Rico/8/1934. Mortality was recorded and group mouse weight was assessed daily for 20 days post-infection.

| **Group #** | **Antigen** | **Antigen dose** | **Formulation** | **Volume, Route** |
|---|---|---|---|---|
| 1 | NIHGen6HASS-foldon RNA | 5 ug | LNP | 100 ul, i.m. |
| 2 | NIHGen6HASS-foldon-TM2 RNA | 5 ug | LNP | 100 ul, i.m. |
| 3 | eH1HA RNA | 10 ug | LNP | 100 ul, i.m. |
| 4 | Naïve | 0 ug | None | None |

To test the sera for the presence of antibody capable of binding to hemagglutinin (HA) from a wide variety of influenza strains, ELISA plates were coated with 100 ng of the following recombinant HAs obtained from Sino Biological Inc.: Influenza A H1N1 (A/New Caledonia/20/99), cat # 11683-V08H; Influenza A H3N2 (A/Aichi/2/1968), cat # 11707-V08H; Influenza A H1N1 (A/California/04/2009), cat # 11055-V08H; Influenza A H1N1 (A/Puerto Rico/8/34), cat # 11684-V08H; Influenza A H1N1 (A/Brisbane/59/2007), cat # 11052-V08H; Influenza A H2N2 (A/Japan/305/1957), cat # 11088-V08H; Influenza A H7N9 (A/Anhui/1/2013), cat # 40103-V08H and Influenza A H3N2 (A/Moscow/10/99), cat #40154-V08. The ELISA assay was performed and endpoint titers were calculated as described above. Fig. 6A depicts the endpoint titers of the pooled serum from animals vaccinated with the test vaccines. The vaccines tested are shown on the x-axis and the binding to HA from each of the different strains of influenza is plotted. The NIHGen6HASS-foldon mRNA vaccine elicited high titers of antibodies that bound all H1, H2 and H7 HAs tested. The binding titers from NIHGen6HASS-TM2 mRNA vaccinated mice were reduced as compared to those from NIHGen6HASS-foldon mRNA vaccinated mice.

Following lethal challenge with mouse-adapted H1N1 A/Puerto Rico/8/1934, all naive animals succumbed to infection by day 15 post-infection (Fig. 6B). In contrast, all animals vaccinated with NIHGen6HASS-foldon mRNA, NIHGen6HASS-TM2 mRNA, or eH1HA RNA survived the challenge. As shown in Fig. 6B, in spite of reduced HA binding titers, the efficacy of the NIHGen6HASS-TM2 vaccine was equivalent to that of the NIHGen6HASS-foldon vaccine.

### Study #3

In this example, animal studies and assays were carried out to evaluate the immune response to influenza virus consensus hemagglutinin (HA) vaccine antigens delivered using an mRNA/LNP platform. The purpose of this study was to evaluate the ability of consensus HA mRNA vaccine antigens to elicit cross-reactive immune responses in the mouse.

To generate consensus HA sequences MRK_sH1_Con and MRK_pH1_Con, 2415 influenza A serotype H1 HA sequences were obtained from the NIAID Influenza Research Database (IRD) (Squires et al., Influenza Other Respir Viruses. 2012 Nov; 6(6): 404-416.) through the web site at http://www.fludb.org. After removal of duplicate sequences and lab strains, 2385 entries remained, including 1735 H1 sequences from pandemic H1N1 strains (pH1N1) and 650 from seasonal H1N1 strains (sH1N1). Pandemic and seasonal H1 sequences were separately aligned, and a consensus sequence was generated for each group using the Matlab 9.0 Bioinformatics toolbox (MathWorks, Natick, MA). Sequence profiles were generated for both groups separately using a modified Seq2Logo program (Thomsen et al., Nucleic Acids Res. 2012 Jul;40 (Web Server issue):W281-7).

Animals tested were 6-8 week old female BALB/c mice obtained from Charles River Laboratories. Test vaccines included the following mRNAs formulated in an LNP (comprised of a cationic lipid, a sterol, a phospholipid and a peg-lipid).: ConH1_ and ConH3 (based on Webby et al., PLoS One. 2015 Oct 15;10(10):e0140702.); Cobra_P1 and Cobra_X3 (based on Carter et al., J Virol. 2016 Apr 14;90(9):4720-34); MRK_pH1_Con and MRK_sH1_Con (pandemic and seasonal consensus sequences described above); and each of the above mentioned six antigens with a ferritin fusion sequence for potential particle formation.

Controls included: an LNP (comprised of a cationic lipid, a sterol, a phospholipid and a peg-lipid; control for effects of LNP); Naive (unvaccinated animals); and vaccination with eH1HA RNA, which encodes the ectodomain of HA from strain H1N1 A/PR/8/34 (positive control for the virus challenge).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and are outlined in the table below. Sera were collected from all animals two weeks after the second dose (week 5).

| **Group #** | **Antigen** | **Antigen dose** | **Formulation** | **Volume, Route** |
|---|---|---|---|---|
| 1 | Con_H1 RNA | 10 ug | LNP | 100 ul, i.m |
| 2 | Con_H3 RNA | 10 ug | LNP | 100 ul, i.m. |
| 3 | MRK_pH1_Con RNA | 10 ug | LNP | 100 ul, i.m. |
| 4 | MRK_sH1_Con RNA | 10 ug | LNP | 100 ul, i.m. |
| 5 | Cobra_P1 RNA | 10 ug | LNP | 100 ul, i.m. |
| 6 | Cobra_X3 RNA | 10 ug | LNP | 100 ul, i.m. |
| 7 | ConH1_ferritin RNA | 10 ug | LNP | 100 ul, i.m. |
| 8 | ConH3 _ferritin RNA | 10 ug | LNP | 100 ul, i.m. |
| 9 | MRK_pH1_Con_ferritin RNA | 10 ug | LNP | 100 ul, i.m. |
| 10 | MRK_sH1_Con_ferritin RNA | 10 ug | LNP | 100 ul, i.m. |
| 11 | Cobra_P1_ferritin RNA | 10 ug | LNP | 100 ul, i.m. |
| 12 | Cobra_X3_ferritin RNA | 10 ug | LNP | 100 ul, i.m. |
| 13 | eH1HA | 10 ug | LNP | 100 ul, i.m. |
| 14 | LNP | 0 ug | LNP | 100 ul, i.m. |
| 15 | Naïve | 0 ug | None | None |

To assess the breadth of the serum neutralizing activity elicited by the consensus HA antigens, neutralization assays were performed using a panel of H1N1 influenza viruses (Fig. 7A). Briefly, Madin-Darby Canine Kidney (MDCK) cells were seeded in 96-well plates with complete media (DMEM containing Glutamax^{®}, 50 µL/mL gentamycin and 10% FBS) and incubated overnight at 37 °C. Duplicate serial dilutions of heat inactivated (35-45 min at 56 °C) serum samples as well as virus were added to cells, and cultures were incubated at 37 °C for 1 hour. Complete media was then replaced with trypsin-containing medium. Cell cultures were incubated at 37 °C for 2 days then fixed with 80% acetone and air-dried before plates were washed with PBS containing 0.1% Tween-20. An ELISA based assay was performed to detect influenza NP protein on the fixed plates. As expected, serum from mice immunized with eH1HA RNA, which encodes the ectodomain of HA from strain H1N1 A/PR/8/34, was only able to robustly neutralize a matched influenza strain (A/Puerto Rico/8/1934). In contrast, serum from mice immunized with the consensus H1 HA antigens was able to neutralize multiple diverse H1N1 strains isolated between 1934 and 2009. This observation was repeated in at least two additional independent studies in which an LNP containing a different cationic lipid nanoparticle ("LNP2"), was used to deliver the mRNA vaccines (Fig. 7B). With the exception of MRK_pH1_Con_ferritin, the ferritin fusion constructs induced at least similar, if not more potent, broadly neutralizing antibody titers as compared to the parental constructs (Fig. 7A).

### Study #4

This study was designed to test the immunogenicity and efficacy in mice of candidate influenza virus vaccines. Animals tested were 6-8 week old female BALB/c mice obtained from Charles River Laboratories. Test vaccines included the following mRNAs formulated in a cationic LNP: MRK_pH1_Con and MRK_sH1_Con (pandemic and seasonal consensus sequences described in study #3), MRK_sHl_Con_v2 (seasonal consensus sequence derived from a different H1N1 sequence database), MRK_pH1_Con ecto and MRK_sH1_Con ecto (soluble ectodomain of pandemic and seasonal consensus sequences) and MRK_pH1_Con_RBD and MRK_sH1_Con_RBD (receptor binding domain of pandemic and seasonal consensus sequences, details on preparation of the constructs were described below in the next sections). A vaccine combining mRNA encoding MRK_pH1_Con and mRNA encoding the nucleoprotein NP from an H3N2 strain was also assessed. Control animals were vaccinated with an mRNA encoding the ectodomain of the HA from H1N1 A/Puerto Rico/8/1934 (eH1HA, positive control), empty LNP, or were not vaccinated (naïve).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and outlined in the table below. Sera were collected from all animals two weeks after the second dose. At week 6, all animals were challenged intranasally while sedated with a mixture of Ketamine and Xylazine with a lethal dose of mouse-adapted influenza virus strain H1N1 A/Puerto Rico/8/1934. Mortality was recorded and group mouse weight was assessed daily for 20 days post-infection.

| **Group #** | **Antigen** | **Antigen dose** | **Formulation** | **Volume, Route** |
|---|---|---|---|---|
| 1 | MRK_sH1_Con RNA | 5 ug | LNP2 | 100 ul, i.m. |
| 2 | MRK_sH1_Con_v2 RNA | 5 ug | LNP2 | 100 ul, i.m. |
| 3 | MRK_sH1_Con_ecto RNA | 5 ug | LNP2 | 100 ul, i.m. |
| 4 | MRK_sH1_Con_RBD RNA | 5 ug | LNP2 | 100 ul, i.m. |
| 5 | MRK_pH1_Con RNA | 5 ug | LNP2 | 100 ul, i.m. |
| 6 | MRK_pH1_Con RNA + NP RNA | 5 ug of each RNA formulated, then mixed | LNP2 | 100 ul, i.m. |
| 7 | MRK_pH1_Con_ecto RNA | 5 ug | LNP2 | 100 ul, i.m. |
| 8 | MRK_pH1_Con_RBD RNA | 5 ug | LNP2 | 100 ul, i.m. |
| 9 | eH1HA RNA | 5 ug | LNP2 | 100 ul, i.m. |
| 10 | Empty LNP2 | 0 ug | LNP2 | 100 ul, i.m. |
| 11 | Naïve | 0 ug | None | None |

To assess the breadth of the serum activity elicited by the consensus HA antigens, hemagglutination inhibition assays (HAI) were performed using a panel of H1N1 influenza viruses (Fig. 8A). Briefly, serum samples were treated with receptor destroying enzyme (RDE) for 18 - 20 hrs at 37°C before inactivation at 56°C for 35 - 45 min. RDE-treated sera was then serially diluted in a 96 well plate and mixed with 4 hemagglutinating units of virus. An equal volume of 0.5% turkey red blood cells was added to each well, and plates were incubated at room temperature for 30 min. The highest dilution with no visible agglutination was assigned as the serum titer. With the exception of the pH1_Con_ecto mRNA vaccine, serum from mice immunized with mRNAs encoding full-length or the soluble ectodomain of consensus HAs were able to inhibit hemagglutination of red blood cells mediated by multiple diverse H1N1 strains isolated between 1934 and 2009. HAI titers were similar from mice immunized with pH1_Con vaccine with or without addition of NP vaccine.

All mice immunized with mRNAs encoding full-length or the soluble ectodomain of consensus H1 HAs survived a lethal PR8 virus challenge, while all naive mice and those vaccinated with empty LNP succumbed to challenge (Fig. 8B). Mice immunized with mRNAs encoding the receptor binding domain of consensus H1 HAs were partially protected: 80% survival for mice immunized with MRK_pH1_Con_RBD and 60% survival for those vaccinated with MRK_sH1_Con_RBD (Fig. 8B). Additionally, mice immunized with MRK_sH1_Con mRNA showed no weight loss post-challenge, in contrast to mice vaccinated with MRK_sH1_Con_v2, MRK_pH1_Con or MRK_pH1_Con_ecto mRNAs which lost, on average, between 7 and 10% of their body weight before recovering fully (Fig. 8C and Fig. 8D). As observed with the NIHGen6HASS-foldon + NP combination mRNA vaccine described in study #1, the MRK_pH1_Con + NP combination mRNA vaccine protected mice better than the MRK_pH1_Con mRNA vaccine alone. Mice in the MRK_pH1_Con + NP group were completely protected from lethal influenza virus challenge and lost no weight post-challenge, similar to mice vaccinated with mRNA expressing an HA antigen homologous to that of the challenge virus (eH1HA) (Fig. 8D).

### Study #5

In this example, two animal studies (A and B) were carried out to assess the ability of candidate influenza mRNA vaccine antigens to elicit cross-protective immune responses in the mouse.

Animals tested were 7-9 week old female BALB/c mice obtained from Envigo. Test vaccines included the following mRNAs formulated in a cationic LNP: MRK_pH1_Con and MRK_sH1_Con (pandemic and seasonal consensus sequences described in study #3); NIHGen6HASS-foldon, NP, a combination of NIHGen6HASS-foldon and NP vaccines, and a combination of NIHGen6HASS-TM2 and NP vaccines. Controls included: empty LNP (control for effects of LNP); Naive (unvaccinated animals); and vaccination with FL_Cal09 mRNA, which encodes the full length HA from strain H1N1 A/California/07/09 (positive control for the virus challenge).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and are outlined in the table below. Sera were collected from all animals two weeks after the second dose (week 5). At week 6, the animals were challenged intranasally while sedated with a mixture of Ketamine and Xylazine with a lethal dose of influenza virus strain H1N1 A/California/07/2009 (Cal09). Mortality was recorded and mouse weight was assessed daily for 21 days post-infection. Mice at less than 80% of their pre-challenge weight were humanely euthanized.

| **Group #** | **Antigen** | **Antigen dose** | **Formulation** | **Volume, Route** |
|---|---|---|---|---|
| 1 | MRK_pH1_Con RNA | 5 ug | LNP2 | 100 ul, i.m. |
| 2 | MRK_sH1_Con RNA | 5 ug | LNP2 | 100 ul, i.m. |
| 3 | NIHGen6HASS-foldon RNA | 5 ug | LNP2 | 100 ul, i.m. |
| 4 | NP RNA | 5 ug | LNP2 | 100 ul, i.m. |
| 5 | NIHGen6HASS-foldon RNA + NP RNA | 5 ug each | LNP2 | 100 ul, i.m. |
| 6 | NIHGen6HASS-TM2 RNA + NP RNA | 5 ug each | LNP2 | |
| 7 | FL_Cal09 RNA | 10 ug | LNP2 | 100 ul, i.m. |
| 8 | Empty LNP2 | 0 ug | LNP2 | 100 ul, i.m. |
| 9 | Naïve | 0 ug | None | None |

Following lethal challenge with H1N1 A/California/07/2009, all naive animals succumbed to infection by day 7 post-infection (Fig. 9A). Between 80 and 100% of mice vaccinated with empty LNP2 also succumbed to infection (Fig. 9A for study A and Fig. 9B for study B). In contrast, all animals vaccinated with candidate vaccines survived the challenge (Figs. 9A and 9B). Although there was no mortality, mice vaccinated with an mRNA encoding NIHGen6HASS-foldon or NP lost a significant amount of body weight, approximately 10% on average, prior to recovery (Fig. 9C). Mice vaccinated with a combination of NIHGen6HASS-foldon and NP mRNAs appeared to be better protected from lethal Cal09 virus challenge, and the group lost, on average, only approximately 5% body weight. In study B , mice vaccinated with a combination of NIHGen6HASS-TM2 and NP mRNAs showed a similar pattern of weight loss and recovery (Fig. 9D). Consistent with the high serum neutralizing titers to the Cal09 strain observed in previously described studies (Figs. 7A and 7B), mice immunized with MRK_pH1_Con mRNA survived the lethal Cal09 virus challenge and lost no weight post-infection. In contrast, mice vaccinated with MRK_sH1_Con mRNA, which does not induce neutralizing titers to Cal09 (Figs. 7A and 7B), lost, on average, approximately 5% of their body weight post-infection, suggesting that partial protection may be mediated by mechanism(s) other than virus neutralization.

### Study #6

This study was designed to test the immunogenicity and efficacy in mice of candidate influenza virus vaccines. Animals tested were 6-8 week old female BALB/c mice obtained from Charles River Laboratories. Test vaccines included the following mRNAs formulated in a cationic LNP: MRK_H1_cot_all, MRK_H3_cot_all, MRK_H3_con_all, MRK_H3_Consensus A and MRK_H3_Consensus B. Consensus H3 sequences were generated similarly as described previously for consensus H1 sequences. COT sequences for H1 and H3 subtypes were generated as described below in the next section. Control animals were vaccinated with an mRNA encoding the HA from H1N1 A/Puerto Rico/8/1934 (FLHA PR8, positive control for PR8 infection), vaccinated with empty LNP, infected with a nonlethal dose of mouse-adapted H3 A/Hong Kong/1/1968, or were not vaccinated (naïve).

At week 0 and week 3, animals were immunized intramuscularly (IM) with a total volume of 100 µL of each test vaccine, which was administered in a 50 µL immunization to each quadricep. Candidate influenza virus vaccines evaluated in this study were described above and outlined in the table below. Sera were collected from all animals two weeks after the second dose. At week 6, all animals were challenged intranasally while sedated with a mixture of Ketamine and Xylazine with a lethal dose of mouse-adapted influenza virus strain H1N1 A/Puerto Rico/8/1934 (PR8) or H3 A/Hong Kong/1/1968 (HK68). Mortality was recorded and group mouse weight was assessed daily for 20 days post-infection.

| **Group #** | **Antigen** | **Antigen dose** | **Formulation** | **Volume, Route** |
|---|---|---|---|---|
| 1 | FLHA_PR8 RNA | 5 ug | LNP2 | 100 ul, i.m. |
| 2 | MRK_H1_cot_all RNA | 10 ug | LNP2 | 100 ul, i.m. |
| 3 | MRK_H3_cot all RNA | 10 ug | LNP2 | 100 ul, i.m. |
| 4 | MRK_H3_con_all RNA | 10 ug | LNP2 | 100 ul, i.m. |
| 5 | MRK_H3_Consensus A RNA | 10 ug | LNP2 | 100 ul, i.m. |
| 6 | MRK_H3_Consensus B RNA | 10 ug | LNP2 | 100 ul, i.m. |
| 7 | Empty LNP2 | 0 ug | LNP2 | 100 ul, i.m. |
| 8 | Mouse-adapted H3 A/Hong Kong/1/1968 virus | 0.1 LD90 | None | 20 ul, i.n. |
| 9 | Naïve | 0 ug | None | None |

To assess the breadth of the serum activity elicited by the antigens, hemagglutination inhibition assays (HAI) were performed using a panel of H1N1 and H3N2 influenza viruses (Figs. 10A and 10B). Briefly, serum samples were treated with receptor destroying enzyme (RDE) for 18 - 20 hrs at 37°C before inactivation at 56°C for 35 - 45 min. RDE-treated sera was then serially diluted in a 96 well plate and mixed with 4 hemagglutinating units of virus. An equal volume of 0.5% turkey red blood cells was added to each well, and plates were incubated at room temperature for 30 min. The highest dilution with no visible agglutination was assigned as the serum titer. While the MRK_H1_cot_all mRNA vaccine elicited titers to only two viruses in the H1 HAI panel (Fig. 10A), the MRK_H3_cot_all, MRK_H3_con_all, MRK_H3_Consensus A and MRK_H3_Consensus B mRNAs induced high HAI titers to multiple H3 strains isolated between 1997 and 2014 (Fig. 10B).

Although mice immunized with MRK_H1_cot_all mRNA did not have detectable HAI titers to the PR8 virus, they were partially protected from lethal challenge with PR8 virus. In contrast to naive or LNP2 vaccinated mice, all MRK_H1_cot_all mRNA immunized mice survived challenge (Fig. 11A), though they lost, on average, approximately 10% of their body weight post-infection (Fig. 11B). Similarly, mice vaccinated with any of the H3 COT or consensus mRNAs tested survived challenge with a lethal dose of HK68 virus (Fig. 11C) but lost between 10 and 15% or their body weight post-infection (Fig. 11D).

### Preparation of COT Constructs

The "Center of Tree" or COT is the point on the phylogenetic tree that represents the minimum of a metric of evolutionary distance. The COT minimizes the evolutionary distance to all sampled circulating strains, while still residing on an evolutionary path to better capture the biological properties of circulating viruses (D.C. Nickle et al., Consensus and ancestral state HIV vaccines, Science 299, 1515 (Mar 7, 2003)). To prepare the COT sequences described herein, hemagglutinin DNA sequences of all of Influenza A collected after 2010 were downloaded from the Influenza Research Database (fludb.org), with H1 genotype forming data set 1 and H3 genotype forming data set 2. Any DNA sequence with aberrant/ambiguous nucleotides was removed. The initial trees for each data set where estimated by aligning the remaining sequences for each data set using the software package MUSCLE (R. C. Edgar, MUSCLE: a multiple sequence alignment method with reduced time and space complexity. BMC bioinformatics 5, 113 (Aug 19, 2004)). Next the alignment was used to estimate a Maximum Likelihood (ML) phylogeny in the software package PhyML under a GTR + G + I model of nucleotide evolution. (S. Guindon et al., New algorithms and methods to estimate maximum-likelihood phylogenies: assessing the performance of PhyML 3.0. Systematic biology 59, 307 (May, 2010)). The estimated ML trees where re-rooted with the COT algorithm defined above - giving rise to a node called COT. The ML state for each and every node including the COT node in the trees was estimated using ML methods under the same model of nucleotide evolution estimated during the initial tree estimation. Because the COT is a node, we can parse out the COT node state DNA sequences from the list of node sequences found throughout the trees. Corresponding protein sequences were derived based on the COT DNA sequences described above.

### Preparation of Receptor Binding Domain Constructs

The following was used as a template sequence for all designs: A/California/4/2009(H1N1) (gi:227809830|UniProtKB:C3W5S1). The Receptor Binding Domain (RBD) was defined as residues 63-278 based on structural analysis (DuBois et al., J. Virology Jan. 2011, p. 865). The 6 constructs are divided into two types: MRK_RBD which uses the native sequence as a template, and RBD1 which adds an additional glycosylation site at position 97 to increase polarity in an exposed hydrophobic patch. The MRK_RBD-Cal09-PC, MRK_RBD-Cal09-PC-Cb, RBD1-Cal09-PC and RBD1-Cal09-PC-Cb constructs are RBDs containing inserted glycosylation recognition motifs to result in a hyper-glycosylated form of the protein upon post-translational modification by the cell (Eggink et al., J. Virology Jan. 2014 (volume 88 number 1) p. 699).

Those skilled in the art will recognize, or be able to ascertain using no more than routine experimentation, many equivalents to the specific embodiments of the disclosure described herein. Such equivalents are intended to be encompassed by the following claims.

All references, including patent documents, disclosed herein are incorporated by reference in their entirety.

### FEATURES

1. An influenza virus vaccine, comprising at least one isolated antigenic polypeptide:
   wherein the at least one antigenic polypeptide comprises an amino acid sequence that has at least 90% identity to a mature amino acid sequence in any one of SEQ ID NOs: 1-4, 8-11, 14-19, 41-61, and 83-88.
2. The vaccine of feature 1, wherein the polypeptide comprises an amino acid sequence that has at least 95% identity to a mature amino acid sequence in any one of SEQ ID NOs: 1-4, 8-11, 14-19, 41-61, and 83-88.
3. The vaccine of feature 1, wherein the polypeptide comprises an amino acid sequence that has 95%-99% identity to a mature amino acid sequence in any one of SEQ ID NOs: 1-4, 8-11, 14-19, 41-61, and 83-88.
4. The vaccine of any one of features 1-3, wherein the polypeptide has conservative mutations compared to a mature amino acid sequence in any one of SEQ ID NOs: 1-4, 8-11, 14-19, 41-61, and 83-88.
5. The vaccine of feature 1, wherein the polypeptide comprises the mature amino acid sequence that is identified in any one of SEQ ID NOs: 1-4, 8-11, 14-19, 41-61, and 83-88.
6. The vaccine of feature 1, wherein the polypeptide consists of the mature amino acid sequence that is identified in any one of SEQ ID NOs: 1-4, 8-11, 14-19, 41-61, and 83-88.
7. The vaccine of feature 1, wherein the polypeptide comprises a mature amino acid sequence that is the extracellular domain of SEQ ID NO: 8 or 10.
8. An influenza virus vaccine comprising two isolated antigenic polypeptides, wherein the first polypeptide comprises an amino acid sequence that has at least 90% identity to the mature amino acid sequence of any one of SEQ ID NOs: 1-5, 8-11, 14-19, 41-63, and 83-88 and the second polypeptide comprises an amino acid sequence that has at least 90% identity to the mature amino acid sequence of SEQ ID NO: 20.
9. The vaccine of feature 8, wherein the first polypeptide comprises an amino acid sequence that has at least 95% identity to the mature amino acid sequence of SEQ ID:NOs: 1-5, 8-11, 14-19, 41-63, and 83-88 and the second polypeptide comprises an amino acid sequence that has at least 95% identity to the mature amino acid sequence of SEQ ID NO: 20.
10. The vaccine of feature 8, wherein the first polypeptide comprises an amino acid sequence that has at least 95-99% identity to the mature amino acid sequence of SEQ NOs: 1-5, 8-11, 14-19, 41-63, and 83-88, and the second polypeptide comprises an amino acid sequence that has at least 95-99% identity to the mature amino acid sequence of SEQ ID NO: 20.
11. The vaccine of any one of features 8-10, wherein the first and second polypeptide has conservative mutations compared to the mature amino acid sequence in any one of SEQ ID NOs: 1-5, 8-11, 14-19, 41-63, 83-88 and 20.
12. The vaccine of feature 8, wherein the first polypeptide comprises the mature amino acid sequence in SEQ ID NO: 4, and the second polypeptide comprises the mature amino acid sequence in SEQ ID NO: 20.
13. The vaccine of feature 8, wherein the first polypeptide consists of the mature amino acid sequence in SEQ ID NO: 4, and the second polypeptide consists of the mature amino acid sequence in SEQ ID NO: 20.
14. The vaccine of any one of features 1-13, wherein the vaccine is multivalent.
15. The vaccine of any one of features 1-14 formulated in an effective amount to produce an antigen-specific immune response.
16. The vaccine of any one of features 1-14 formulated with a Lipid Nanoparticle (LNP) comprising one or more cationic lipids and a poly(ethyleneglycol)-lipid (PEG-lipid).
17. The vaccine of feature 16, wherein the one or more cationic lipids are ionizable cationic lipids.
18. The vaccine of feature 17, wherein the ionizable cationic lipids are selected from DLinDMA; DlinKC2DMA; DLin-MC3-DMA; CLinDMA; S-Octyl CLinDMA;
   (2 S )-1-{7-[(3 β)-cholest-5-en-3-yloxy]heptyloxy}-3-[(4 Z )-dec-4-en-1-yloxy]- *N, N-*dimethylpropan-2-amine;
   (2 R )-1-{4-[(3 β)-cholest-5-en-3-yloxy]butoxy}-3-[(4 Z )-dec-4-en-1-yloxy]- N, N - dimethylpropan-2-amine;
   1-[(2 R )-1-{4-[(3 β )-cholest-5-en-3-yloxy]butoxy}-3-(octyloxy)propan-2-yl]guanidine;
   1-[(2 R )-1-{7-[(3 β )-cholest-5-en-3-yloxy]heptyloxy}-N,N-dimethyl-3-[(9 Z, 12 Z )-octadeca-9,12-dien-1-yloxy]propan-2-amine;
   1-[(2 R )-1-{4-[(3 β )-cholest-5-en-3-yloxy]butoxy}-N,N-dimethyl-3-[(9 Z, 12 Z )-octadeca-9,12-dien-1-yloxy]propan-2-amine;
   (2S)-1-({6-[(3β))-cholest-5-en-3-yloxy]hexyl}oxy)-N,N-dimethyl-3-[(9 Z)-octadec-9-en-1-yloxy]propan-2-amine;
   (3β)-3-[6-{ [ (2S)-3-[ (9Z)-octadec-9-en-1-yloxyl]-2-(pyrrolidin-1-yl)propyl]oxy}hexyl)oxy] cholest-5-ene;
   (2R)-1-{4-[(3β)-cholest-5-en-3-yloxy]butoxy}-3-(octyloxy)propan-2-amine;
   (2R)-1-({8-[(3β)-cholest-5-en-3-yloxy]octyl}oxy)-N,N-dimethyl-3-(pentyloxy)propan-2-amine;
   (2R)-1-({8-[(3β)-cholest-5-en-3-yloxy]octyl}oxy)-3-(heptyloxy)-N,N-dimethylpropan-2-amine;
   (2R)-1-({8-[(3β)-cholest-5-en-3-yloxy]octyl}oxy)-N,N-dimethyl-3-[(2Z)-pent-2-en-1-yloxy]propan-2-amine;
   (2S)-1-butoxy-3-({8-[(3β)-cholest-5-en-3-yloxy]octyl}oxy)-N,N-dimethylpropan-2-amine;
   (2S-1-({8-[(3β)-cholest-5-en-3-yloxy]octyl}oxy)-3-[2,2,3,3,4,4,5,5,6,6,7,7,8,8,9,9-hexadecafluorononyl)oxy]-N,N-dimethylpropan-2-amine;
   2-amino-2-{[(9Z,12Z)-octadeca-9,12-dien-1-yloxy]methyl}propane-1,3-diol;
   2-amino-3-((9-(((3S,10R,13R)-10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)nonyl)oxy)-2-((((9Z,12Z)-octadeca-9,12-dien-1-yl)oxy)methyl)propan-1-ol;
   2-amino-3-((6-(((3S,10R,13R)-10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)hexyl)oxy)-2-((((Z)-octadec-9-en-1-yl)oxy)methyl)propan-1-ol;
   (20Z,23Z)-N,N-dimethylnonacosa-20,23-dien-10-amine;
   (17Z,20Z)-N,N-dimethylhexacosa-17,20-dien-9-amine;
   (16Z,19Z)-N,N-dimethylpentacosa-16,19-dien-8-amine;
      (13Z,16Z)-N,N-dimethyldocosa-13,16-dien-5-amine;
      (12Z,15Z)-N,N-dimethylhenicosa-12,15-dien-4-amine;
      (14Z,17Z)-N,N-dimethyltricosa-14,17-dien-6-amine;
      (15Z,18Z)-N,N-dimethyltetracosa-15,18-dien-7-amine;
      (18Z,21Z)-N,N-dimethylheptacosa-18,21-dien-10-amine;
      (15Z,18Z)-N,N-dimethyltetracosa-15,18-dien-5-amine;
      (14Z,17Z)-N,N-dimethyltricosa-14,17-dien-4-amine;
      (19Z,22Z)-N,N-dimethyloctacosa-19,22-dien-9-amine;
      (18Z,21Z)-N,N-dimethylheptacosa-18,21-dien-8-amine;
      (17Z,20Z)-N,N-dimethylhexacosa-17,20-dien-7-amine;
      (16Z,19Z)-N,N-dimethylpentacosa-16,19-dien-6-amine;
      (22Z,25Z)-N,N-dimethylhentriaconta-22,25-dien-10-amine;
      (21Z,24Z)-N,N-dimethyltriaconta-21,24-dien-9-amine;
      (18Z)-N,N-dimethylheptacos-18-en-10-amine;
      (17Z)-N,N-dimethylhexacos-17-en-9-amine;
      (19Z,22Z)-N,N-dimethyloctacosa-19,22-dien-7-amine;
      N,N-dimethylheptacosan-10-amine;
      (20Z,23Z)-N-ethyl-N-methylnonacosa-20,23-dien-10-amine;
      1-[(HZ,14Z)-1-nonylicosa-11,14-dien-1-yl]pyrrolidine;
      (20Z)-N,N-dimethylheptacos-20-en-10-amine;
      (15Z)-N,N-dimethylheptacos-15-en-10-amine;
      (14Z)-N,N-dimethylnonacos-14-en-10-amine;
      (17Z)-N,N-dimethylnonacos-17-en-10-amine;
      (24Z)-N,N-dimethyltritriacont-24-en-10-amine;
      (20Z)-N,N-dimethylnonacos-20-en-10-amine;
      (22Z)-N,N-dimethylhentriacont-22-en-10-amine;
      (16Z)-N,N-dimethylpentacos-16-en-8-amine;
      (12Z,15Z)-N,N-dimethyl-2-nonylhenicosa-12,15-dien-1-amine;
      (13Z,16Z)-N,N-dimethyl-3-nonyldocosa-13,16-dien-1-amine;
      N,N-dimethyl-1-[(1S,2R)-2-octylcyclopropyl]heptadecan-8-amine;
      1-[(1S,2R)-2-hexylcyclopropyl]-N,N-dimethylnonadecan-10-amine;
      N,N-dimethyl-1-[(1S,2R)-2-octylcyclopropyl]nonadecan-10-amine;
      N,N-dimethyl-21-[(1S,2R)-2-octylcyclopropyl]henicosan-10-amine;
      N,N-dimethyl-1-[(1S,2S)-2-{[(1R,2R)-2-pentylcyclopropyl]methyl}cyclopropyl]nonadecan-10-amine;
      N,N-dimethyl-1-[(1S,2R)-2-octylcyclopropyl]hexadecan-8-amine;
      N,N-dimethyl-1-[(1R,2S)-2-undecylcyclopropyl]tetradecan-5-amine;
      N,N-dimethyl-3-{7-[(1S,2R)-2-octylcyclopropyl]heptyl}dodecan-1-amine;
      1-[(1R,2S)-2-heptylcyclopropyl]-N,N-dimethyloctadecan-9-amine;
      1-[(1S,2R)-2-decylcyclopropyl]-N,N-dimethylpentadecan-6-amine;
      N,N-dimethyl-1-[(1S,2R)-2-octylcyclopropyl]pentadecan-8-amine; and
      (11E,20Z,23Z)-N,N-dimethylnonacosa-11,20,23-trien-10-amine;
   or a pharmaceutically acceptable salt thereof, or a stereoisomer of any of the foregoing.
19. The vaccine of feature 17, wherein the ionizable cationic lipids are selected from the group consisting of (2S)-1-({6-[(3β))-cholest-5-en-3-yloxy]hexyl}oxy)-N,N-dimethyl-3-[(9Z)-octadec-9-en-1-yloxy]propan-2-amine; (13Z,16Z)-N,N-dimethyl-3-nonyldocosa-13,16-dien-1-amine; and N,N-dimethyl-1-[(1S,2R)-2-octylcyclopropyl]heptadecan-8-amine;or a pharmaceutically acceptable salt thereof, or a stereoisomer of any of the foregoing.
20. The vaccine of feature 17, which comprises 30-75 mole % ionizable cationic lipid and 0.1-20 mole % PEG-lipid.
21. The vaccine of any one of features 17-20, further comprising one or more non-cationic lipids selected from a phospholipid, a phospholipid derivative, a fatty acid, a sterol, or a combination thereof.
22. The vaccine of feature 21, wherein the sterol is cholesterol, stigmasterol or stigmastanol.
23. The vaccine of feature 21, wherein the phospholipid is selected from phosphatidylserine, 1,2-Distearoyl-sn-glycero-3-phosphocholine (DSPC), 1,2-dipalmitoleoyl-sn-glycero-3-phosphocholine, 1,2-dimyristoyl-sn-glycero-3-phosphocholine (DMPC), dilauroylphosphatidylcholine (DLPC), 1,2-dieicosenoyl-sn-glycero-3-phosphocholine, and 1,2-dioleoyl-*sn*-glycero-3-phosphocholine (DOPC).
24. The vaccine of any one of features 16-23, wherein the PEG-lipid is 1,2-Dimyristoyl-sn-glycerol methoxypolyethylene glycol (PEG-DMG), PEG-disteryl glycerol (PEG-DSG), PEG-dipalmetoleyl, PEG-dioleyl, PEG-distearyl, PEG-diacylglycamide (PEG-DAG), PEG-dipalmitoyl phosphatidylethanolamine (PEG-DPPE), or PEG-1,2-dimyristyloxlpropyl-3-amine (PEG-c-DMA).
25. The vaccine of feature 16-24, which comprises 20-99.8 mole % ionizable cationic lipids, 0.1-65 mole % non-cationic lipids, and 0.1-20 mole % PEG-lipid.
26. The vaccine of feature 25, wherein the non-cationic lipids comprise a mixture of cholesterol and DSPC.
27. The vaccine of feature 16, which comprises 34-59 mole % ionizable cationic lipids selected from the group consisting of (2S)-1-({6-[(3β))-cholest-5-en-3-yloxy]hexyl}oxy)-N,N-dimethyl-3-[(9Z)-octadec-9-en-1-yloxy]propan-2-amine; (13Z,16Z)-N,N-dimethyl-3-nonyldocosa-13,16-dien-1-amine; and N,N-dimethyl-1-[(1S,2R)-2-octylcyclopropyl]heptadecan-8-amine, 30-48 mole % cholesterol, 10-24% DSPC and 1-2 mole % PEG-DMG.
28. The vaccine composition of any one of features 1-27 that is formulated with an aluminum adjuvant MAA or APA.
29. A method of inducing an immune response in a subject, the method comprising administering to the subject the vaccine of any one of features 1-28 in an amount effective to produce an antigen-specific immune response in the subject.
30. The method of feature 29, wherein the antigen specific immune response comprises a T cell response or a B cell response.
31. The method of feature 29 or 30, wherein the subject is administered a single dose of the vaccine.
32. The method of feature 29 or 30, wherein the subject is administered a booster dose of the vaccine.
33. The method of any one of features 29-32, wherein the vaccine is administered to the subject by intradermal injection or intramuscular injection.
34. The method of any one of features 29-33, wherein the subject has been exposed to the virus, wherein the subject is infected with the virus, or wherein the subject is at risk of infection by the virus.
35. The vaccine of any one of features 1-28 for use in a method of inducing an antigen specific immune response in a subject, the method comprising administering to the subject the vaccine in an amount effective to produce an antigen specific immune response in the subject.
36. Use of the vaccine of any one of features 1-28 in the manufacture of a medicament for use in a method of inducing an antigen specific immune response in a subject, the method comprising administering to the subject the vaccine in an amount effective to produce an antigen specific immune response in the subject.
37. A method of inducing cross-reactivity against a variety of influenza strains in a mammal, the method comprising administering to the mammal in need thereof the vaccine of any one of features 1-28.
38. The method of feature 37, wherein at least two vaccines are administered to the mammal separately or simultaneously.

## Claims

1. An influenza virus vaccine, comprising at least one isolated antigenic polypeptide:
wherein the at least one antigenic polypeptide comprises an amino acid sequence that has at least 90% - 99% identity to a mature amino acid sequence in any one of SEQ ID NOs: 10, 1-4, 8, 9, 11, 14-19, 41-61, and 83-88.

2. The vaccine of claim 1, wherein the polypeptide has conservative mutations compared to a mature amino acid sequence in any one of SEQ ID NOs: 10, 1-4, 8, 9, 11, 14-19, 41-61, and 83-88; or
comprises the mature amino acid sequence that is identified in any one of SEQ ID NOs: 10, 1-4, 8, 9, 11, 14-19, 41-61, and 83-88; or
consists of the mature amino acid sequence that is identified in any one of SEQ ID NOs: 10, 1-4, 8, 9, 11, 14-19, 41-61, and 83-88.

3. The vaccine of claim 1, wherein the polypeptide comprises a mature amino acid sequence that is the extracellular domain of SEQ ID NO: 10 or 8.

4. An influenza virus vaccine comprising two isolated antigenic polypeptides, wherein the first polypeptide comprises an amino acid sequence that has at least 90%-99% identity to the mature amino acid sequence of any one of SEQ ID NOs: 10, 1-5, 8, 9, 11, 14-19, 41-63, and 83-88 and the second polypeptide comprises an amino acid sequence that has at least 90%-99% identity to the mature amino acid sequence of SEQ ID NO: 20.

5. The vaccine of claim 4, wherein the first and second polypeptide has conservative mutations compared to the mature amino acid sequence in any one of SEQ ID NOs: 10, 1-5, 8, 9, 11, 14-19, 41-63, 83-88 and 20.

6. The vaccine of claim 4, wherein the first polypeptide comprises the mature amino acid sequence in SEQ ID NO: 10, and the second polypeptide comprises the mature amino acid sequence in SEQ ID NO: 20; or wherein the first polypeptide consists of the mature amino acid sequence in SEQ ID NO: 10, and the second polypeptide consists of the mature amino acid sequence in SEQ ID NO: 20.

7. The vaccine of any one of claims 1-6, wherein the vaccine is multivalent.

8. The vaccine of any one of claims 1-7 formulated in an effective amount to produce an antigen-specific immune response.

9. The vaccine of any one of claims 1-7 formulated with a Lipid Nanoparticle (LNP) comprising one or more cationic lipids and a poly(ethyleneglycol)-lipid (PEG-lipid).

10. The vaccine of claim 9, wherein the one or more cationic lipids are ionizable cationic lipids.

11. The vaccine of claim 10, wherein the ionizable cationic lipids are selected from DLinDMA; DlinKC2DMA; DLin-MC3-DMA; CLinDMA; S-Octyl CLinDMA;
(2 S )-1-{7-[(3 β)-cholest-5-en-3-yloxy]heptyloxy}-3-[(4 Z )-dec-4-en-1-yloxy]- *N, N* - dimethylpropan-2-amine;
(2 R )-1-{4-[(3 β)-cholest-5-en-3-yloxy]butoxy}-3-[(4 Z )-dec-4-en-1-yloxy]- N, N - dimethylpropan-2-amine;
1-[(2 R )-1-{4-[(3 β )-cholest-5-en-3-yloxy]butoxy}-3-(octyloxy)propan-2-yl]guanidine;
1-[(2 R )-1-{7-[(3 β )-cholest-5-en-3-yloxy]heptyloxy}-N,N-dimethyl-3-[(9 Z, 12 Z )-octadeca-9,12-dien-1-yloxy]propan-2-amine;
1-[(2 R )-1-{4-[(3 β )-cholest-5-en-3-yloxy]butoxy}-N,N-dimethyl-3-[(9 Z, 12 Z )-octadeca-9,12-dien-1-yloxy]propan-2-amine;
(2S)-1-({6-[(3β))-cholest-5-en-3-yloxy]hexyl}oxy)-N,N-dimethyl-3-[(9 Z)-octadec-9-en-1-yloxy]propan-2-amine;
(3β)-3-[6-{ [ (2S)-3-[ (9Z)-octadec-9-en-1-yloxyl]-2-(pyrrolidin-1-yl)propyl]oxy}hexyl)oxy] cholest-5-ene;
(2R)-1-{4-[(3β)-cholest-5-en-3-yloxy]butoxy}-3-(octyloxy)propan-2-amine;
(2R)-1-({8-[(3β)-cholest-5-en-3-yloxy]octyl}oxy)-N,N-dimethyl-3-(pentyloxy)propan-2-amine;
(2R)-1-({8-[(3β)-cholest-5-en-3-yloxy]octyl}oxy)-3-(heptyloxy)-N,N-dimethylpropan-2-amine;
(2R)-1-({8-[(3β)-cholest-5-en-3-yloxy]octyl}oxy)-N,N-dimethyl-3-[(2Z)-pent-2-en-1-yloxy]propan-2-amine;
(2S)-1-butoxy-3-({8-[(3β)-cholest-5-en-3-yloxy]octyl}oxy)-N,N-dimethylpropan-2-amine;
(2S-1-({8-[(3β)-cholest-5-en-3-yloxy]octyl}oxy)-3-[2,2,3,3,4,4,5,5,6,6,7,7,8,8,9,9-hexadecafluorononyl)oxy]-N,N-dimethylpropan-2-amine;
2-amino-2-{[(9Z,12Z)-octadeca-9,12-dien-1-yloxy]methyl}propane-1,3-diol;
2-amino-3-((9-(((3S,10R,13R)-10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)nonyl)oxy)-2-((((9Z,12Z)-octadeca-9,12-dien-1-yl)oxy)methyl)propan-1-ol;
2-amino-3-((6-(((3S,10R,13R)-10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)hexyl)oxy)-2-((((Z)-octadec-9-en-1-yl)oxy)methyl)propan-1-ol;
(20Z,23Z)-N,N-dimethylnonacosa-20,23-dien-10-amine;
(17Z,20Z)-N,N-dimethylhexacosa-17,20-dien-9-amine;
(16Z, 19Z)-N,N-dimethylpentacosa-16,19-dien-8-amine;
(13Z,16Z)-N,N-dimethyldocosa-13,16-dien-5-amine;
(12Z,15Z)-N,N-dimethylhenicosa-12,15-dien-4-amine;
(14Z,17Z)-N,N-dimethyltricosa-14,17-dien-6-amine;
(15Z,18Z)-N,N-dimethyltetracosa-15,18-dien-7-amine;
(18Z,21Z)-N,N-dimethylheptacosa-18,21-dien-10-amine;
(15Z,18Z)-N,N-dimethyltetracosa-15,18-dien-5-amine;
(14Z,17Z)-N,N-dimethyltricosa-14,17-dien-4-amine;
(19Z,22Z)-N,N-dimethyloctacosa-19,22-dien-9-amine;
(18Z,21Z)-N,N-dimethylheptacosa-18,21-dien-8-amine;
(17Z,20Z)-N,N-dimethylhexacosa-17,20-dien-7-amine;
(16Z,19Z)-N,N-dimethylpentacosa-16,19-dien-6-amine;
(22Z,25Z)-N,N-dimethylhentriaconta-22,25-dien-10-amine;
(21Z,24Z)-N,N-dimethyltriaconta-21,24-dien-9-amine;
(18Z)-N,N-dimethylheptacos-18-en-10-amine;
(17Z)-N,N-dimethylhexacos-17-en-9-amine;
(19Z,22Z)-N,N-dimethyloctacosa-19,22-dien-7-amine;
N,N-dimethylheptacosan-10-amine;
(20Z,23Z)-N-ethyl-N-methylnonacosa-20,23-dien-10-amine;
1-[(11Z,14Z)-1-nonylicosa-11,14-dien-1-yl]pyrrolidine;
(20Z)-N,N-dimethylheptacos-20-en-10-amine;
(15Z)-N,N-dimethylheptacos-15-en-10-amine;
(14Z)-N,N-dimethylnonacos-14-en-10-amine;
(17Z)-N,N-dimethylnonacos-17-en-10-amine;
(24Z)-N,N-dimethyltritriacont-24-en-10-amine;
(20Z)-N,N-dimethylnonacos-20-en-10-amine;
(22Z)-N,N-dimethylhentriacont-22-en-10-amine;
(16Z)-N,N-dimethylpentacos-16-en-8-amine;
(12Z,15Z)-N,N-dimethyl-2-nonylhenicosa-12,15-dien-1-amine;
(13Z,16Z)-N,N-dimethyl-3-nonyldocosa-13,16-dien-1-amine;
N,N-dimethyl-1-[(1S,2R)-2-octylcyclopropyl]heptadecan-8-amine;
1-[(1S,2R)-2-hexylcyclopropyl]-N,N-dimethylnonadecan-10-amine;
N,N-dimethyl-1-[(1S,2R)-2-octylcyclopropyl]nonadecan-10-amine;
N,N-dimethyl-21-[(1S,2R)-2-octylcyclopropyl]henicosan-10-amine;
N,N-dimethyl-1-[(1S,2S)-2-{[(1R,2R)-2-pentylcyclopropyl]methyl}cyclopropyl]nonadecan-10-amine;
N,N-dimethyl-1-[(1S,2R)-2-octylcyclopropyl]hexadecan-8-amine;
N,N-dimethyl-1-[(1R,2S)-2-undecylcyclopropyl]tetradecan-5-amine;
N,N-dimethyl-3-{7-[(1S,2R)-2-octylcyclopropyl]heptyl}dodecan-1-amine;
1-[(1R,2S)-2-heptylcyclopropyl]-N,N-dimethyloctadecan-9-amine;
1-[(1S,2R)-2-decylcyclopropyl]-N,N-dimethylpentadecan-6-amine;
N,N-dimethyl-1-[(1S,2R)-2-octylcyclopropyl]pentadecan-8-amine; and
(11E,20Z,23Z)-N,N-dimethylnonacosa-11,20,23-trien-10-amine;
or a pharmaceutically acceptable salt thereof, or a stereoisomer of any of the foregoing.

12. The vaccine of claim 10, which comprises 30-75 mole % ionizable cationic lipid and 0.1-20 mole % PEG-lipid.

13. The vaccine of any one of claims 10-12, further comprising one or more non-cationic lipids selected from a phospholipid, a phospholipid derivative, a fatty acid, a sterol, or a combination thereof.

14. The vaccine of claim 13, wherein the sterol is cholesterol, stigmasterol or stigmastanol.

15. The vaccine of claim 13, wherein the phospholipid is selected from phosphatidylserine, 1,2-Distearoyl-sn-glycero-3-phosphocholine (DSPC), 1,2-dipalmitoleoyl-sn-glycero-3-phosphocholine, 1,2-dimyristoyl-sn-glycero-3-phosphocholine (DMPC), dilauroylphosphatidylcholine (DLPC), 1,2-dieicosenoyl-sn-glycero-3-phosphocholine, and 1,2-dioleoyl-*sn*-glycero-3-phosphocholine (DOPC).

16. The vaccine of any one of claims 9-15, wherein the PEG-lipid is 1,2-Dimyristoyl-sn-glycerol methoxypolyethylene glycol (PEG-DMG), PEG-disteryl glycerol (PEG-DSG), PEG-dipalmetoleyl, PEG-dioleyl, PEG-distearyl, PEG-diacylglycamide (PEG-DAG), PEG-dipalmitoyl phosphatidylethanolamine (PEG-DPPE), or PEG-1,2-dimyristyloxlpropyl-3-amine (PEG-c-DMA).

17. The vaccine of claims 9-16, which comprises 20-99.8 mole % ionizable cationic lipids, 0.1-65 mole % non-cationic lipids, and 0.1-20 mole % PEG-lipid.

18. The vaccine of claim 17, wherein the non-cationic lipids comprise a mixture of cholesterol and DSPC.

19. The vaccine of claim 9, which comprises 34-59 mole % ionizable cationic lipids selected from the group consisting of (2S)-1-({6-[(3β))-cholest-5-en-3-yloxy]hexyl}oxy)-N,N-dimethyl-3-[(9 Z)-octadec-9-en-1-yloxy]propan-2-amine; (13Z,16Z)-N,N-dimethyl-3-nonyldocosa-13,16-dien-1-amine; and N,N-dimethyl-1-[(1S,2R)-2-octylcyclopropyl]heptadecan-8-amine, 30-48 mole % cholesterol, 10-24% DSPC and 1-2 mole % PEG-DMG.

20. The vaccine of any one of claims 1-19 for use in a method of inducing an antigen specific immune response in a subject, the method comprising administering to the subject the vaccine in an amount effective to produce an antigen specific immune response in the subject.
